# EUROPEAN PATENT SPECIFICATION

(11) **EP 0 769 011 B1**
(45) Date of publication and mention of the grant of the patent: **29.08.2001**
(21) Application number: 95924935.0
(22) Date of filing: 26.06.1995
(51) Int. Cl.: C07D 285/00, C07D 417/00, C07F 7/10, A01N 43/72, A01N 55/10

(54) **HERBICIDAL 1,2,4,6-THIATRIAZINES**
HERBIZIDE 1,2,4,6-THIATRIAZINE
1,2,4,6-THIATRIAZINES HERBICIDES

(30) Priority: 07.07.1994 CH 217594
(43) Date of publication of application: 23.04.1997
(73) Proprietor: Syngenta Participations AG, 4058 Basel (CH)
(72) Inventor: STOLLER, André, F-68730 Blotzheim (FR); HAAKE, Manfred, D-35043 Marburg (DE); ZONDLER, Helmut, CH-4103 Bottmingen (CH)
(86) International application number: EP9502478
(87) International publication number: WO9601814

(56) References cited:
- EP-A- 0 075 117
- Chem. Ber., vol. 121, pp. 383-386, 1988

## Description

The present invention relates to novel herbicidally active thiatriazine derivatives, to processes for their preparation, to compositions comprising these compounds, and to their use for controlling weeds, especially in crops of useful plants or for inhibiting plant growth.

Thiatriazine compounds are described, for example, in Chem. Ber. 121, 383-386 (1988), Z. Naturforsch. 43, 763-768 (1988), Chem. Ber. 126, 2601-2607 (1993), J. Am. Chem. Soc. 1989, 111, 1180-1185, DD 113 006 and in the PhD thesis of W. Jürgler, Philipps-University Marburg/Lahn, 1988 ("1λ⁴- und 1λ⁶-2.4.6-Thiatriazine aus Sulfodiimiden" ["1λ⁴- and 1λ⁶-2,4,6-thiatriazines from sulfodiimides]).

2H-1,2,4,6-Thiatriazin-1,1-dioxide compounds are described as herbicides in EP-A-0 075 117.

There has now been found a novel and simple synthetic method for preparing novel thiatriazine derivatives which are substituted by a variety of substituents. Advantages of this synthetic method are not only the ready accessibility of thiatriazine derivatives which are substituted by a variety of substituents, but also the low number of synthetic steps (converging). In addition, the herbicidal and growth-inhibiting properties of the thiatriazine derivatives were found for the first time.

The present invention therefore provides compounds of the formula I 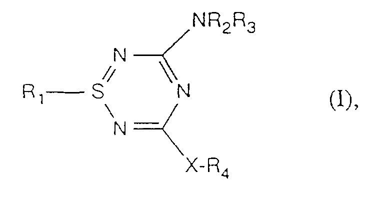 in which
R₁ is C₁-C₂₀alkyl, C₃-C₈cycloalkyl, C₃-C₈cycloalkyl-C₁-C₁₇alkyl, C₁-C₁₇alkyl-C₃-C₈cycloalkyl, C₂-C₁₇alkenyl, C₃-C₈cycloalkyl-C₂-C₁₇alkenyl, C₂-C₁₇alkenyl-C₃-C₈cycloalkyl, C₂-C₁₇alkynyl, C₃-C₈cycloalkyl-C₂-C₁₇alkynyl, C₂-C₁₇alkynyl-C₃-C₈cycloalkyl, C₅-C₈cycloalkenyl, C₅-C₈cycloalkenyl-C₁-C₁₇alkyl, C₁-C₁₇alkyl-C₅-C₈cycloalkenyl, C₅-C₈cycloalkenyl-C₂-C₁₇alkenyl, C₂-C₁₇alkenyl-C₅-C₈cycloalkenyl, C₅-C₈cycloalkenyl-C₂-C₁₇alkynyl, C₂-C₁₇alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₁₇alkynyl-C₂-C₁₇alkenyl or C₂-C₁₇alkenyl-C₂-C₁₇alkynyl, it being possible for these substituents to be substituted by halogen, cyano, nitro, =O, -OR₅, -S(O)ₙR₆,-COOR₇, -CONR₈R₉, -NR₁₀R₁₁, =NR₁₂, =N-NR₁₃R₁₄, phenyl, biphenyl, naphthyl or saturated or unsaturated heterocylyl having 3 to 8 ring atoms which, in addition to carbon atoms, has at least one hetero atom selected from nitrogen, oxygen and sulfur, it being possible for these phenyl, naphthyl and heterocyclyl substituents to be substituted by halogen, cyano, nitro, -OR₅, -NR₁₀R₁₁ or -CONR₈R₉, or
R₁ is saturated or unsaturated heterocylyl having 3 to 8 ring atoms which, in addition to carbon atoms, has at least one hetero atom selected from nitrogen, oxygen and sulfur, the heterocycle being bonded to the thiatriazine ring via one of its carbon atoms and it being possible for the heterocycle to be substituted by halogen, cyano, nitro, =O, -OR₅, S(O)ₙR₆, -COOR₇, -CONR₈R₉, -NR₁₀R₁₁, =NR₁₂, =N-NR₁₃R₁₄, C₁-C₁₇alkyl, C₂-C₁₇alkenyl, C₂-C₁₇alkynyl, C₃-C₈cycloalkyl, C₅-C₈cycloalkenyl, phenyl, biphenyl, naphthyl, C₁-C₁₇alkyl which is substituted by halogen, cyano, nitro, -OR₅, -CONR₈R₉ or -NR₁₀R₁₁, C₂-C₁₇alkenyl which is substituted by halogen, cyano, nitro, -OR₅,-CONR₈R₉ or -NR₁₀R₁₁, C₂-C₁₇alkynyl which is substituted by halogen, cyano, nitro,-OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁, phenyl which is substituted by halogen, cyano, nitro, -OR₅, -CONR₈R₉ or -NR₁₀R₁₁, biphenyl which is substituted by halogen, cyano, nitro, -OR₅, -CONR₈R₉ or -NR₁₀R₁₁ or naphthyl which is substituted by halogen, cyano, nitro, -OR₅, -CONR₈R₉ or -NR₁₀R₁₁, or
R₁ is phenyl, biphenyl, naphthyl, C₁-C₁₀alkylphenyl, C₁-C₁₀alkylbiphenyl, C₁-C₁₀alkylnaphthyl, C₂-C₁₀alkenylphenyl, C₂-C₁₀alkenylbiphenyl, C₂-C₁₀alkenylnaphthyl, C₂-C₁₀alkynylphenyl, C₂-C₁₀alkynylbiphenyl or C₂-C₁₀alkynylnaphthyl, it being possible for these substituents to be substituted by halogen, cyano, nitro, -OR₅, -S(O)ₙR₆, -COOR₇, -CONR₈R₉ or -NR₁₀R₁₁, in which R₅ is hydrogen, C₁-C₆alkyl, C₁-C₆haloalkyl, C₂-C₅alkoxyalkyl, C₁-C₆cyanoalkyl, phenyl, halophenyl, C₁-C₄alkoxyphenyl, phenyl-C₁-C₄alkyl, C₂-C₇alkylcarbonyl, benzoyl, halobenzoyl, C₁-C₆alkylamino, C₂-C₈dialkylamino, -N=CH₂, -N=CH-C₁-C₄alkyl, -N=C(C₁-C₄alkyl)₂, C₃-C₆trialkylsilyl, C₃-C₇cycloalkyl, C₂-C₇alkenyl or C₃-C₇alkynyl,
n is 0, 1 or 2,
R₆ is hydrogen or cyano if n is 0, or
R₆ is C₁-C₅alkyl, C₁-C₅haloalkyl, C₁-C₅hydroxyalkyl, phenyl, naphthyl, C₁-C₄alkylphenyl, C₁-C₄alkylnaphthyl, halophenyl, halonaphthyl, saturated or unsaturated heterocyclyl or haloheterocyclyl having 3 to 8 ring atoms which, in addition to carbon atoms, at least one hetero atom selected from nitrogen, oxygen and sulfur, or C₃-C₇cycloalkyl,
R₇ is hydrogen, C₁-C₁₂alkyl, C₁-C₁₂haloalkyl, C₁-C₆nitroalkyl, C₁-C₆cyanoalkyl, phenyl, naphthyl, halophenyl, halonaphthyl, C₂-C₁₀alkoxyalkyl, C₂-C₁₀alkylcarbonyl, saturated or unsaturated heterocyclyl or haloheterocyclyl having 3 to 8 ring atoms which, in addition to carbon atoms, at least one hetero atom selected from nitrogen, oxygen and sulfur, C₃-C₇cycloalkyl, C₃-C₇halocycloalkyl, -N=CH₂, -N=CH-C₁-C₄-alkyl, -N=C(C₁-C₄alkyl)₂ or C₂-C₆dialkylamino,
R₈ and R₉ independently of one another are hydrogen, phenyl, naphthyl, halophenyl, halonaphthyl, C₁-C₈alkyl, C₁-C₈haloalkyl, C₁-C₈alkoxy, phenoxy, naphthoxy, halophenoxy, halonaphthoxy, C₂-C₇cyanoalkyl, C₃-C₇alkenyl, C₃-C₇alkynyl, C₂-C₈alkoxyalkyl, -N=CH₂, -N=CH-C₁-C₄alkyl, -N=C(C₁-C₄alkyl)₂, C₁-C₆alkylamino or C₂-C₆dialkylamino, or
R₈ and R₉ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur,
R₁₀ and R₁₁ independently of one another are hydrogen, phenyl, naphthyl, halophenyl, halonaphthyl, C₁-C₈alkyl, C₁-C₈haloalkyl, C₁-C₈alkoxy, phenoxy, naphthoxy, halophenoxy, halonaphthoxy, C₂-C₇cyanoalkyl, C₃-C₇alkenyl, C₃-C₇alkynyl, C₂-C₈alkoxyalkyl, -N=CH₂, -N=CH-C₁-C₄alkyl, -N=C(C₁-C₄alkyl)₂, C₁-C₆alkylamino, C₂-C₆dialkylamino, formyl, C₂-C₈alkylcarbonyl, phenylcarbonyl or naphthylcarbonyl, it being possible therein for the phenyl or naphthyl moiety to be substituted by C₁-C₄alkyl, halogen, C₁-C₄alkoxy, hydroxyl, cyano, nitro or C₂-C₆alkoxycarbonyl, or
R₁₀ and R₁₁ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur,
R₁₂ is hydrogen, C₁-C₈alkyl, C₁-C₈haloalkyl, phenyl, halophenyl, cyanophenyl, nitrophenyl, C₁-C₄alkylphenyl, C₁-C₄alkoxyphenyl, naphthyl, cyanonaphthyl, nitronaphthyl, halonaphthyl, C₁-C₄alkylnaphthyl, C₁-C₄alkoxynaphthyl, phenyl-C₁-C6alkyl or naphthyl- C₁-C₆alkyl, and
R₁₃ and R₁₄ independently of one another are hydrogen, C₁-C₆alkyl, C₂-C₆alkylcarbonyl, phenyl, halophenyl, cyanophenyl, nitrophenyl, C₁-C₄alkylphenyl, C₁-C₄alkoxyphenyl, naphthyl, cyanonaphthyl, nitronaphthyl, halonaphthyl, C₁-C₄alkylnaphthyl or C₁-C₄alkoxynaphthyl,
R₂ and R₃ independently of one another are hydrogen, C₁-C₆alkyl, C₁-C₆alkyl which is substituted by halogen, cyano, nitro, C₁-C₈alkoxy, phenyl, naphthyl, phenoxy, naphthoxy, phenyl which is substituted by halogen, cyano, nitro, -OR₅, -NR₁₀R₁₁ or-CONR₈R₉, naphthyl which is substituted by halogen, cyano, nitro, -OR₅, -NR₁₀R₁₁ or-CONR₈R₉, phenoxy which is substituted by halogen, cyano, nitro, -OR₅, -NR₁₀R₁₁ or-CONR₈R₉, naphthoxy with is substituted by halogen, cyano, nitro, -OR₅, -NR₁₀R₁₁ or-CONR₈R₉, C₁-C₈-alkylthio, phenthio, naphthio, phenthio which is substituted by halogen, cyano, nitro, -OR₅, -NR₁₀R₁₁ or -CONR₈R₉, naphthio which is substituted by halogen, cyano, nitro, -OR₅, -NR₁₀R₁₁ or -CONR₈R₉, C₃-C₆trialkylsilyl, hydroxyl, amino, ammonium, tri-C₁-C₄alkylammonium, C₂-C₈alkoxycarbonyl, -COOH, -COOM, in which M is ammonium or alkali metal atom or alkaline earth metal atom, or C₃-C₈cycloalkyl, C₂-C₅alkylcarbonyloxy, phenylcarbonyloxy, naphthylcarbonyloxy, C₁-C₆alkylamino, C₂-C₅alkoxycarbonyl or C₂-C₁₂dialkylamino, or
R₂ and R₃ independently of one another are C₂-C₆alkenyl, C₂-C₆alkenyl which is substituted by halogen, cyano, nitro, C₁-C₈alkoxy, phenoxy, naphthoxy, phenoxy which is substituted by halogen, cyano, nitro, -OR₅, -NR₁₀R₁₁ or -CONR₈R₉, naphthoxy which is substituted by halogen, cyano, nitro, -OR₅, -NR₁₀R₁₁ or -CONR₈R₉, C₁-Cgalkylthio, phenthio which is substituted by halogen, cyano, nitro, -OR₅, -NR₁₀R₁₁ or -CONR₈R₉, naphthio which is substituted by halogen, cyano, nitro, -OR₅, -NR₁₀R₁₁ or -CONR₈R₉, C₃-C₆trialkylsilyl, hydroxyl, amino, ammonium, tri-C₁-C₄alkylammonium, C₂-C₈alkoxycarbonyl, -COOH, -COOM, in which M is ammonium or an alkali metal atom or alkaline earth metal atom, or C₃-C₈cycloalkyl, C₂-C₅alkylcarbonyloxy, phenylcarbonyloxy or naphthylcarbonyloxy, C₁-C₆alkylamino, C₂-C₅alkoxycarbonyl or C₂-C₁₂dialkylamine, or
R₂ and R₃ independently of one another are C₂-C₆alkynyl, C₂-C₆alkynyl which is substituted by halogen, cyano, nitro, C₁-C₈alkoxy, phenoxy, naphthoxy, C₁-C₈alkylthio, phenthio, naphthio, phenoxy which is substituted by halogen, cyano, nitro, -OR₅,-NR₁₀R₁₁ or -CONR₈R₉, naphthoxy which is substituted by halogen, cyano, nitro, -OR₅, NR₁₀R₁₁ or -CONR₈R₉, phenthio which is substituted by halogen, cyano, nitro, -OR₅, -NR₁₀R₁₁ or -CONR₈R₉, naphthio which is substituted by halogen, cyano, nitro, -OR₅, -NR₁₀R₁₁ or -CONR₈R₉, C₃-C₆trialkylsilyl, hydroxyl, amino, ammonium, tri-C₁-C₄alkylammonium, C₂-C₈alkoxycarbonyl, -COOH, -COOM, in which M is ammonium or an alkali metal atom or alkaline earth metal atom, or C₃-C₈cycloalkyl, or
R₂ and R₃ independently of one another are formyl, C₂-C₁₅alkylcarbonyl, C₂-C₁₅alkenylcarbonyl, C₄-C₉cycloalkylcarbonyl, C₆-C₉cycloalkenylcarbonyl or C₃-C₈cycloalkyl-C₂-C₆alkylcarbonyl, it being possible for these substituents to be substituted by halogen, cyano, nitro, hydroxyl, amino, C₁-C₆alkylamino, C₂-C₁₂dialkylamino, -COOH, -COOM, in which M is ammonium or an alkali metal atom or alkaline earth metal atom, or C₂-C₈alkoxycarbonyl, C₄-C₁₀cycloalkoxycarbonyl, C₂-C₈alkylaminocarbonyl or C₃-C₁₂dialkylaminocarbonyl, or
R₂ and R₃ independently of one another are saturated or unsaturated heterocyclyl or heterocyclylcarbonyl having 3 to 8 ring atoms which, in addition to carbon atoms, at least one hetero atom selected from nitrogen, oxygen and sulfur, heterocyclyl as defined above which is substituted by halogen, cyano, nitro, C₁-C₅alkyl, C₁-C₅alkoxy, C₂-C₅alkylcarbonyl, C₂-C₅alkylcarbonyloxy, C₂-C₆alkoxycarbonyl, aminocarbonyl, C₂-C₆alkylamino or C₃-C₁₁ dialkylaminocarbonyl, or heterocyclylcarbonyl as defined above which is substituted by halogen, cyano, nitro, C₁-C₅alkyl, C₁-C₅alkoxy, C₂-C₅alkylcarbonyl, C₂-C₆alkoxylcarbonyl, aminocarbonyl, C₂-C₆alkylamino or C₂-C₅alkylcarbonyloxy, or
R₂ and R₃ independently of one another are phenylcarbonyl, biphenylcarbonyl, naphthylcarbonyl, phenyl-C₂-C₆alkylcarbonyl, biphenyl-C₂-C₆alkylcarbonyl, naphthyl-C₂-C₆alkylcarbonyl, phenyl-C₃-C₆alkenylcarbonyl, biphenyl-C₃-C₆alkenylcarbonyl, naphthyl-C₃-C₆alkenylcarbonyl, phenyl-C₃-C₆alkynylcarbonyl, biphenyl-C₃-C₆ alkynylcarbonyl or naphthyl-C₃-C₆alkynylcarbonyl, it being possible for these substituents to be substituted by C₁-C₅alkyl, C₁-C₅alkoxy, C₁-C₅alkylthio, C₁-C₅haloalkyl, C₂-C₅alkylcarbonyl, halogen, cyano, amino, nitro, -COOH, C₂-C₈alkoxycarbonyl, hydroxyl, C₂-C₆alkylaminocarbonyl or C₂-C₁₂dialkylaminocarbonyl, or
R₂ and R₃ independently of one another are phenyl-C₁-C₆alkyl, biphenyl-C₁-C₆alkyl, naphthyl-C₁-C₆alkyl, phenyl-C₂-C₆alkenyl, biphenyl-C₂-C₆alkenyl, naphthyl-C₂-C₆alkenyl, phenyl-C₂-C₆alkynyl, biphenyl-C₂-C₆alkynl or naphthyl-C₂-C₆alkynyl, it being possible for these substituents to be substituted by C₁-C₅alkyl, C₂-C₅alkenyl, C₂-C₅alkinyl, hydroxyl, C₁-C₅alkoxy, formyl, C₂-C₆alkylcarbonyl, C₂-C₆alkoxycarbonyl, C₂-C₆alkylaminocarbonyl, C₃-C₉dialkylaminocarbonyl, nitro or C₁-C₅alkylthio, or
R₂ and R₃ independently of one another are phenyl, naphthyl or saturated or unsaturated heterocylyl having 3 to 8 ring atoms which, in addition to carbon atoms, has at least one hetero atom selected from nitrogen, oxygen and sulfur, it being possible for these substituents to be substituted by halogen, cyano, nitro, C₁-C₅alkyl, C₁-C₅alkoxy, C₁-C₅alkylthio, -COOH, -CONH₂, C₂-C₆alkylaminocarbonyl, C₃-C₁₀di alkylaminocarbonyl, C₂-C₅alkylcarbonyl or C₂-C₅alkoxycarbonyl, or
R₂ and R₃ together with the nitrogen atom to which they are bonded form a saturated or unsaturated heterocyclic ring having 3 to 8 ring atoms which, in addition to carbon atoms, at least one hetero atom selected from nitrogen, oxygen and sulfur, which heterocyclic ring is unsubstituted or substituted by C₁-C₅alkyl, C₁-C₅alkoxy, halogen, cyano or nitro, or
R₂ and R₃ independently of one another are amino, C₁-C₆alkylamino, C₂-C₈dialkylamino, phenylamino, naphthylamino, C₂-C₆alkylcarbonylamino, C₂-C₁₀alkoxycarbonylamino, hydroxyl, C₁-C₆alkoxy, C₂-C₆alkylcarbonyloxy, phenoxy, biphenyloxy or naphthoxy,
X is O or S(O)ₓ, in which x is 0, 1 or 2, and
R₄ is C₁-C₈alkyl, C₂-C₈alkenyl, C₂-C₈alkynyl, C₃-C₈cycloalkyl, C₅-C₈cycloalkenyl, C₃-C₈cycloalkyl-C₁-C₄alkyl or C₁-C₄alkyl-C₃-C₈cycloalkyl, it being possible for these substituents to be substituted by halogen, cyano, nitro, =O or -OR₅, or
R₄ is phenyl, naphthyl, saturated or unsaturated heterocylyl having 3 to 8 ring atoms which, in addition to carbon atoms, has at least one hetero atom selected from nitrogen, oxygen and sulfur, C₁-C₄alkylphenyl, C₁-C₄alkylnaphthyl, phenyl-C₁-C₄alkyl or
naphthyl-C₁-C₄alkyl, it being possible for these substituents to be substituted by halogen, cyano, nitro, amino, -COOH, hydroxyl, C₁-C₁₀alkyl, C₁-C₁₀alkyloxy, C₁-C₁₀alkylthio, C₁-C₁₀haloalkyl, C₁-C₁₀haloalkoxy, C₁-C₁₀haloalkylthio, C₃-C₁₀alkoxycarbonylalkoxy, C₁-C₆alkylsulfinyl, C₁-C₆alkylsulfonyl, C₂-C6alkyloxycarbonyl, C₂-C₆alkylcarbonyl, -CONH₂, formyl, C₂-C₇alkylaminocarbonyl, C₃-C₁₁dialkylaminocarbonyl, C₃-C₆trialkylsilyl, C₂-C₁₀alkylcarbonylamino, C₂-C₁₀alkylcarbonyloxy, phenoxy, halophenoxy, pyridyloxy or pyridyloxy which is substituted by halogen, C₁-C₄alkyl, C₁-C₄alkoxy, cyano, nitro or amino, or 2 adjacent substituents on the phenyl or naphthyl ring R₄ form a carbocyclic or saturated or unsaturated heterocyclic ring having 3 to 8 ring atoms which, in addition to carbon atoms, at least one hetero atom selected from nitrogen, oxygen and sulfur, which carbocyclic or heterocyclic ring is unsubstituted or substituted by halogen, cyano, nitro, amino, -COOH, =O, C₁-C₁₀alkyl, C₁-C₁₀alkoxy, C₁-C₁₀alkylthio, C₁-C₁₀haloalkyl, hydroxyl, C₃-C₁₀alkoxycarbonylalkoxy, C₁-C₆alkylsulfinyl, C₁-C₆alkylsulfonyl, C₂-C₆alkyloxycarbonyl, C₂-C₆alkylcarbonyl, -CONH₂, formyl, C₂-C₇alkylaminocarbonyl, C₃-C₁₁dialkylaminocarbonyl, C₃-C₆trialkylsilyl, C₂-C₁₀alkylcarbonylamino, C₂-C₁₀alkylcarbonyloxy, phenoxy, pyridyloxy or pyridyloxy which is substituted by halogen, C₁-C₄alkyl, C₁-C₄alkoxy, cyano, nitro or amino,
and salts of the compounds of the formula I,
the compounds of the formulae Ia, Ia₁, Ia₂, Ia₃ and Ia₄ being excluded: 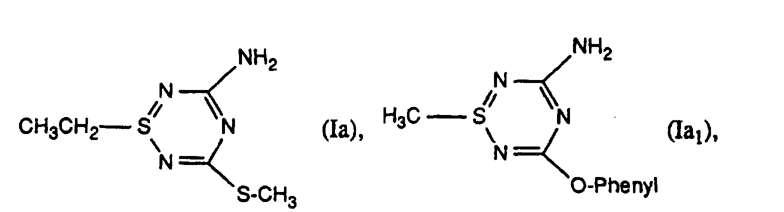 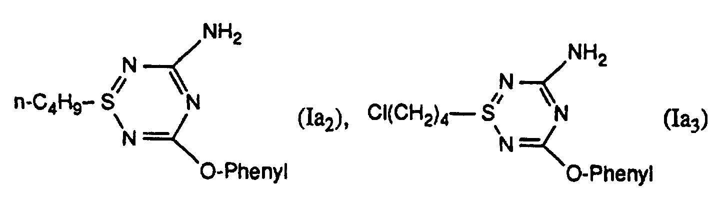 and

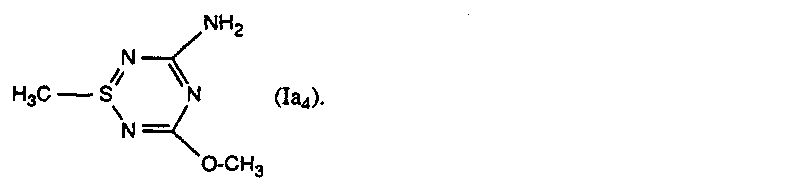

The present invention also relates to compounds of the formula I 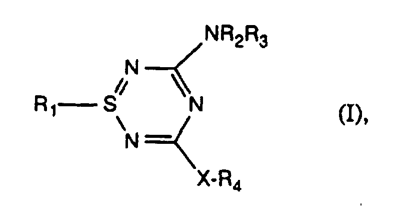 in which
R₁ is C₁-C₂₀alkyl, C₃-C₈cycloalkyl, C₃-C₈cycloalkyl-C₁-C₁₇alkyl, C₁-C₁₇alkyl-C₃-C₈cycloalkyl, C₂-C₁₇alkenyl, C₃-C₈cycloalkyl-C₂-C₁₇alkenyl, C₂-C₁₇alkenyl-C₃-C₈cycloalkyl, C₂-C₁₇alkynyl, C₃-C₈cycloalkyl-C₂-C₁₇alkynyl, C₂-C₁₇alkynyl-C₃-C₈cycloalkyl, C₅-C₈cycloalkenyl, C₅-C₈cycloalkenyl-C₁-C₁₇alkyl, C₁-C₁₇alkyl-C₅-C₈cycloalkenyl, C₅-C₈cycloalkenyl-C₂-C₁₇alkenyl, C₂-C₁₇alkenyl-C₅-C₈cycloalkenyl, C₅-C₈cycloalkenyl-C₂-C₁₇alkynyl, C₂-C₁₇alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₁₇alkynyl-C₂-C₁₇alkenyl or C₂-C₁₇alkenyl-C₂-C₁₇alkynyl, it being possible for the substituents to be unsubstituted or substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, phenyl, biphenyl, naphthyl or saturated or unsaturated heterocylyl having 3 to 8 ring atoms which, in addition to carbon atoms, has at least one hetero atom selected from nitrogen, oxygen and sulfur, it being possible for these phenyl, naphthyl and heterocyclyl substituents to be unsubstituted or substituted by halogen, cyano, nitro, -OR₅, -NR₁₀R₁₁ or -CONR₈R₉, or R₁ is saturated or unsaturated heterocylyl having 3 to 8 ring atoms which, in addition to carbon atoms, has at least one hetero atom selected from nitrogen, oxygen and sulfur, the heterocycle being bonded to the thiatriazine ring via one of its carbon atoms and it being possible for the heterocycle to be substituted by halogen, cyano, nitro, =O, -OR₅,-S(O)ₙR₆, -COOR₇, -CONR₈R₉, -NR₁₀R₁₁, =NR₁₂, =N-NR₁₃R₁₄, C₁-C₁₇alkyl, C₂-C₁₇alkenyl, C₂-C₁₇alkynyl, C₃-C₈cycloalkyl, C₅-C₈cycloalkenyl, phenyl, biphenyl, naphthyl, C₁-C₁₇alkyl which is substituted by halogen, cyano, nitro, -OR₅, -CONR₈R₉ or -NR₁₀R'₁₁, C₂-C₁₇alkenyl which is substituted by halogen, cyano, nitro, -OR₅,-CONR₈R₉ or -NR₁₀R₁₁, C₂-C₁₇alkynyl which is substituted by halogen, cyano, nitro,-OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁, phenyl which is substituted by halogen, cyano, nitro, -OR₅, -CONR₈R₉ or -NR₁₀R₁₁, biphenyl which is substituted by halogen, cyano, nitro, -OR₅, -CONR₈R₉ or -NR₁₀R₁₁, or naphthyl which is substituted by halogen, cyano, nitro, -OR₅, -CONR₈R₉ or -NR₁₀R₁₁, or
R₁ is phenyl, biphenyl, naphthyl, C₁-C₁₀alkylphenyl, C₁-C₁₀alkylbiphenyl, C₁-C₁₀alkylnaphthyl, C₂-C₁₀alkenylphenyl, C₂-C₁₀alkenylbiphenyl, C₂-C₁₀alkenylnaphthyl, C₂-C₁₀alkynylphenyl, C₂-C₁₀alkynylbiphenyl or C₂-C₁₀alkynylnaphthyl, it being possible for these substituents to be unsubstituted or substituted by halogen, cyano, nitro, -OR₅, -S(O)ₙR₆, -COOR₇, -CONR₈R₉ or-NR₁₀R₁₁, in which
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₇alkylcarbonyl, benzoyl, halobenzoyl, C₁-C₆alkylamino, C₂-C₈dialkylamino, -N=CH₂, -N=CH-C₁-C₄alkyl, -N=C(C₁-C₄alkyl)₂, C₃-C₆trialkylsilyl, C₃-C₇cycloalkyl, C₂-C₇alkenyl or C₃-C₇alkynyl,
n is 0, 1 or 2,
R₆ is hydrogen or cyano if n is 0 or
R₆ is C₁-C₅alkyl, C₁-C₅haloalkyl, C₁-C₅hydroxyalkyl, phenyl, naphthyl, C₁-C₄alkylphenyl, C₁-C₄alkylnaphthyl, halophenyl, halonaphthyl, saturated or unsaturated heterocylyl having 3 to 8 ring atoms which, in addition to carbon atoms, has at least one hetero atom selected from nitrogen, oxygen and sulfur, saturated or unsaturated haloheterocylyl having 3 to 8 ring atoms which, in addition to carbon atoms, has at least one hetero atom selected from nitrogen, oxygen and sulfur or C₃-C₇cycloalkyl,
R₇ is hydrogen, C₁-C₁₂alkyl, C₁-C₁₂haloalkyl, C₁-C₆nitroalkyl, C₁-C₆cyanoalkyl, phenyl, naphthyl, halophenyl, halonaphthyl, C₂-C₁₀alkoxyalkyl, C₁-C₁₀alkylcarbonyl, saturated or unsaturated heterocyclyl or haloheterocyclyl having 3 to 8 ring atoms which, in addition to carbon atoms, at least one hetero atom selected from nitrogen, oxygen and sulfur, C₃-C₇cycloalkyl, C₃-C₇halocycloalkyl, -N=CH₂, -N=CH-C₁-C₄alkyl, -N=C(C₁-C₄alkyl)₂ or C₂-C₆dialkylamino,
R₈ and R₉ independently of one another are hydrogen, phenyl, naphthyl, halophenyl, halonaphthyl, C₁-C₈alkyl, C₁-C₈haloalkyl, C₁-C₈alkoxy, phenoxy, naphthoxy, halophenoxy, halonaphthoxy, C₁-C₇cyanoalkyl, C₃-C₇alkenyl, C₃-C₇alkynyl or C₂-C₈alkoxyalkyl, or
R₈ and R₉ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur,
R₁₀ and R₁₁ independently of one another are hydrogen, phenyl, naphthyl, halophenyl, halonaphthyl, C₁-C₈alkyl, C₁-C₈haloalkyl, C₁-C₈alkoxy, phenoxy, naphthoxy, halophenoxy, halonaphthoxy, C₁-C₇cyanoalkyl, C₃-C₇alkenyl, C₃-C₇alkynyl, C₂-C₈ alkoxyalkyl, C₁-C₆alkylamino, C₂-C₆dialkylamino, formyl, C₁-C₈alkylcarbonyl, phenylcarbonyl or naphthylcarbonyl, it being possible therein for the phenyl or naphthyl moiety to be substituted by C₁-C₄alkyl, halogen, C₁-C₄alkoxy, hydroxy, cyano, nitro or C₁-C₆alkoxycarbonyl, or
R₁₀ and R₁₁ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur,
R₁₂ is hydrogen, C₁-C₈alkyl, C₁-C₈haloalkyl, phenyl, halophenyl, cyanophenyl, nitrophenyl, C₁-C₄alkylphenyl, C₁-C₄alkoxyphenyl, naphthyl, cyanonaphthyl, nitronaphthyl, halonaphthyl, C₁-C₄alkylnaphthyl, C₁-C₄alkoxynaphthyl, phenyl-C₁-C₆alkyl or naphthyl-C₁-C₆alkyl, and
R₁₃ and R₁₄ independently of one another are hydrogen, C₁-C₆alkyl, C₁-C₆alkylcarbonyl, phenyl, halophenyl, cyanophenyl, nitrophenyl, C₁-C₄alkylphenyl, C₁-C₄alkoxyphenyl, naphthyl, cyanonaphthyl, nitronaphthyl, halonaphthyl, C₁-C₄alkylnaphthyl or C₁-C₄alkoxynaphthyl,
R₂ and R₃ independently of one another are hydrogen, C₁-C₆alkyl, C₁-C₆alkyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₃-C₈cycloalkyl, C₁-C₅alkylcarbonyloxy, phenylcarbonyloxy, naphthylcarbonyloxy, C₁-C₆alkylamino, C₁-C₅alkoxycarbonyl, C₂-C₁₂dialkylamino, phenyl, naphthyl, phenoxy, naphthoxy, biphenyl, biphenyloxy, phenthio or naphthio, it being possible for these aromatic rings to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or CONR₈R₉
R₂ and R₃ independently of one another are C₂-C₆alkenyl, C₂-C₆alkenyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₃-C₈cycloalkyl, C₂-C₅alkylcarbonyloxy, phenylcarbonyloxy, naphthylcarbonyloxy, C₁-C₆alkylamino, C₂-C₅alkoxycarbonyl, C₂-C₁₂dialkylamino, phenyl, naphthyl, phenoxy, naphthoxy, biphenyl, biphenyloxy, phenthio or naphthio, it being possible for these aromatic rings to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or CONR₈R₉ , or
R₂ and R₃ independently of one another are C₃-C₆alkynyl, C₃-C₆alkynyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₃-C₈cycloalkyl, C₁-C₅alkylcarbonyloxy, phenylcarbonyloxy, naphthylcarbonyloxy, C₁-C₆alkylamino, C₁-C₅alkoxycarbonyl, C₂-C₁₂dialkylamino, phenyl, naphthyl, phenoxy, naphthoxy, biphenyl, biphenyloxy, phenthio or naphthio, it being possible for these aromatic rings to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or CONR₈R₉, or
R₂ and R₃ independently of one another are formyl, C₁-C₁₅alkylcarbonyl, C₂-C₁₅alkenylcarbonyl, C₄-C₉cycloalkylcarbonyl, C₆-C₉cycloalkenylcarbonyl or C₃-C₈cycloalkyl-C₁-C₆alkylcarbonyl, it being possible for these substituents to be substituted by halogen, cyano, nitro, hydroxyl, amino, C₁-C₆alkylamino, C₂-C₁₂dialkylamino, -COOH, -COOM, in which M is ammonium or an alkali metal atom or alkaline earth metal atom, or C₁-C₈alkoxycarbonyl, C₄-C₁₀cycloalkoxycarbonyl, C₁-C₈alkylaminocarbonyl or C₂-C₁₂dialkylaminocarbonyl, or
R₂ and R₃ independently of one another are saturated or unsaturated heterocyclyl or heterocyclylcarbonyl having 3 to 8 ring atoms which, in addition to carbon atoms, at least one hetero atom selected from nitrogen, oxygen and sulfur, heterocyclyl as defined above which is substituted by halogen, cyano, nitro, C₁-C₅alkyl, C₁-C₅alkoxy, C₁-C₅alkylcarbonyl, C₁-C₅alkylcarbonyloxy, C₁-C₆alkoxycarbonyl, aminocarbonyl, C₁-C₆alkylaminocarbonyl or C₂-C₁₁ dialkylaminocarbonyl, or heterocyclylcarbonyl as defined above which is substituted by halogen, cyano, nitro, C₁-C₅alkyl, C₁-C₅alkoxy, C₁-C₅alkylcarbonyl, C₁-C₆alkoxycarbonyl, aminocarbonyl, C₁-C₆alkylamino or C₁-C₅alkylcarbonyloxy, or
R₂ and R₃ independently of one another are phenylcarbonyl, biphenylcarbonyl, naphthylcarbonyl, phenyl-C₁-C₆alkylcarbonyl, biphenyl-C₁-C₆alkylcarbonyl, naphthyl-C₁-C₆alkylcarbonyl, phenyl-C₂-C₆alkenylcarbonyl, biphenyl-C₂-C₆alkenylcarbonyl, naphthyl-C₂-C₆alkenylcarbonyl,
   phenyl-C₃-C₆alkynylcarbonyl, biphenyl-C₃-C₆alkynylcarbonyl or naphthyl-C₃-C₆alkynylcarbonyl, it being possible for these substituents to be substituted by C₁-C₅alkyl, C₁-C₅alkoxy, C₁-C₅alkylthio, C₁-C₅haloalkyl, C₁-C₅alkylcarbonyl, halogen, cyano, amino, nitro, -COOR₇, C₁-C₈alkoxycarbonyl, hydroxyl, C₁-C₅alkylsulfinyl, C₁-C₅alkylsulfonyl, C₁-C₆alkylaminocarbonyl or C₂-C₁₂dialkylaminocarbonyl, or
R₂ and R₃ independently of one another are phenyl, naphthyl or saturated or unsaturated heterocylyl having 3 to 8 ring atoms which, in addition to carbon atoms, has at least one hetero atom selected from nitrogen, oxygen and sulfur, it being possible for these substituents to be substituted by halogen, cyano, nitro, C₁-C₅alkyl, C₁-C₅alkoxy, C₁-C₅alkylthio, -COOH, -CONH₂, C₁-C₆alkylaminocarbonyl, C₂-C₁₀dialkylaminocarbonyl, C₁-C₅alkylcarbonyl or C₁-C₅alkoxycarbonyl, or
R₂ and R₃ together with the nitrogen atom to which they are bonded form a saturated or unsaturated heterocyclic ring having 3 to 8 ring atoms which, in addition to carbon atoms, at least one hetero atom selected from nitrogen, oxygen and sulfur, which heterocyclic ring is unsubstituted or substituted by C₁-C₅alkyl, C₁-C₅alkoxy, halogen, cyano or nitro, or
R₂ and R₃ independently of one another are amino, C₁-C₆alkylamino, C₂-C₈dialkylamino, phenylamino, naphthylamino, C₁-C₆alkylcarbonylamino, C₁-C₁₀alkoxycarbonylamino, hydroxyl, C₁-C₆alkoxy, C₁-C₆alkylcarbonyloxy, phenoxy, biphenyloxy or naphthoxy,
X is O or S(O)ₓ, in which x is 0, 1 or 2, and
R₄ is C₁-C₈alkyl, C₂-C₈alkenyl, C₃-C₈alkynyl, C₃-C₈cycloalkyl, C₅-C₈cycloalkenyl, C₃-C₈cycloalkyl-C₁-C₄alkyl or C₁-C₄alkyl-C₃-C₈cycloalkyl, it being possible for these substituents to be unsubstituted or substituted by halogen, cyano, nitro, =O or -OR₅, or R₄ is phenyl, biphenyl, naphthyl, saturated or unsaturated heterocylyl having 3 to 8 ring atoms which, in addition to carbon atoms, has at least one hetero atom selected from nitrogen, oxygen and sulfur, C₁-C₄alkylphenyl, C₁-C₄alkylnaphthyl, phenyl-C₁-C₄alkyl or naphthyl-C₁-C₄alkyl, it being possible for these substituents to be unsubstituted or substituted by halogen, cyano, nitro, amino, -COOH, hydroxyl, C₁-C₁₀alkyl, C₁-C₁₀alkyloxy, C₁-C₆alkylamino, di-C₂-C₈alkylamino, C₁-C₁₀alkylthio, C₁-C₁₀haloalkyl, C₁-C₁₀haloalkoxy, C₁-C₁₀haloalkylthio, C₂-C₁₀alkoxycarbonylalkoxy, C₁-C₆alkylsulfinyl, C₁-C₆alkylsulfonyl, C₁-C₆alkyloxycarbonyl, C₁-C₆alkylcarbonyl,-CONH₂, formyl, C₁-C₇alkylaminocarbonyl, C₂-C₁₁ dialkylaminocarbonyl, C₃-C₆trialkylsilyl, C₁-C₁₀alkylcarbonylamino, C₁-C₁₀alkylcarbonyloxy, phenoxy, halophenoxy, pyridyloxy or pyridyloxy which is substituted by halogen, C₁-C₄alkyl, C₁-C₄alkoxy, cyano, nitro or amino, or 2 adjacent substituents on the phenyl or naphthyl ring R₄ form a carbocyclic or saturated or unsaturated heterocyclic ring having 3 to 8 ring atoms which, in addition to carbon atoms, at least one hetero atom selected from nitrogen, oxygen and sulfur, which carbocyclic or heterocyclic ring is unsubstituted or substituted by halogen, cyano, nitro, amino, -COOH, =O, C₁-C₁₀alkyl, C₁-C₁₀alkoxy, C₁-C₁₀alkylthio, C₁-C₁₀haloalkyl, hydroxyl, C₃-C₁₀alkoxycarbonylalkoxy, C₁-C₆alkylsulfinyl, C₁-C₆alkylsulfonyl, C₂-C₆alkyloxycarbonyl, C₂-C₆alkylcarbonyl,-CONH₂, formyl, C₂-C₇alkylaminocarbonyl, C₃-C₁₁ dialkylaminocarbonyl, C₃-C₆trialkylsilyl, C₂-C₁₀alkylcarbonylamino, C₂-C₁₀alkylcarbonyloxy, phenoxy, pyridyloxy or pyridyloxy which is substituted by halogen, C₁-C₄alkyl, C₁-C₄alkoxy, cyano, nitro or amino,
and salts of the compounds of the formula I,
the compounds of the formulae Ia, Ia₁, Ia₂, Ia₃ and Ia₄ being excluded: 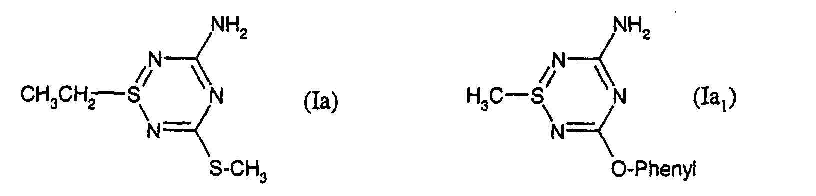 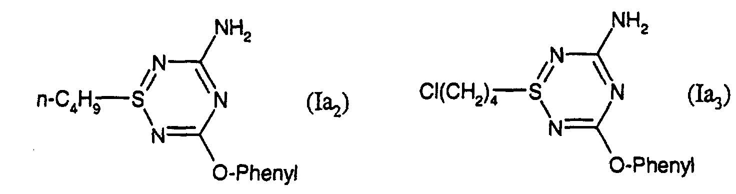 and 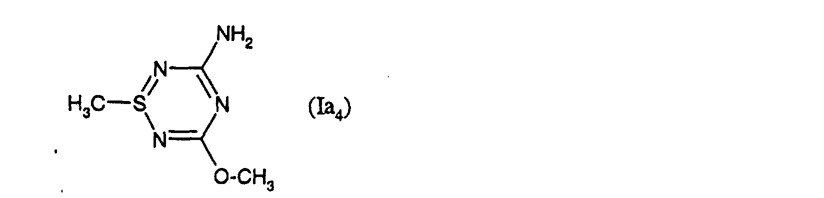 (Ia), (Ia₁), (Ia₂), (Ia₃) and (Ia₄).

The alkyl groups in the definitions of the substituents can be straight-chain or branched and are, for example, methyl, ethyl, propyl, butyl, pentyl, hexyl, heptyl, octyl, nonyl, decyl, undecyl, dodecyl, tetradecyl, hexadecyl, octadecyl or eicosyl, and the branched isomers of these. Alkoxy, alkenyl and alkynyl radicals are derived from the abovementioned alkyl radicals. The alkenyl and alkynyl radicals can be mono- or polyunsaturated.

Suitable cycloalkyl substituents have 3 to 8 carbon atoms and are, for example, cyclopropyl, cyclobutyl, cyclopentyl, cyclohexyl, cycloheptyl or cyclooctyl. The corresponding cycloalkenyl substituents can be mono- or else polyunsaturated, for example cyclopentadienyl or cyclooctatetraenyl.

If alkyl, alkenyl or alkynyl are substituents on a cycloalkyl, cycloalkenyl, phenyl, biphenyl, naphthyl or saturated or unsaturated heterocylyl having 3 to 8 ring atoms which, in addition to carbon atoms, has at least one hetero atom selected from nitrogen, oxygen and sulfur, these ring systems can also be polysubstituted by alkyl, alkenyl or alkynyl.

Halogen is, as a rule, fluorine, chlorine, bromine or iodine. The same also applies to halogen in connection with other meanings, such as haloalkyl or halophenyl.

Heterocyclyl is to be understood as meaning ring systems which, in addition to carbon atoms, have at least one hetero atom, such as nitrogen, oxygen and/or sulfur. They can be saturated or unsaturated. Such ring systems preferably have 3 to 8 ring atoms. This also applies to those heterocycles which are formed by 2 substituents bonded to a nitrogen atom, as is the case in groups such as -NR₁₀R₁₁.

Preferred heterocycles R₁ which are suitable are, for example, oxiranyl, dioxolanyl, dioxanyl, 2,3-dihydro[1,4]dioxinyl, pyrrolidinyl, piperidinyl, morpholinyl, thiomorpholinyl, pyridinyl, pyrimidinyl, pyrrolyl, imidazolyl, tetrahydrofuranyl, tetrahydropyranyl, dihydrofuranyl, thiophenyl, 2,3-dihydrothiophenyl, tetrahydrothiophenyl and dihydropyranyl.

Heterocyclyl as a substituent of a group R₁ can be, for example, oxiranyl, dioxolanyl, pyrrolidinyl, piperidinyl, morpholinyl, pyridinyl, imidazolyl, tetrahydrofuranyl, tetrahydropyranyl, dihydrofuranyl, dihydropyranyl or phthalimidyl.

Heterocycles R₆ are preferably, inter alia, pyridinyl, pyrimidinyl and triazinyl.

Preferred heterocycles R₇ are, for example, oxetanyl, pyridyl, thiophenyl and furanyl.

R₈ and R₉ preferably form rings such as piperidinyl, morpholinyl and pyrrolidine.

Preferred examples of heterocycles formed by R₁₀ and R₁₁, or R₂ and R₃, together with the nitrogen atom to which these radicals are bonded, are piperidinyl, morpholinyl, pyrrolidinyl and imidazolyl.

Heterocycles R₉ are preferably thiophenyl, furanyl, pyridinyl and oxetanyl.

Phenyl and naphthyl rings R₄ can be substituted by carbo- or heterocyclic radicals which are formed by 2 adjacent substituents from these phenyl or naphthyl rings. The carbocycles have preferably 4 to 6 carbon atoms, such as cyclobutyl, cyclopentyl and cyclohexyl. Suitable heterocyclic groups are, in particular, dioxolanyl and tetrahydrofuranyl.

Heterocycles R₄ such as succinimidyl, pyridinyl, thiophenyl or furanyl can have fused to them carbocycles, such as phenyl or cyclohexenyl.

Heterocycles R₂ and R₃ are preferably pyridinyl, pyrrolidinyl and pyrimidinyl.

Heterocycles formed by -NR₂R₃ encompass, for example, succinimidyl, imidazolyl and triazolyl, it being possible for such groups to have fused to them carbocycles, such as phenyl or cyclohexene.

Heterocyclylcarbonyl R₂ or R₃ is, for example, pyridinyl, pyrrolidinyl, triazolyl, thiophenyl, furanyl or isoxazolyl.

In substituents such as -N=C(C₁-C₄-alkyl)₂, the alkyl radicals can be identical or different. They are preferably identical. The same also applies to the alkyl radicals in dialkylamino, dialkylaminocarbonyl, trialkylammonium and trialkylsilyl substituents.

In the definitions cyanoalkyl, alkoxycarbonyl, alkylcarbonyloxy, alkylcarbonyl, alkylcarbonylamino, alkylaminocarbonyl, dialkylaminocarbonyl, alkoxycarbonylalkoxy and alkenylcarbonyl, the cyano or carbonyl carbon atom is not included in the upper and lower limits of the number of carbon atoms in each case specified.

Suitable alkali metals and alkaline earth metals are, for example, lithium, sodium, potassium, magnesium, calcium or barium.

The invention also encompasses the salts which the compounds of the formula I can form, in particular with amines, alkali metal bases and alkaline earth metal bases or quaternary ammonium bases.

Salt formers amongst the alkali metal hydroxides and alkaline earth metal hydroxides are, in particular, the hydroxides of lithium, sodium, potassium, magnesium or calcium, but especially those of sodium or potassium.

Examples of amines which are suitable for the formation of ammonium salts are ammonia as well as primary, secondary and tertiary C₁-C₁₈alkylamines, C₁-C₄hydroxyalkylamines and C₂-C₄alkoxyalkylamines, for example methylamine, ethylamine, n-propylamine, isopropylamine, the four butylamine isomers, n-amylamine, isoamylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, di-isopropylamine, di-n-butylamine, di-n-amylamine, di-isoamylamine, dihexylamine, diheptylamine, dioctylamine, ethanolamine, n-propanolamine, isopropanolamine, N,N-diethanolamine, N-ethylpropanolamine, N-butylethanolamine, allylamine, n-butenyl-2-amine, n-pentenyl-2-amine, 2,3-dimethylbutenyl-2-amine, dibutenyl-2-amine, n-hexenyl-2-amine, propylenediamine, trimethylamine, triethylamine, tri-n-propylamine, tri-isopropylamine, tri-n-butylamine, triisobutylamine, tri-sec-butylamine, tri-n-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, o, m, p-toluidines, phenylenediamines, benzidines, naphthylamines and o, m, p-chloroanilines; but in particular triethylamine, isopropylamine and di-isopropylamine.

The compounds of the formula I have an asymmetric centre in the sulfur atom of the thiatriazine ring. This is why the preparation of these compounds gives racemates, which can be separated into the corresponding enantiomers by customary separation methods. If the substituents of the thiatriazine ring have further asymmetric centres, it is also possible to separate the corresponding diastereoisomers in the customary manner. The present invention also encompasses such diastereoisomers and enantiomers.

In preferred compounds of the formula I, R₁ is C₁-C₂₀alkyl, C₃-C₈cycloalkyl, C₃-C₈cycloalkyl-C₁-C₁₇alkyl, C₁-C₁₇alkyl-C₃-C₈cycloalkyl, C₂-C₁₇alkenyl, C₃-C₈cycloalkyl-C₂-C₁₇alkenyl, C₂-C₁₇alkenyl-C₃-C₈cycloalkyl, C₂-C₁₇alkynyl, C₃-C₈cycloalkyl-C₂-C₁₇alkynyl, C₂-C₁₇alkynyl-C₃-C₈cycloalkyl, C₅-C₈cycloalkenyl, C₅-C₈cycloalkenyl-C₁-C₁₇alkyl, C₁-C₁₇alkyl-C₅-C₈cycloalkenyl, C₅-C₈cycloalkenyl-C₂-C₁₇alkenyl, C₂-C₁₇alkenyl-C₅-C₈cycloalkenyl, C₅-C₈cycloalkenyl-C₂-C₁₇alkynyl, C₂-C₁₇alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₁₇alkynyl-C₂-C₁₇alkenyl or C₂-C₁₇alkenyl-C₂-C₁₇alkynyl, it being possible for these substituents to be substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, phenyl, biphenyl, naphthyl or heterocyclyl, it being possible for these phenyl, naphthyl and heterocyclyl substituents to be substituted by halogen, cyano, nitro, -OR₅, -NR₁₀R₁₁ or -CONR₈R₉, or
R₁ is heterocyclyl, the heterocycle being bonded to the thiatriazine ring via one of its carbon atoms and it being possible for the heterocycle to be substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, C₁-C₁₇alkyl, C₂-C₁₇alkenyl, C₂-C₁₇alkynyl, C₃-C₈cycloalkyl, C₅-C₈cycloalkenyl, phenyl, biphenyl, naphthyl, C₁-C₁₇alkyl which is substituted by halogen, cyano, nitro, -OR₅, -CONR₈R₉ or -NR₁₀R₁₁, C₂-C₁₇alkenyl which is substituted by halogen, cyano, nitro, -OR₅, -CONR₈R₉ or -NR₁₀R₁₁, C₂-C₁₇alkynyl which is substituted by halogen, cyano, nitro, -OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁, phenyl which is substituted by halogen, cyano, nitro, -OR₅, -CONR₈R₉ or -NR₁₀R₁₁, biphenyl which is substituted by halogen, cyano, nitro, -OR₅, -CONR₈R₉ or -NR₁₀R₁₁or naphthyl which is substituted by halogen, cyano, nitro, -OR₅, -CONR₈R₉ or -NR₁₀R₁₁, and R₅, R₆, R₇, R₈, R₉, R₁₀, R₁₁, R₁₂, R₁₃, R₁₄ and n are as defined above.

In a group of preferred compounds of the formula I, R₁ is C₁-C₂₀alkyl, C₃-C₈cycloalkyl, C₃-C₈cycloalkyl-C₁-C₁₇alkyl, C₁-C₁₇alkyl-C₃-C₈cycloalkyl, C₂-C₁₇alkenyl, C₃-C₈cycloalkyl-C₂-C₁₇alkenyl, C₂-C₁₇alkenyl-C₃-C₈cycloalkyl, C₂-C₁₇alkynyl, C₃-C₈cycloalkyl-C₂-C₁₇alkynyl or C₂-C₁₇alkynyl-C₃-C₈cycloalkyl, it being possible for these substituents to be substituted by halogen or -OR₅, and R₅ is as defined above.

In a further preferred group of the compounds of the formula I, R₁ is a non-aromatic heterocyclyl, the heterocycle being bonded to the thiatriazine ring via one of its carbon atoms and it being possible for the heterocycle to be substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, C₁-C₁₇alkyl, C₂-C₁₇alkenyl, C₂-C₁₇alkynyl, C₃-C₈cycloalkyl, C₅-C₈cycloalkenyl, phenyl, biphenyl, naphthyl, C₁-C₁₇alkyl which is substituted by halogen, cyano, nitro, -OR₅, -CONR₈R₉ or -NR₁₀R₁₁, C₂-C₁₇alkenyl which is substituted by halogen, cyano, nitro, -OR₅, -CONR₈R₉ or -NR₁₀R₁₁, C₂-C₁₇alkynyl which is substituted by halogen, cyano, nitro, -OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁, phenyl which is substituted by halogen, cyano, nitro, -OR₅, -CONR₈R₉ or -NR₁₀R₁₁, biphenyl which is substituted by halogen, cyano, nitro, -OR₅, -CONR₈R₉ or -NR₁₀R₁₁ or naphthyl which is substituted by halogen, cyano, nitro, -OR₅, -CONR₈R₉ or -NR₁₀R₁₁.

Particularly preferred amongst this group of compounds of the formula I are those in which R₁ can be substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, C₁-C₁₇alkyl, C₂-C₁₇alkenyl, C₂-C₁₇alkynyl, C₃-C₈cycloalkyl, C₅-C₈cycloalkenyl, C₁-C₁₇alkyl which is substituted by halogen, cyano, nitro, -OR₅, -CONR₈R₉ or -NR₁₀R₁₁, C₂-C₁₇alkenyl which is substituted by halogen, cyano, nitro, -OR₅, -CONR₈R₉ or -NR₁₀R₁₁, C₂-C₁₇alkynyl which is substituted by halogen, cyano, nitro, -OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁.

In another group of particularly suitable compounds of the formula I, R₂ and R₃ independently of one another are hydrogen, C₁-C₆alkyl, C₁-C₆alkyl which is substituted by halogen, cyano, nitro, C₁-C₈alkoxy, phenyl, phenoxy, phenyl which is substituted by halogen, cyano, nitro, -OR₅, -NR₁₀R₁₁ or -CONR₈R₉, phenoxy which is substituted by halogen, cyano, nitro, -OR₅, -NR₁₀R₁₁ or -CONR₈R₉, C₁-C₈alkylthio, phenthio, phenthio which is substituted by halogen, cyano, nitro, -OR₅, -NR₁₀R₁₁ or -CONR₈R₉, or
R₂ and R₃ independently of one another are C₂-C₆alkenyl, C₂-C₆alkenyl which is substituted by halogen, cyano, nitro, C₁-C₈alkoxy, phenoxy, phenoxy which is substituted by halogen, cyano, nitro, -OR₅, -NR₁₀R₁₁ or -CONR₈R₉, C₁-C₈alkylthio, phenthio which is substituted by halogen, cyano, nitro, -OR₅, -NR₁₀R₁₁ or -CONR₈R₉, or
R₂ and R₃ independently of one another are C₂-C₆alkynyl, C₂-C₆alkynyl which is substituted by halogen, cyano, nitro, C₁-C₈alkoxy, phenoxy, naphthoxy, C₁-C₈alkylthio, phenthio, phenoxy which is substituted by halogen, cyano, nitro, -OR₅, -NR₁₀R₁₁ or -CONR₈R₉, phenthio which is substituted by halogen, cyano, nitro, -OR₅, -NR₁₀R₁₁ or -CONR₈R₉, or
R₂ and R₃ independently of one another are formyl, C₂-C₁₅alkylcarbonyl, C₂-C₁₅alkenylcarbonyl, C₄-C₉cycloalkylcarbonyl, C₆-C₉cycloalkenylcarbonyl or C₃-C₈cycloalkyl-C₂-C₆alkylcarbonyl, it being possible for these substituents to be substituted by halogen, cyano, nitro, hydroxyl, amino, C₁-C₆alkylamino, C₂-C₁₂dialkylamino, -COOH, C₂-C₈alkoxycarbonyl, C₄-C₁₀cycloalkoxycarbonyl, C₂-C₈alkylaminocarbonyl or C₃-C₁₂dialkylaminocarbonyl, or
R₂ and R₃ independently of one another are heterocyclylcarbonyl, or
R₂ and R₃ independently of one another are phenylcarbonyl, phenyl-C₂-C₆alkylcarbonyl, phenyl-C₃-C₆alkenylcarbonyl or phenyl-C₃-C₆alkynylcarbonyl, it being possible for these substituents to be substituted by C₁-C₅alkyl, C₁-C₅alkoxy, C₁-C₅alkylthio, C₁-C₅haloalkyl, C₂-C₅alkylcarbonyl, halogen, cyano, amino, nitro, -COOH, C₂-C₈alkoxycarbonyl, hydroxyl, C₂-C₆alkylaminocarbonyl or
C₂-C₁₂dialkylaminocarbonyl, or
R₂ and R₃ independently of one another are phenyl-C₁-C₆alkyl, phenyl-C₂-C₆alkenyl or phenyl-C₂-C₆alkynyl, it being possible for these substituents to be substituted by C₁-C₅alkyl, C₂-C₅alkenyl, C₂-C₅alkynyl, hydroxyl, C₁-C₅alkoxy, formyl, C₂-C₆alkylcarbonyl, C₂-C₆alkoxycarbonyl, C₂-C₆alkylaminocarbonyl, C₃-C₉dialkylaminocarbonyl, nitro or C₁-C₅alkylthio, or
R₂ and R₃ independently of one another are phenyl or phenyl which is substituted by halogen, cyano, nitro, C₁-C₅alkyl, C₁-C₅alkoxy, C₁-C₅alkylthio, -COOH, -CONH₂, C₂-C₆alkylaminocarbonyl, C₃-C₁₀dialkylaminocarbonyl, C₂-C₅alkylcarbonyl or C₂-C₅alkoxycarbonyl, or
R₂ and R₃ independently of one another are amino, C₁-C₆alkylamino, C₂-C₈dialkylamino, phenylamino, C₂-C₆alkylcarbonylamino, C₂-C₁₀alkoxycarbonylamino, hydroxyl, C₁-C₆alkoxy, C₂-C₆alkylcarbonyloxy or phenoxy.

Amongst these, particularly suitable compounds of the formula I are those in which R₂ and R₃ independently of one another are hydrogen, C₁-C₆alkyl, C₂-C₆alkenyl, C₂-C₆alkynyl, C₂-C₁₅alkylcarbonyl, C₂-C₁₅alkenylcarbonyl, C₄-C₉cycloalkylcarbonyl, it being possible for these substituents to be substituted by halogen, cyano, nitro or hydroxyl, or
R₂ and R₃ independently of one another are amino, C₁-C₆alkylamino, C₂-C₈dialkylamino, phenylamino, C₂-C₆alkylcarbonylamino or C₂-C₁₀alkoxycarbonylamino.

In other groups of preferred compounds of the formula I, X is O or R₄ is phenyl or phenyl which is substituted by halogen.

Preferred compounds of the formula I are those in which
R₁ is C₁-C₁₀alkyl, C₃-C₈cycloalkyl, C₃-C₈cycloalkyl-C₁-C₆alkyl, C₁-C₈alkyl-C₃-C₈cycloalkyl, C₂-C₁₀alkenyl, C₃-C₈cycloalkyl-C₂-C₆alkenyl, C₂-C₈alkenyl-C₃-C₈cycloalkyl, C₂-C₁₀alkynyl, C₃-C₈cycloalkyl-C₂-C₆alkynyl, C₂-C₈alkynyl-C₃-C₈cycloalkyl, C₅-C₈cycloalkenyl, C₅-C₈cycloalkenyl-C₁-C₆alkyl, C₁-C₈alkyl-C₅-C₈cycloalkenyl, C₅-C₈cycloalkenyl-C₂-C₆alkenyl, C₂-C₈alkenyl-C₅-C₈cycloalkenyl, C₅-C₈cycloalkenyl-C₂-C₆alkynyl, C₂-C₈alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₈alkynyl-C₂-C₆alkenyl or C₂-C₈alkenyl-C₂-C₆alkynyl, it being possible for these substituents to be unsubstituted or substituted by halogen, cyano, nitro, =O, -OR₅, -S(O)ₙR₆, -COOR₇, -CONR₈R₉, -NR₁₀R₁₁, =NR₁₂, phenyl, biphenyl, naphthyl or heterocyclyl, it being possible for these phenyl, naphthyl and heterocyclyl substituents to be unsubstituted or substituted by halogen, cyano, nitro, -OR₅, -NR₁₀R₁₁ or -CONR₈R₉, or
R₁ is heterocyclyl, the heterocycle being bonded to the thiatriazine ring via one of its carbon atoms and it being possible for the heterocycle to be substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, C₁-C₈alkyl, C₂-C₈alkenyl, C₂-C₈alkynyl, C₃-C₈cycloalkyl, C₅-C₈cycloalkenyl, phenyl, biphenyl, naphthyl, C₁-C₈alkyl which is substituted by halogen, cyano, nitro, -OR₅, -CONR₈R₉ or -NR₁₀R₁₁, C₂-C₈alkenyl which is substituted by halogen, cyano, nitro, -OR₅, -CONR₈R₉ or -NR₁₀R₁₁, C₂-C₈alkynyl which is substituted by halogen, cyano, nitro, -OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁, phenyl which is substituted by halogen, cyano, nitro, C₁-C₆alkyl, C₁-C₆haloalkyl, -OR₅, -CONR₈R₉ or -NR₁₀R₁₁, biphenyl which is substituted by halogen, cyano, nitro, -OR₅, -CONR₈R₉ or -NR₁₀R₁₁, or naphthyl which is substituted by halogen, cyano, nitro, -OR₅, -CONR₈R₉ or -NR₁₀R₁₁, or R₁ is phenyl, biphenyl, naphthyl, C₁-C₄alkylphenyl, C₁-C₄alkylbiphenyl, C₁-C₄alkylnaphthyl, C₂-C₄alkenylphenyl, C₂-C₄alkenylbiphenyl, C₂-C₄alkenylnaphthyl, C₂-C₄alkynylphenyl, C₂-C₄alkynylbiphenyl or C₂-C₄alkynylnaphthyl, it being possible for these substituents to be substituted by halogen, cyano, nitro, -OR₅, -S(O)ₙR₆, -COOR₇, -CONR₈R₉ or -NR₁₀R₁₁, in which
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₄alkylcarbonyl, benzoyl, halobenzoyl, C₁-C₄alkylamino, C₂-C₆dialkylamino, C₃-C₆trialkylsilyl, C₃-C₆cycloalkyl, C₂-C₄alkenyl or C₃-C₄alkynyl,
n is 0, 1 or 2,
R₆ is hydrogen or cyano if n is 0, or
R₆ is C₁-C₄alkyl, C₁-C₄haloalkyl, phenyl, C₁-C₄alkylphenyl, halophenyl, heterocyclyl, haloheterocyclyl or C₃-C₆cycloalkyl,
R₇ is hydrogen, C₁-C₄alkyl, C₁-C₄haloalkyl, C₁-C₄cyanoalkyl, phenyl, halophenyl, C₂-C₄alkoxyalkyl, heterocyclyl or haloheterocyclyl,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or
R₈ and R₉ together with the nitrogen atom to which they are bonded form a heterocycle which can be halogenated,
R₁₀ and R₁₁ independently of one another are hydrogen, phenyl, halophenyl, C₁-C₄alkyl, C₁-C₄haloalkyl, C₁-C₄-cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be substituted by C₁-C₄alkyl, halogen, C₁-C₄alkoxy, hydroxyl, cyano, nitro or C₁-C₄alkoxycarbonyl, or
R₁₀ and R₁₁ together with the nitrogen atom to which they are bonded to form a heterocycle which can be halogenated, and
R₁₂ is hydrogen, C₁-C₄alkyl, C₁-C₄haloalkyl, phenyl, halophenyl, cyanophenyl, nitrophenyl, C₁-C₄alkylphenyl, C₁-C₄alkoxyphenyl or phenyl-C₁-C₄alkyl.

Particularly preferred amongst these compounds are those in which
R₁ is C₁-C₁₀alkyl, C₃-C₈cycloalkyl, C₃-C₈cycloalkyl-C₁-C₆alkyl, C₁-C₈alkyl-C₃-C₈cycloalkyl, C₂-C₁₀alkenyl, C₃-C₈cycloalkyl-C₂-C₆alkenyl, C₂-C₈alkenyl-C₃-C₈cycloalkyl, C₂-C₁₀alkynyl, C₃-C₈cycloalkyl-C₂-C₆alkynyl, C₂-C₈alkynyl-C₃-C₈cycloalkyl, C₅-C₈cycloalkenyl, C₅-C₈cycloalkenyl-C₁-C₆alkyl, C₁-C₈alkyl-C₅-C₈cycloalkenyl, C₅-C₈cycloalkenyl-C₂-C₆alkenyl, C₂-C₈alkenyl-C₅-C₈cycloalkenyl, C₅-C₈cycloalkenyl-C₂-C₆alkynyl, C₂-C₈alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₈alkynyl-C₂-C₆alkenyl or
C₂-C₈alkenyl-C₂-C₆alkynyl, it being possible for these substituents to be unsubstituted or substituted by halogen, cyano, =O, -OR₅, -COOR₇, -CONR₈R₉, -NR₁₀R₁₁ or phenyl, it being possible for this phenyl ring to be unsubstituted or substituted by halogen, or
R₁ is heterocyclyl, the heterocycle being bonded to the thiatriazine ring via one of its carbon atoms and it being possible for the heterocycle to be substituted by halogen, cyano, =O, -OR₅, -S(O)ₙR₆, -COOR₇, -CONR₈R₉, -NR₁₀R₁₁, C₁-C₈alkyl, C₂-C₈alkenyl, C₂-C₅alkynyl, C₃-C₈cycloalkyl, C₅-C₈cycloalkenyl, phenyl, C₁-C₈alkyl which is substituted by halogen, cyano, -OR₅, -CONR₈R₉ or -NR₁₀R₁₁, C₂-C₈alkenyl which is substituted by halogen, cyano, -OR₅, -CONR₈R₉ or -NR₁₀R₁₁, C₂-C₈alkynyl which is substituted by halogen, cyano, -OR₅, -CONR₈R₉ or -NR₁₀R₁₁, C₃-C₈cycloalkyl which is substituted by halogen, cyano, -OR₅, -CONR₈R₉ or -NR₁₀R₁₁, C₅-C₈cycloalkenyl which is substituted by halogen, cyano, -OR₅, -CONR₈R₉ or -NR₁₀R₁₁, phenyl which is substituted by halogen, cyano, nitro, C₁-C₄alkyl, C₁-C₆haloalkyl, -OR₅, -CONR₈R₉ or -NR₁₀R₁₁, or R₁ is phenyl, biphenyl, naphthyl, C₁-C₄alkylphenyl, C₁-C₄alkylbiphenyl, C₁-C₄alkylnaphthyl, C₂-C₄alkenylphenyl, C₂-C₄alkenylbiphenyl, C₂-C₄alkenylnaphthyl, C₂-C₄alkynylphenyl, C₂-C₄alkynylbiphenyl or C₂-C₄alkynylnaphthyl, it being possible for these substituents to be unsubstituted or substituted by halogen, cyano, -OR₅, -S(O)ₙR₆, -COOR₇, -CONR₈R₉ or -NR₁₀R₁₁, in which
R₅ is hydrogen, C₁-C₆alkyl, C₁-C₆haloalkyl, C₂-C₆alkoxyalkyl, C₁-C₆cyanoalkyl, phenyl-C₁-C₄alkyl, C₁-C₄alkylcarbonyl, benzoyl, halobenzoyl, C₃-C₆trialkylsilyl, C₃-C₆cycloalkyl, C₂-C₄alkenyl or C₃-C₄alkynyl,
n is 0, 1 or 2,
R₆ is hydrogen or cyano if n is 0 or
R₆ is C₁-C₄alkyl, C₁-C₄haloalkyl, phenyl or C₁-C₄alkylphenyl,
R₇ is hydrogen, C₁-C₄alkyl, C₁-C₄haloalkyl, C₁-C₄cyanoalkyl, phenyl, halophenyl, C₂-C₄alkoxyalkyl, heterocyclyl or haloheterocyclyl,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or R₈ and R₉ together with the nitrogen atom to which they are bonded form a heterocycle which can be halogenated,
R₁₀ and R₁₁ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be substituted by C₁-C₄alkyl, halogen, C₁-C₄alkoxy, hydroxyl, cyano, nitro or C₁-C₄alkoxycarbonyl, or
R₁₀ and R₁₁ together with the nitrogen atom to which they are bonded form a heterocycle which can be halogenated.

Very particularly preferred compounds of the formula I are those in which R₁ is C₁-C₁₀alkyl, C₃-C₈cycloalkyl, C₃-C₈cycloalkyl-C₁-C₆alkyl, C₁-C₈alkyl-C₃-C₈cycloalkyl, C₂-C₁₀alkenyl, C₃-C₈cycloalkyl-C₂-C₆alkenyl, C₂-C₈alkenyl-C₃-C₈cycloalkyl, C₂-C₁₀alkynyl, C₃-C₈cycloalkyl-C₂-C₆alkynyl, C₂-C₈alkynyl-C₃-C₈cycloalkyl, C₅-C₈cycloalkenyl, C₅-C₈cycloalkenyl-C₁-C₆alkyl, C₁-C₈alkyl-C₅-C₈cycloalkenyl, C₅-C₈cycloalkenyl-C₂-C₆alkenyl, C₂-C₈alkenyl-C₅-C₈cycloalkenyl, C₅-C₈cycloalkenyl-C₂-C₆alkynyl, C₂-C₈alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₈alkynyl-C₂-C₆alkenyl or C₂-C₈alkenyl-C₂-C₆alkynyl, it being possible for these substituents to be unsubstituted or substituted by halogen, cyano, =O, -OR₅, -COOR₇, -CONR₈R₉, -NR₁₀R₁₁ or phenyl, it being possible for this phenyl ring to be unsubstituted or substituted by halogen, or
R₁ is heterocyclyl, the heterocycle being bonded to the thiatriazine ring via one of its carbon atoms and it being possible for the heterocycle to be substituted by halogen, cyano, =O, C₁-C₄alkoxy, C₁-C₈alkyl, C₂-C₈alkenyl, C₂-C₈alkynyl, phenyl, C₁-C₈alkyl which is substituted by halogen, C₂-C₈alkenyl which is substituted by halogen, C₂-C₈alkynyl which is substituted by halogen, phenyl which is substituted by halogen, cyano, nitro, C₁-C₄alkyl or C₁-C₄alkoxy, or
R₁ is phenyl, biphenyl, naphthyl, C₁-C₄alkylphenyl, C₁-C₄alkylbiphenyl, C₁-C₄alkylnaphthyl, C₂-C₄alkenylphenyl or C₂-C₄alkynylphenyl, it being possible for these substituents to be unsubstituted or substituted by halogen, cyano, -OR₅, -S(O)ₙR₆,
-COOR₇, -CONR₈R₉ or -NR₁₀R₁₁, in which
R₅ is hydrogen, C₁-C₆alkyl, C₁-C₆haloalkyl, C₂-C₆alkoxyalkyl, C₁-C₆cyanoalkyl, phenyl-C₁-C₄alkyl, C₁-C₄alkylcarbonyl, benzoyl, halobenzoyl, C₃-C₆trialkylsilyl, C₃-C₆cycloalkyl, C₂-C₄alkenyl or C₃-C₄alkynyl,
n is 0, 1 or 2,
R₆ is hydrogen or cyano if n is 0, or
R₆ is C₁-C₄alkyl, C₁-C₄haloalkyl, phenyl or C₁-C₄alkylphenyl,
R₇ is hydrogen, C₁-C₄alkyl, C₁-C₄haloalkyl, C₁-C₄cyanoalkyl, phenyl, halophenyl, C₂-C₄alkoxyalkyl, heterocyclyl or haloheterocyclyl,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or R₈ and R₉ together with the nitrogen atom to which they are bonded form a heterocycle which can be halogenated,
R₁₀ and R₁₁ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be unsubstituted or substituted by C₁-C₄alkyl, halogen, C₁-C₄alkoxy, hydroxyl, cyano, nitro or C₁-C₄alkoxycarbonyl, or
R₁₀ and R₁₁ together with the nitrogen atom to which they are bonded form a heterocycle which can be halogenated.

Other preferred compounds of the formula I are those in which R₂ and R₃ independently of one another are hydrogen, C₁-C₆alkyl, C₁-C₆alkyl which is substituted by halogen, cyano, nitro, C₁-C₄alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₃-C₈cycloalkyl, C₁-C₅alkylcarbonyloxy, phenylcarbonyloxy, naphthylcarbonyloxy, C₁-C₆alkylamino, C₁-C₅alkoxycarbonyl, C₂-C₆dialkylamino or phenyl, it being possible for the phenyl ring to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or -CONR₈R₉, or R₂ and R₃ independently of one another are C₂-C₆alkenyl, C₂-C₆alkenyl which is substituted by halogen, cyano, nitro, C₁-C₄alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₁-C₆alkylamino, C₂-C₅alkoxycarbonyl, C₂-C₆dialkylamino or phenyl, it being possible for the phenyl ring to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or -CONR₈R₉, or
R₂ and R₃ independently of one another are C₃-C₆alkynyl, C₃-C₆alkynyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₁-C₆alkylamino, C₂-C₅alkoxycarbonyl, C₂-C₆dialkylamino or phenyl, it being possible for the phenyl ring to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or -CONR₈R₉, or
R₂ and R₃ independently of one another are formyl, C₁-C₈alkylcarbonyl, C₂-C₈alkenylcarbonyl, C₄-C₉cycloalkylcarbonyl, C₆-C₉cycloalkenylcarbonyl or C₃-C₈cycloalkyl-C₁-C₆alkylcarbonyl, it being possible for these substituents to be unsubstituted or substituted by halogen, cyano, nitro, hydroxyl, amino, C₁-C₆alkylamino, C₂-C₇dialkylamino, -COOH, -COOM, in which M is ammonium or an alkali metal atom or alkaline earth metal atom, or C₂-C₈alkoxycarbonyl, C₄-C₁₀cycloalkoxycarbonyl, C₁-C₈alkylaminocarbonyl or C₂-C₈dialkylaminocarbonyl, or
R₂ and R₃ independently of one another are heterocyclyl, heterocyclylcarbonyl, heterocyclyl which is substituted by halogen, cyano, nitro, C₁-C₅alkyl, C₁-C₅alkoxy, C₁-C₅alkylcarbonyl, C₁-C₅alkylcarbonyloxy, C₁-C₆alkoxycarbonyl, aminocarbonyl, C₁-C₆alkylaminocarbonyl or C₂-C₈dialkylaminocarbonyl, or heterocyclylcarbonyl which is substituted by halogen, cyano, nitro, C₁-C₄alkyl, C₁-C₄alkoxy, C₁-C₄alkylcarbonyl, C₁-C₄alkoxycarbonyl, aminocarbonyl, C₁-C₆alkylamino or C₁-C₅alkylcarbonyloxy, or
R₂ and R₃ independently of one another are phenylcarbonyl, biphenylcarbonyl, naphthylcarbonyl, phenyl-C₁-C₆alkylcarbonyl, biphenyl-C₁-C₆alkylcarbonyl, naphthyl-C₁-C₆alkylcarbonyl, phenyl-C₂-C₆alkenylcarbonyl, biphenyl-C₂-C₆alkenylcarbonyl or naphthyl-C₂-C₆alkenylcarbonyl, it being possible for these substituents to be unsubstituted or substituted by C₁-C₄alkyl, C₁-C₄alkoxy, C₁-C₄alkylthio, C₁-C₄haloalkyl, C₁-C₄alkylcarbonyl, halogen, cyano, amino, nitro, -COOR₇, C₁-C₅alkoxycarbonyl, hydroxyl, C₁-C₄alkylsulfonyl, C₁-C₅alkylaminocarbonyl or C₂-C₆dialkylaminocarbonyl, or
R₂ and R₃ independently of one another are phenyl, naphthyl or heterocyclyl, it being possible for these substituents to be unsubstituted or substituted by halogen, cyano, nitro, C₁-C₄alkyl, C₁-C₄alkoxy, C₁-C₄alkylthio, -COOH, -CONH₂, C₁-C₅alkylaminocarbonyl, C₂-C₇dialkylaminocarbonyl, C₁-C₄alkylcarbonyl or C₁-C₅alkoxycarbonyl, or
R₂ and R₃ together with the nitrogen atom to which they are bonded form a heterocyclic ring which can be substituted by C₁-C₄alkyl, C₁-C₄alkoxy, halogen, cyano or nitro, or
R₂ and R₃ independently of one another are amino, C₁-C₄alkylamino, C₂-C₆dialkylamino, phenylamino, C₁-C₅alkylcarbonylamino, C₁-C₅alkoxycarbonylamino, hydroxyl, C₁-C₄alkoxy, C₁-C₅alkylcarbonyloxy or phenoxy,
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₄alkylcarbonyl, benzoyl, halobenzoyl, C₁-C₄-alkylamino, C₂-C₆dialkylamino, C₃-C₆trialkylsilyl, C₃-C₆cycloalkyl, C₂-C₄alkenyl or C₃-C₄alkynyl,
R₇ is hydrogen, C₁-C₄alkyl, C₁-C₄halogenalkyl, C₁-C₄cyanoalkyl, phenyl, halophenyl, C₂-C₄alkoxyalkyl, heterocyclyl or haloheterocyclyl,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or R₈ and R₉ together with the nitrogen atom to which they are bonded form a heterocycle which can be halogenated,
R₁₀ and R₁₁ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be substituted by C₁-C₄alkyl, halogen, C₁-C₄alkoxy, hydroxyl, cyano, nitro or C₁-C₄alkoxycarbonyl, or
R₁₀ and R₁₁ together with the nitrogen atom to which they are bonded form a heterocycle which can be halogenated.

Amongst these, particularly preferred compounds are those in which R₂ and R₃ independently of one another are hydrogen, C₁-C₆alkyl, C₁-C₆alkyl which is substituted by halogen, hydroxyl, amino, ammonium, tri-C₁-C₄alkylammonium, -COOH, -COOM, where M is ammonium or an alkali metal atom or alkaline earth metal atom, or C₁-C₅alkylcarbonyloxy, phenylcarbonyloxy, C₁-C₆alkylamino, C₁-C₅alkoxycarbonyl or C₂-C₆dialkylamino, or
R₂ and R₃ independently of one another are formyl, C₁-C₈alkylcarbonyl, C₂-C₈alkenylcarbonyl, C₄-C₉cycloalkylcarbonyl, C₆-C₉cycloalkenylcarbonyl or C₃-C₈cycloalkyl-C₁-C₆alkylcarbonyl, it being possible for these substituents to be unsubstituted or substituted by halogen, cyano, hydroxyl, amino, -COOH or -COOM, where M is ammonium or an alkali metal atom or alkaline earth metal atom, or
R₂ and R₃ independently of one another are heterocyclyl, heterocyclylcarbonyl, heterocyclyl which is substituted by halogen, cyano, nitro, C₁-C₅alkyl, C₁-C₅alkoxy or C₁-C₆alkoxycarbonyl, or heterocyclylcarbonyl which is substituted by halogen, cyano, nitro, C₁-C₄alkyl, C₁-C₄alkoxy or C₁-C₄alkoxycarbonyl, or
R₂ and R₃ independently of one another are phenylcarbonyl, biphenylcarbonyl, naphthylcarbonyl, phenyl-C₁-C₆alkylcarbonyl, biphenyl-C₁-C₆alkylcarbonyl, naphthyl-C₁-C₆alkylcarbonyl, phenyl-C₂-C₆alkenylcarbonyl, biphenyl-C₂-C₆alkenylcarbonyl or naphthyl-C₂-C₆alkenylcarbonyl, it being possible for these substituents to be unsubstituted or substituted by C₁-C₄alkyl, C₁-C₄alkoxy, C₁-C₄alkylthio, C₁-C₄haloalkyl, C₁-C₄alkylcarbonyl, halogen, cyano, amino, nitro, -COOH, C₁-C₅alkoxycarbonyl, hydroxyl or C₁-C₄alkylsulfonyl, or
R₂ and R₃ independently of one another are phenyl, naphthyl or heterocyclyl, it being possible for these substituents to be unsubstituted or substituted by halogen, cyano, C₁-C₄alkyl, C₁-C₄alkoxy, C₁-C₄alkylthio, -COOH or C₁-C₅alkoxycarbonyl, or
R₂ and R₃ together with the nitrogen atom to which they are bonded form a heterocyclic ring which can be substituted by C₁-C₄alkyl, C₁-C₄alkoxy, halogen or cyano.

Very particularly preferred compounds of the formula I are those in which R₂ and R₃ are hydrogen, or
R₂ and R₃ independently of one another are formyl, C₁-C₈alkylcarbonyl, C₂-C₈alkenylcarbonyl, C₄-C₉cycloalkylcarbonyl, C₆-C₉cycloalkenylcarbonyl or C₃-C₈cycloalkyl-C₁-C₆alkylcarbonyl, it being possible for these substituents to be unsubstituted or substituted by halogen, cyano, hydroxyl or amino, or
R₂ and R₃ are phenylcarbonyl, it being possible for the phenyl ring to be unsubstituted or substituted by C₁-C₄-alkyl, C₁-C₄-alkoxy, C₁-C₄-alkylthio, C₁-C₄haloalkyl, halogen, cyano, nitro, -COOH, C₁-C₅alkoxycarbonyl, hydroxyl or C₁-C₄alkylsulfonyl.

Other preferred compounds of the formula I are those in which X is O or S(O)ₓ, where x is 0, 1 or 2, and
R₄ is methyl which is substituted by halogen, cyano, nitro or OR₅, or
R₄ is C₂-C₈alkyl, C₂-C₈alkenyl, C₃-C₈alkynyl, C₃-C₈cycloalkyl, C₅-C₈cycloalkenyl, C₃-C₈cycloalkyl-C₁-C₄alkyl or C₁-C₄alkyl-C₃-C₈cycloalkyl, it being possible for these substituents to be unsubstituted or substituted by halogen, cyano, nitro, =O or -OR₅, or
R₄ is phenyl which is substituted by halogen, cyano, nitro, amino, -COOH, hydroxyl, C₁-C₄alkyl, C₁-C₄alkyloxy, C₁-C₄alkylthio, C₁-C₄haloalkyl, C₁-C₄haloalkoxy, C₁-C₄haloalkylthio, C₂-C₆alkoxycarbonylalkoxy, C₁-C₄alkylsulfinyl, C₁-C₄alkylsulfonyl, C₁-C₆alkyloxycarbonyl, C₁-C₆alkylcarbonyl, -CONH₂, formyl, C₁-C₅alkylaminocarbonyl, C₂-C₇dialkylaminocarbonyl, C₁-C₄alkylamino, C₂-C₆dialkylamino, C₃-C₆trialkylsilyl, C₁-C₆alkylcarbonylamino, C₁-C₆alkylcarbonyloxy, phenoxy, halophenoxy or pyridyloxy, or
R₄ is biphenyl, naphthyl, heterocyclyl, C₁-C₄alkylphenyl, C₁-C₄alkylnaphthyl, phenyl-C₁-C₄alkyl or naphthyl-C₁-C₄alkyl, it being possible for these substituents to be unsubstituted or substituted by halogen, cyano, nitro, amino, -COOH, hydroxyl, C₁-C₄alkyl, C₁-C₄alkyloxy, C₁-C₄alkylthio, C₁-C₄haloalkyl, C₁-C₄haloalkoxy, C₁-C₄haloalkylthio, C₂-C₆-alkoxycarbonylalkoxy, C₁-C₄alkylsulfinyl, C₁-C₄alkylsulfonyl, C₁-C₆alkyloxycarbonyl, C₁-C₆alkylcarbonyl, -CONH₂, formyl, C₁-C₅alkylaminocarbonyl, C₂-C₇dialkylaminocarbonyl,
   C₃-C₆trialkylsilyl, C₁-C₆alkylcarbonylamino, C₁-C₆alkylcarbonyloxy, phenoxy, halophenoxy or pyridyloxy, and
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₄alkylcarbonyl, benzoyl, halobenzoyl, C₁-C₄alkylamino, C₂-C₆dialkylamino, C₃-C₆trialkylsilyl, C₃-C₆cycloalkyl, C₂-C₄alkenyl or C₃-C₄alkynyl.

Amongst these, particularly preferred compounds are those in which X is O or S, and
R₄ is methyl which is substituted by halogen or cyano, or
R₄ is C₂-C₈alkyl, C₂-C₈alkenyl, C₃-C₈alkynyl, C₃-C₈cycloalkyl, C₅-C₈cycloalkenyl, C₃-C₈cycloalkyl-C₁-C₄alkyl or C₁-C₄alkyl-C₃-C₈cycloalkyl, it being possible for these substituents to be unsubstituted or substituted by halogen or cyano, or
R₄ is phenyl which is substituted by halogen, cyano, nitro, amino, C₁-C₄alkyl, C₁-C₄alkyloxy, C₁-C₄alkylthio, C₁-C₄haloalkyl, C₁-C₄haloalkoxy, C₂-C₆alkoxycarbonylalkoxy, C₁-C₄alkylsulfonyl, C₁-C₆alkyloxycarbonyl, C₁-C₆alkylcarbonyl, formyl, C₁-C₄alkylamino, C₂-C₆dialkylamino, C₃-C₆trialkylsilyl or C₂-C₆alkylcarbonyloxy, or
R₄ is biphenyl, naphthyl, heterocyclyl, C₁-C₄alkylphenyl, C₁-C₄alkylnaphthyl, phenyl-C₁-C₄alkyl or naphthyl-C₁-C₄alkyl, it being possible for these substituents to be unsubstituted or substituted by halogen, cyano, nitro, amino, C₁-C₄alkyl, C₁-C₄alkyloxy, C₁-C₄alkylthio, C₁-C₄haloalkyl, C₁-C₄haloalkoxy, C₂-C₆alkoxycarbonylalkoxy, C₁-C₄alkylsulfonyl, C₁-C₆alkyloxycarbonyl, C₁-C₆alkylcarbonyl, C₃-C₆trialkylsilyl or C₁-C₆alkylcarbonyloxy.

Very particularly preferred compounds of the formula I are those in which X is O, and R₄ is phenyl which is substituted by halogen, cyano, nitro, amino, C₁-C₄alkyl, C₁-C₄alkyloxy, C₁-C₄alkylthio, C₁-C₄haloalkyl, halomethoxy, C₁-C₄alkylsulfonyl, C₁-C₄alkylamino or C₂-C₆dialkylamino.

The compounds of the formula I can be prepared, on the one hand, via process steps which are known per se using known starting materials, and, on the other hand, via a process which was not known per se. The latter process, which was not known per se, comprises starting from the trihalogenated thiatriazine of the formula II 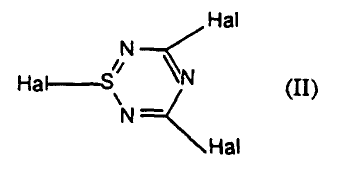 in which Hal substituents independently of one another are fluorine, bromine or, in particular, chlorine, this thiatriazine together with its preparation being described in Chem. Ber. (1991) 124, 1347-1352 and Z. Chem. 16 (1976) 358-359, and reacting it, to introduce the substituent R₁, with the corresponding organometal compound of the formula III

R₁-M (III)

in which R₁ is as defined above and M is a monovalent or polyvalent metal atom which, depending on the valency, can carry a corresponding number of R₁ groups. Examples of suitable metals are, in particular, lithium, magnesium, zinc, aluminium, silicon and tin, and furthermore also manganese and titanium. The polyvalent metal atoms can also carry, besides one or more R₁ groups, further substituents such as halogen, cyano, C₁-C₄alkyl or tetrafluoroborate. The organometal compound of the formula III can furthermore be used in combination with salts, such as aluminium chloride, tin chloride, zinc chloride or cerium chloride, or aluminium bromide, zinc bromide and/or copper bromide, amongst which aluminum chloride, aluminium bromide, zinc chloride and zinc bromide are preferred.

The compounds of the formula III can be prepared by customary methods, for example by reacting the corresponding halide R₁-halogen with the metal M, if appropriate with an addition of a metal halide, or by halogen/metal exchange of R₁-Br or R₁-I with a reactive organometal compound, for example butyllithium, and reaction with a compound of the metal M which carries at least one leaving group, such as halogen, or by abstraction of a proton of the molecule R₁-H with a strong base, for example a metal alkoxide, for example potassium tert-butoxide, or metal hydride, for example sodium hydride or lithium aluminium hydride, or a metal amide, for example lithium diisopropylamide, or, in particular, an organometal compound, for example butyllithium, and treatment with a compound of the metal M which carries at least one leaving group, for example cyano, alkoxy or, in particular, halogen, and, if appropriate, one or more C₁-C₄alkyl groups.

Introduction of the R₁ group can be effected in an aprotic solvent, such as a hydrocarbon, for example hexane, heptane or toluene, or an ether, such as dioxane, diethyl ether or, in particular, tetrahydrofuran, at temperatures from -100°C to 150°C, in particular -80°C to 50°C (depending on the solvent).

To prepare the compounds of the formula IV in which the sulfur atom of the thiatriazine is bonded to an aromatic or heteroaromatic part of R₁, the reaction of the corresponding compound R₁-H with a compound of the formula II can be carried out under the conditions of a Friedel-Crafts reaction, i.e. in the presence of a Lewis acid, for example aluminium trichloride, zinc chloride or tin tetrachloride, in an aprotic solvent, for example benzene, nitrobenzene or dichloromethane.

In the case of compounds of the formula I in which R₁ additionally to the abovementioned definitions is also a group of the formula IX or X 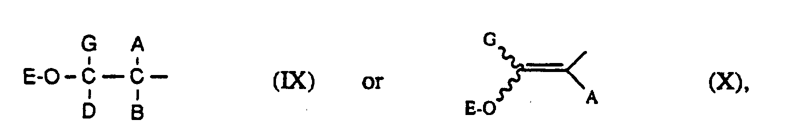 in which A is hydrogen, fluorine, chlorine, bromine, iodine, C₁-C₇alkyl, C₁-C₇alkyl which is substituted by halogen or C₁-C₃alkoxy, or C₁-C₄alkoxy; B is hydrogen, fluorine, chlorine, bromine, iodine, C₁-C₇alkyl, C₁-C₇alkyl which is substituted by halogen or C₁-C₃alkoxy, or C₁-C₄alkoxy; G is hydrogen, C₁-C₇alkyl, C₁-C₇alkyl which is substituted by halogen or C₁-C₃alkoxy, C₁-C₄alkoxy or trimethylsilyl; D is fluorine, chlorine, bromine, iodine, C₁-C₆alkoxy, C₁-C₆alkylthio, thiocyanato, cyano or
C₁-C₅alkylcarbonyloxy; and E is C₁-C₈alkyl, C₁-C₈alkyl which is substituted by C₁-C₄alkoxy, acetoxy, benzyloxy or halogen, C₃-C₈cycloalkyl, C₃-C₈cycloalkyl which is substituted by C₁-C₄alkoxy, acetoxy, benzyloxy or halogen, C₁-C₅alkylcarbonyl, C₆-C₈aryl or (C₁-C₄alkyl)₃silyl, the compounds of the formula IV can be prepared advantageously from compounds of the formula XI 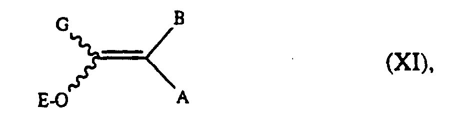 in which the substituents A and E, A and B, A and G, and G and E in-each case together can form a ring, for example in the case of A and E: 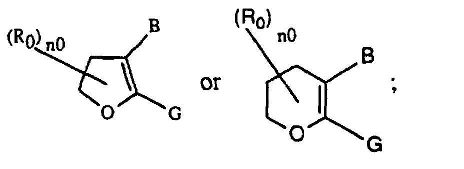 in the case of A and B: 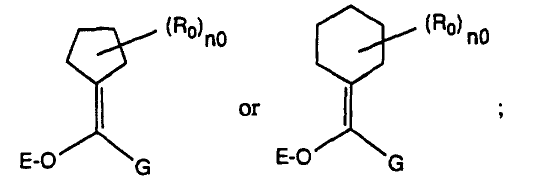 in the case of A and G: 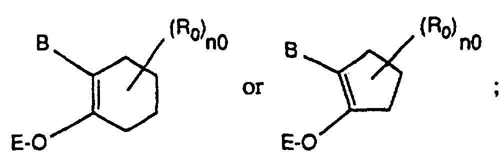 and
in the case of G and E: 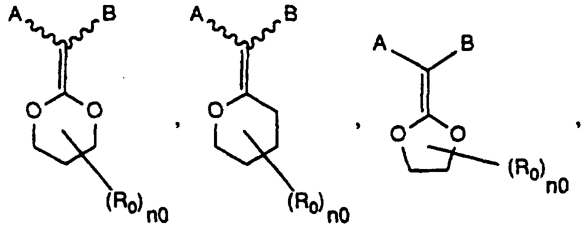 or 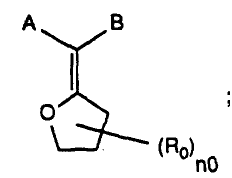 in which R₀ is C₁-C₃alkyl, C₁-C₃alkoxy, acetoxy, benzyloxy or halogen; and n₀ is 0, 1, 2 or 3, by reacting the compound of the formula XI with a compound of the formula II in the presence or absence of a Lewis acid, for example aluminium trichloride, zinc chloride or tin tetrachloride, in an aprotic solvent, for example hexane, dichloromethane, chloroform, carbon tetrachloride, 1,2-dichloroethane, ether or tetrahydrofuran, at temperatures from -78°C to the boiling point of the solvent, but in particular at -40°C to +40°C, and, if desired, the crude product obtained is subsequently treated with an acid-binding agent, such as tertiary amine, for example triethylamine or pyridine, or a mineral base, for example sodium hydrogen carbonate, sodium carbonate or potassium carbonate, if appropriate in aqueous solution, to neutralize the acid H-halogen.

If the reactivity of the substituent R₁ in the compound of the formula I makes difficult the preparation of the organometal compound of the formula III, then this labile functional group R₁ can advantageously be protected and subsequently re-eliminated at the level of the compounds of the formulae IV, VI, VIII or I, analogously to the procedure described in, for example, 'Protective Groups in Organic Synthesis', Editors Th. W. Greene and P. G. M. Wuts, Wiley Interscience, 1991. For example,
a) an alcohol can be protected in the form of a tert-butyl dimethylsilyl ether, and this protective group can be re-eliminated with tetrabutylammonium fluoride; or
b) a carbonyl group can be protected in the form of 1,3-dioxane and then again hydrolyzed under acidic conditions to give the carbonyl group; or
c) a carboxylic acid can be protected in the form of the ortho-ester and then be recovered under aqueous-acidic and subsequently basic conditions.

The reactive substituents in R₁ in the compounds of the formula I can also be introduced by known methods from less reactive precursors at the level of the compounds of the formulae IV, VI, VIII or I, for example by the method described in 'Advanced Organic Chemistry: Reactions, Mechanisms and Structure', Editor J. March, McGraw-Hill, 1983, or in 'Comprehensive Organic Transformations', Editor R. C. Larock, VCH, 1989. Such less reactive precursors can be, for example, halogens, such as bromine, chlorine or iodine, carbon-carbon double or triple bonds, or ethers or thioethers.
A large number of suitable conversion reactions for the preparation of functional groups is described in the specialist literature. For example,
a) a halide can be converted by reduction to give the corresponding alkane or by nucleophilic substitution to give the corresponding nitrile, ether, thioether, ester, azide or thiocyanate; or
b) a carbon-carbon double bond can be hydrogenated, halogenated on the allyl position, oxidized to give the corresponding epoxide or cis-diol, or subjected to ozonolysis to give the carbonyl derivative or to oxidative hydroborination to give the alkanol; or
c) a nitrile group can be hydrolyzed to give the carboxamide or the carboxylic acid; or
d) a thioether can be oxidized to give the sulfoxide or the sulfone, it being possible for these latter compounds to be deprotonated in the α-position and further reacted with electrophilic reagents; or
e) an azide can be reduced to give the corresponding amine and, if desired, subsequently acylated.

The resulting compounds of the formula IV 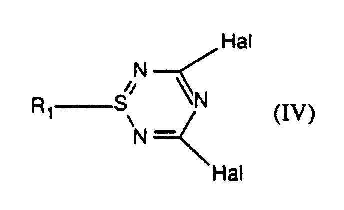 are novel and thus also provided by the present invention.

The substituents -NR₂R₃- and -XR₄ can then be introduced into the compound of the formula IV in any desired sequence.

The reaction of the compounds of the formula IV with the compounds of the formula V

H-XR₄ (V),

in which X and R₄ are as defined above, to give the compounds of the formula VI 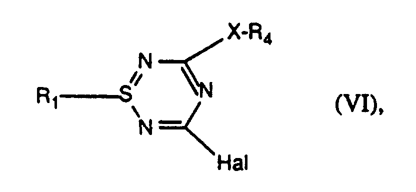 is advantageously carried out in such a way that, prior to the reaction with the compounds of the formula IV, the compounds of the formula V are pretreated with a base, such as a metal hydride, for example lithium hydride, sodium hydride or potassium hydride, a metal hydroxide, such as sodium hydroxide or potassium hydroxide, or a basic salt such as sodium carbonate or potassium carbonate, preferably in equivalent amounts. Alternatively, it is also possible to treat the reaction mixture of the compounds of the formulae IV and V with the abovementioned bases. Solvents which are suitable for this step are hydrocarbons such as hexane or toluene, halogenated hydrocarbons such as chlorobenzene, ethers such as tetrahydrofuran, dioxane or diethyl ether, and tertiary amides, such as dimethylformamide. It is also possible to employ a mixture of these solvents with water, in which case it is advantagoeus to use a phase transfer catalyst. As a rule, the reaction temperatures are between -50°C and 100°C, preferably between 0°C and 40°C.

The compounds of the formula V are known and can be prepared in a manner known to the expert.

The compounds of the formula VI are novel and are also provided by the present invention.

They can be converted into the end products of the formula I by means of a reaction with a compound of the formula VII

M₁-NR₂R₃ (VII),

in which R₂ and R₃ are as defined above and M₁ is hydrogen or a metal atom such as lithium, sodium, potassium or calcium, the process advantageously being carried out in the presence of a base. If R₂ and/or R₃ are hydrogen, then M₁ is preferably hydrogen. If R₂ or R₃ is an acyl group, M₁ is preferably lithium, sodium or potassium.

Solvents which are suitable for this reaction are hydrocarbons such as hexane or toluene, halogenated hydrocarbons such as chlorobenzene or dichloromethane, ethers such as diethyl ether, dioxane or tetrahydrofuran, alcohols such as ethanol or isopropanol, esters such as ethyl acetate, nitriles such as acetonitrile, or water. The reaction temperatures are in the range of -70°C to 100°C, in particular 0°C to 40°C.

If M₁ is hydrogen, an acid binder is preferably used for trapping the HHal acid. This can be, for example, a second equivalent of the compound of the formula VII or a tertiary amine such as triethylamine, pyridine or an inorganic base such as sodium carbonate, potassium carbonate or sodium hydrogencarbonate. In the event that R₂, R₃ and M₁ are hydrogen, an excess of base can be used. The process may be carried out under pressure.

The compounds of the formula VII and their preparation are described in the literature.

The reaction conditions to be adhered to for the reaction of compound VI with compound VII must also be observed when the compound of the formula IV is first to react with the base of the formula VII. The resulting compounds of the formula VIII 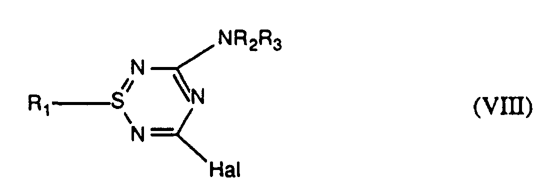 are also novel and provided by the present invention.

The further reaction of the compounds of the formula VIII with the compounds of the formula V is effected analogously to the procedure in the reaction of the compounds of the formulae IV and V. However, an amine such as triethylamine is additionally added to the reaction mixture in amounts which range from catalytic to an excess.

The end products of the formula I can be isolated in the customary manner by concentrating and/or evaporating the solvent and purified by recrystallization or trituration of the solid residue in solvents in which they are not readily soluble, such as ethers, aromatic hydrocarbons or chlorinated hydrocarbons, or by chromatographic methods.

Conventional derivatization of compounds of the formula I allows other compounds of the formula I to be prepared.

A variety of methods and techniques are suitable for the use according to the invention for the compounds of the formula I, IV, VI or VIII or for compositions comprising these compounds, for example the following:

### i) Seed dressing

a) The seeds are dressed by shaking them, in a vessel, together with an active ingredient formulated as a wettable powder until the active ingredient is distributed uniformly on the seed surface (dry seed dressing). Up to 4 g of active ingredient of the formula I, IV, VI, VIII (in the case of a 50% formulation: up to 8.0 g of wettable powder) per kg seed is used.
b) Seed dressing with an emulsion concentrate of the active ingredient or with an aqueous solution of the active ingredient of the formula I, IV, VI or VIII formulated as a wettable powder by method a) (wet seed dressing).
c) Dressing by immersing the seed into a mixture comprising up to 1000 ppm of active ingredient of the formula I, IV, VI or VIII for 1 to 72 hours, if desired followed by drying of the seeds (seed soaking).

Naturally, dressing the seed or treating the seedling which has just begun to germinate are the preferred methods of application because the treatment with the active ingredient is directed entirely towards the target crop. As a rule, 0.001 g to 4.0 g of active ingredient are used per kg of seed, it being possible to deviate from these limit concentrations up or down, depending on the method, which also makes possible the addition of other active ingredients or micronutrients (repeated dressing).

### ii) Controlled release of active ingredient

The active ingredient is applied in solution to mineral granule carriers or polymerized granules (urea/formaldehyde) and allowed to dry. If desired, a coating can be applied additionally (coated granules) which allows controlled release of the active ingredient over a certain period.

The compounds of the formula I, IV, VI or VIII can be used as pure active ingredients, i.e. as they are obtained in synthesis, but they are preferably processed in the customary manner together with the auxiliaries conventionally used in the art of formulation, for example to give emulsifiable concentrates, directly sprayable or dilutable solutions, dilute emulsions, wettable powders, soluble powders, dusts, granules or microcapsules. The methods of application, such as spraying, atomizing, dusting, wetting, scattering or pouring, as well as the nature of the compositions, are chosen to suit the intended aims and the prevailing circumstances.

The formulations, i.e. the compositions, preparations or combinations comprising the active ingredient of the formula I, IV, VI or VIII or at least one active ingredient of the formula I, IV, VI or VIII and, if desired, one or more solid or liquid additives, are prepared in a known manner, for example by intimately mixing and/or grinding the active ingredients with the additives, for example solvents or solid carriers. Furthermore, surface-active compounds (surfactants) can additionally be used for the preparation of the formulations.

The following are suitable as solvents: aromatic hydrocarbons, in particular the fractions C₈ to C₁₂, such as mixtures of alkylbenzenes, for example xylene mixtures or alkylated naphthalenes; aliphatic and cycloaliphatic hydrocarbons such as paraffins, cyclohexane or tetrahydronaphthalene; alcohols such as ethanol, propanol or butanol; glycols as well as their ethers and esters, such as propylene glycol or dipropylene glycol ether, ketones such as cyclohexanone, isophorone or diacetone alcohol, strongly polar solvents such as N-methyl-2-pyrrolidone, dimethyl sulfoxide or water; vegetable oils as well as their esters, such as rapeseed oil, castor oil or soya oil; if appropriate, also silicone oils.

Solid carriers which are used, for example, for dust and dispersible powders are, as a rule, ground natural minerals, such as calcite, talc, kaolin, montmorillonite or attapulgite. To improve the physical properties, it is also possible to add highly-disperse silica or highly-disperse absorptive polymers. Possible particulate, adsorptive carriers for granules are either porous types, for example pumice, brick grit, sepiolite or bentonite, and suitable non-sorptive carrier materials are, for example, calcite or sand. Moreover, a large number of pregranulated materials of inorganic or organic nature, such as, in particular, dolomite or comminuted plant residues, can be used.

Suitable surface-active compounds are non-ionic, cationic and/or anionic surfactants having good emulsifying, dispersing and wetting properties, depending on the nature of the active ingredient of the formula I to be formulated. Surfactants are also to be understood as meaning surfactant mixtures.

Suitable anionic surfactants can be either so-called water-soluble soaps or water-soluble, synthetic surface-active compounds.

Soaps which may be mentioned are the alkali metal salts, alkaline earth metal salts or substituted or unsubstituted ammonium salts of higher fatty acids (C₁₀-C₂₂) for example the sodium or potassium salts of oleic or stearic acid, or of natural mixtures of fatty acids which can be obtained from, for example, coconut oil or tallow oil. Mention must also be made of the fatty acid methyltaurinates.

However, so-called synthetic surfactants are used more frequently, in particular fatty alcohol sulfonates, fatty alcohol sulfates, sulfonated benzimidazole derivatives or alkylarylsulfonates.

As a rule, the fatty alcohol sulfonates or fatty alcohol sulfates are in the form of alkali metal salts, alkaline-earth metal salts or substituted or unsubstituted ammonium salts and have, as a rule, an alkyl radical having 8 to 22 carbon atoms, alkyl also including the alkyl moiety of acyl radicals, for example the sodium salt or calcium salt of lignosulfonic acid, of the dodecylsulfuric ester or of a fatty alcohol sulfate mixture prepared from natural fatty acids. This group also includes the salts of the sulfuric esters and the sulfonic acids of fatty alcohol/ethylene oxide addition products. The sulfonated benzimidazole derivatives preferably have 2 sulfonyl groups and a fatty acid radical having approximately 8-22 carbon atoms. Examples of alkylarylsulfonates are the sodium, calcium or triethanolamine salts of dodecylbenzenesulfonic acid, of dibutylnaphthalenesulfonic acid, or of a naphthalenesulfonic acid/formaldehyde condensation product.

Suitable phosphates, for example salts of the phosphoric ester of a p-nonylphenol-(4-14) ethylene oxide adduct, or phospholipids, are also possible.

Possible non-ionic surfactants are mainly polyglycol ether derivatives of aliphatic or cycloaliphatic alcohols, saturated or unsaturated fatty acids and alkylphenols, which can have 3 to 30 glycol ether groups and 8 to 20 carbon atoms in the (aliphatic) hydrocarbon moiety and 6 to 18 carbon atoms in the alkyl moiety of the alkylphenols.

Other suitable non-ionic surfactants are the water-soluble polyethylene oxide addition products with polypropylene glycol, ethylene diaminopolypropylene glycol and alkyl polypropylene glycol which have 1 to 10 carbon atoms in the alkyl chain and have 20 to 250 ethylene glycol ether groups and 10 to 100 propylene glycol ether groups. The abovementioned compounds conventionally have 1 to 5 ethylene glycol units per propylene glycol unit.

Examples of non-ionic surfactants which may be mentioned are nonylphenolpolyethoxyethanols, castor oil polyglycol ethers, polypropylene/polyethylene oxide addition products, tributylphenoxypolyethoxyethanol, polyethylene glycol and octylphenoxypolyethoxyethanol.

Other substances which are suitable are fatty acid esters of polyoxyethylene sorbitan, such as polyoxyethylene sorbitan trioleate.

The cationic surfactants are mainly quaternary ammonium salts which have, as N substituent, at least one alkyl radical having 8 to 22 carbon atoms and as further substituents lower, halogenated or unhalogenated alkyl, benzyl or lower hydroxyalkyl radicals. The salts are preferably in the form of halides, methylsulfates or ethylsulfates, for example stearyltrimethylammonium chloride or benzyldi(2-chloroethyl)ethylammonium bromide.

The surfactants conventionally used in the art of formulation which can also be used in the compositions according to the invention are described, inter alia, in the following publications:
- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch" [Surfactants Guide], Carl Hanser Verlag, Munich/Vienna 1981.

As a rule, the herbicidal preparations comprise as a rule 0.1 to 99 %, in particular 0.1 to 95 %, of active ingredient of the formula I, 1 to 99 % of a solid or liquid additive and 0 to 25 %, in particular 0.1 to 25 %, of a surfactant.

While concentrated compositions are more preferred as commercially available goods, the end consumer uses, as a rule, dilute compositions.

The compositions can also comprise further additives such as stabilizers, for example unepoxidized or epoxidized vegetable oils (epoxidized coconut oil, rapeseed oil or soya oil), antifoams, for example silicone oil, preservatives, viscosity regulators, binders, tackifiers as well as fertilizers or other active ingredients for achieving specific effects.

In particular, preferred formulations are composed as follows: (% = percent by weight)

| Emulsifiable concentrates: | |
|---|---|
| Active ingredient | 1 to 90 %, preferably 5 to 50 % |
| Surface-active agent | 5 to 30 %, preferably 10 to 20 % |
| Solvent | 15 to 94 %, preferably 70 to 85 % |

| Dusts: | |
|---|---|
| Active ingredient | 0.1 to 50 %, preferably 0.1 to 1 % |
| Solid carrier | 99.9 to 90 %, preferably 99.9 to 99 % |

| Suspension concentrates: | |
|---|---|
| Active ingredient: | 5 to 75 %, preferably 10 to 50 % |
| Water | 94 to 24 %, preferably 88 to 30 % |
| Surface active agent: | 1 to 40 %, preferably 2 to 30 % |

| Wettable powders: | |
|---|---|
| Active ingredient | 0.5 to 90 %, preferably 1 to 80 % |
| Surface-active agent | 0.5 to 20 %, preferably 1 to 15 % |
| Solid carrier | 5 to 95 %, preferably 15 to 90% |

| Granules: | |
|---|---|
| Active ingredient | 0.1 to 30 %, preferably 0.1 to 15 % |
| Solid carrier | 99.5 to 70 %, preferably 97 to 85 % |

As a rule, the active ingredients of the formula I are successfully employed at rates of application of 0.001 to 5 kg/ha, in particular 0.005 to 2 kg/ha. The dosage required for the desired effect can be determined by experiments. It depends on the nature of the effect, the development stage of the crop plant and of the weed as well as on the application (location, timing, method) and can vary within wide limits due to these parameters.

The compounds of the formula I are distinguished by growth-inhibiting and herbicidal properties which make them outstandingly suitable for use in crops of useful plants, in particular in cereals, cotton, soya beans, oilseed rape, maize and rice.

The examples which follow illustrate the invention in greater detail without imposing any limitation.

The nomenclature used hereinbelow is based on the numbering showed below:

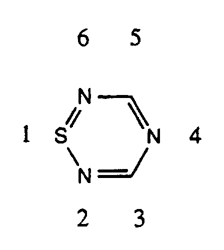

### Preparation Examples:

### Example H1: Preparation of 3,5-dichloro-1-pentylthiatriazine

A solution of 13.4 g of AlCl₃ (0.1 mol) in 400 ml of THF is cooled to -70°C. A solution of pentylmagnesium bromide in ether is then added dropwise, and the mixture is allowed to come to 20°C and, after 15 minutes, is again cooled to -70°C. 20.5 g of trichlorothiatriazine in 50 ml of THF are subsequently added with stirring. The reaction mixture is kept for 2 hours at this temperature. It is then allowed to warm to room temperature and poured into 400 ml of ice-water. The organic phase is treated with dilute aqueous hydrochloric acid and then washed with water until neutral and dried using sodium sulfate. After filtration and concentration of the phase, an oily residue is obtained, and this is taken up in 200 ml of hexane. Insoluble components are removed and the solution which remains is cooled. White crystals of the compound given above form which have a melting point of 34°C after recrystallization.

### Example H2: Preparation of 3,5-dichloro-1-methylthiatriazine

A solution of 18.5 g of trichlorothiatriazine (0.09 mol) in 200 ml of methylene chloride is cooled to -75°C. A solution of dimethylaluminium chloride in hexane is then added dropwise under protective gas. The temperature should not exceed -65°C. The reaction mixture is then held for 40 minutes at -70°C. It is then allowed to come to -45°C, and 9 ml of water are added dropwise. After the mixture has warmed to room temperature, it is filtered, and the filtrate is dried using sodium sulfate. After concentration, a residue is obtained, and this is taken up in 50 ml of hexane and 20 ml of ether. After the crude product has been filtered off, it is washed with hexane and dried in vacuo. This gives colourless crystals of the compound given above having a melting point of 101°C (decomposition).

### Example H3: Preparation of 3-(2-ethoxycarbonylphenyloxy)-5-chloro-1-phenylthiatriazine

3.4 g of ethyl salicylate are dissolved in 15 ml of DMF and the solution is treated with 0.88 g of sodium hydride (55 % in oil) at 0-10°C. This solution is then added dropwise to a solution of 5.0 g of 3,5-dichloro-1-phenylthiatriazine (prepared analogously to Example H1) in 10 ml of DMF. After the mixture has been stirred for 12 hours at room temperature, it is filtered and concentrated. The residue is taken up in methylene chloride, washed with water, dried using sodium sulfate and evaporated. After pre-purification by chromatography on silica gel using a mixture of hexane and ethyl acetate (7:1) and recrystallization from the abovementioned mixture, crystals of the abovementioned compound having a melting point of 88-90°C are obtained.

### Example H4: Preparation of 3-amino-5-chloro-1-methylthiatriazine

Ammonia is passed at a temperature of 10-20°C into a solution of 11.04 g of 3,5-dichloro-1-methylthiatriazine (obtained as described in Example H2) in 1.5 l of acetonitrile. After the mixture has been stirred for 1.5 hours at 20°C, 120 ml of water are added. The mixture is cooled to 5°C, solid is filtered off, and the residue is washed with water and cold acetonitrile and ether and dried. This gives the abovementioned product having a melting point of 156°C (decomposition).

### Example H5: Preparation of 3-chloro-5-isopropylamino-1-methylthiatriazine

14.8 g of isopropylamine are added dropwise at room temperature to a solution of 23.0 g of dichloromethylthiatriazine (obtained as described in Example H2) in 175 ml of THF. The reaction mixture is then treated with ethyl acetate and water. The product is extracted with ethyl acetate, this phase is washed with water and dried using sodium sulfate. After the mixture has been filtered and the filtrate concentrated, the crude product is taken up in methylene chloride and refiltered. The filtrate is treated with hexane, stirred for 2 hours, filtered, and the residue is washed with pentane and dried. This gives the abovementioned compound in crystalline form having a melting point of 120-122°C.

### Example H6: Preparation of 3-amino-5-(2,6-dichlorophenoxy)-n-pentylthiatriazine

2.2 g of aminochloropentylthiatriazine (obtained by the methods described in Examples H1 and H4), 1.6 g of dichlorophenyl, 0.4 g of sodium hydroxide (dissolved in 1 ml of water) and 1.48 g of a 40% aqueous solution of trimethylamine are introduced into 100 ml of THF. The mixture is stirred for 12 hours and concentrated, and the residue is stirred with water and diethyl ether. After filtration, the solid is washed with water and dried. Recrystallization from a THF/hexane mixture gives the abovementioned compound having a melting point of 200°C (decomposition).

### Example H7: Preparation of 3-methoxy-1-methyl-5-methylaminothiatriazine

123 mmol of sodium methylate are added to a suspension of 10.0 g of 3-chloro-1-methyl-5-methylaminothiatriazine (obtained analogously to the method described in Example H4) in 250 ml of methanol, which has been cooled to 15°C, and 2.6 g of trimethylamine are then passed in. After the mixture has been stirred for 14 hours it is evaporated, the residue is stirred with methylene chloride, the mixture is filtered, and the solid is washed with pentane and dried. This gives the abovementioned compound having a melting point of 153-154°C.

### Example H8: Preparation of 3,5-dichloro-1-phenylthiatriazine

0.15 g of aluminium trichloride is added at -20°C to a solution of 10.2 g of trichlorothiatriazine (0.05 mol) and 7.5 g of phenyltrimethylsilane (0.05 mol) in 40 ml of dichloromethane. This reaction mixture is heated slowly at 20°C and stirred for 2 hours at this temperature. After this, 300 g of ice-water are added and the mixture is extracted using dichloromethane. The combined organic phases are washed with water until neutral and dried over sodium sulfate. After the desiccant has been filtered off and the filtrate concentrated, the desired product is obtained as crystals of m.p. 91-94°C.

### Example H9: Preparation of 1-(tert-butylethynyl)-3,5-dichlorotriazine

A solution of lithium tetrakis(tert-butylethynyl)alanate, prepared from 3.24 g of lithium aluminium hydride (0.0855 mol), 0.2 g of diethylamine and 33.7 g of tert-butylacetylene (0.41 mol) in 250 ml of tetrahydrofuran in analogy to Bull. Acad. Sci. of USSR (engl. transl.) 1965, pages 165-167, editor L.I. Zakharkin et al., is added dropwise at -75°C to a solution of 52.4 g of trichlorothiatriazine (0.256 mol) in 400 ml of dichloromethane. The reaction mixture is kept for 1 hour at this temperature. A cold solution of 48 g of Rochelle salt and 9 g of potassium carbonate in 450 ml of water is added. After a further addition of 1 litre of water and 1 litre of ether, the phases are separated. The aqueous phase is extracted with ether and the organic phase washed with saturated sodium hydrogencarbonate solution until neutral and dried over sodium sulfate. Removal of the desiccant by filtration and evaporation of the filtrate gives the desired product as crystals. After recrystallization from hexane, a melting point of 73-74.5°C is determined.

### Example H10: Preparation of 3,5-dichloro-1-phenylthiatriazine

A solution of 5.12 g of trichlorothiatriazine (0.025 mol) in 20 ml of benzene is slowly added dropwise at 0°C to a vigorously stirred suspension of 6.0 g of aluminium trichloride (0.045 mol) in 80 ml of benzene and 10 ml of pentane. The reaction mixture is stirred for 2 hours at 0°C and subsequently for a further hour at 20°C. The reaction mixture is again cooled to 0°C and treated with ice and ether. The aqueous phase is extracted with ether and the combined organic phases are washed with aqueous saturated sodium hydrogencarbonate solution until neutral and dried over sodium sulfate. After removal of the desiccant by filtration and concentration of the filtrate, the crude product obtained is the desired compound which after purification by chromatography is identical with the compound obtained in Example H8.

### Example H11: Preparation of 3-amino-1-cyclohexyl-5-methanesulfinylthiatriazine

A solution of 6.45 g of 3-chloroperbenzoic acid (0.02057 mol) in 110 ml of chloroform is added dropwise in the course of 1.5 hours at 20°C to a vigorously stirred suspension of 4.57 g of 3-amino-1-cyclohexyl-5-methylthiothiatriazine (0.0187 mol) in 70 ml of chloroform and 1.9 g of sodium hydrogencarbonate. After stirring of the reaction mixture has continued for 4 hours, it is treated with 180 ml of aqueous semisaturated sodium hydrogencarbonate solution. The phases are separated and the water phase is extracted using dichloromethane. The combined organic phases are dried over sodium sulfate. After the desiccant has been removed by filtration and the filtrate concentrated, the residue is subjected to flash chromatography over silica gel using ethyl acetate/ethanol 4/1. Recrystallization from ethyl acetate/methylene chloride gives the desired product as crystals having an m.p. of 134-138°C (decomposition).

### Example H12: Preparation of 1-cyclohexyl-3-isopropylthio-5-propylaminothiatriazine

0.77 g of propylamine (0.013 mol) in 5 ml of tetrahydrofuran is added dropwise at 20°C to a solution of 1.75 g of 3-chloro-1-cyclohexyl-5-isopropylthiothiatriazine (0.006 mol) in 25 ml of tetrahydrofuran. After 1 hour, the reaction mixture is filtered off with suction over silica gel and the residue is washed with tetrahydrofuran. The filtrate is evaporated and the residue recrystallized from ethyl acetate/hexane. The desired compound is obtained as crystals having an m.p. of 73-74°C.

### Example H13: Preparation of 3-amino-1-(3-bromo-2-methoxytetrahydropyran-3-yl)-5-chlorothiatriazine

A suspension of 1.16 g of 3-amino-5-chloro-1-(5,6-dihydro-4H-pyran-3-yl)thiatriazine (0.005 mol) in 40 ml of methanol is treated at 5°C with a solution of 0.80 g of bromine (0.005 mol) in 10 ml of methanol and stirring is subsequently continued for 1 hour at 20°C. After this, the reaction mixture is evaporated in vacuo, the residue is taken up in dichloromethane, and the mixture is extracted by shaking with a saturated sodium hydrogencarbonate solution. The organic phase is separated off and dried over sodium sulfate. After the desiccant has been removed by filtration and the filtrated concentrated, the residue obtained is triturated with ether. The desired compound is obtained as crystals having an m.p. of 188°C (decomposition).

### Example H14: Preparation of 3-amino-5-chloro-1-(2-methoxytetrahydropyran-3-yl)-thiatriazine

A suspension of 0.61 g of 3-amino-1-(3-bromo-2-methoxytetrahydropyran-3-yl)-5-chlorothiatriazine (0.00177 mol), 0.06 g of bisazobutyronitrile and 0.62 g of tri-n-butyltin hydride in 50 ml of benzene is stirred at reflux temperature. After 1 hour, the mixture is evaporated in vacuo and the residue obtained is stirred with hexane. After filtration and washing the crystals with hexane, the product is recrystallized from diisopropyl ether. The desired product has an m.p. of 167-168°C (decomposition).

### Example H15: Preparation of 3,5-dichloro-1-(2,3-dimethylpropyl)thiatriazine

A Grignard solution prepared from 29.9 g of 1-bromo-2,2-dimethylpropane (0.198 mol) and 5.0 g of magnesium (0.208 mol) in 120 ml of tetrahydrofuran is added slowly to a mixture of 26.9 g of zinc chloride (0.198 mol) and 400 ml of tetrahydrofuran at -20°C. After the mixture has been heated to 0°C, it is again cooled to -30°C, and a solution of 40.5 g of 1,3,5-trichlorothiatriazine in 200 ml of tetrahydrofuran is added dropwise at this temperature. The temperature is slowly raised to 0°C and kept for 2 hours at 0°C. The reaction mixture is subsequently poured into 600 g of ice-water and extracted with pentane. The organic phase is washed with aqueous sodium hydrogencarbonate solution until neutral and dried over sodium sulfate. After the desiccant has been removed by filtration and the filtrate concentrated, the residue is recrystallized from pentane. The crystals of the desired product show an m.p. of 48-51°C.

### Example H16: Preparation of 2-(1-cyclohexyl-5-phenoxythiatriazin-3-yl)-isoindole-1,3-dione

A mixture of 1.0 g of 3-amino-1-cyclohexyl-5-phenoxythiatriazine (0.00344 mol), 0.69 ml of pyridine (0.0086 mol), 0.55 ml of phthalic acid dichloride (0.00378 mol) in 30 ml of 1,2-dimethoxyethane is stirred for 6 hours at reflux temperature. After the mixture has cooled, it is poured into 70 ml of saturated aqueous sodium hydrogencarbonate solution and the mixture is extracted with ethyl acetate. The organic phase is dried over sodium sulfate. After filtration and concentration of the organic phase, the residue is subjected to flash chromatography over silica gel using ethyl acetate/hexane 35/65. Recrystallization from ether/hexane gives the desired compound as crystals having an m.p. of 145-146°C.

### Example H17: Preparation of 3-methoxy-1-methyl-5-methylaminothiatriazine

2.6 g of trimethylamine (0.045 mol) are passed at 15°C into a suspension of 10.0 g of 3-chloro-1-methyl-5-methylaminothiatriazine (0.056 mol) in 250 ml of methanol. The reaction mixture is stirred for 14 hours and subsequently evaporated in vacuo. The residue is stirred with dichloromethane and filtered off over kieselguhr. The filtrate obtained is evaporated and the product is stirred with pentane and filtered with suction. Washing of the solid obtained by filtration with suction with pentane and drying gives the desired compound as crystals having an m.p. of 153-154°C.

### Example H18: Preparation of 3-(3-cyanopropylamino)-1-cyclohexyl-5-phenoxythiatriazine

0.35 g of sodium hydroxide (0.00875 mol) is added to a solution of 2.98 g 3-(N-3-cyanopropylacetamido)-1-cyclohexyl-5-phenoxythiatriazine (0.00746 mol) in 50 ml of ethanol. After the reaction mixture has been stirred for 0.5 hour at 22°C, it is treated with 40 ml of water and the suspension obtained is filtered with suction. The solid obtained by filtration with suction is washed with water and dried. This gives the desired compound as crystals having an m.p. of 170-172°C.

### Example H19: Preparation of 3-(N-3-cyanopropylacetamido)-1-cyclohexyl-5-phenoxythiatriazine

A solution of 2.5 g of 3-acetamido-1-cyclohexyl-5-phenoxythiatriazine (0.00752 mol) in 50 ml of N,N-dimethylformamide is treated at 40°C with 0.22 g of sodium hydride (0.00902 mol). After the evolution of gas has ceased, 1.14 mg of 4-bromopropionitrile (0.001128 mol) are added at 22°C and the mixture is stirred for 1 hour. After water has been added carefully, the reaction mixture is poured into 80 ml of water and the product extracted using ethyl acetate. The organic phase is washed with brine and dried over sodium sulfate. After the desiccant has been removed by filtration and the filtrate concentrated, the crude product is subjected to flash chromatography over silica gel using ethyl acetate/hexane 1/1. Evaporation of the fractions obtained allows the desired compound to be isolated as an oil.

### Example H20: Preparation of 1-cyclohexyl-3-valeroylamino-5-phenoxythiatriazine

0.92 g of 2-valeroyl-3-oxo-4,5-benzo-1,2-thiazoline-1,1-dioxide (0.00344 mol), prepared analogously to Synthetic communications 14(4), 353-362 (1984), 0.72 ml of triethylamine (0.00516 mol) and a catalytic amount of 4-dimethylaminopyridine are added to a suspension of 1.0 g of 3-amino-1-cyclohexyl-5-phenoxythiatriazine (0.00344 mol) in 40 ml of 1,2-dichloroethane. The reaction mixture is stirred for 12 hours at reflux temperature and subsequently evaporated in vacuo. The residue is purified by chromatography over silica gel using toluene/ethyl acetate 4/1. Recrystallization from ether/hexane gives the desired product as crystals having an m.p. of 116°C.

### Example H21: Preparation of 3-acetamido-1-pentyl-5-phenylthiothiatriazine

0.75 g of pyridine (0.0095 mol) and 0.97 g of acetic anhydride (0.0095 mol) are added to a solution of 1.4 g of 3-amino-1-pentyl-5-phenylthiothiatriazine (0.00475 mol) in 50 ml of 1,2-dimethoxyethane and the reaction mixture is stirred for 12 hours at reflux temperature. The reaction mixture is then evaporated in vacuo and the residue treated with ethyl acetate and water. The organic phase is separated off, washed with water and dried over sodium sulfate. After the desiccant has been removed by filtration, the filtrate is concentrated and the residue obtained is subjected to flash chromatography over silica gel using ethyl acetate/hexane. Concentration of the fractions gives the desired compound as crystals having an m.p. of 89-90°C.

### Example H22: Preparation of (E)-3,5-dichloro-1-(2-methylhex-1-enyl)thiatriazine

A solution of (E)-dimethyl-1-(2-methylhex-1-enyl)alane is prepared analogously to Tetrahedron 1994, 5189-5202 from 16.43 g 1-hexyne (0.2 mol), 310 ml of a 2.0 molar trimethylaluminium solution in hexane (0.62 mol) and 8.0 g of ZrCl₂(Cp)₂ (0.0274 mol) in 600 ml of dichlormethane. This solution is added dropwise in the course of 1.25 hours to a solution of 39.07 g of trichlorothiatriazine (0.191 mol) in 400 ml of tetrahydrofuran cooled to -40°C. Stirring of the reaction mixture obtained is continued for 1 hour under cold conditions, 40 ml of water are then carefully added at -30°C, and the mixture is allowed to defrost slowly (evolution of gas). The mixture is then subjected to filtration with suction over kieselguhr, the filtrate is extracted with water and washed with sodium hydrogencarbonate solution until neutral and the organic phase is dried over sodium sulfate. Filtration and concentration of the filtrate give a residue which is recrystallized from hexane. The crystals obtained having an m.p. of 59-62°C are those of the desired compound.

### Example H23: Preparation of 3,5-dichloro-1-(5,6-dihydro-4H-pyran-3-yl)thiatriazine

A solution of 61.3 g of trichlorothiatriazine (0.3 mol) in 600 ml of dichloromethane is cooled in an ice bath and treated with 0.41 g of zinc chloride. 27.7 g of 3,4-dihydro-2H-pyran is slowly added dropwise to this mixture with vigorous stirring. After this, stirring is continued for 1 hour, and 60 ml of triethylamine are subsequently added dropwise. Stirring of this suspension is continued for 0.5 hour at 22°C, and the mixture is then filtered with suction. The residue obtained is washed with ether, and the filtrate is concentrated and treated with water. After the mixture has been filtered, the residue obtained is washed with water and dried. The crude product is subjected to flash chromatography over silica gel using dichloromethane/ether 1:1. After the fractions obtained have been evaporated, crystals of the desired compound having an m.p. of 127°C (decomposition) form.

The compounds listed in the tables below can be prepared analogously to Examples H1 to H23.

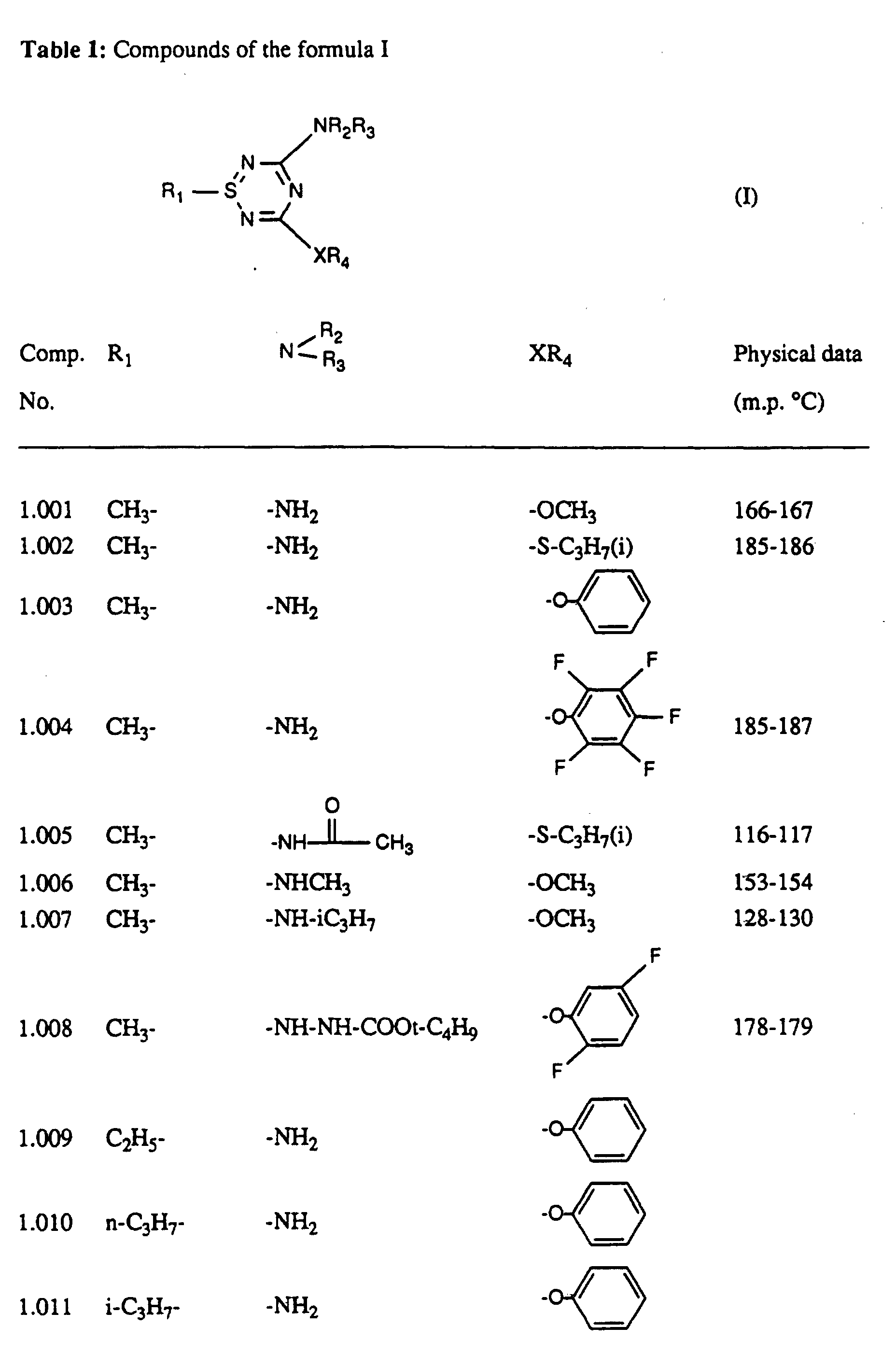

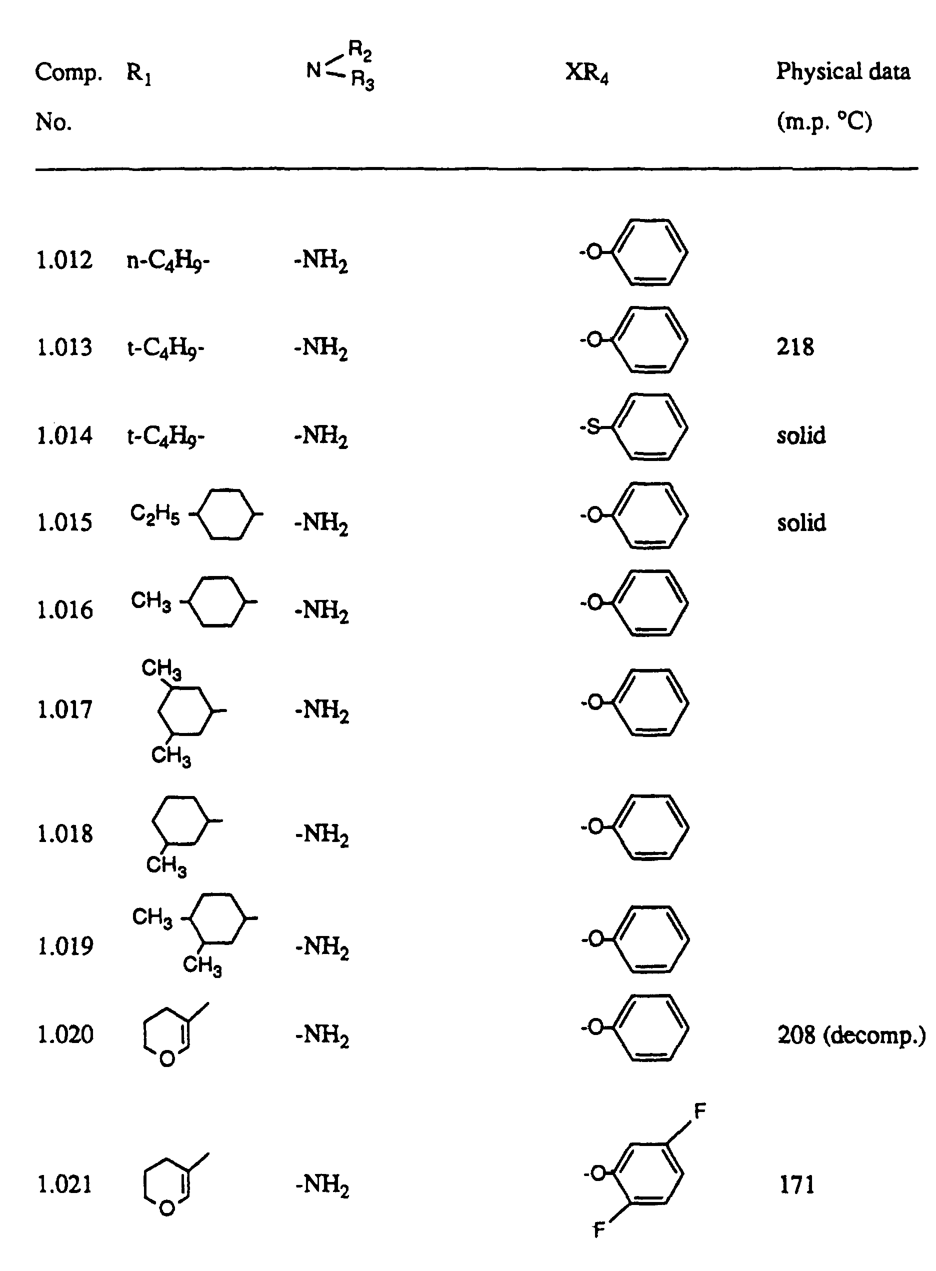

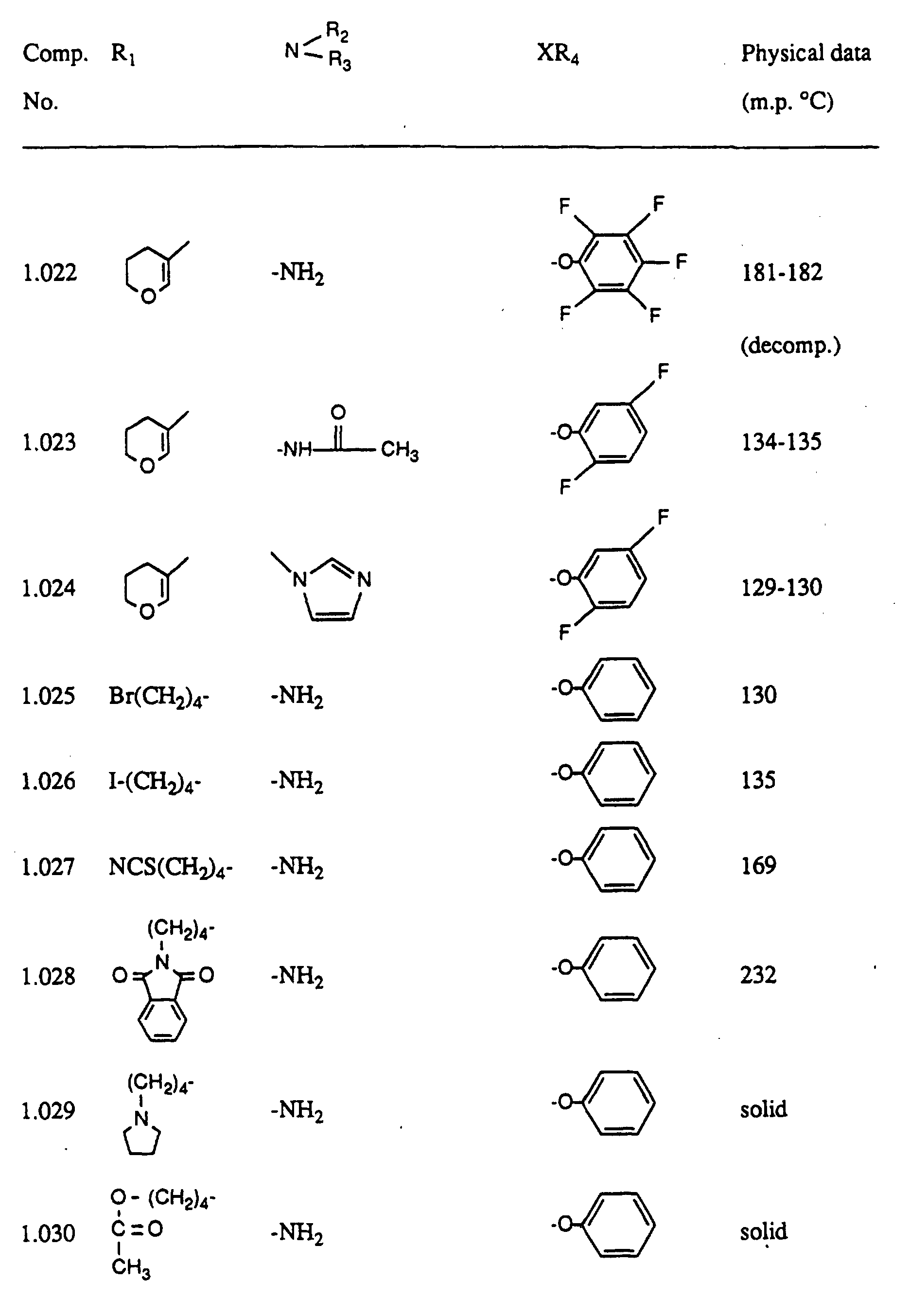

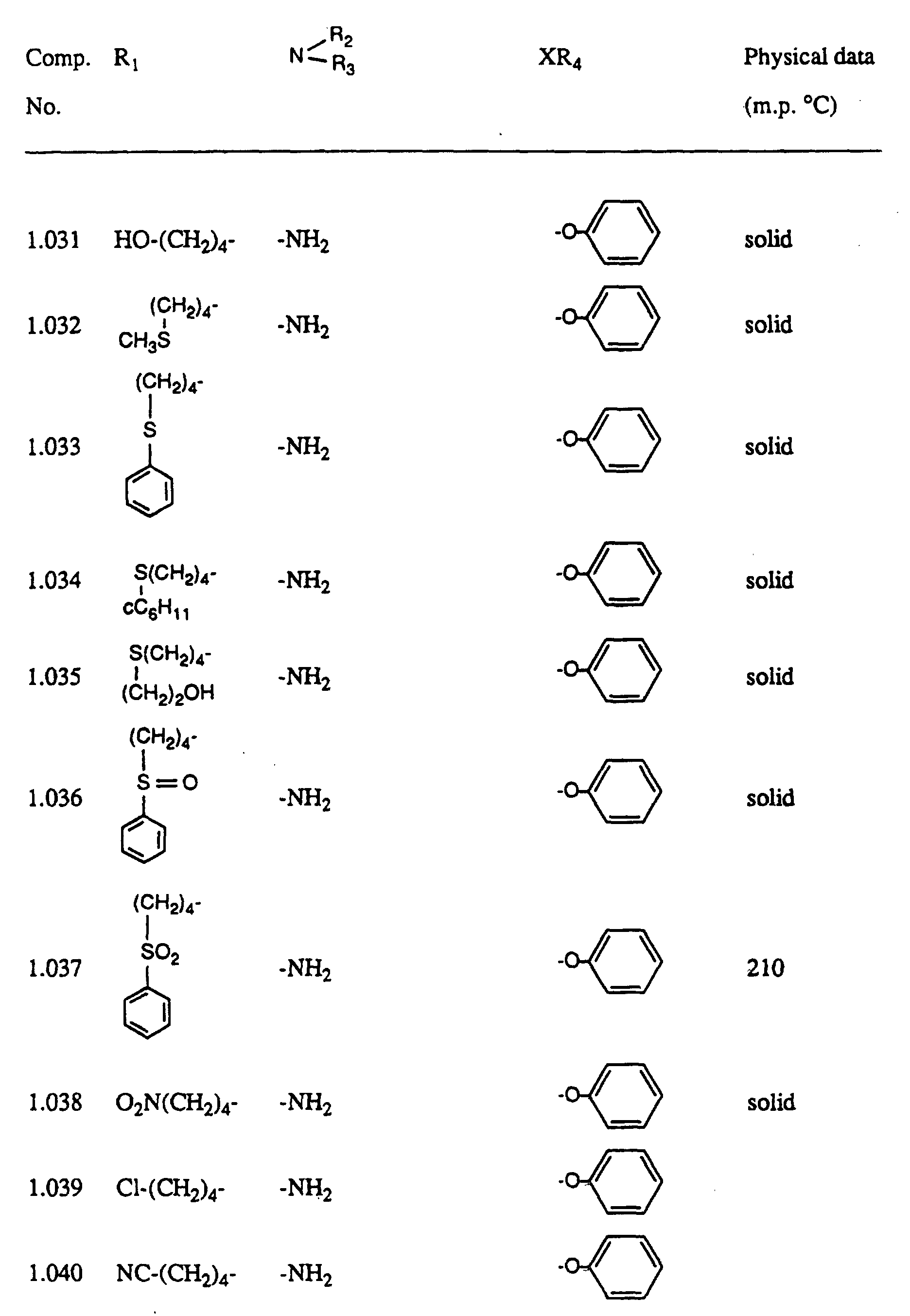

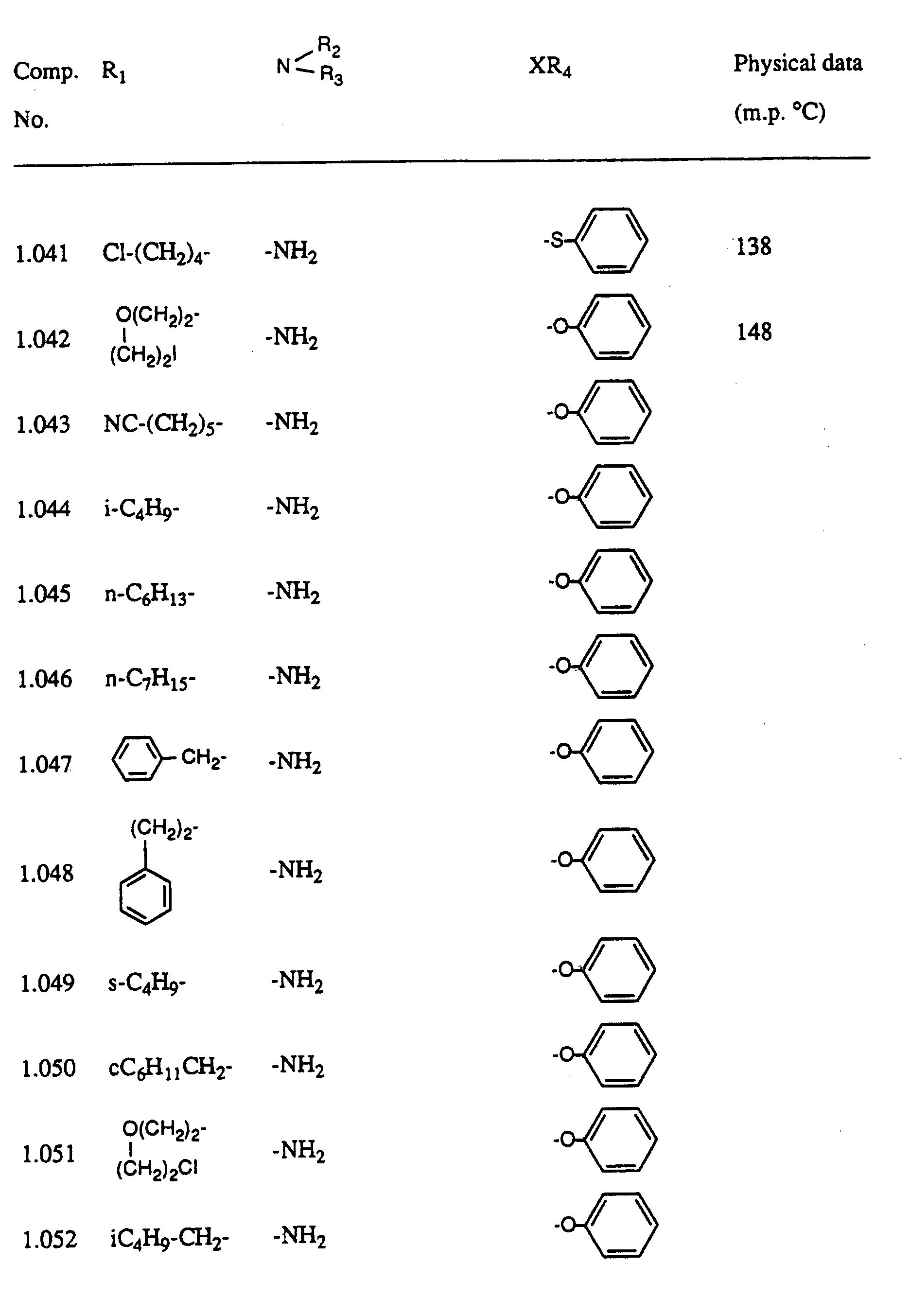

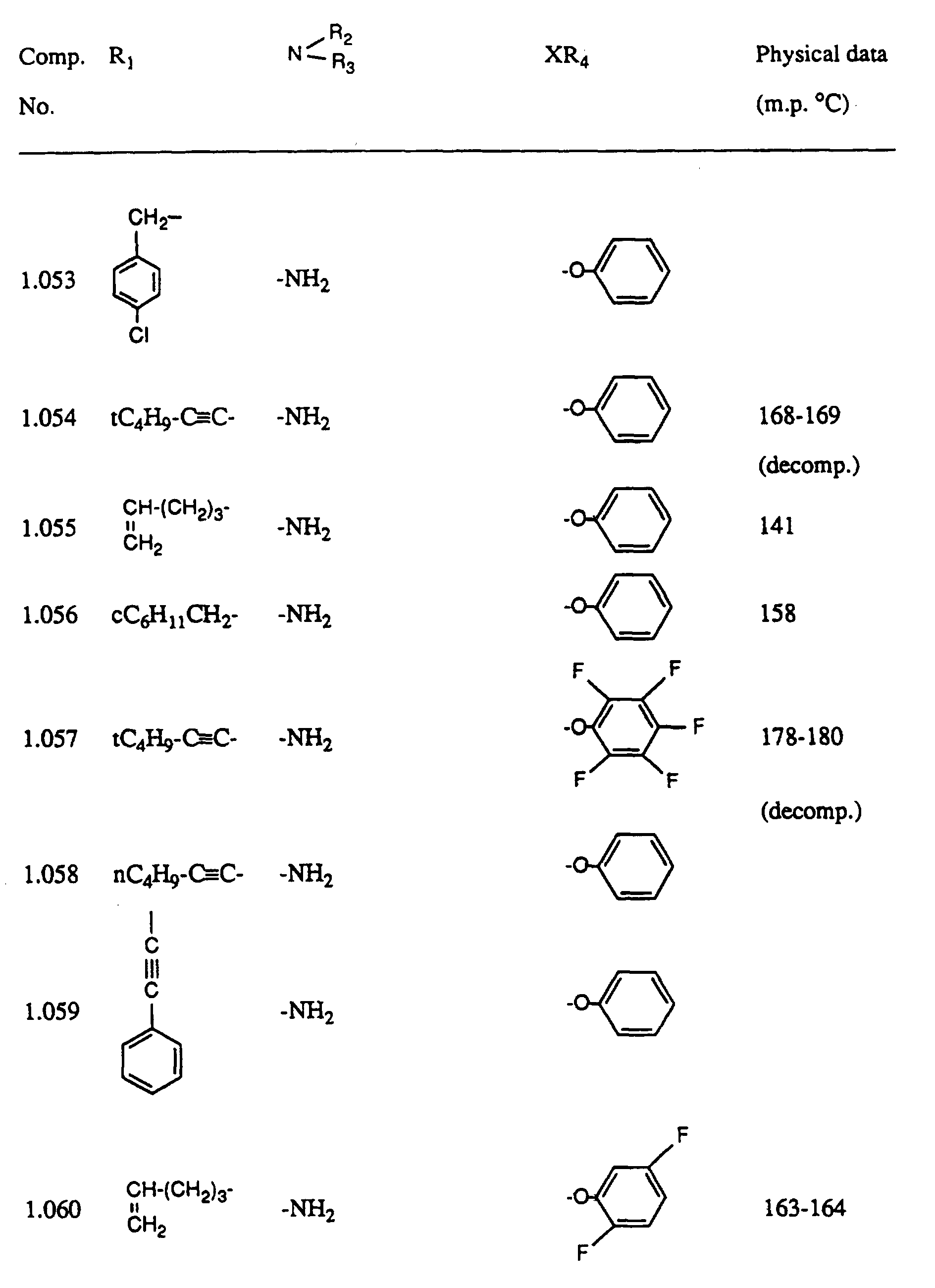

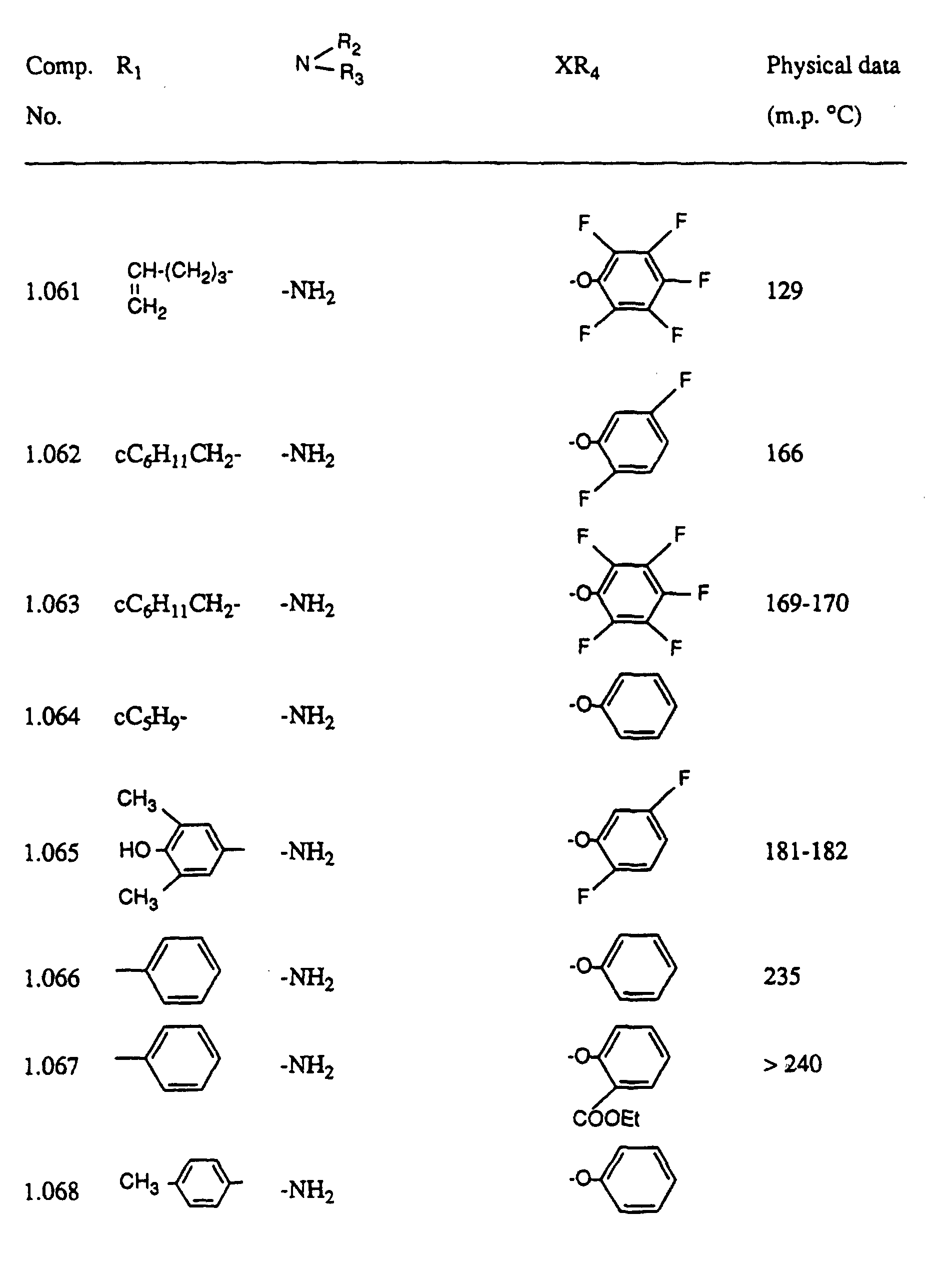

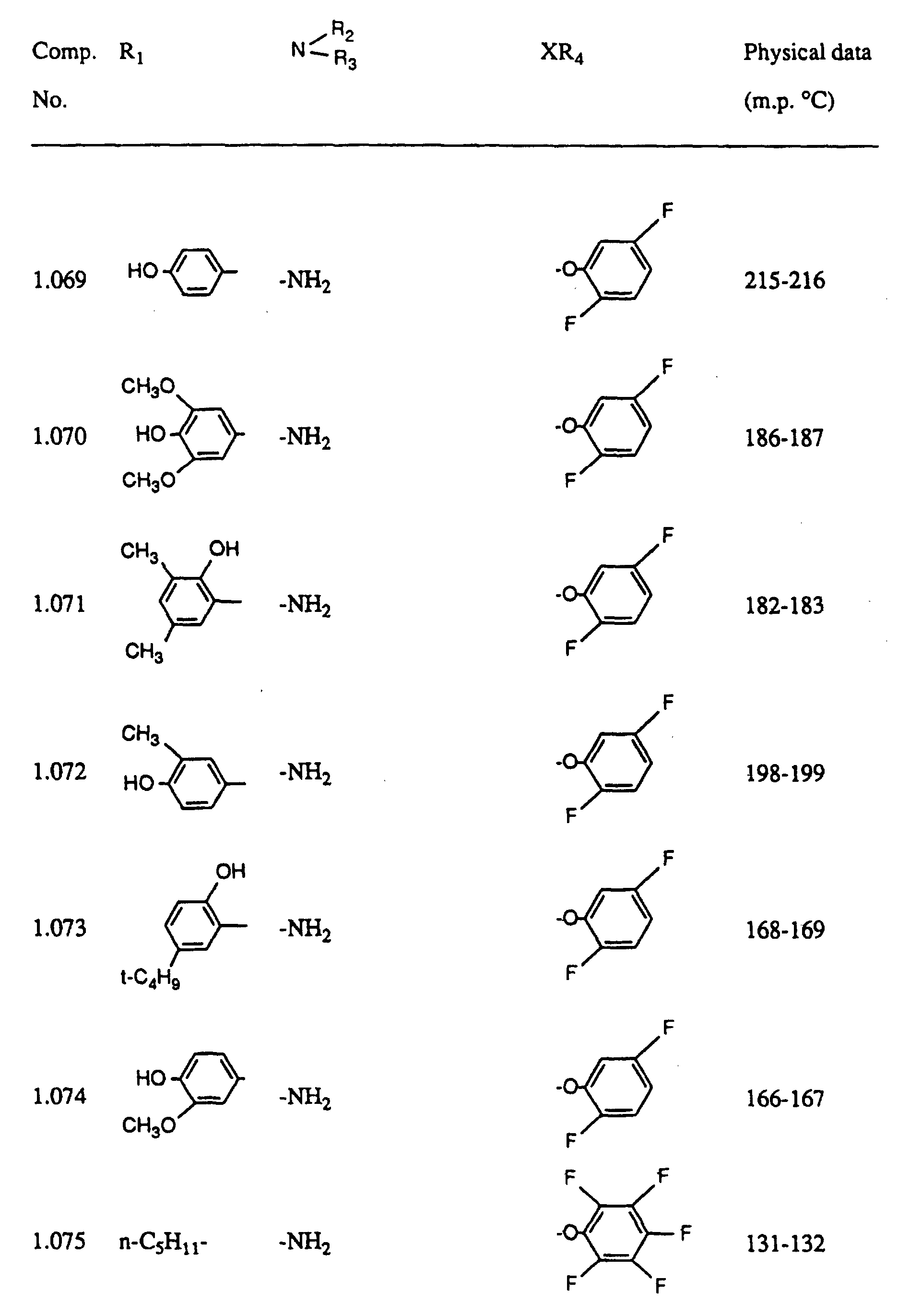

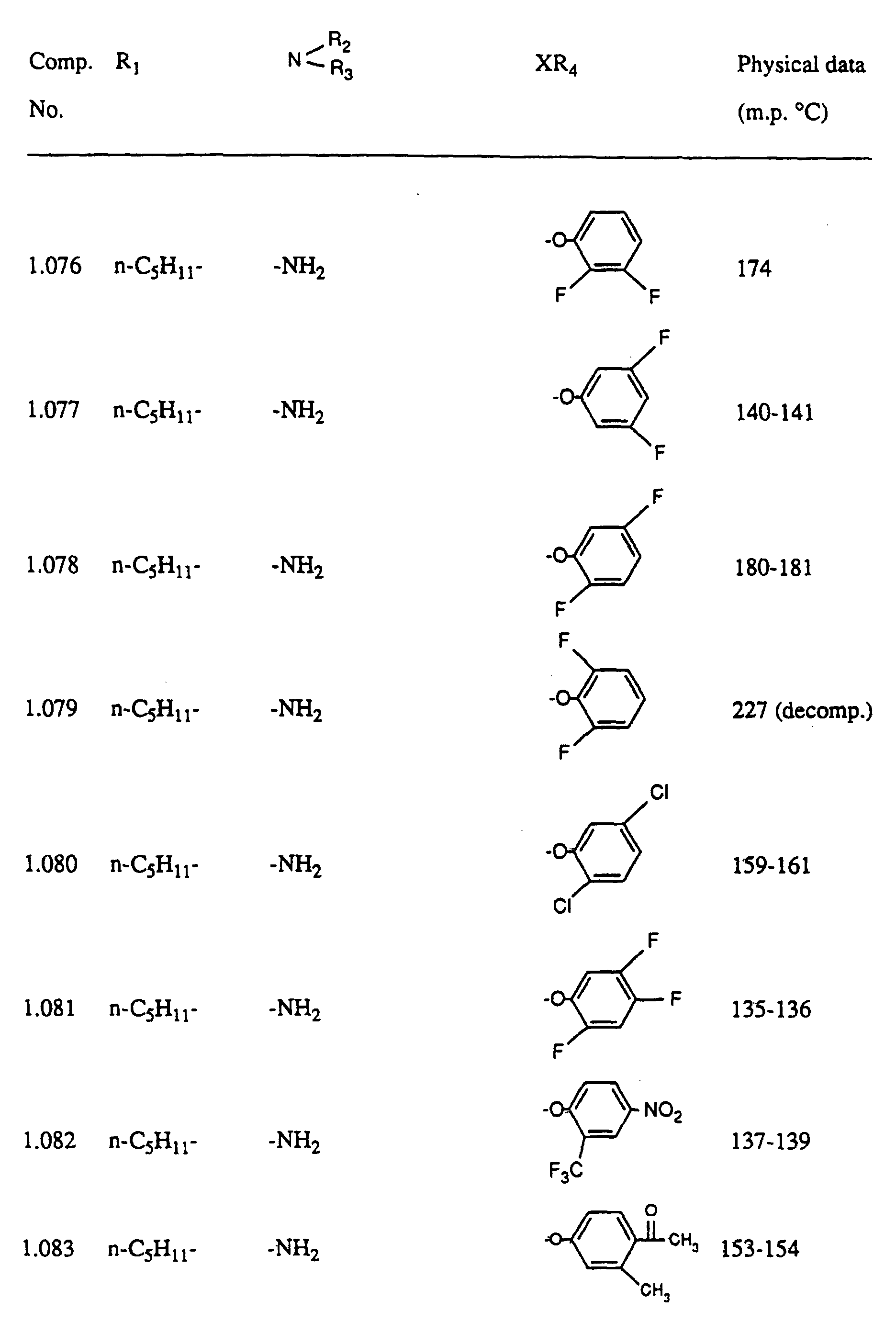

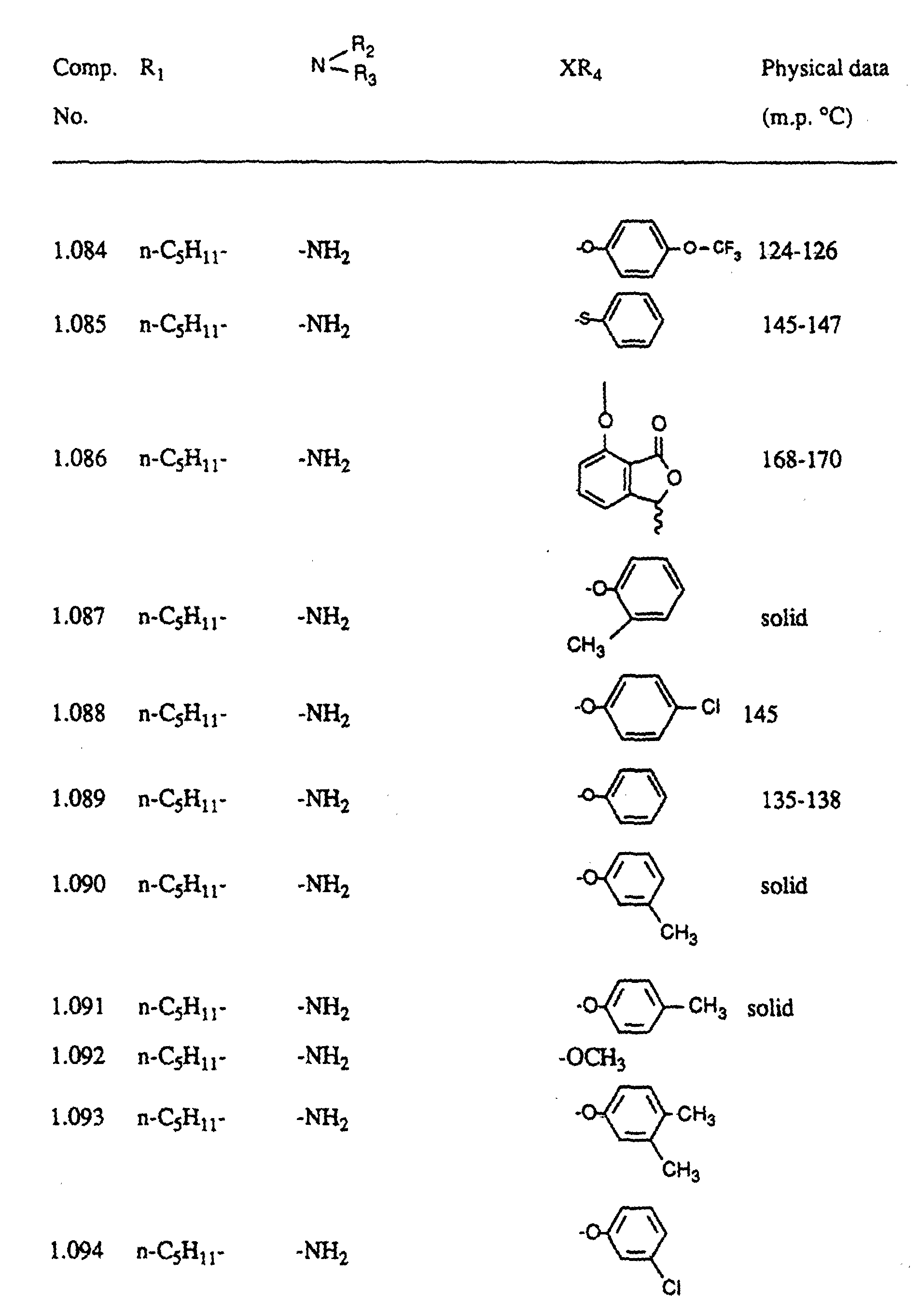

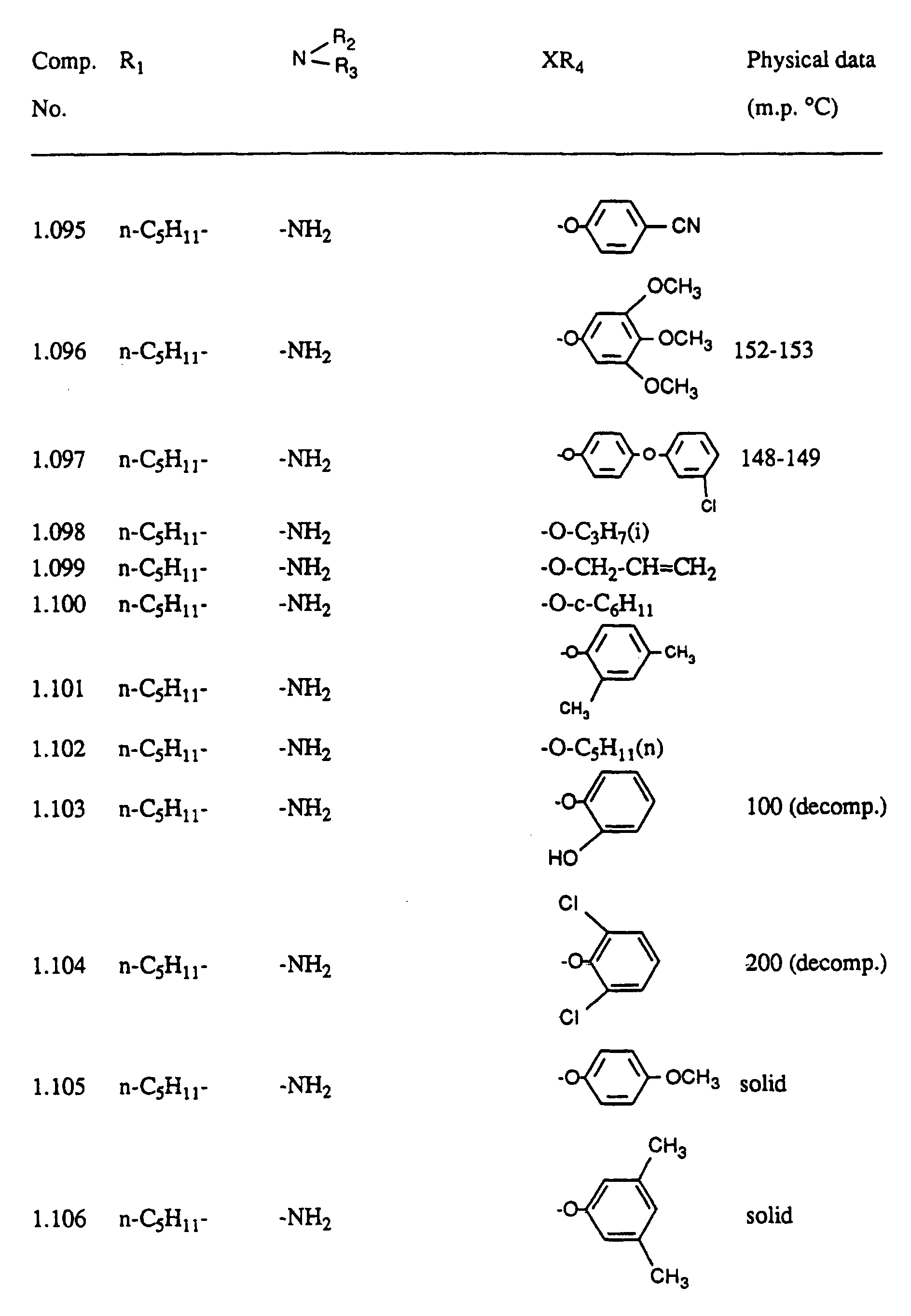

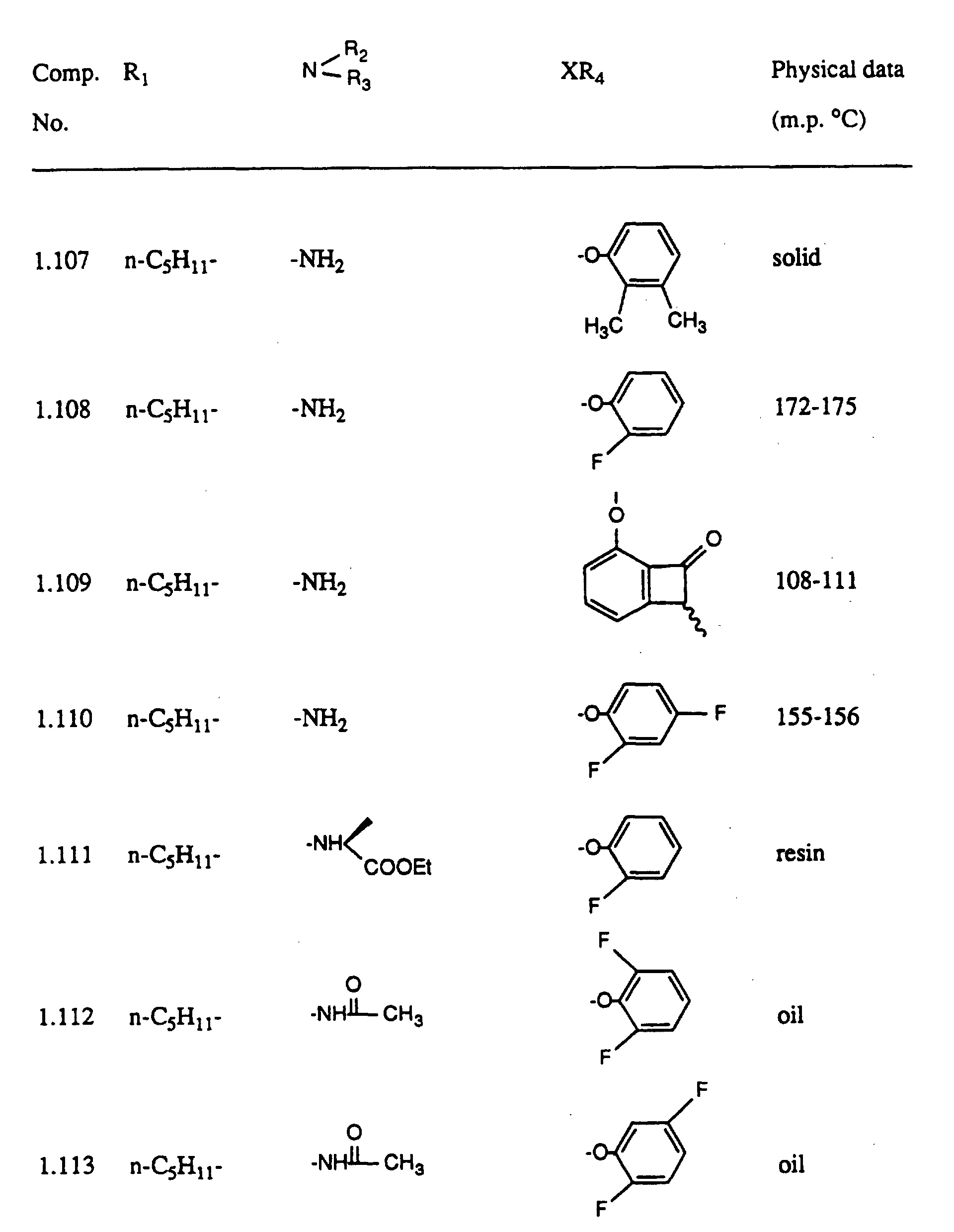

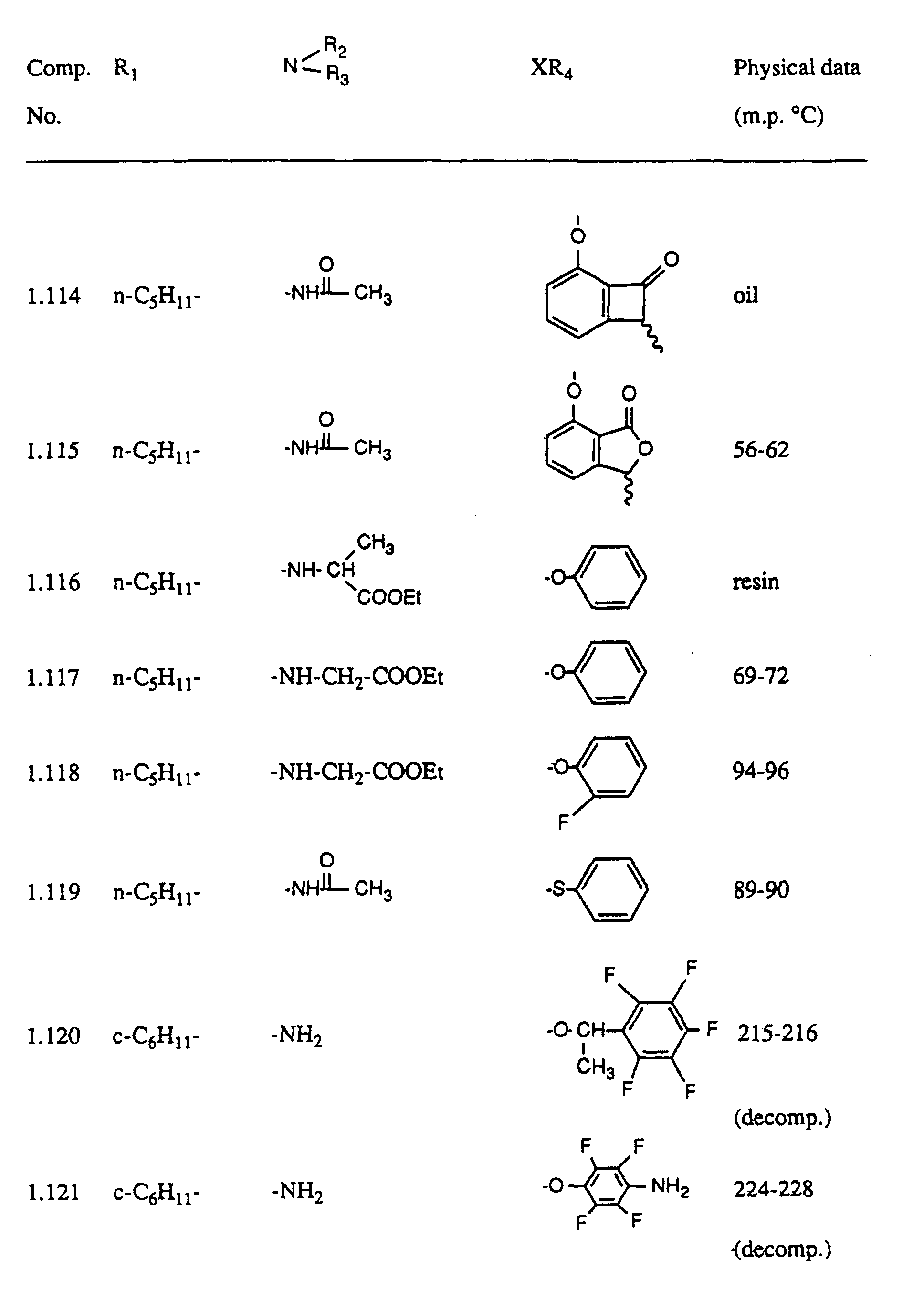

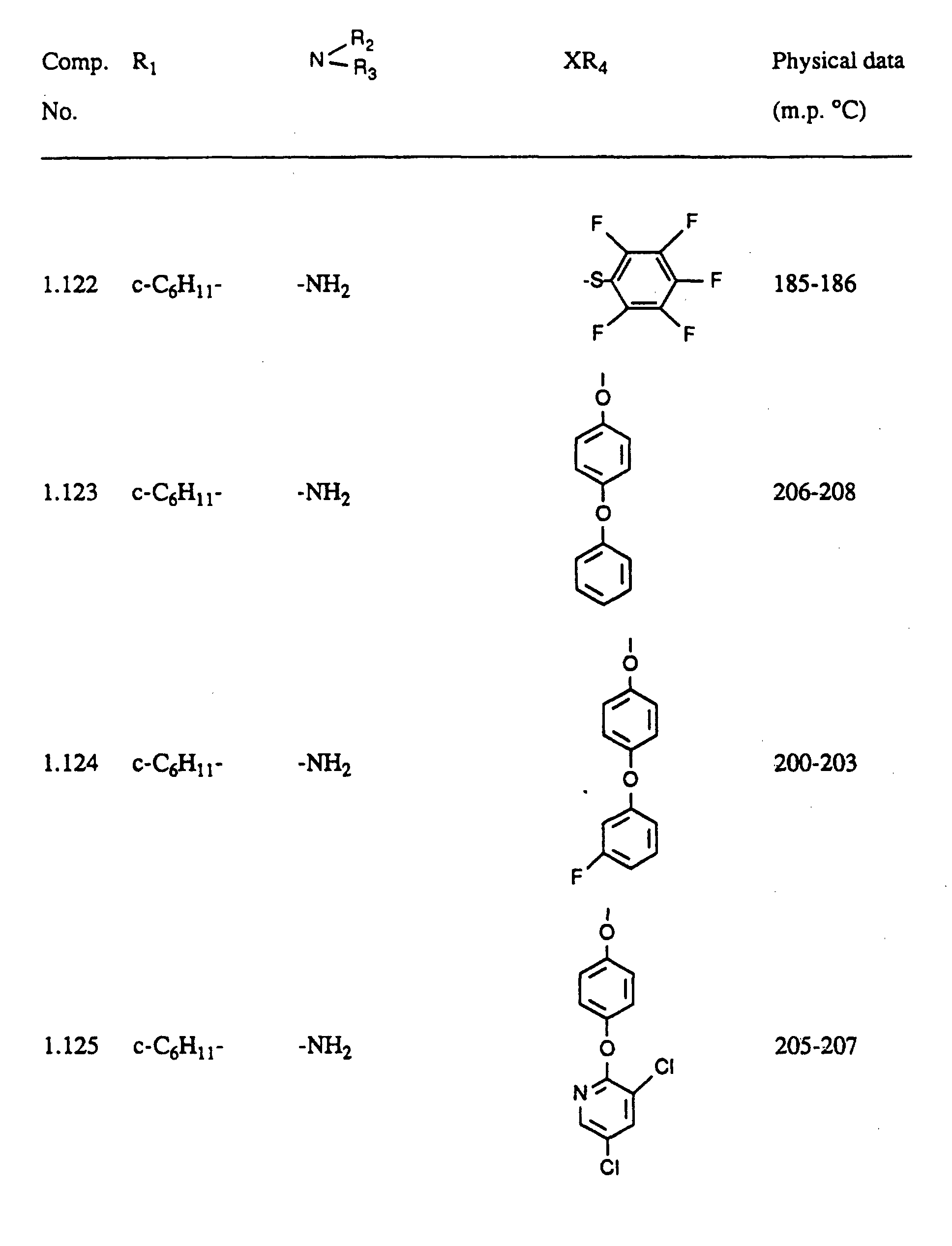

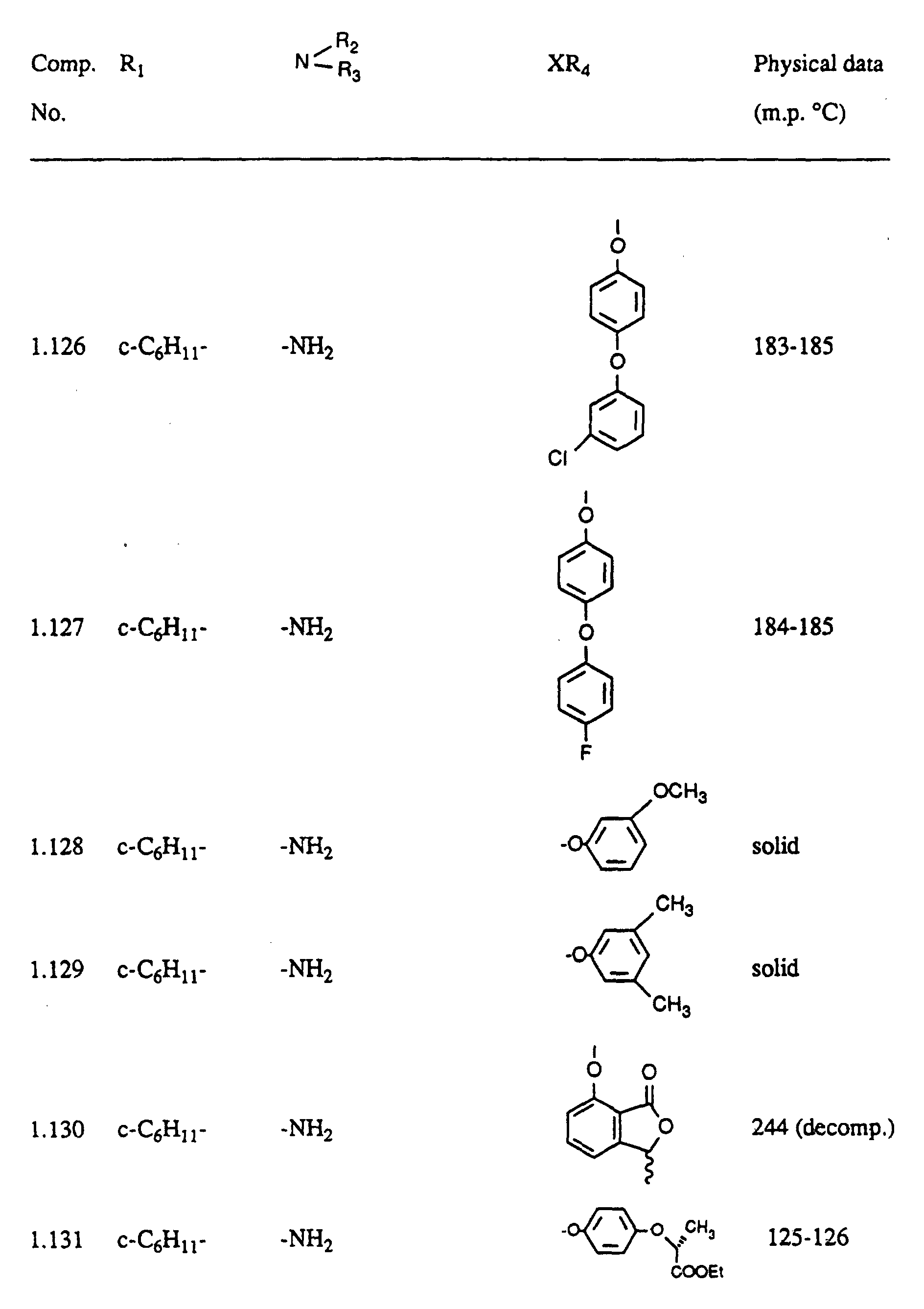

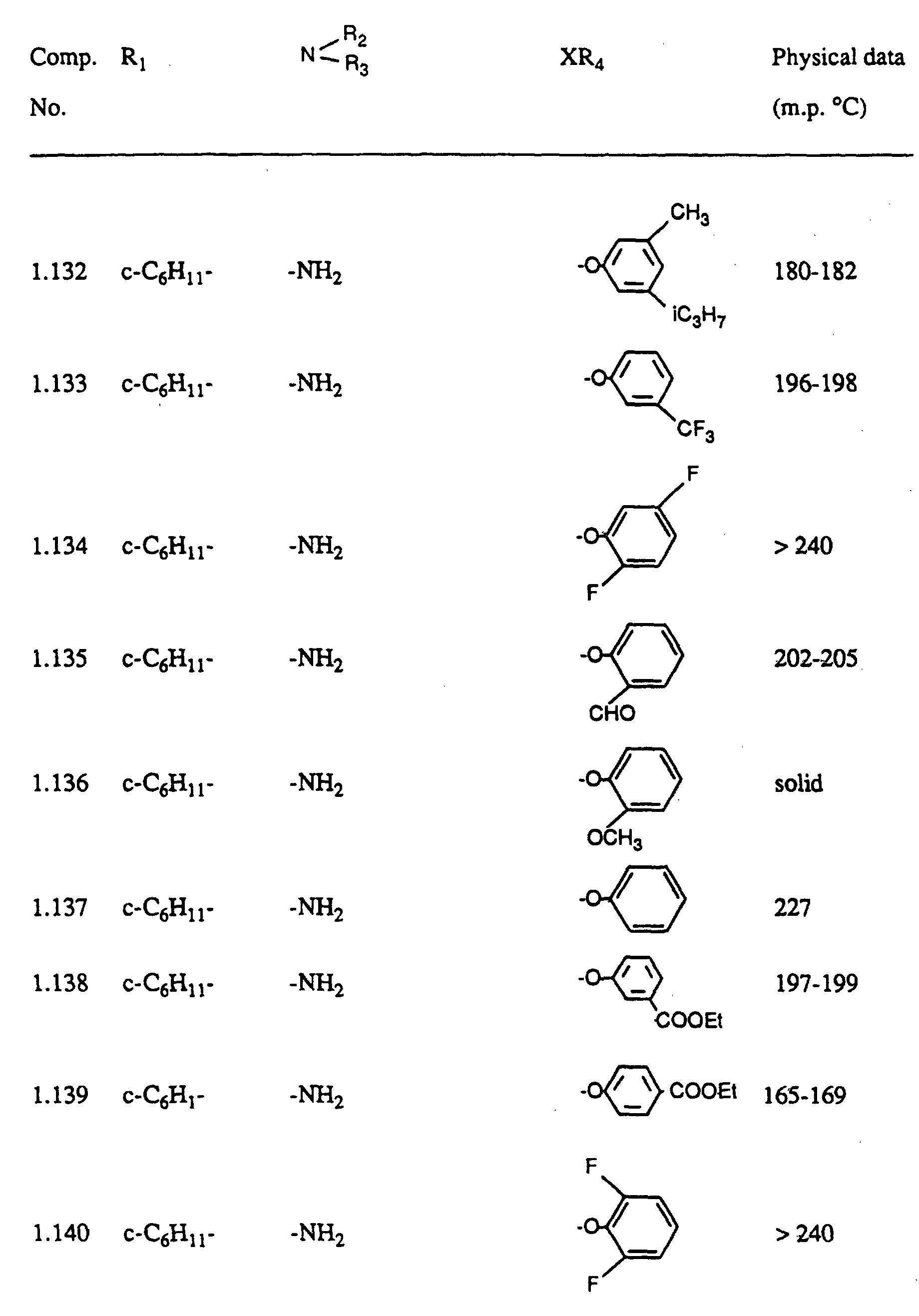

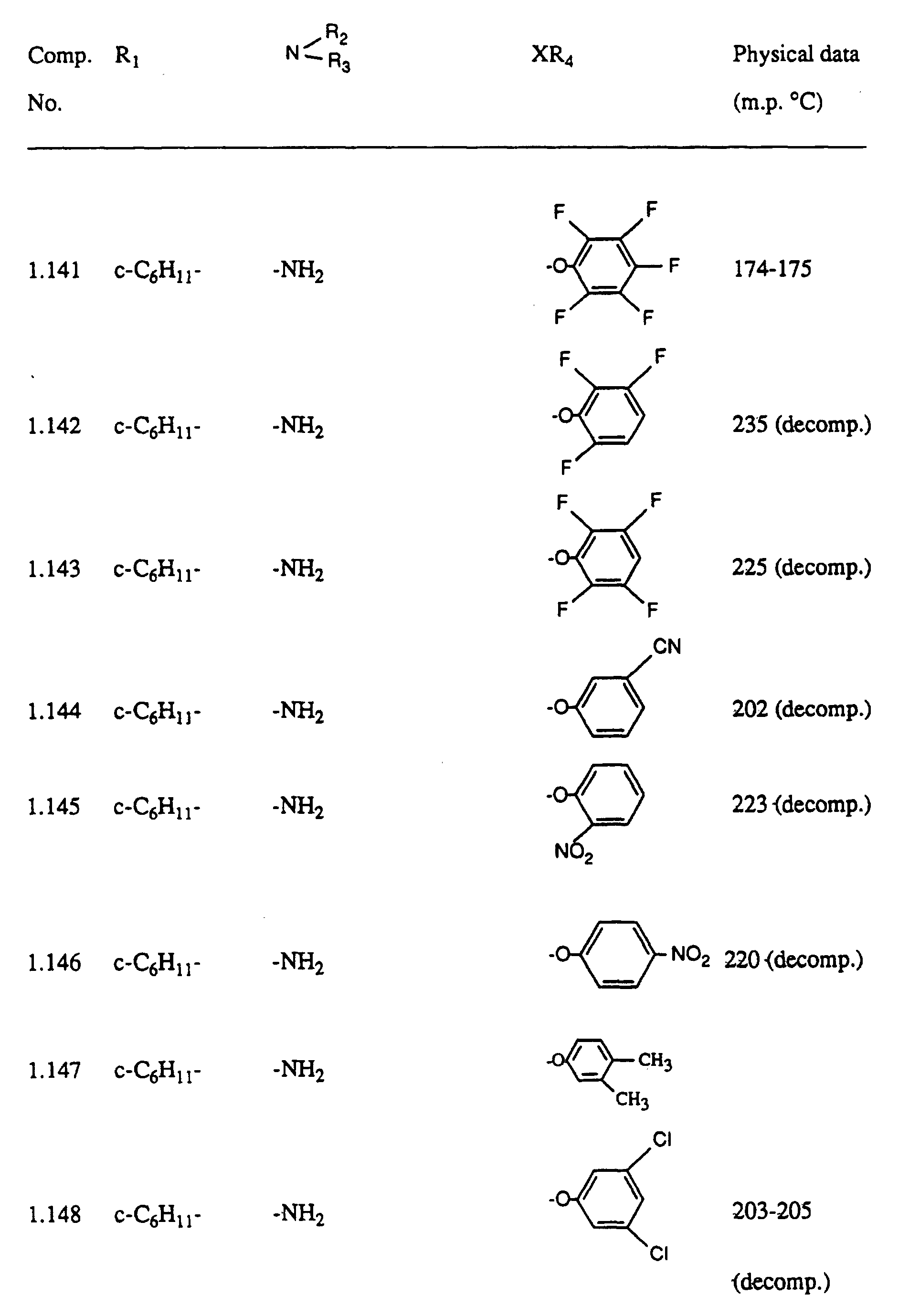

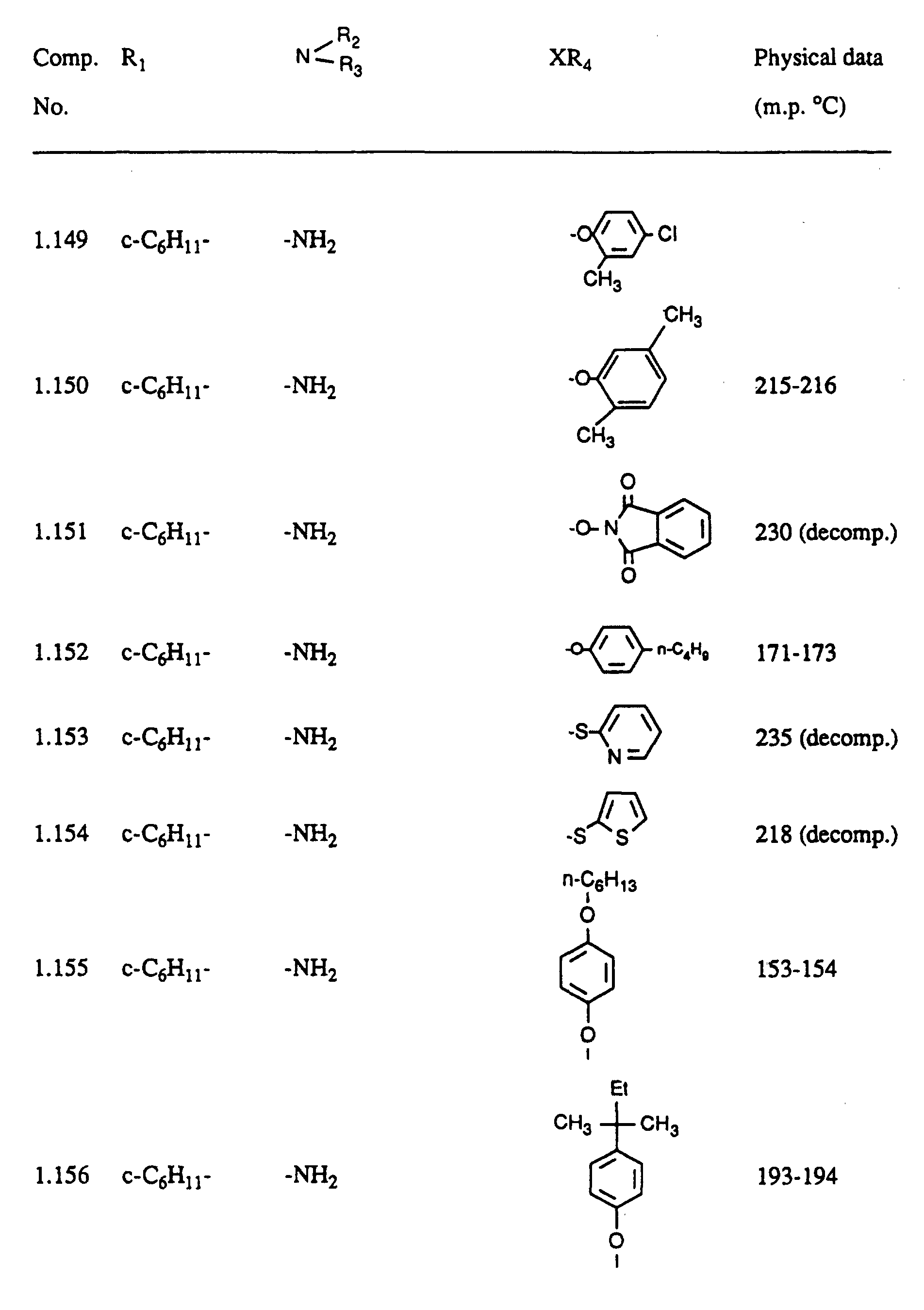

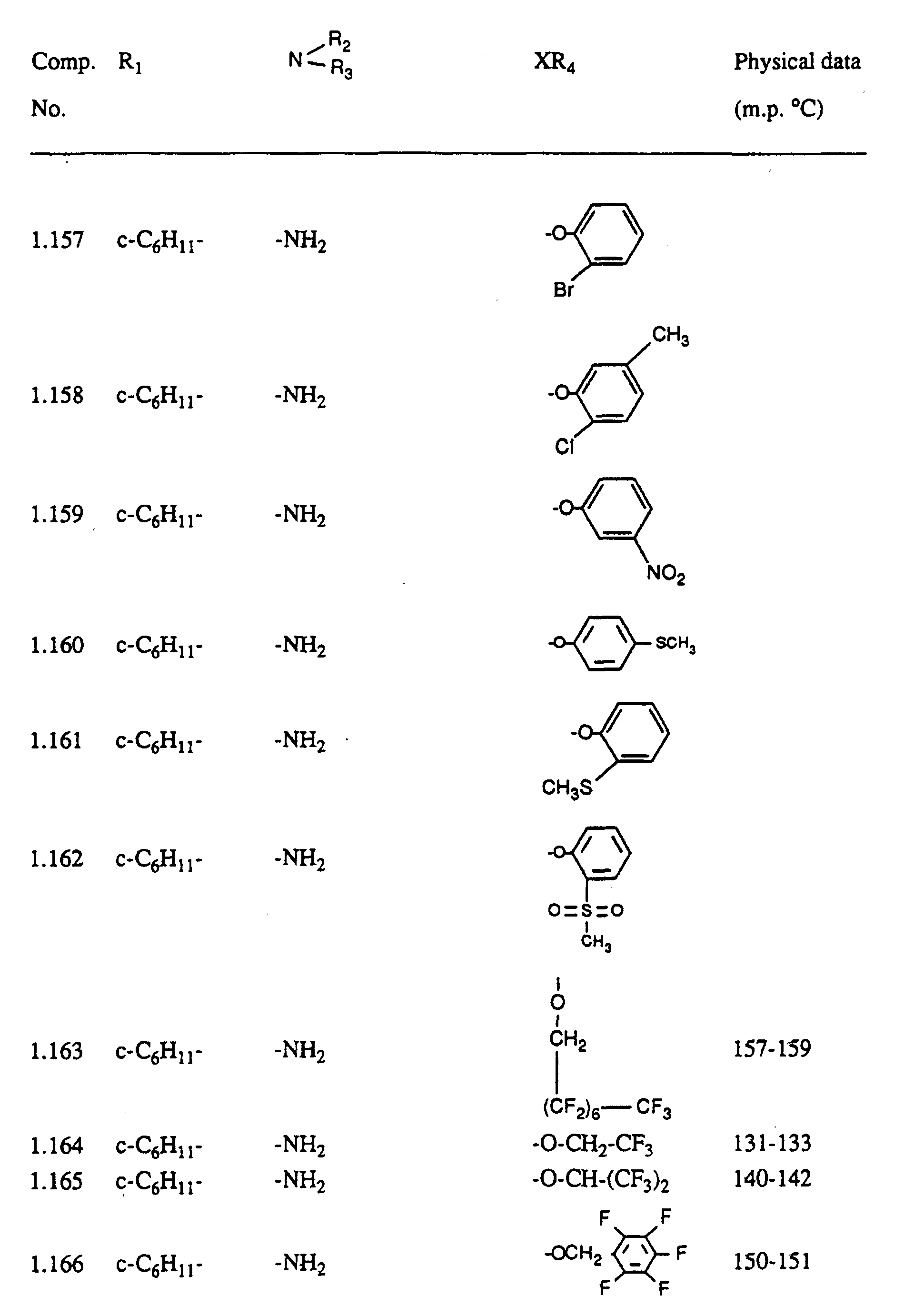

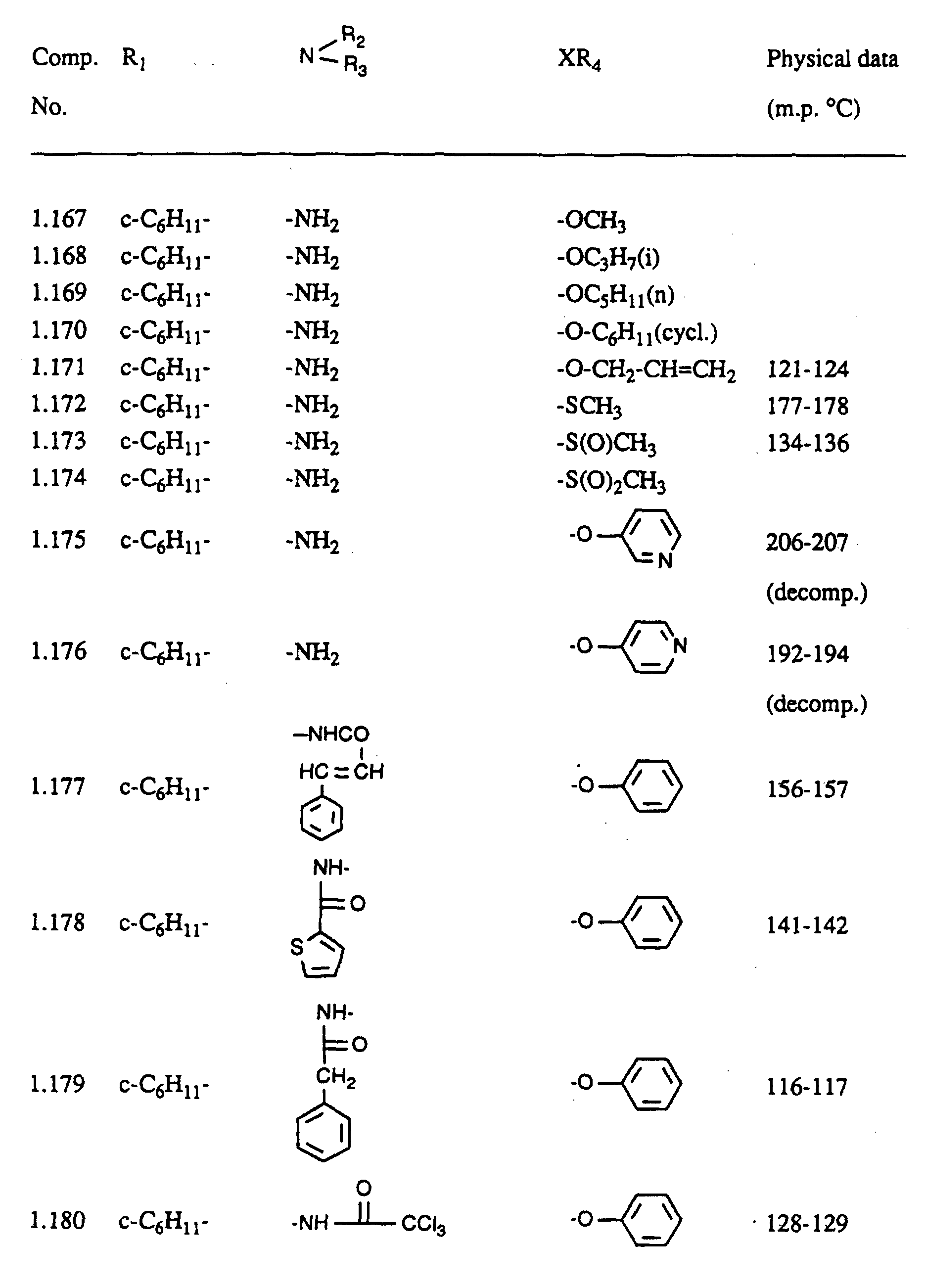

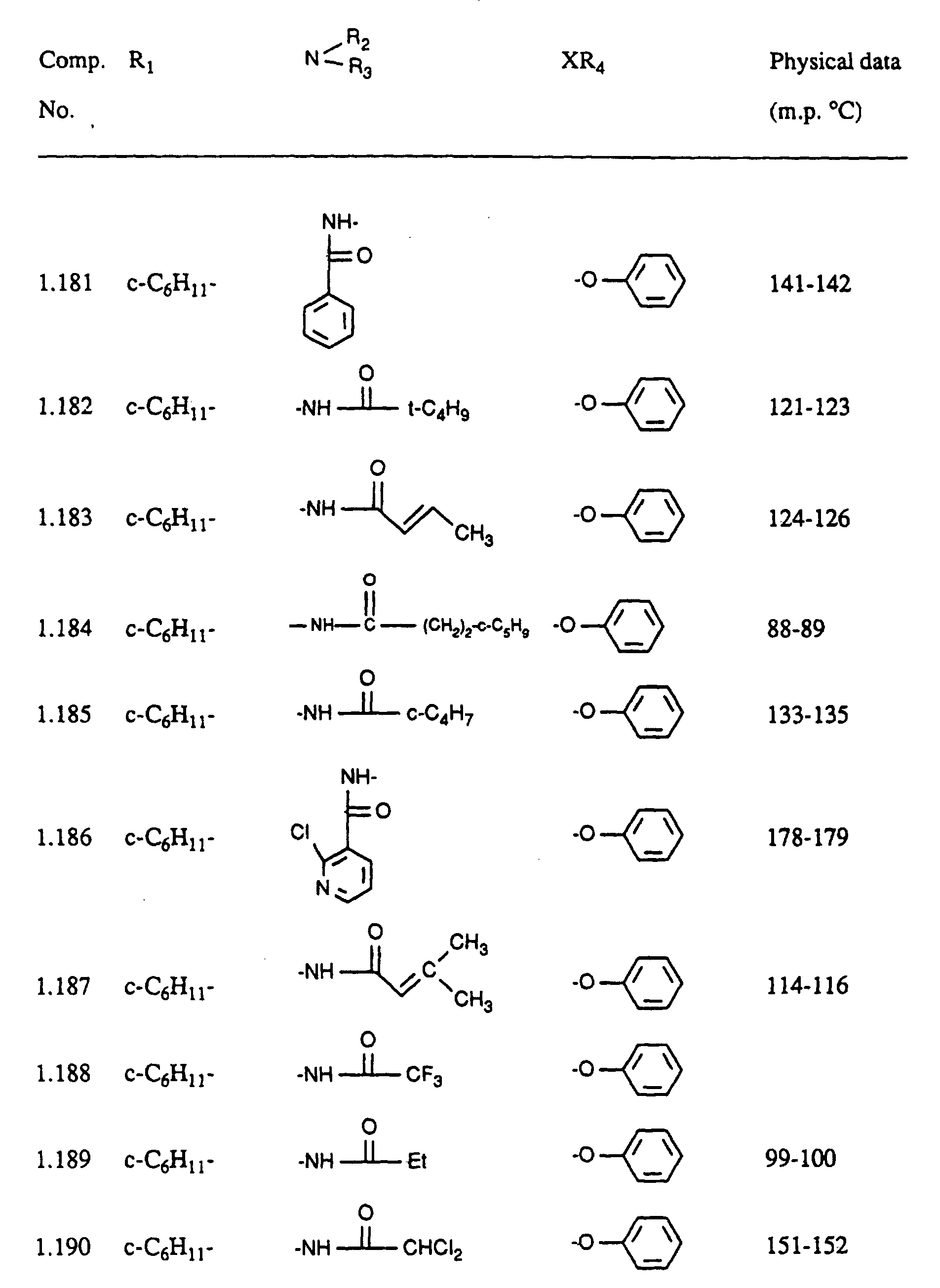

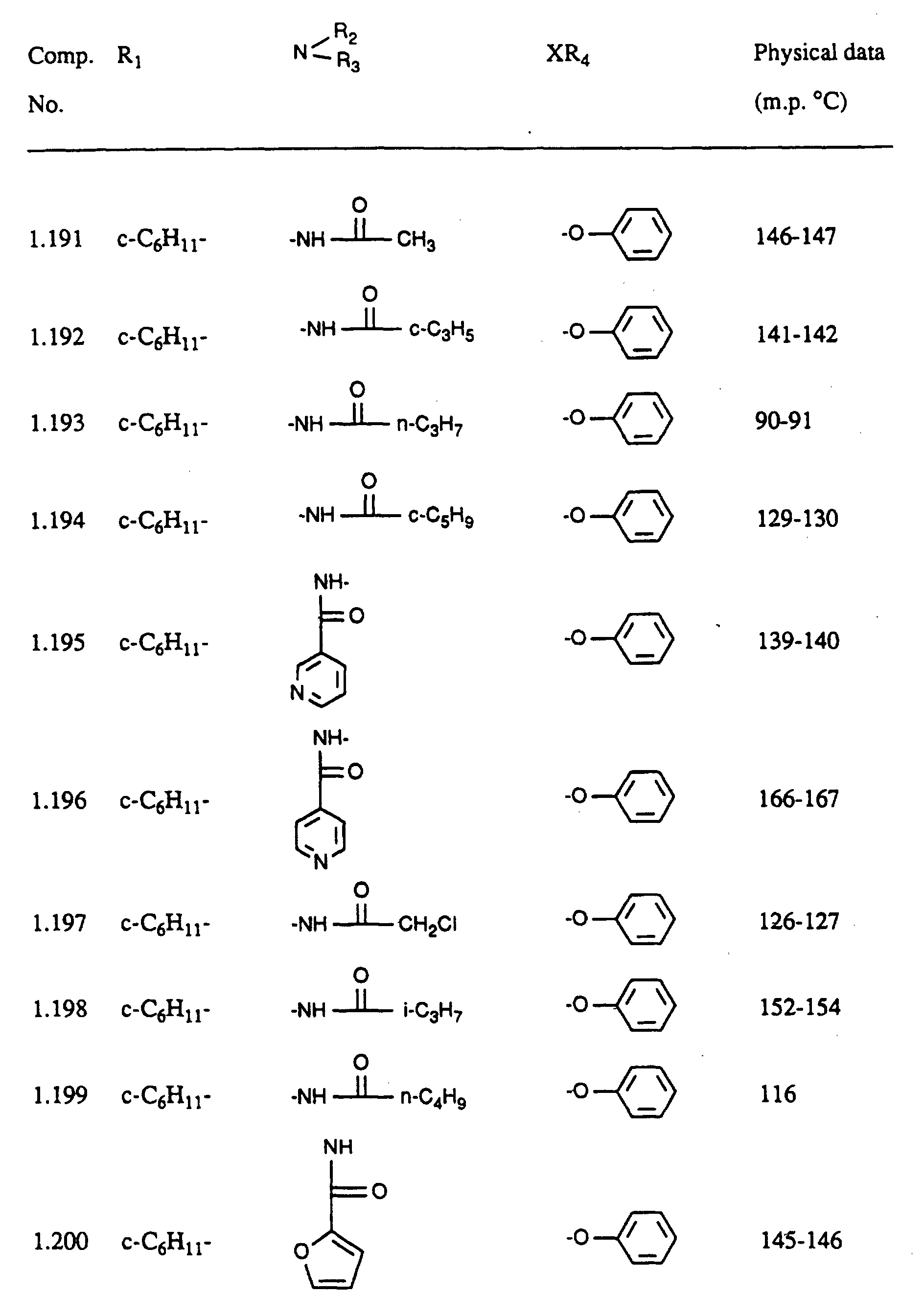

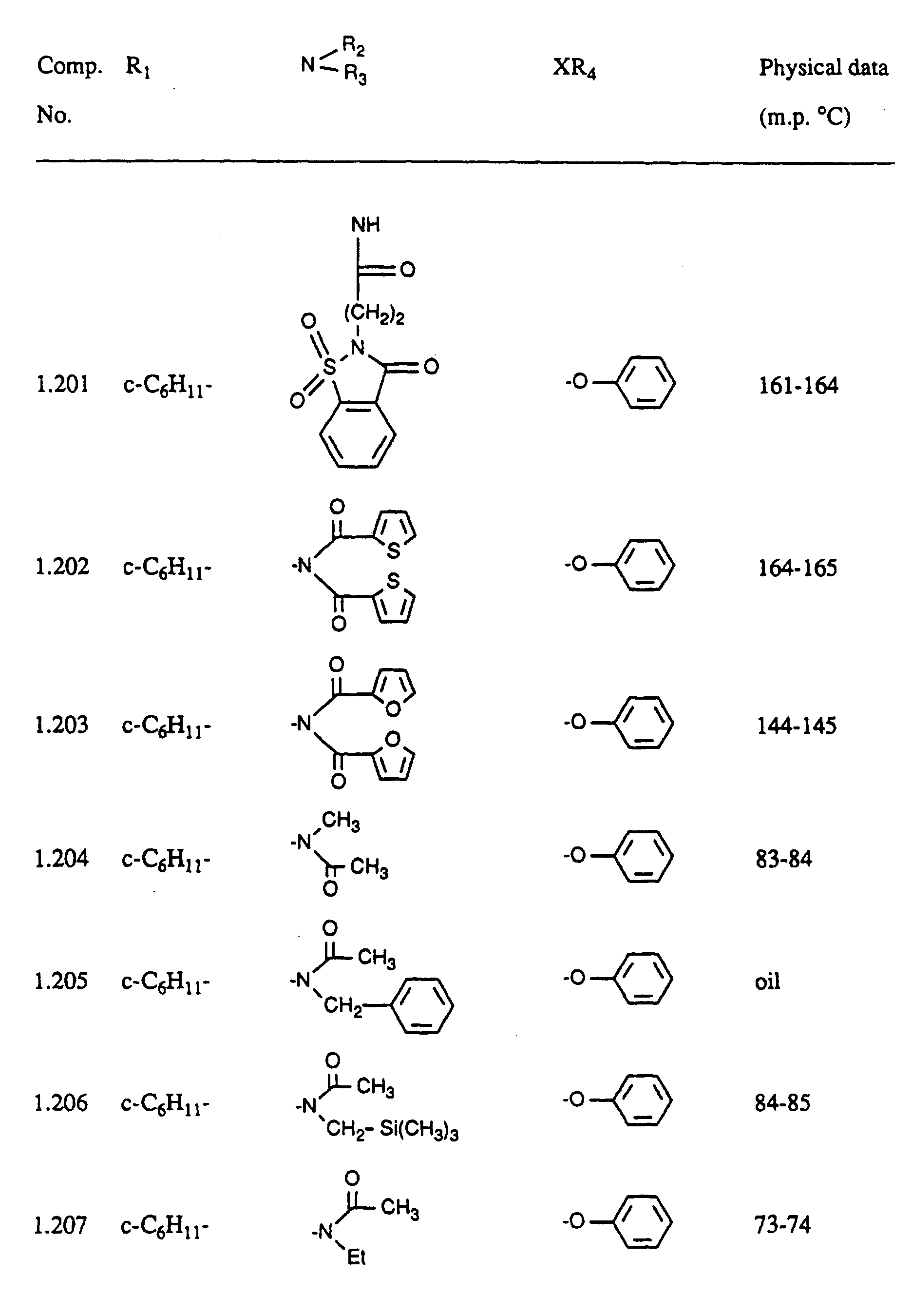

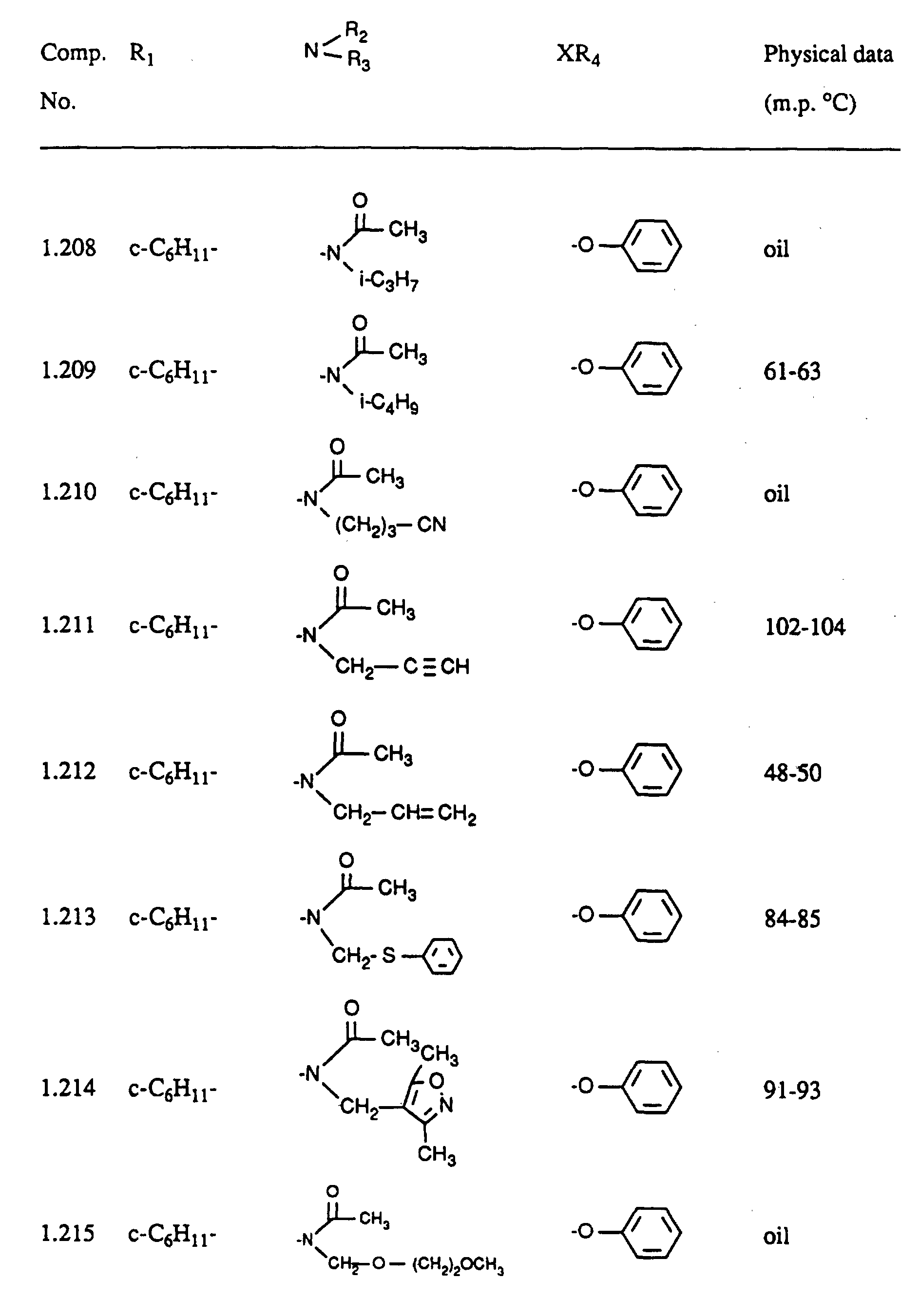

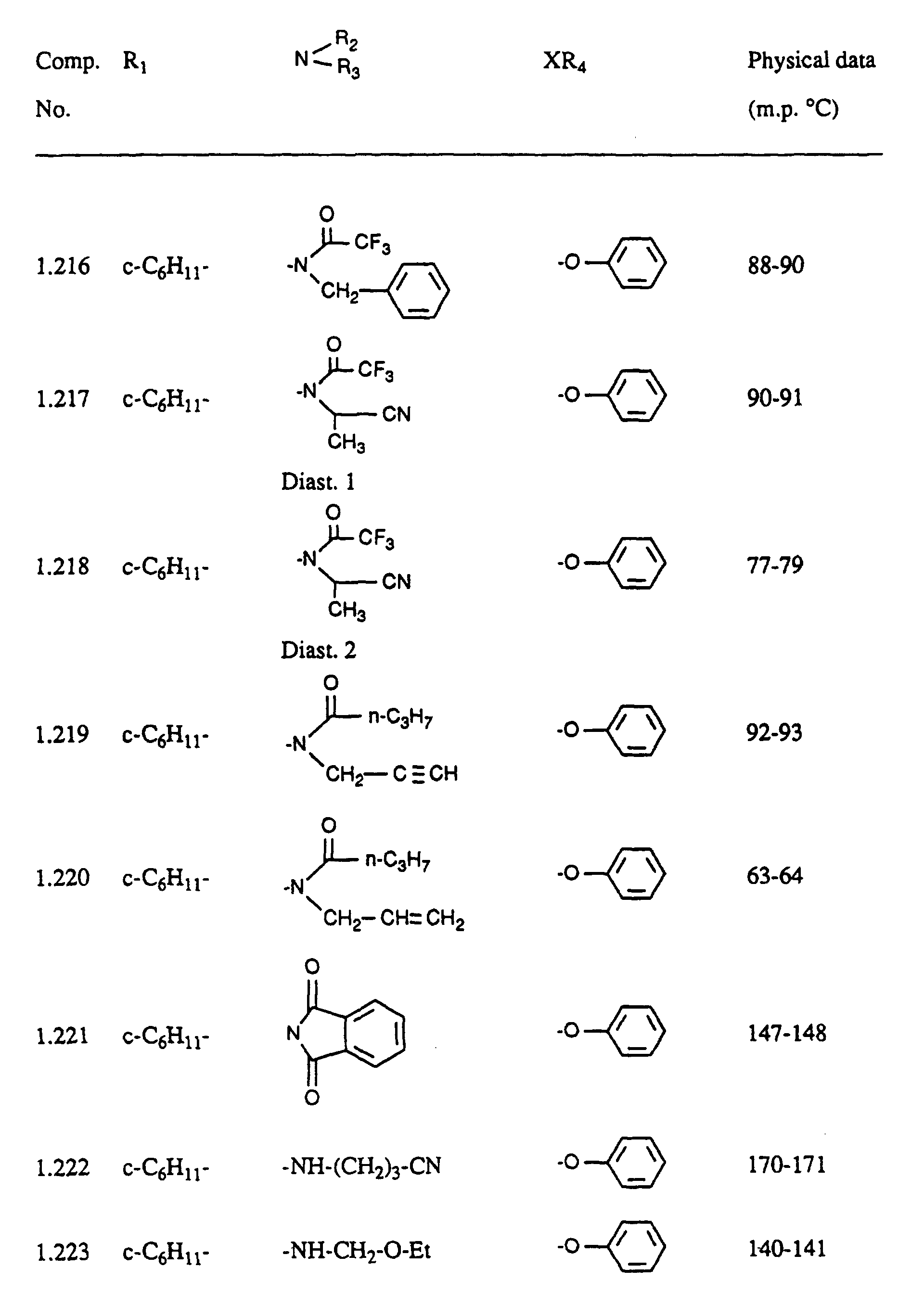

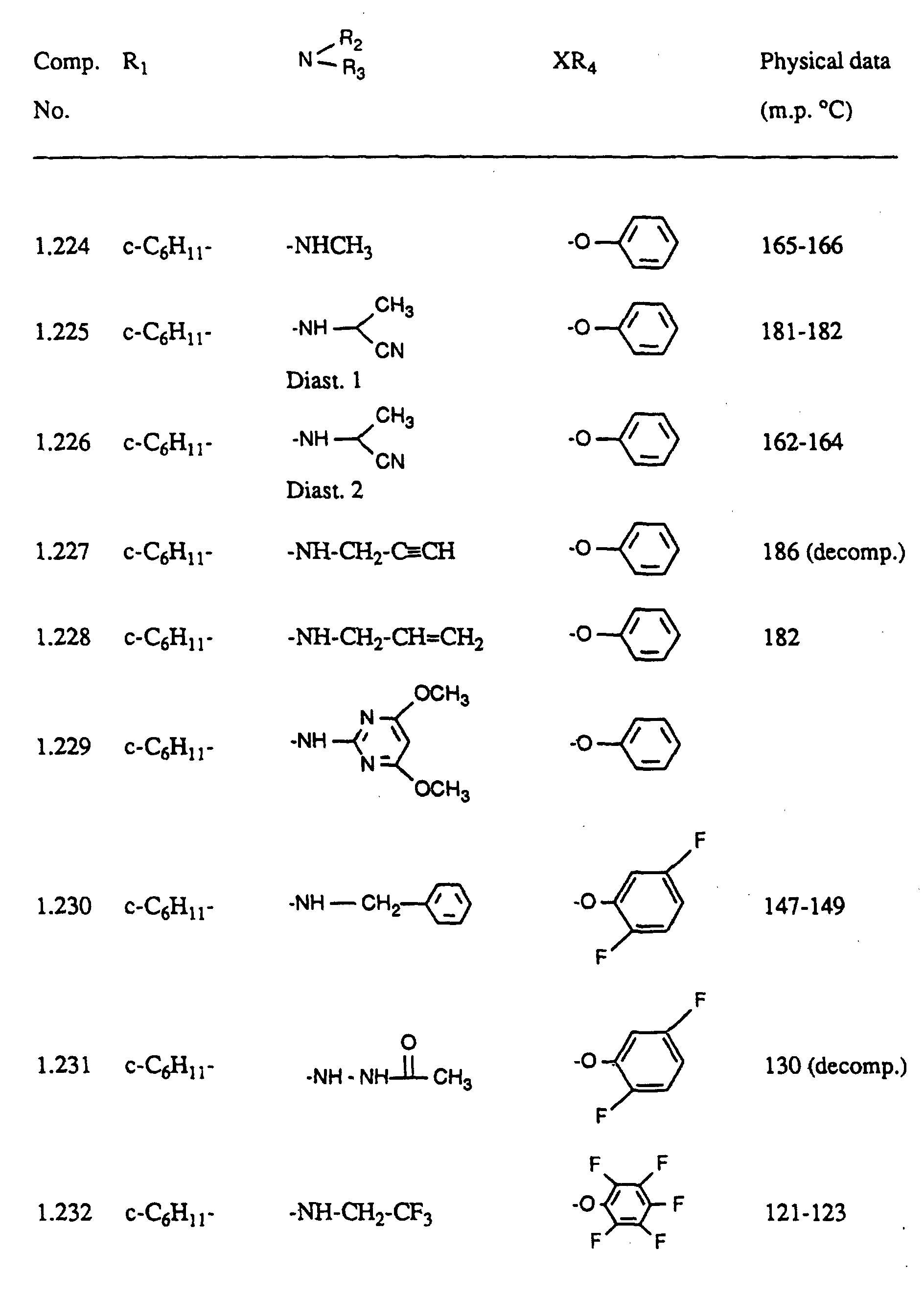

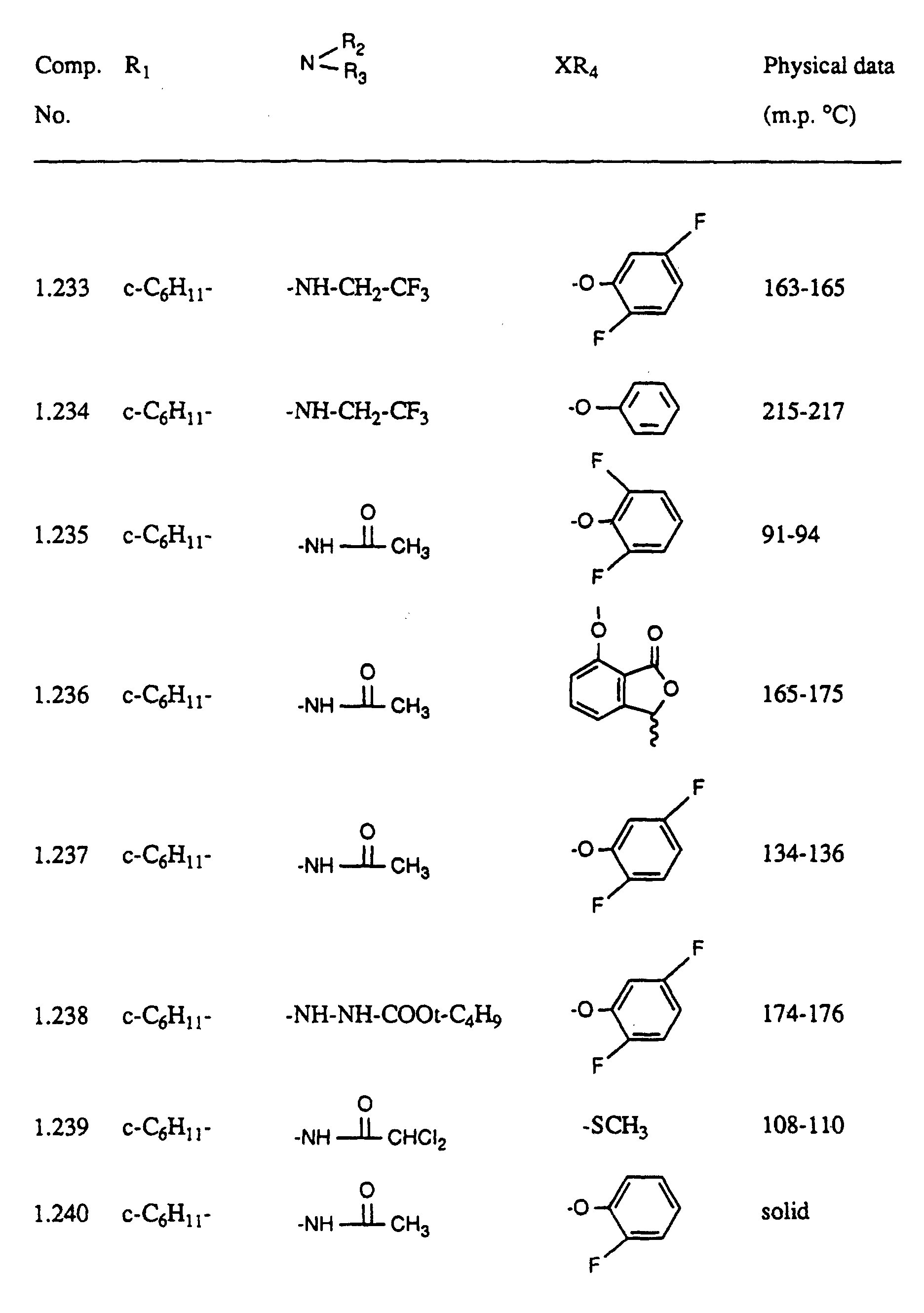

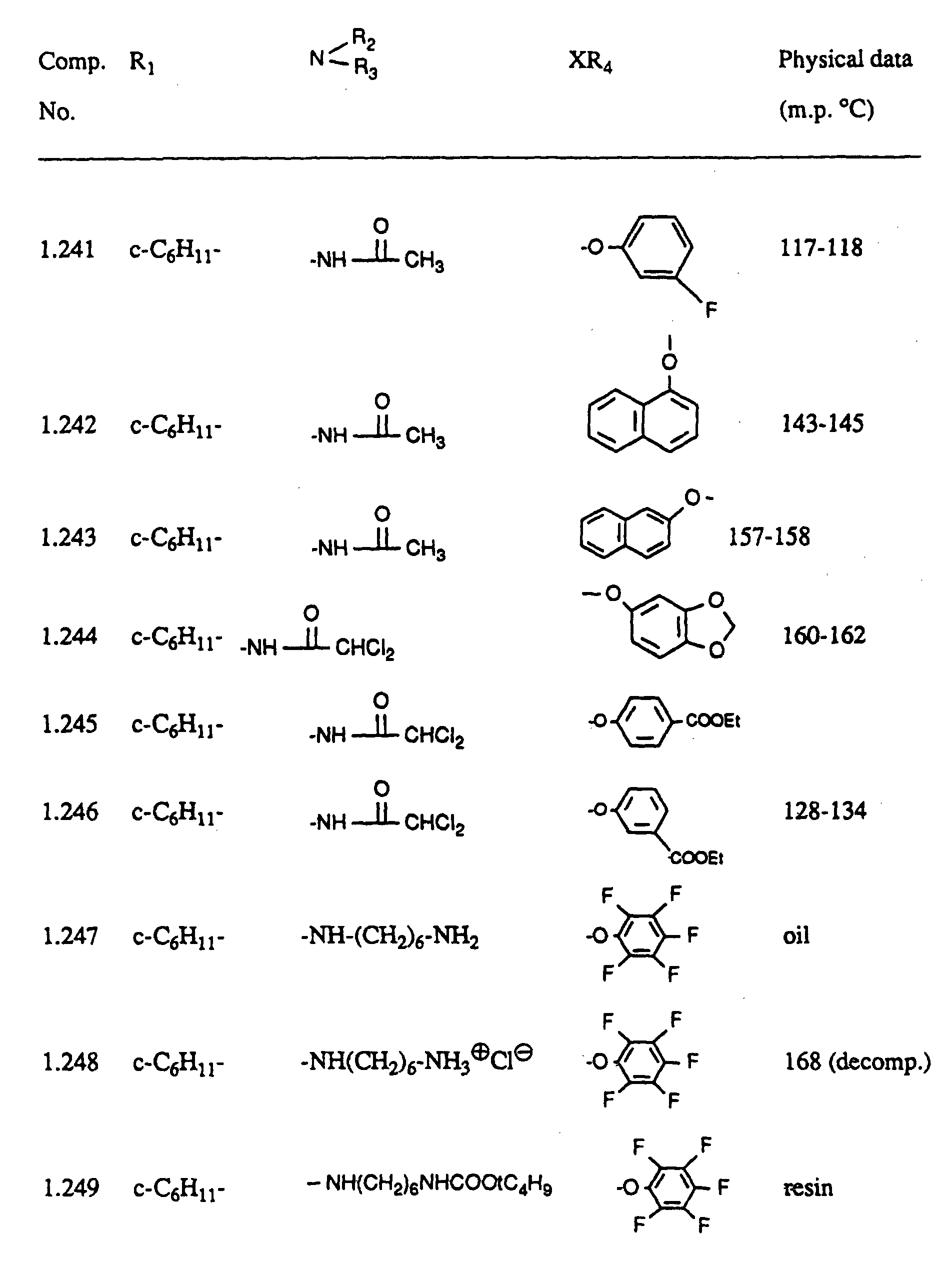

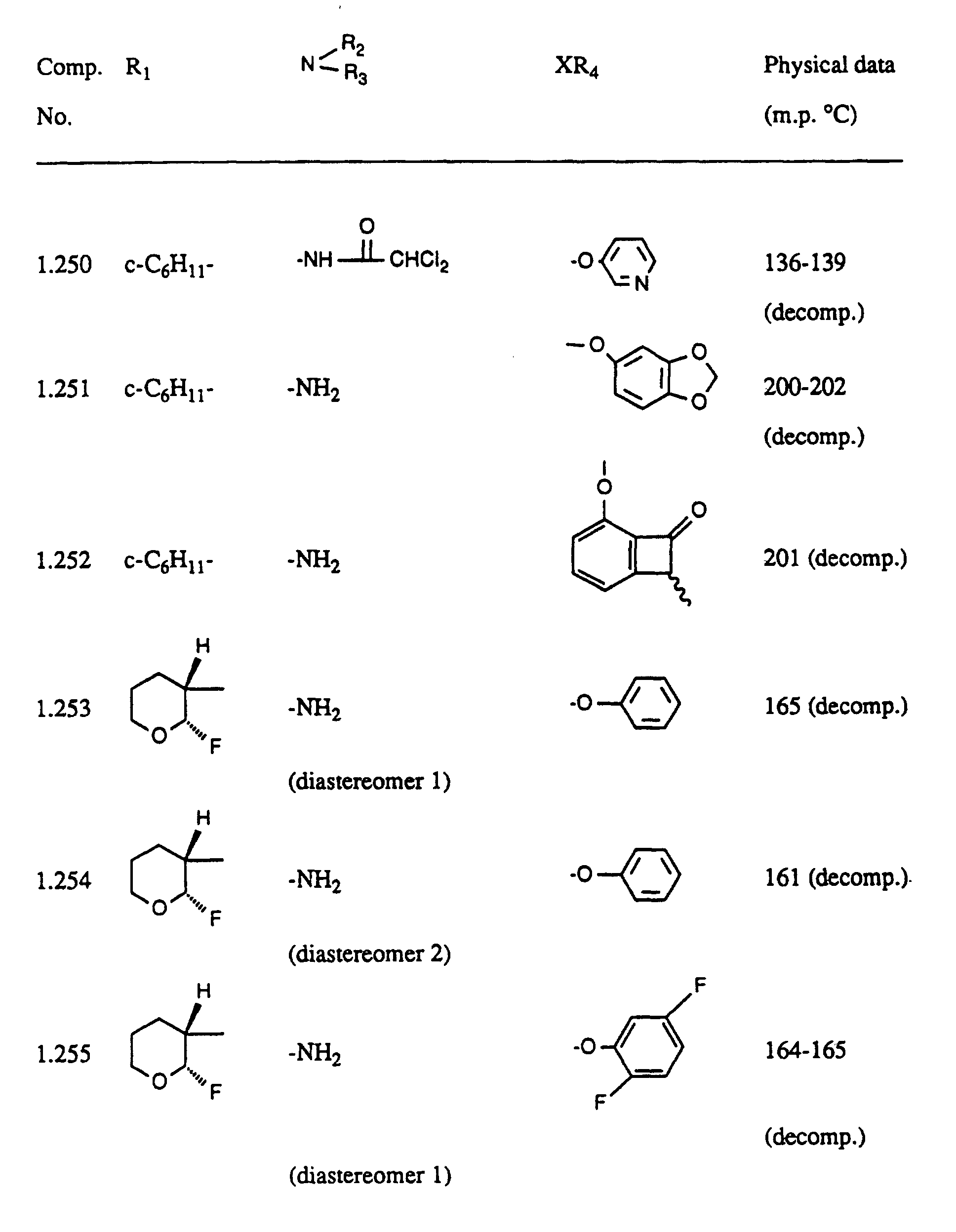

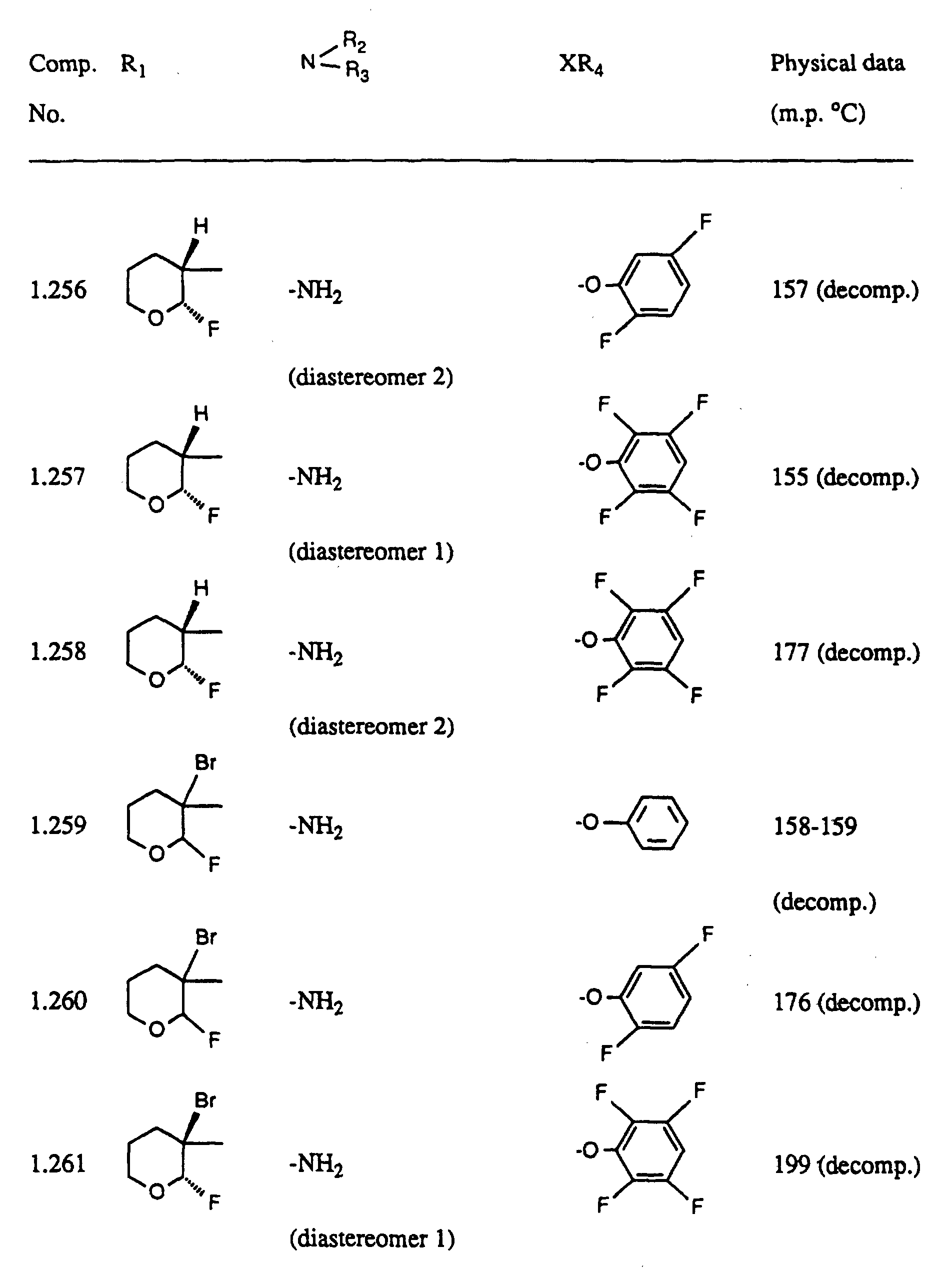

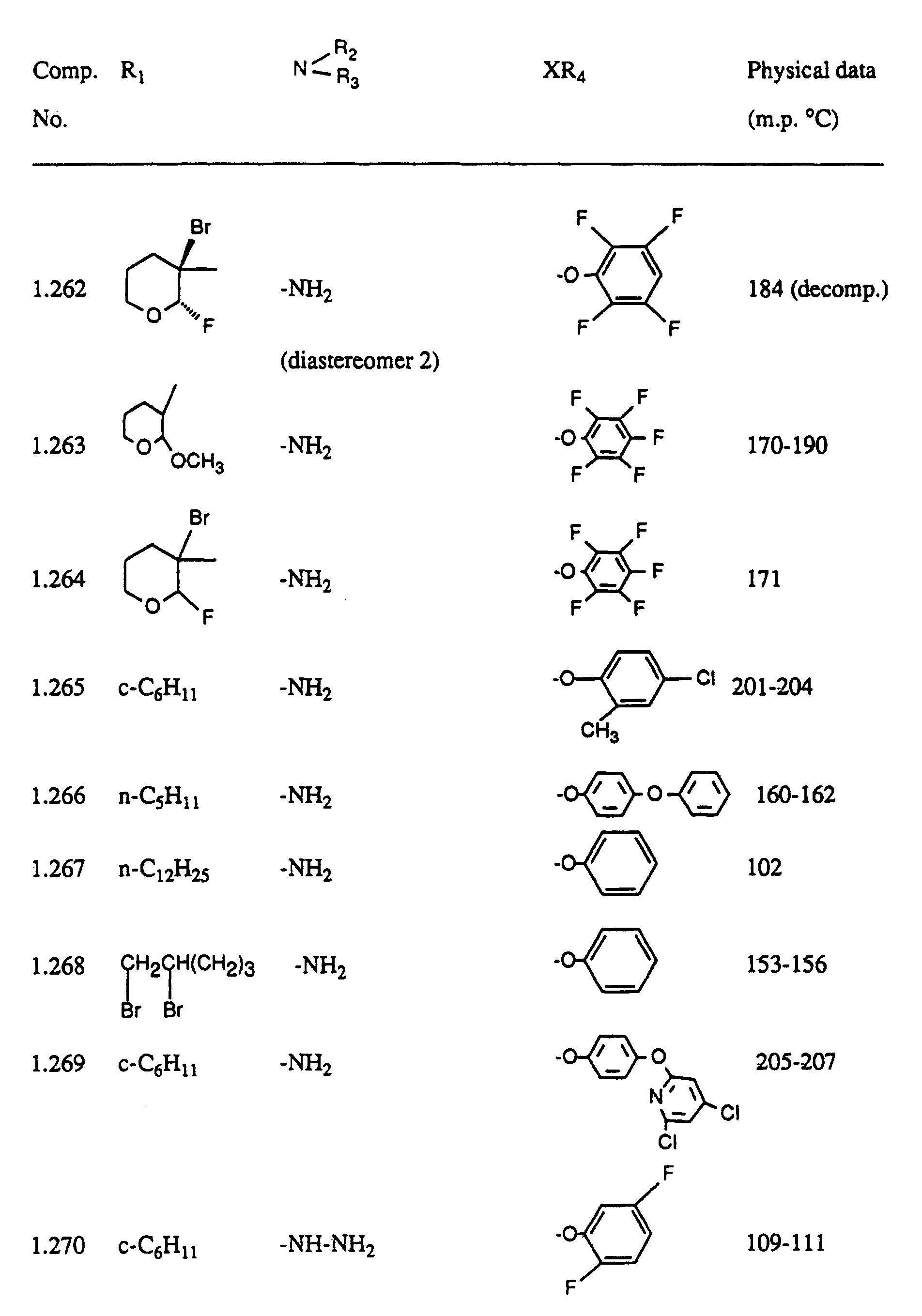

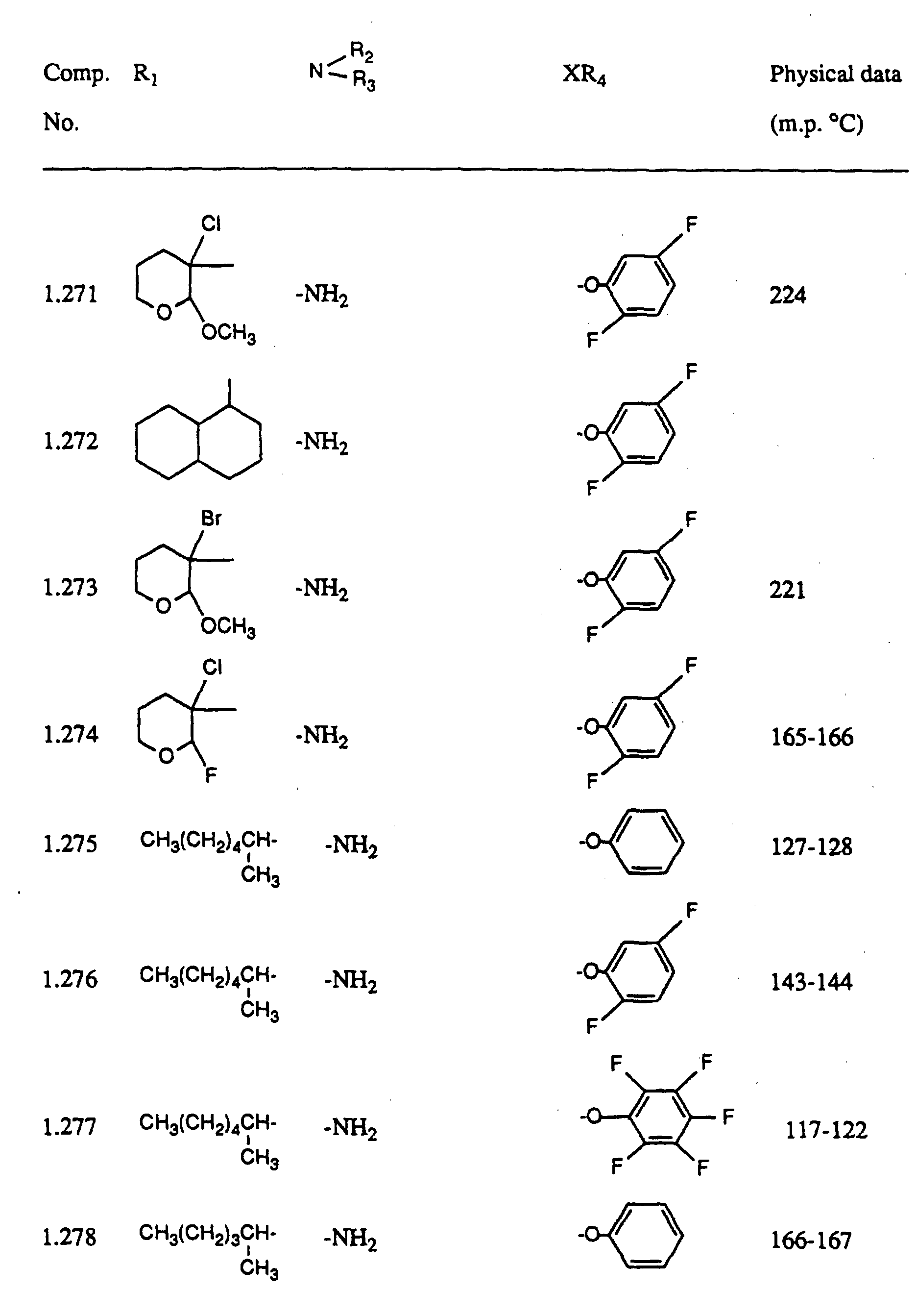

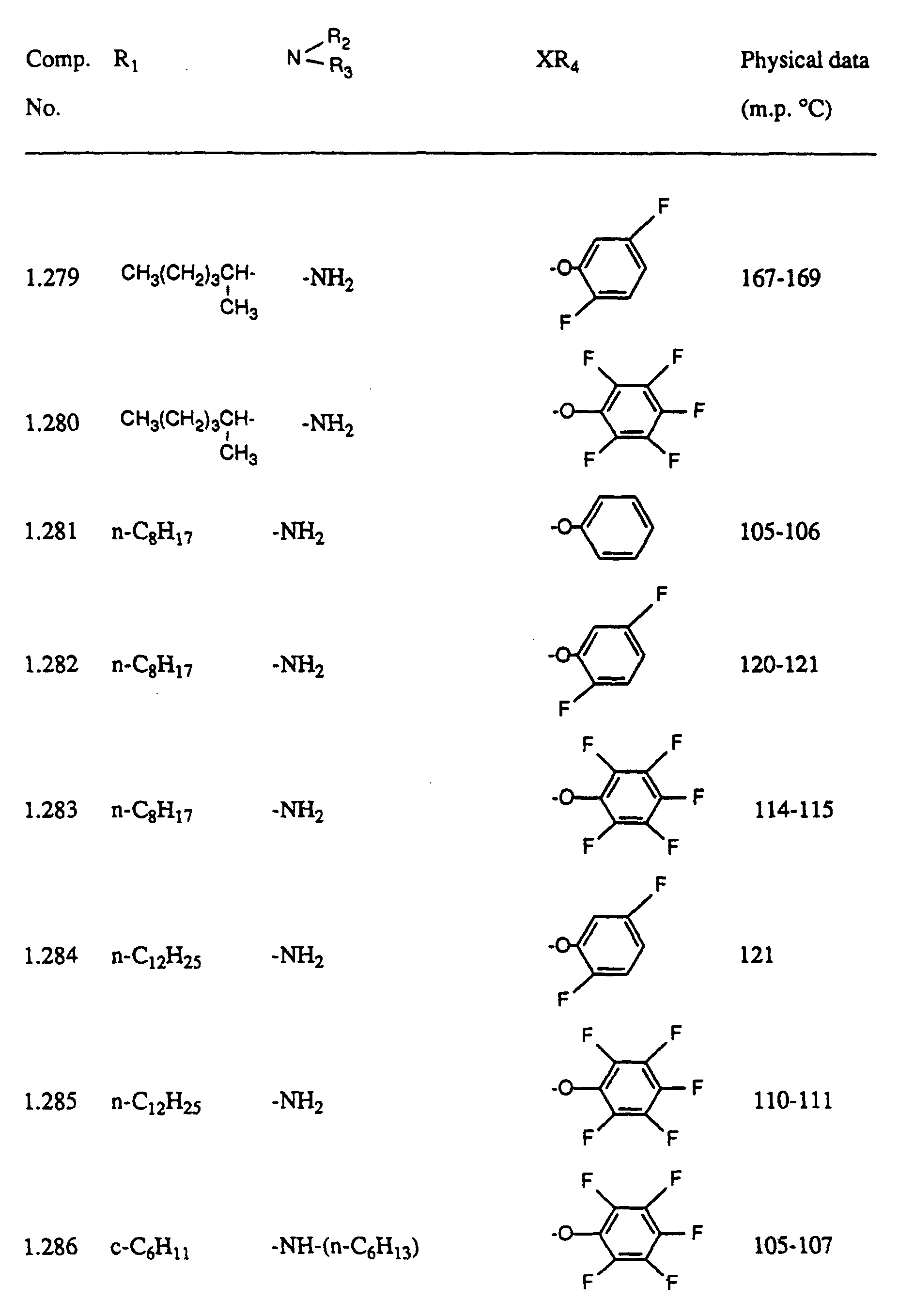

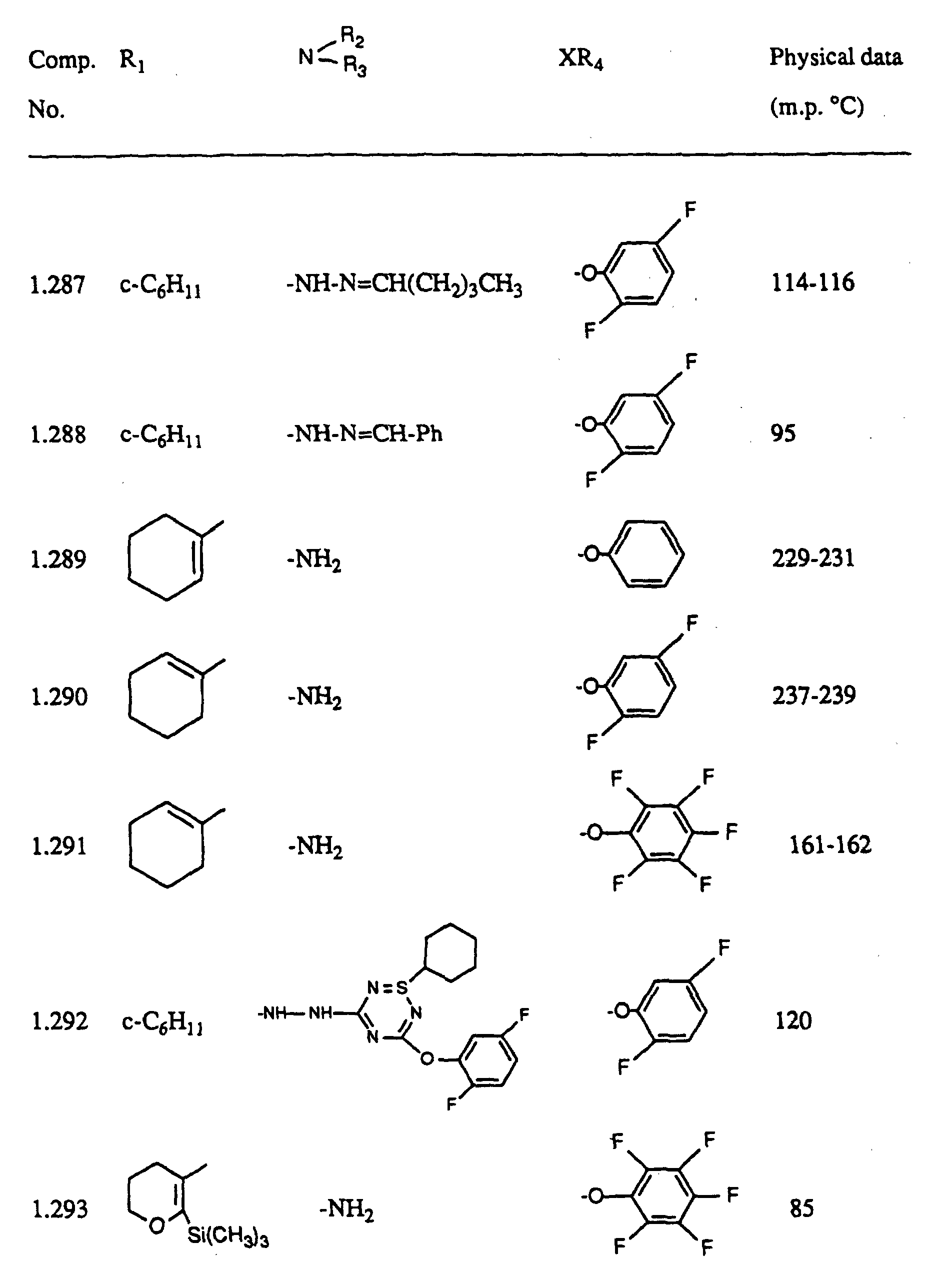

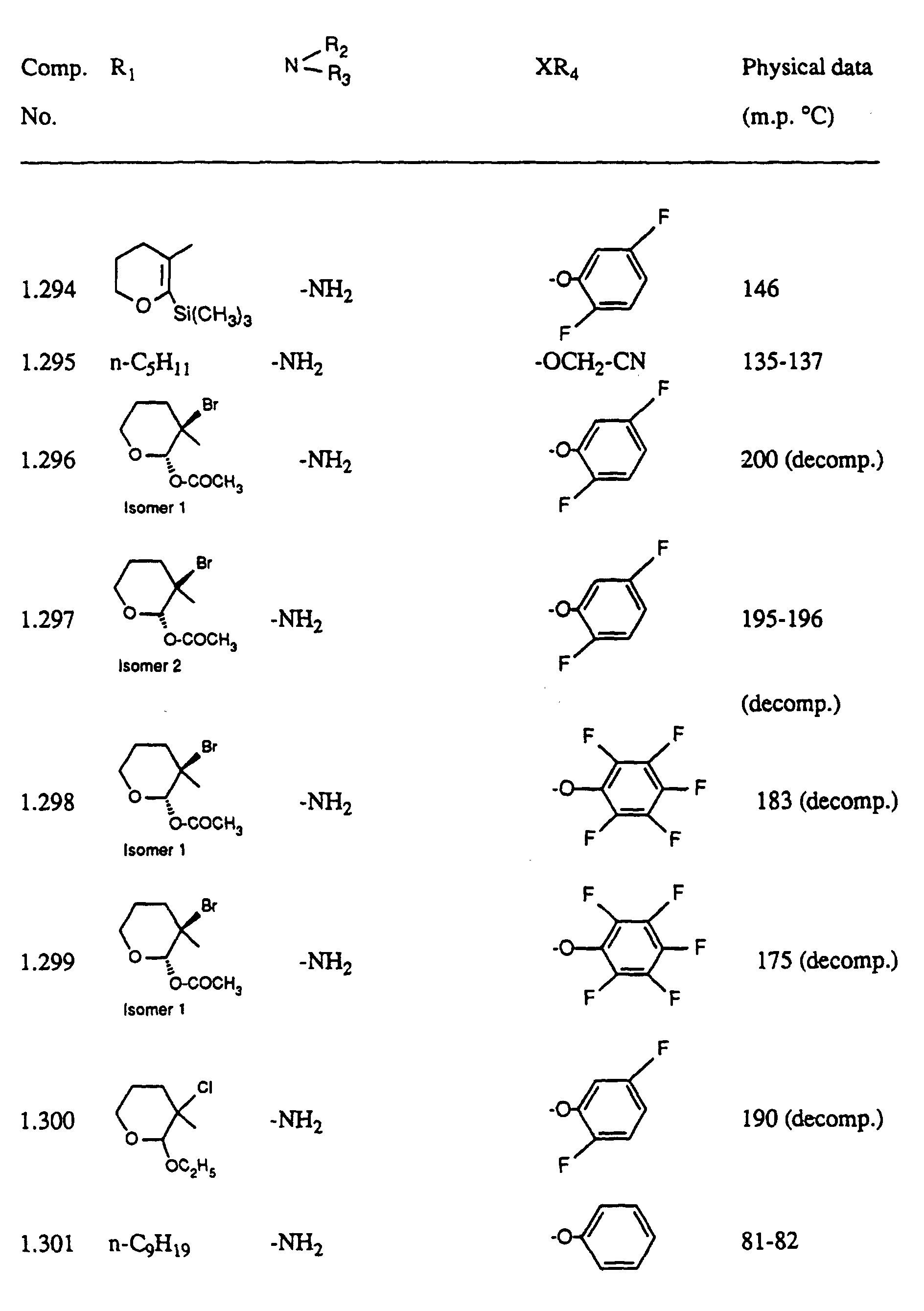

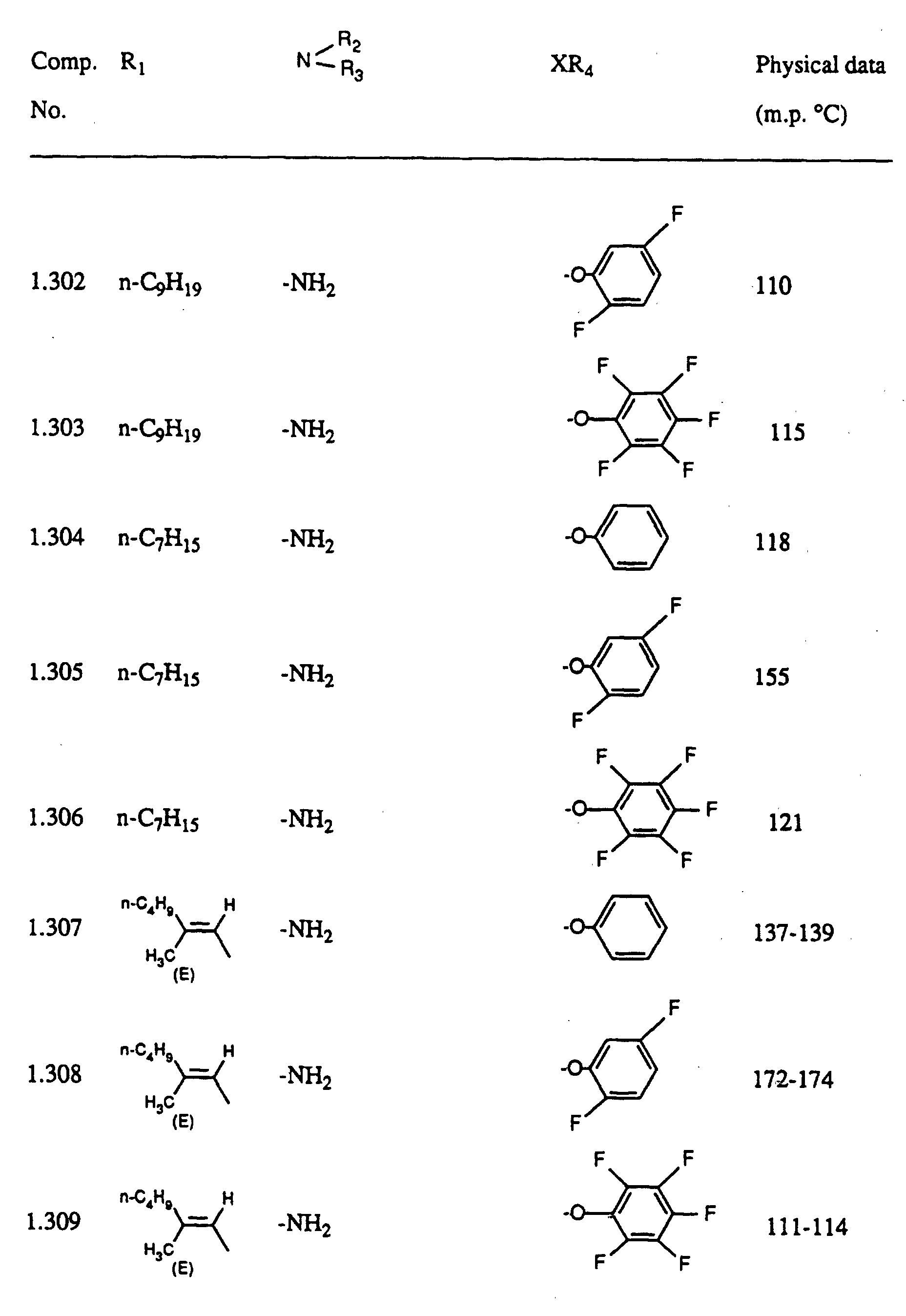

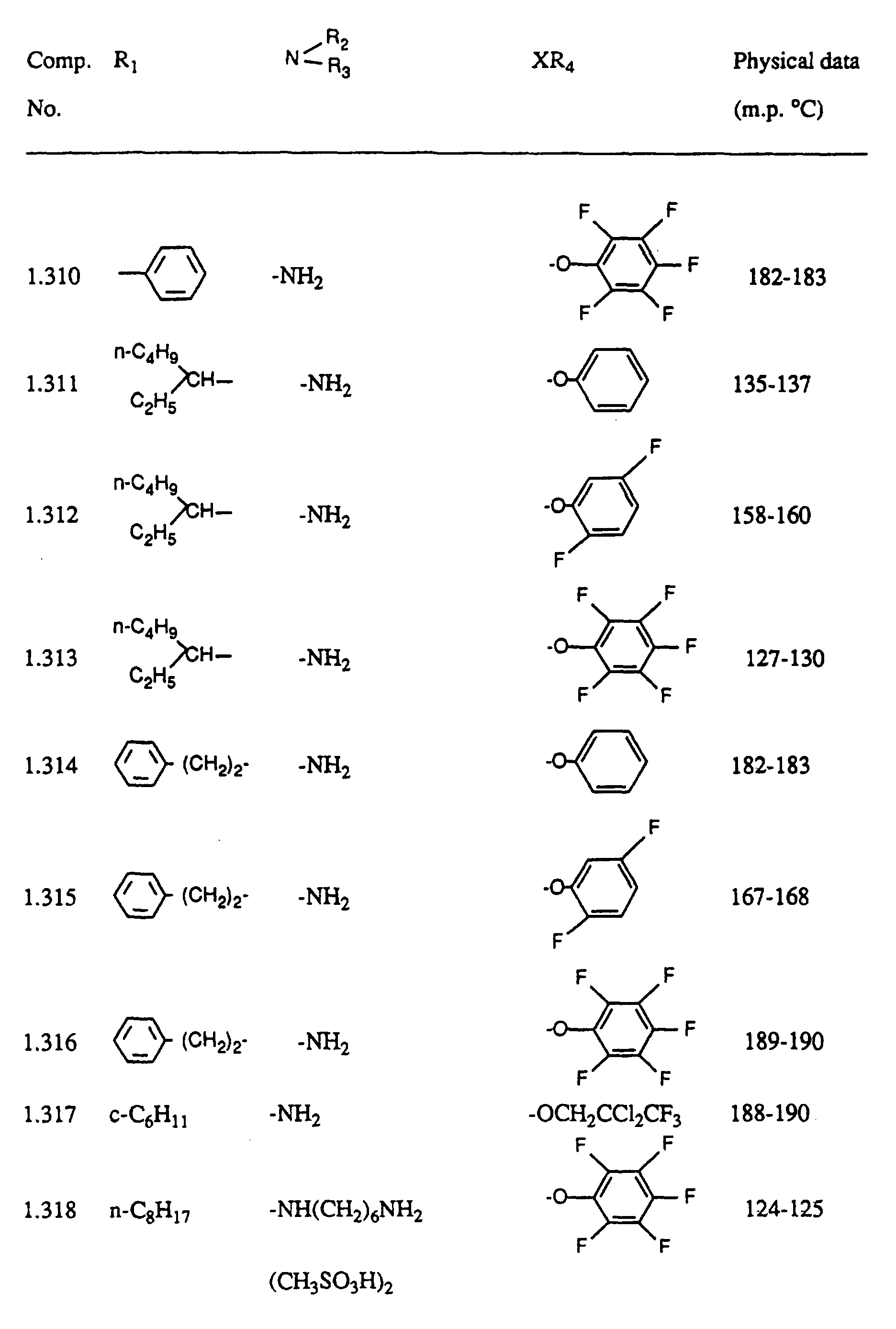

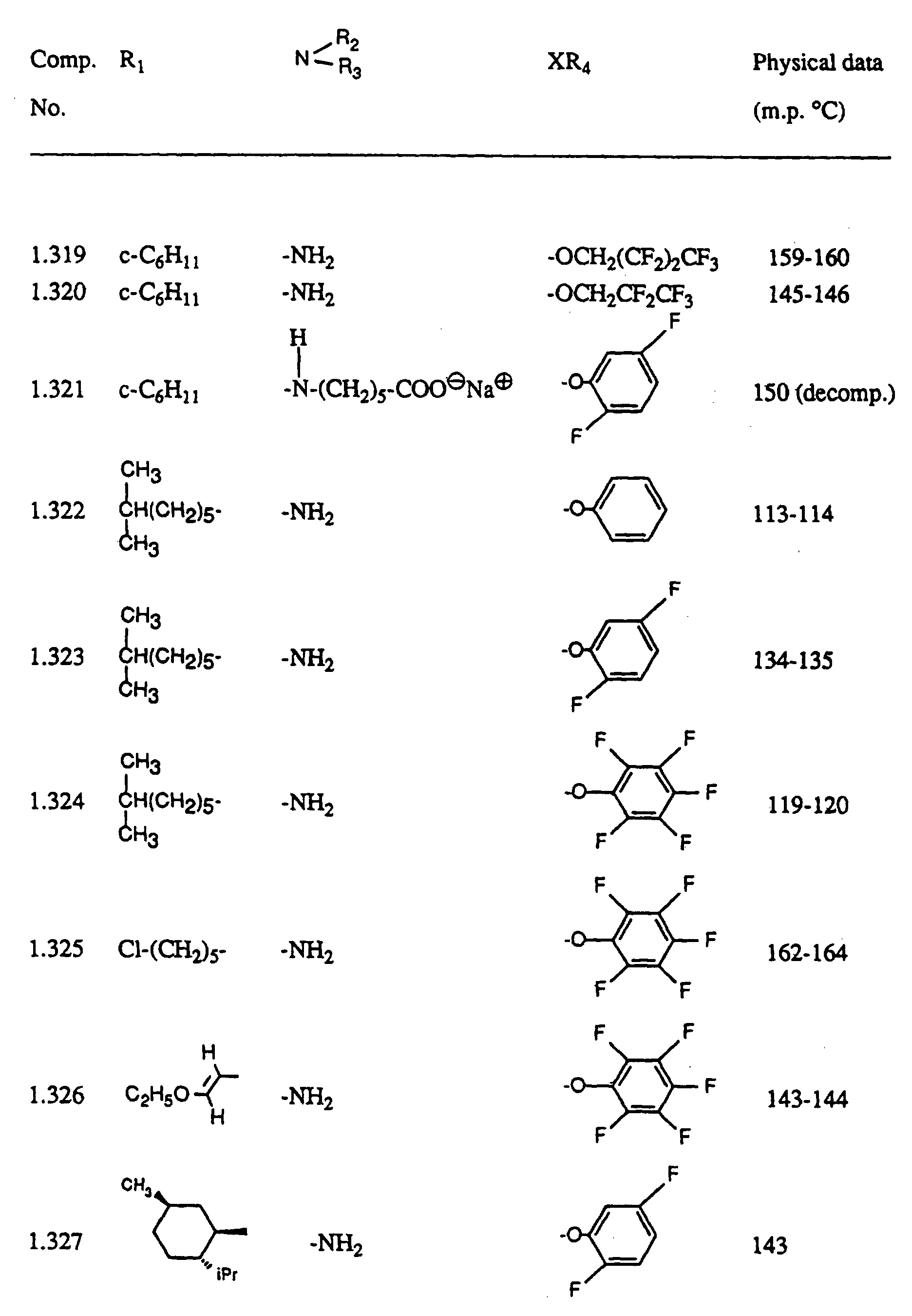

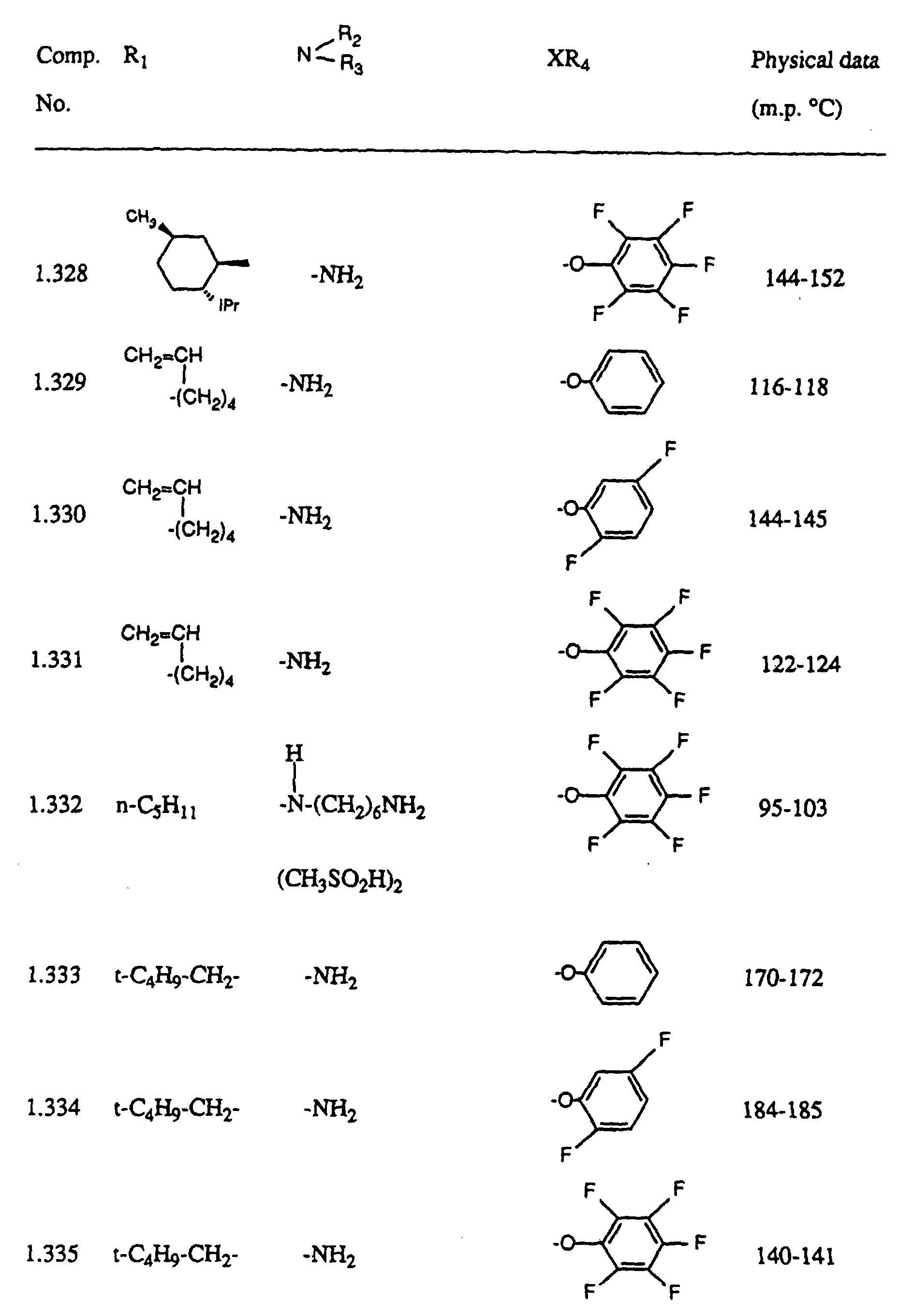

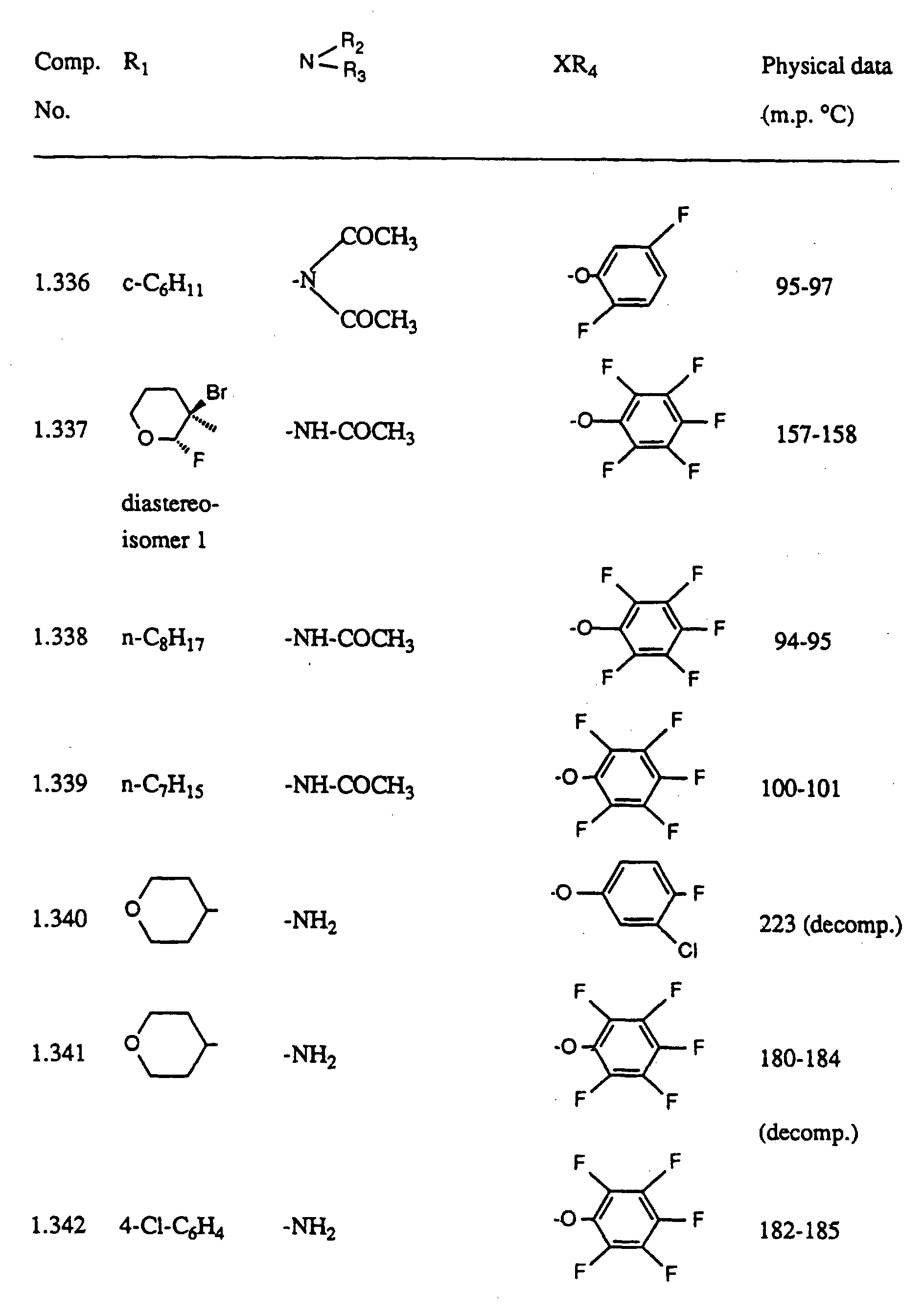

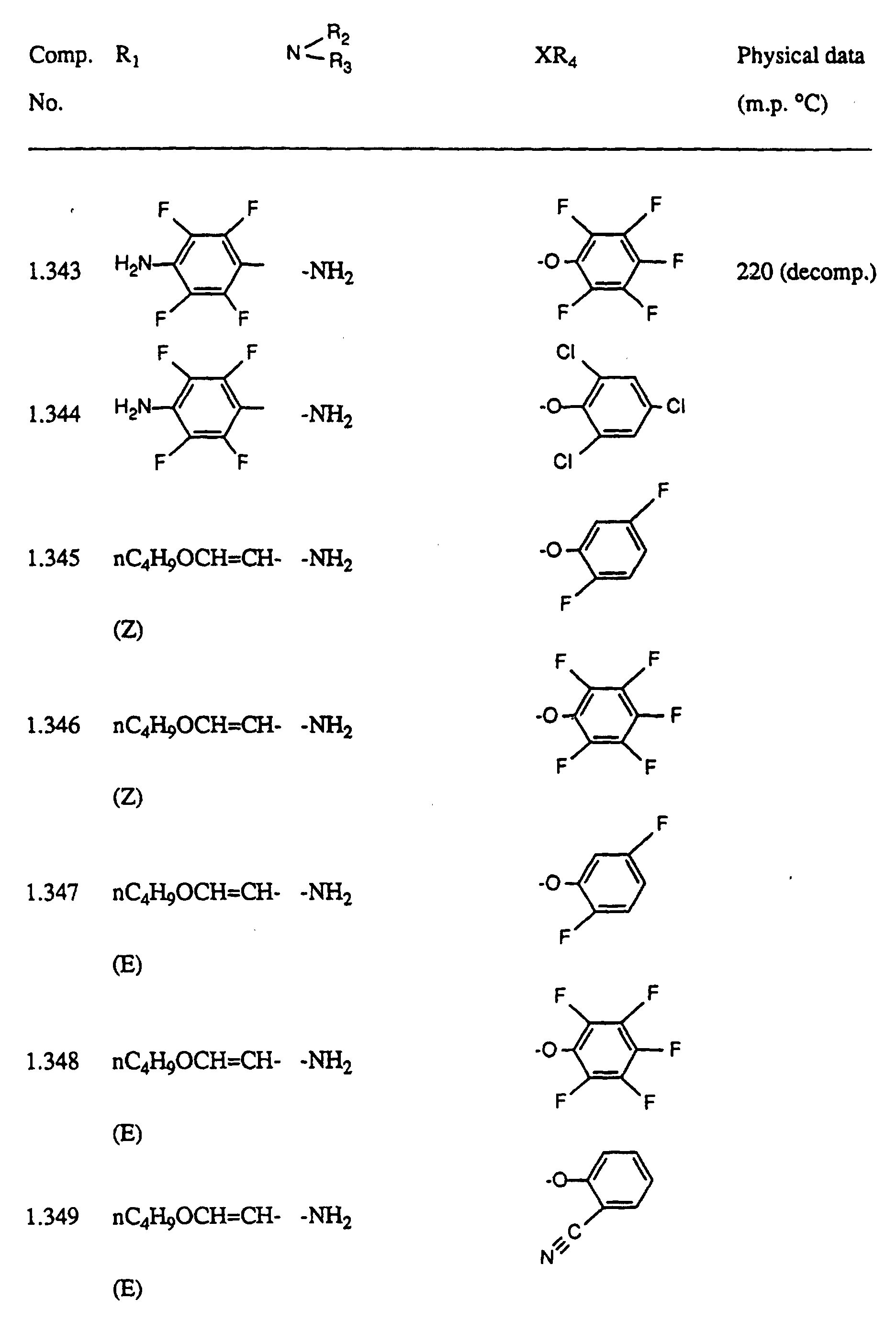

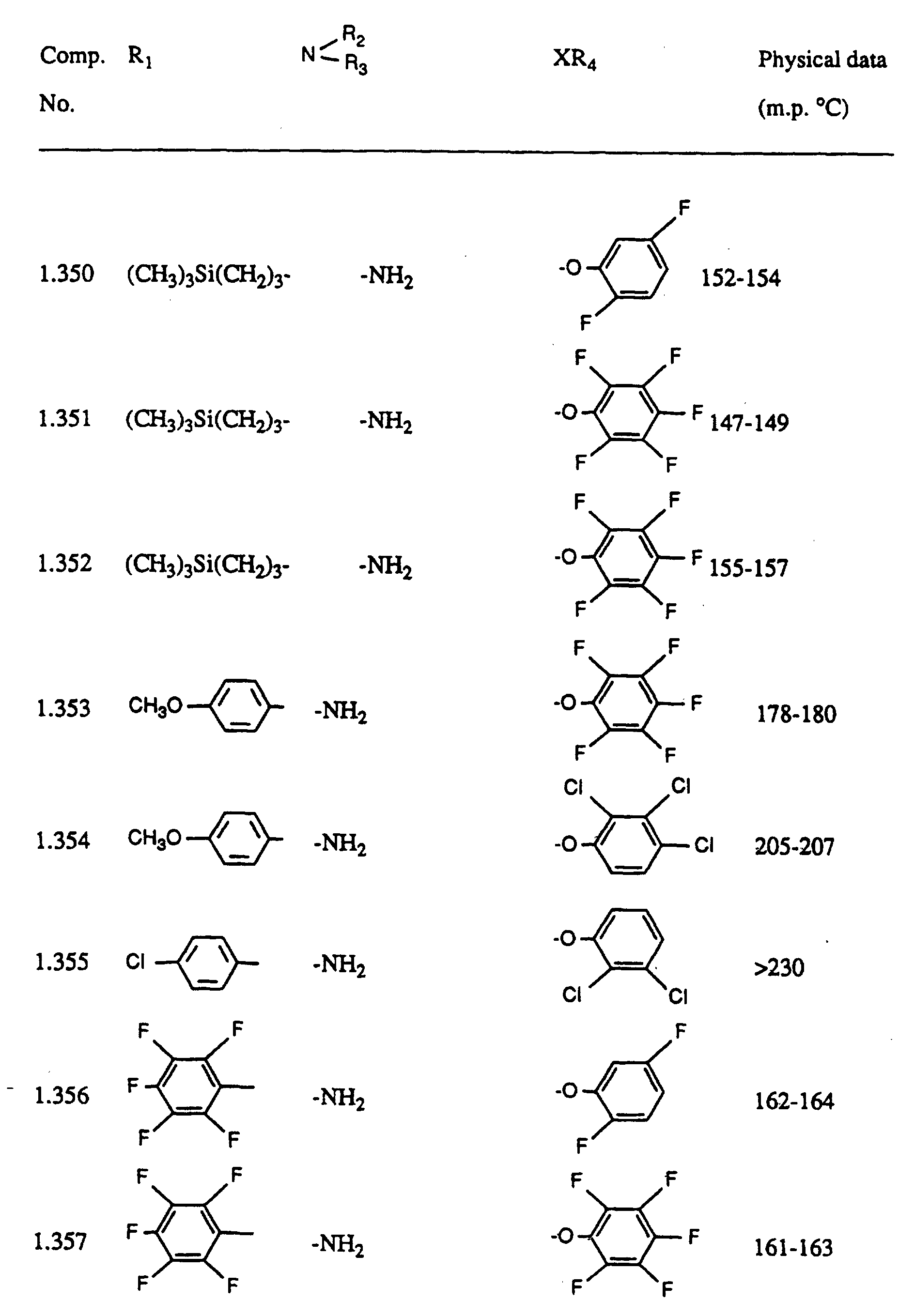

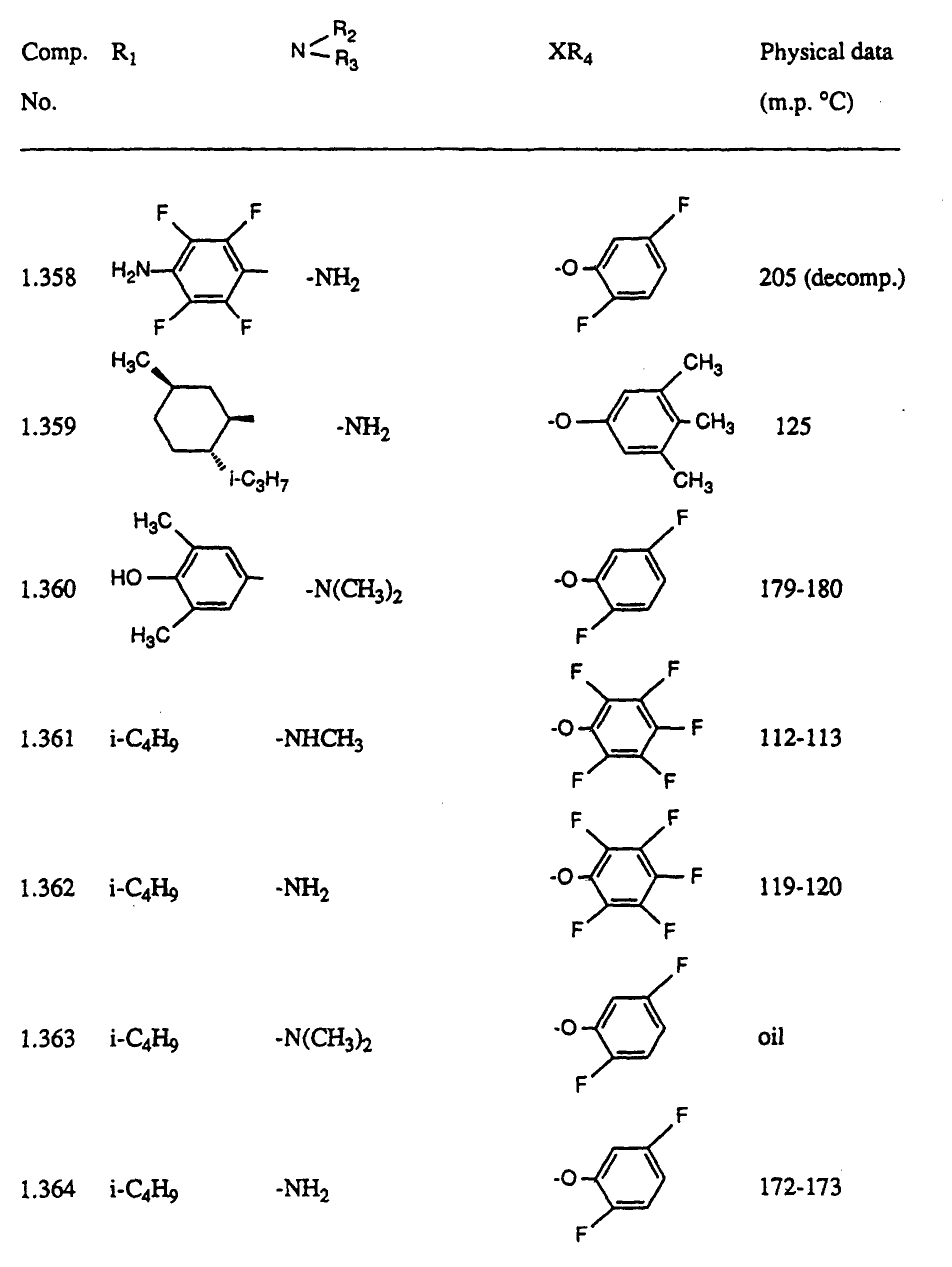

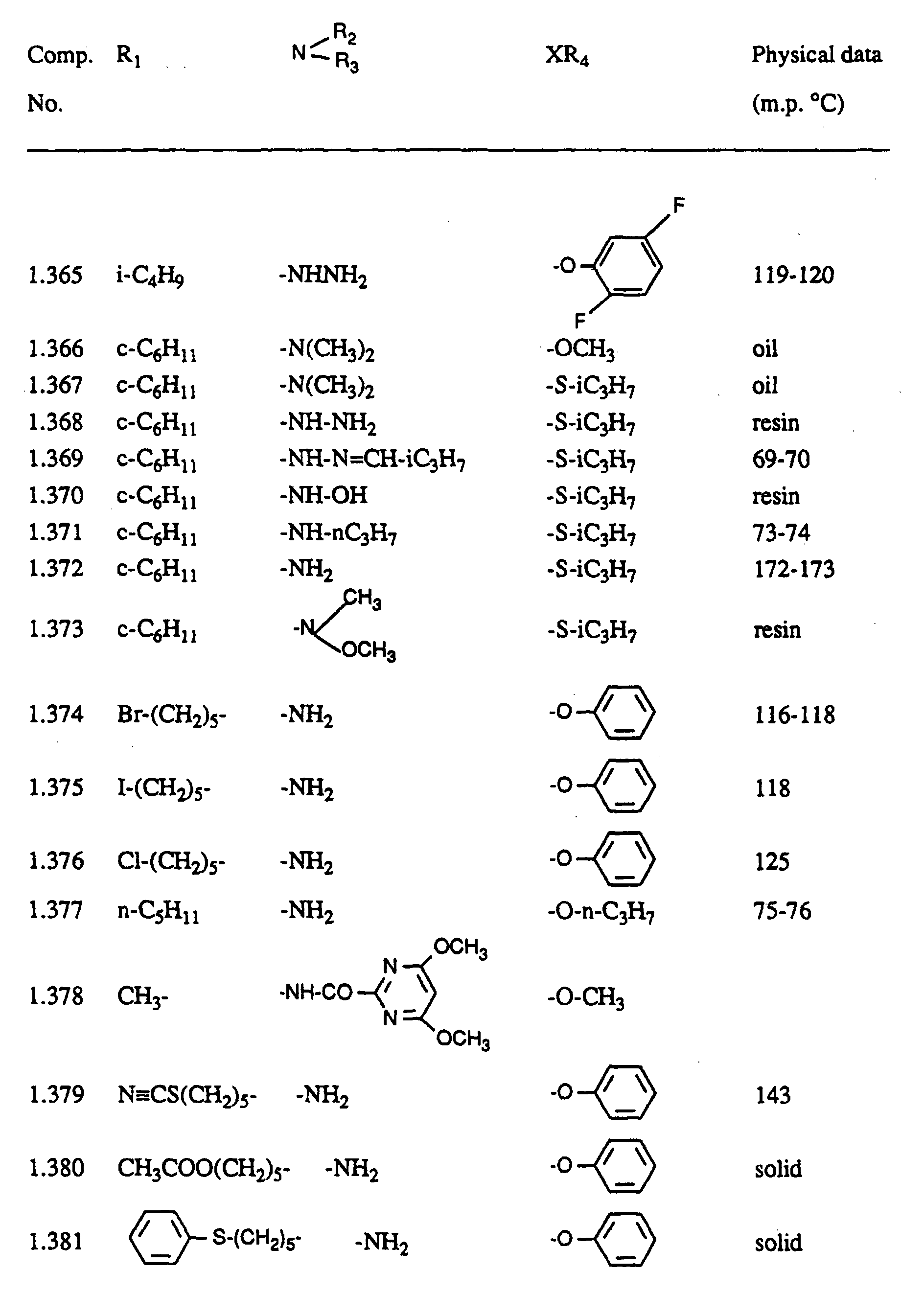

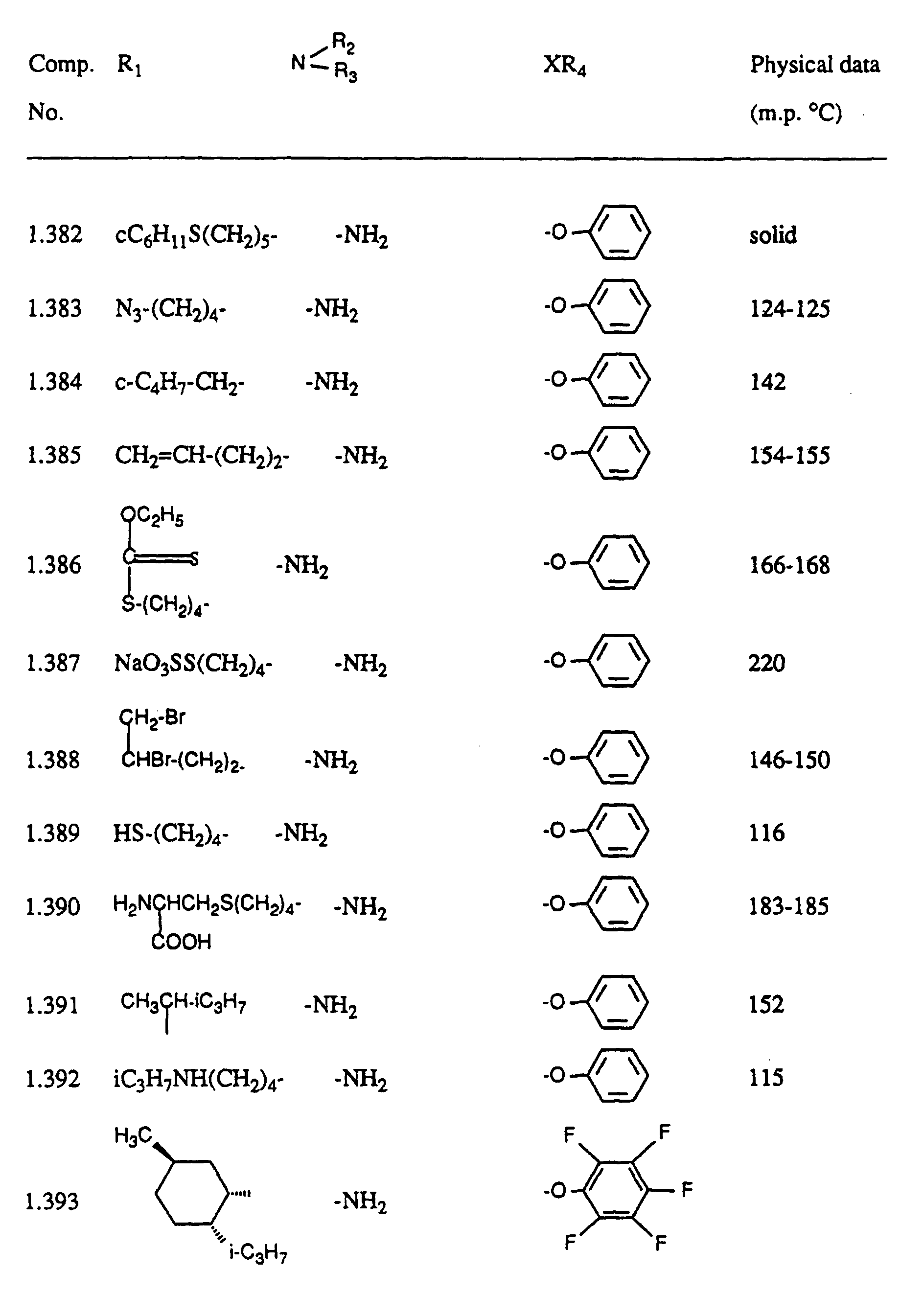

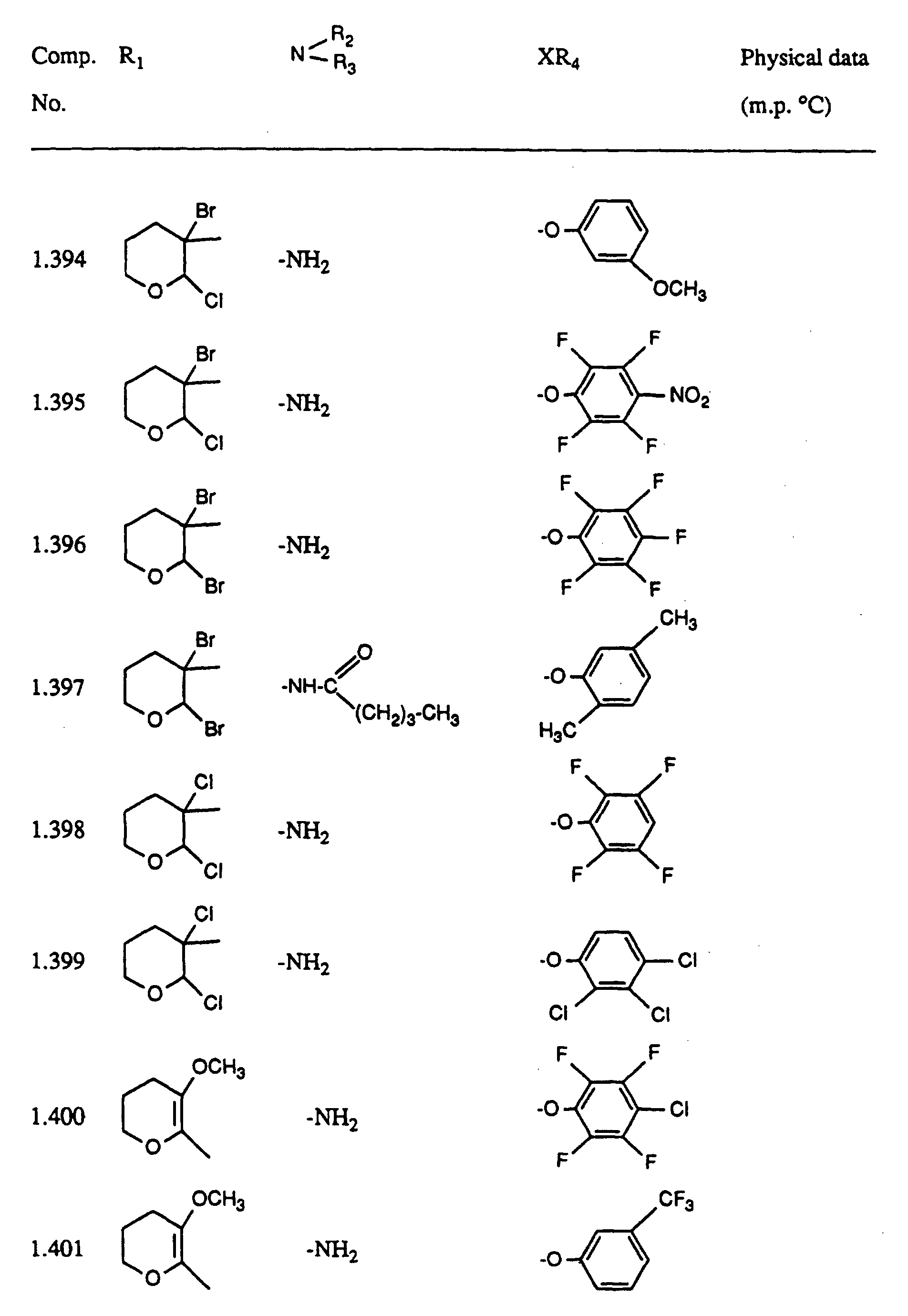

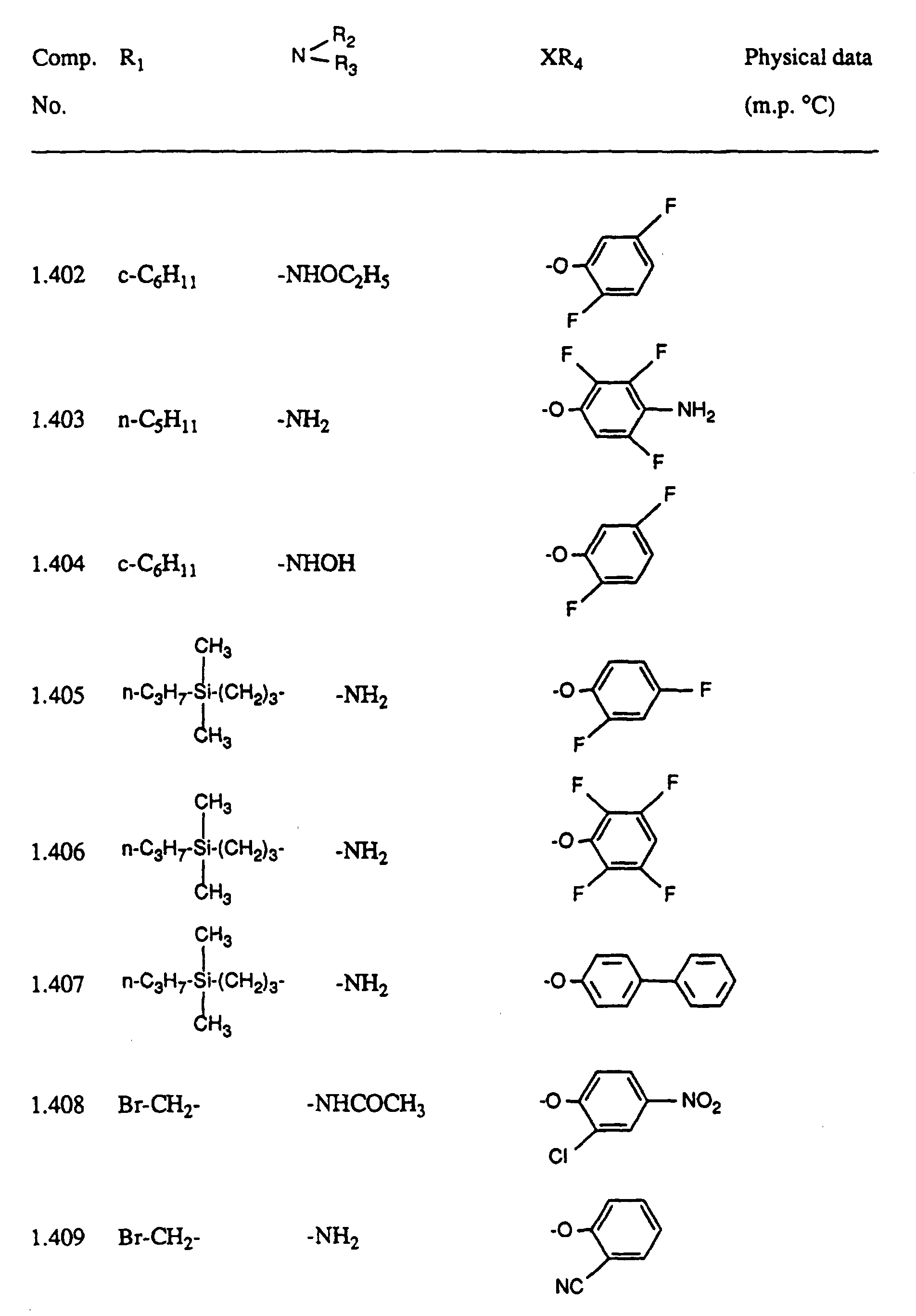

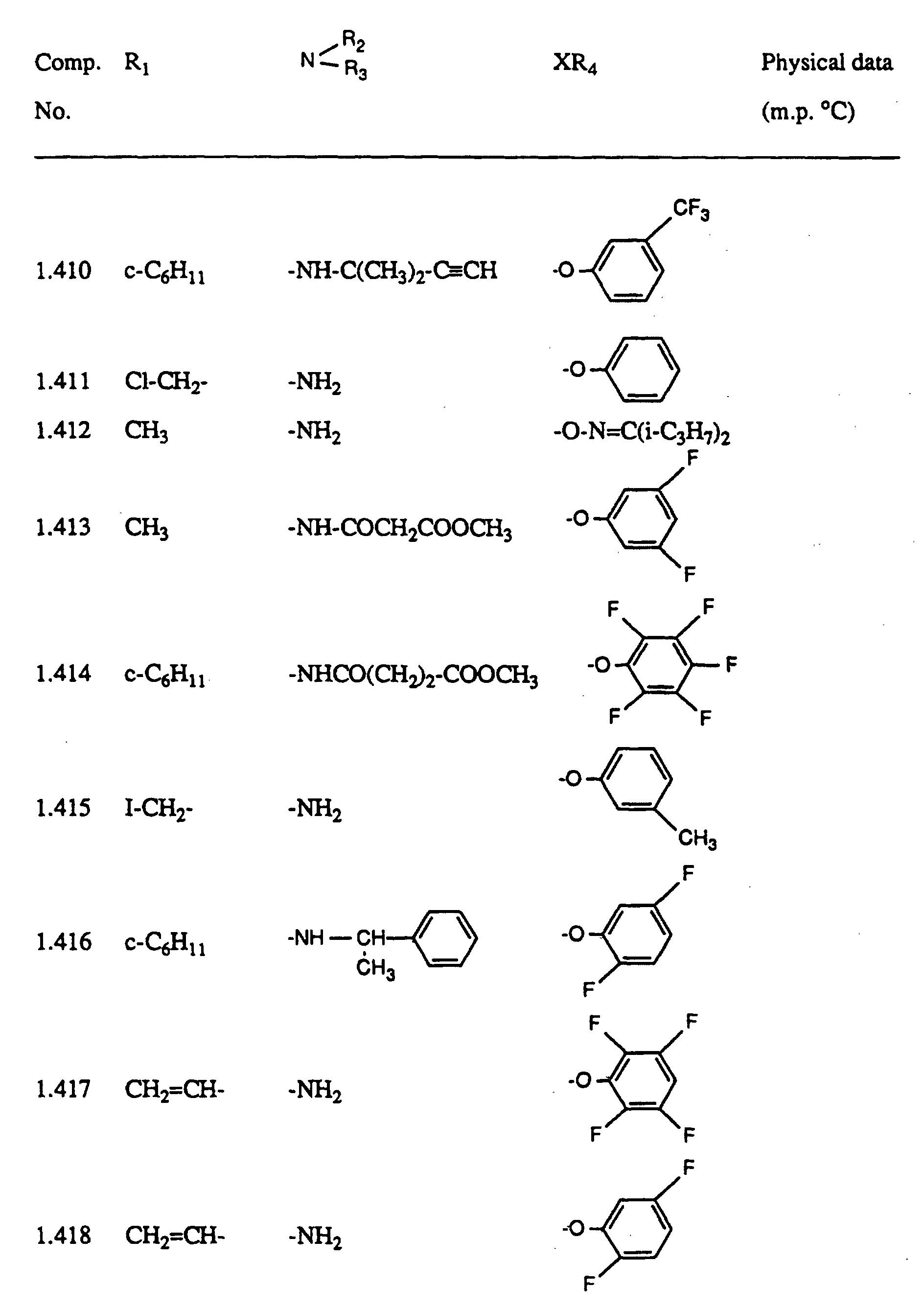

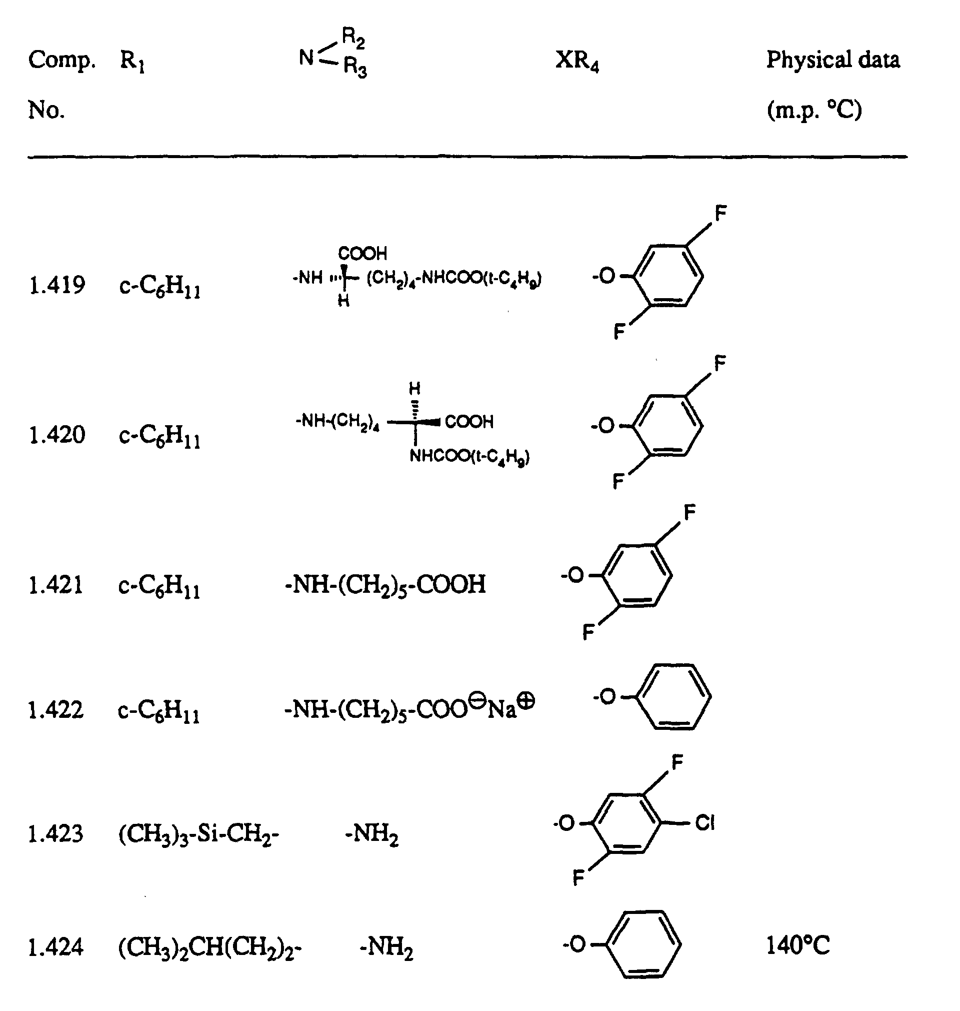

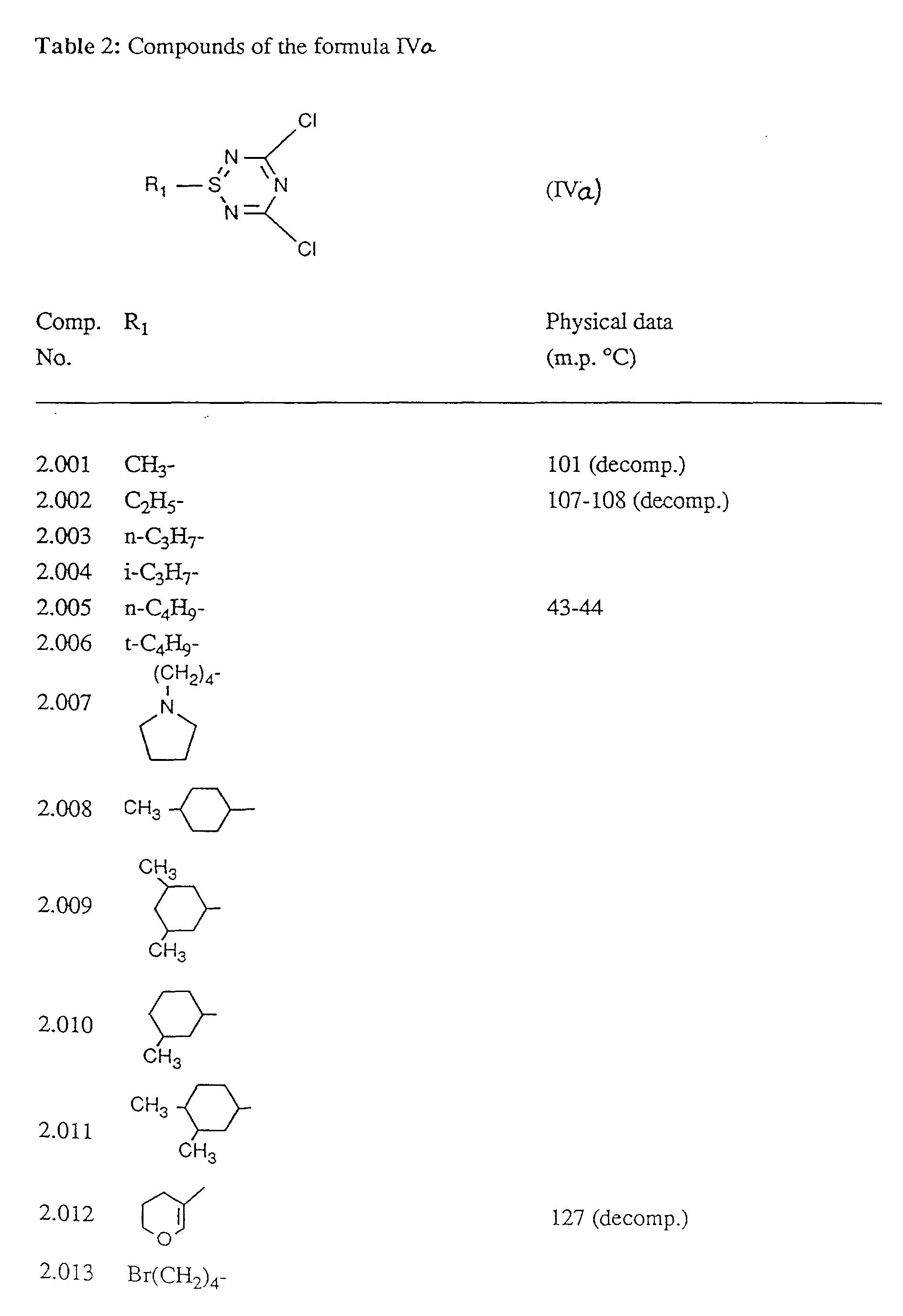

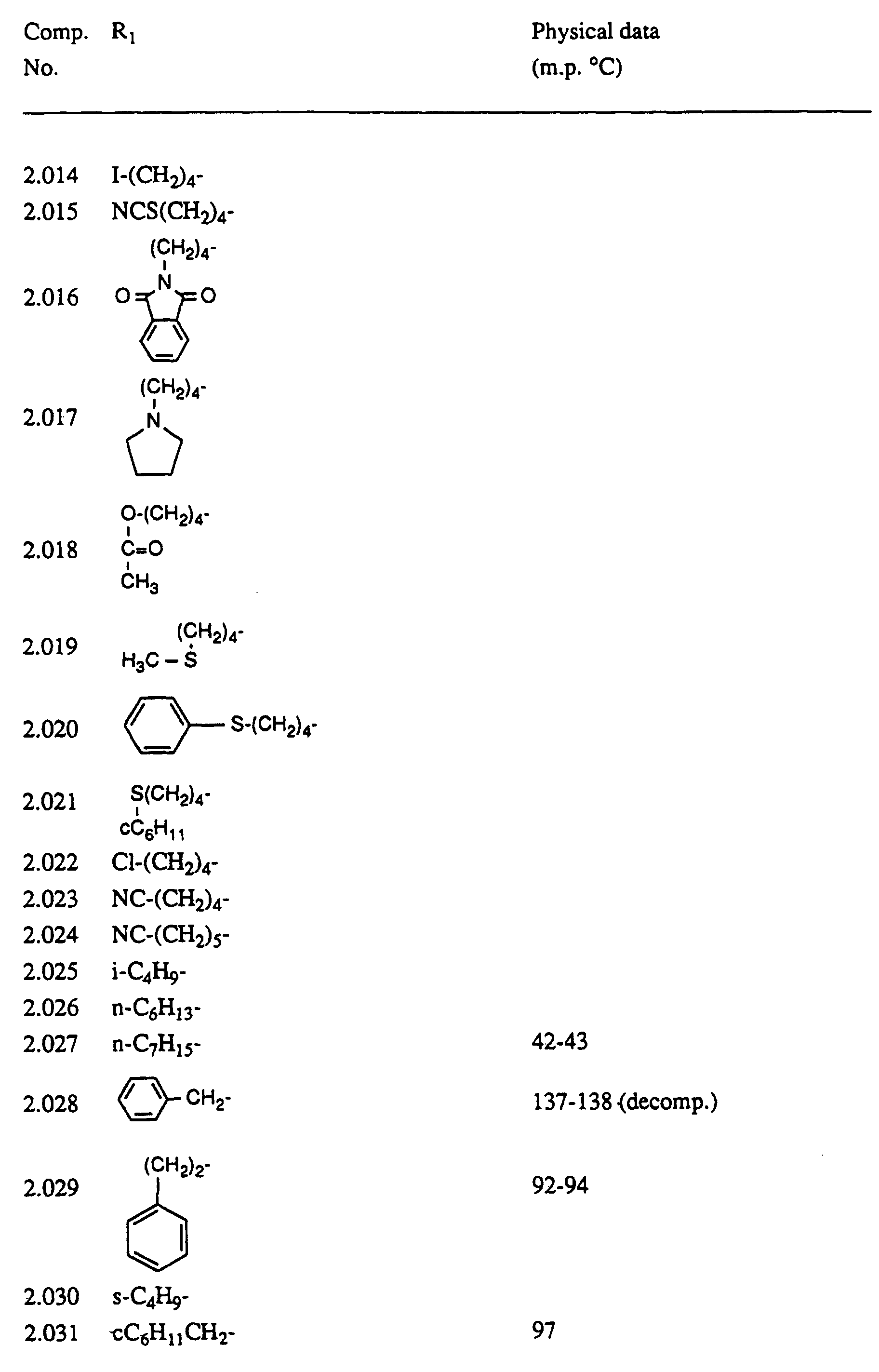

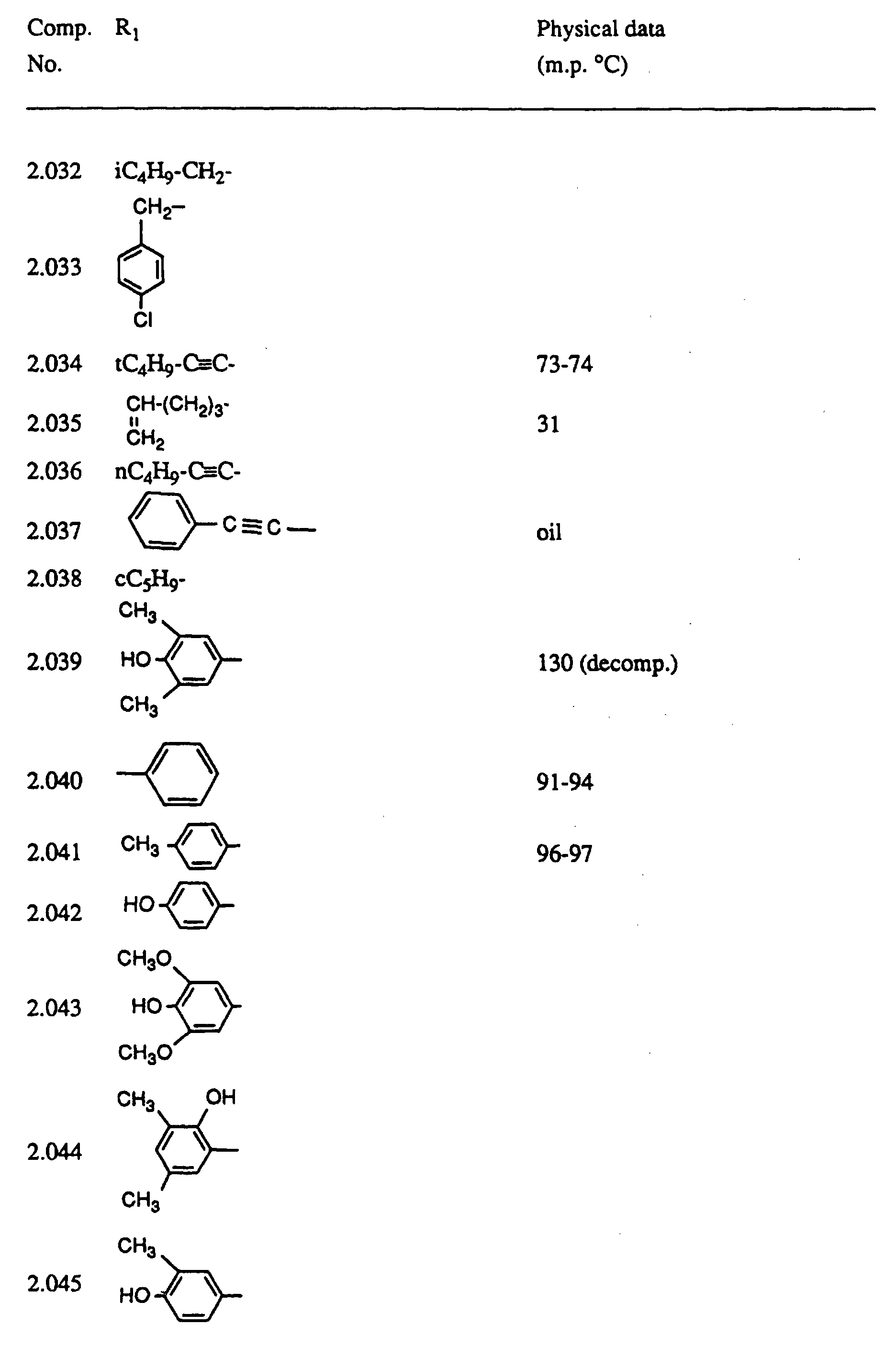

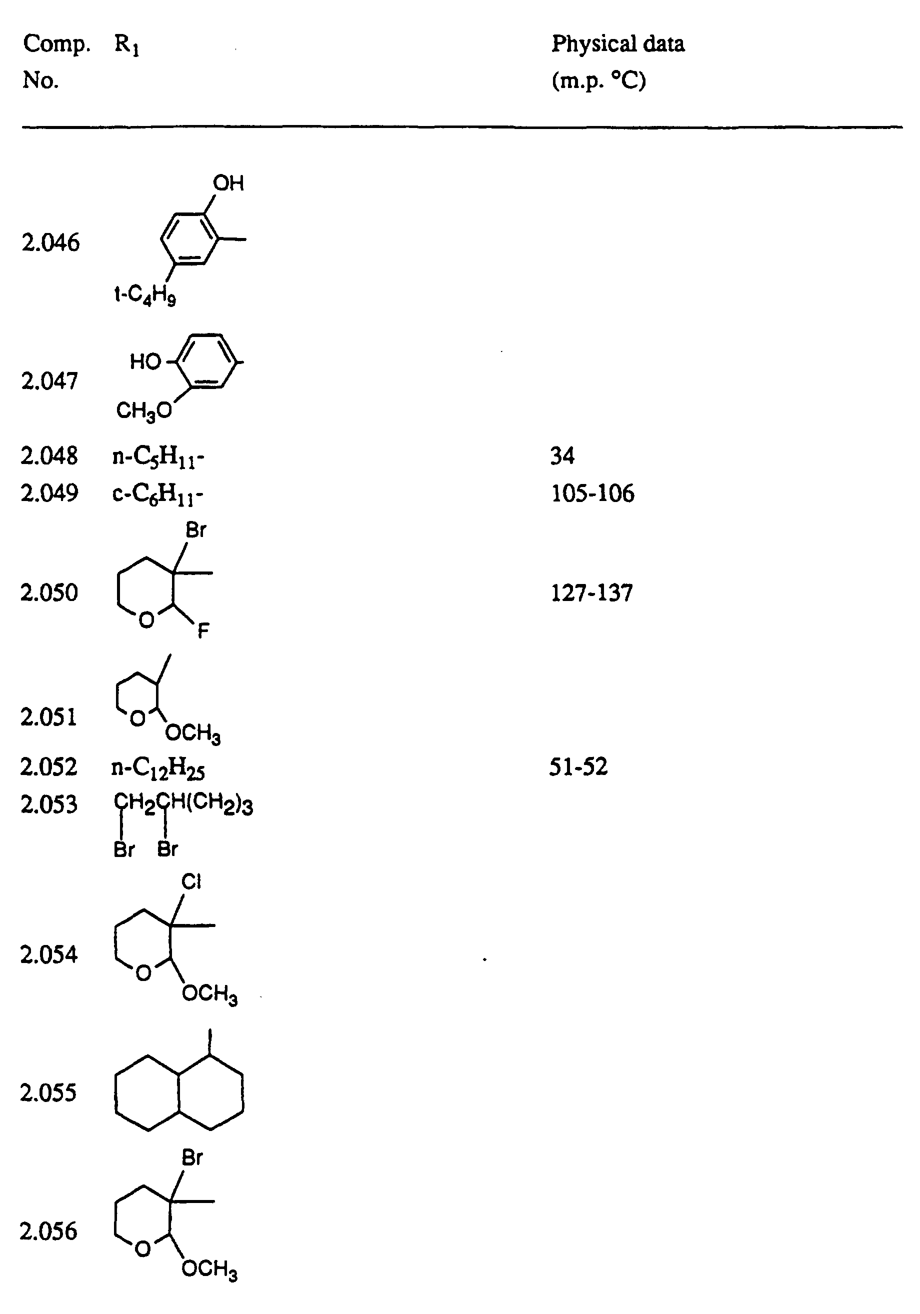

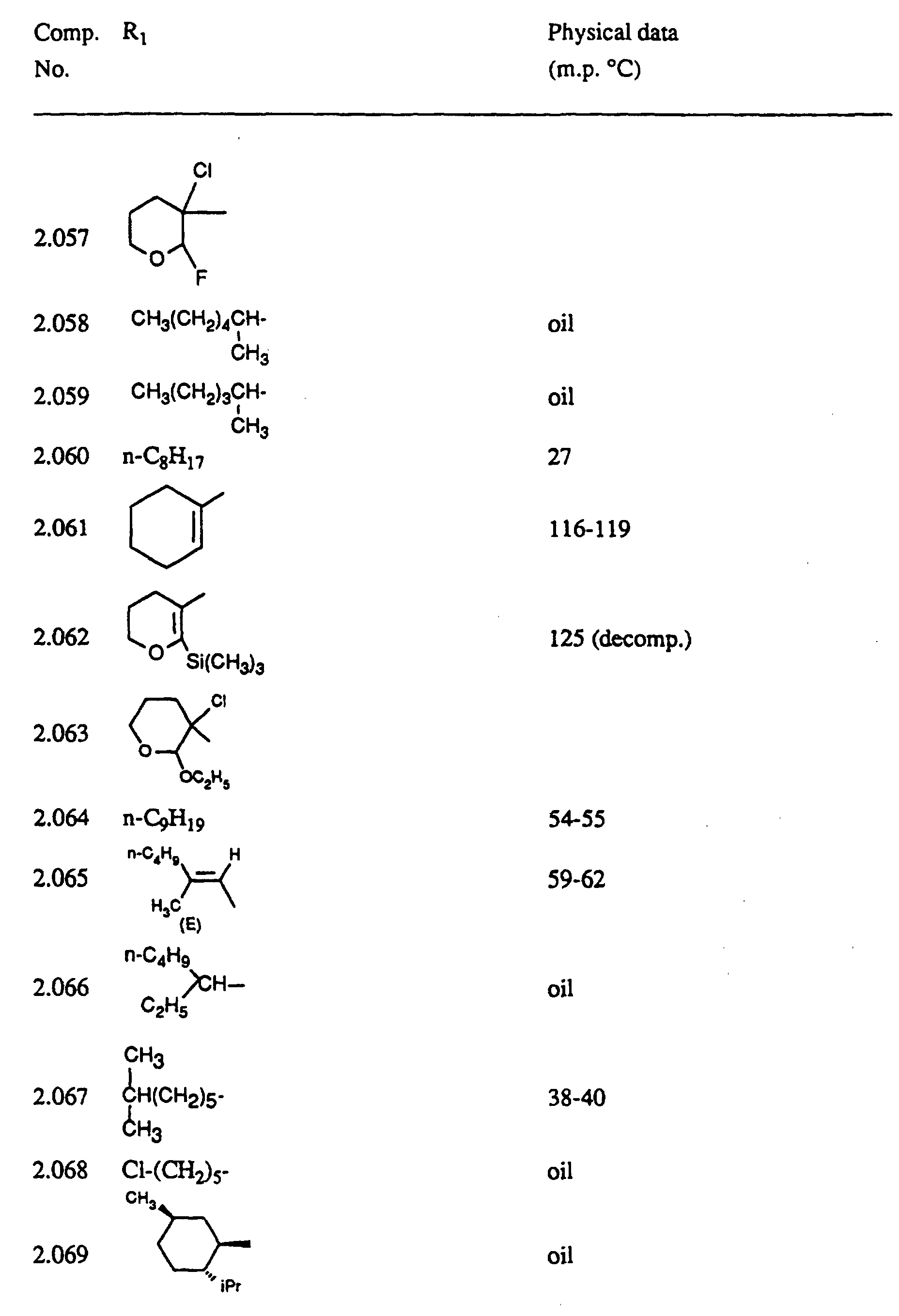

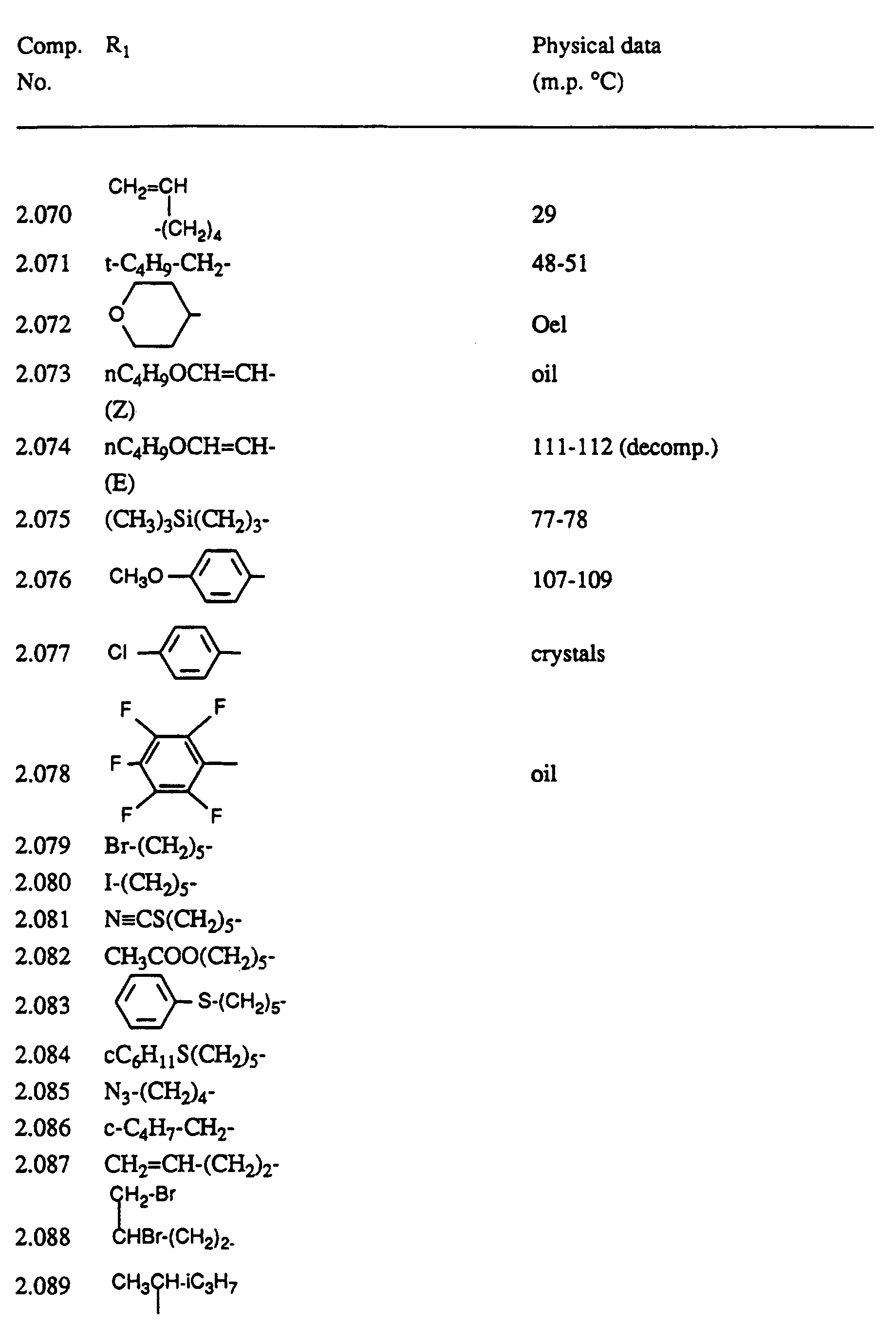

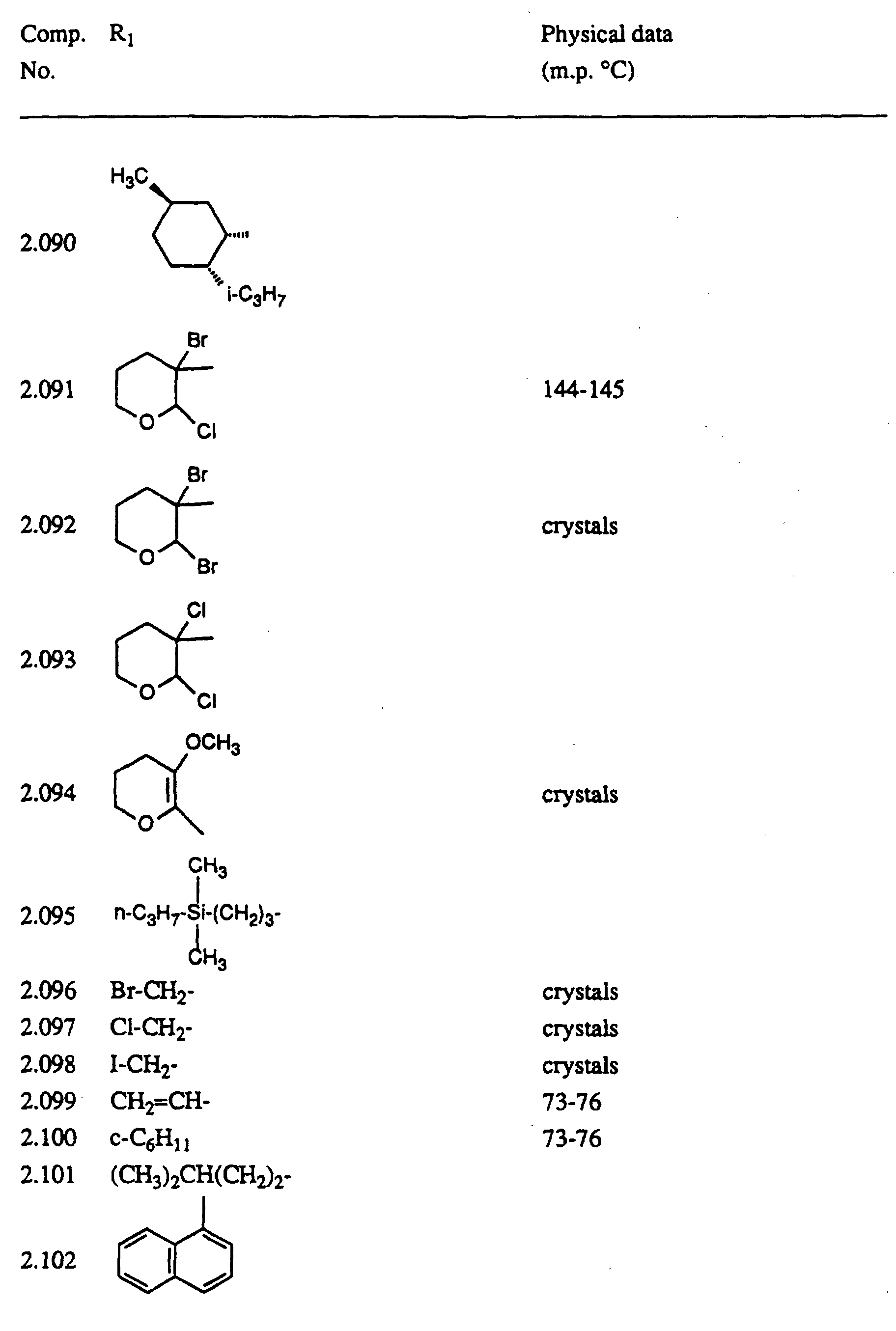

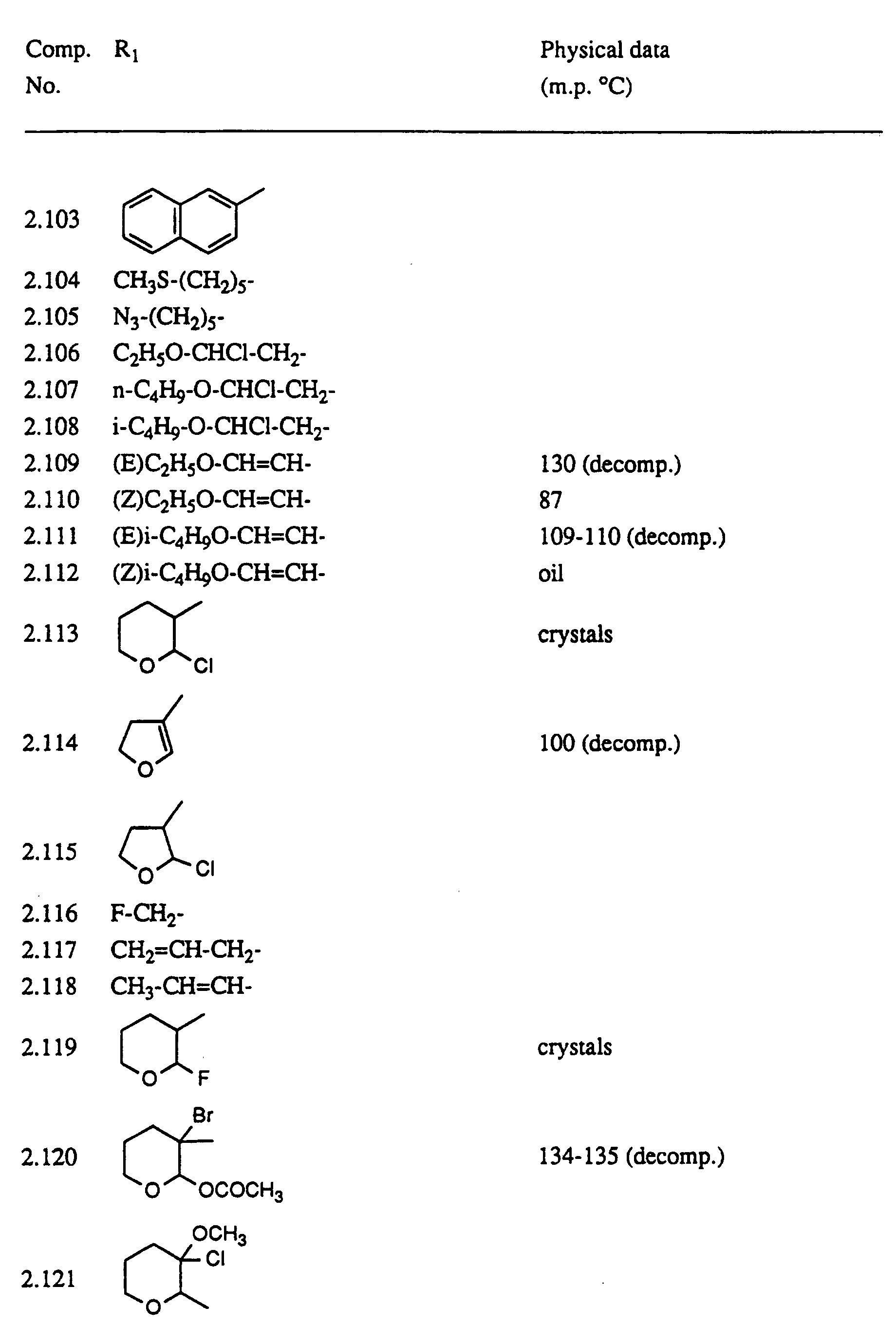

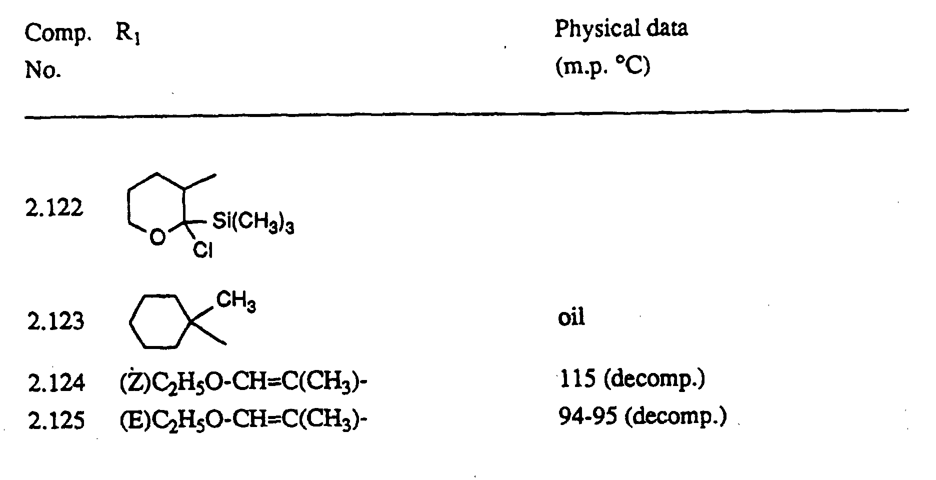

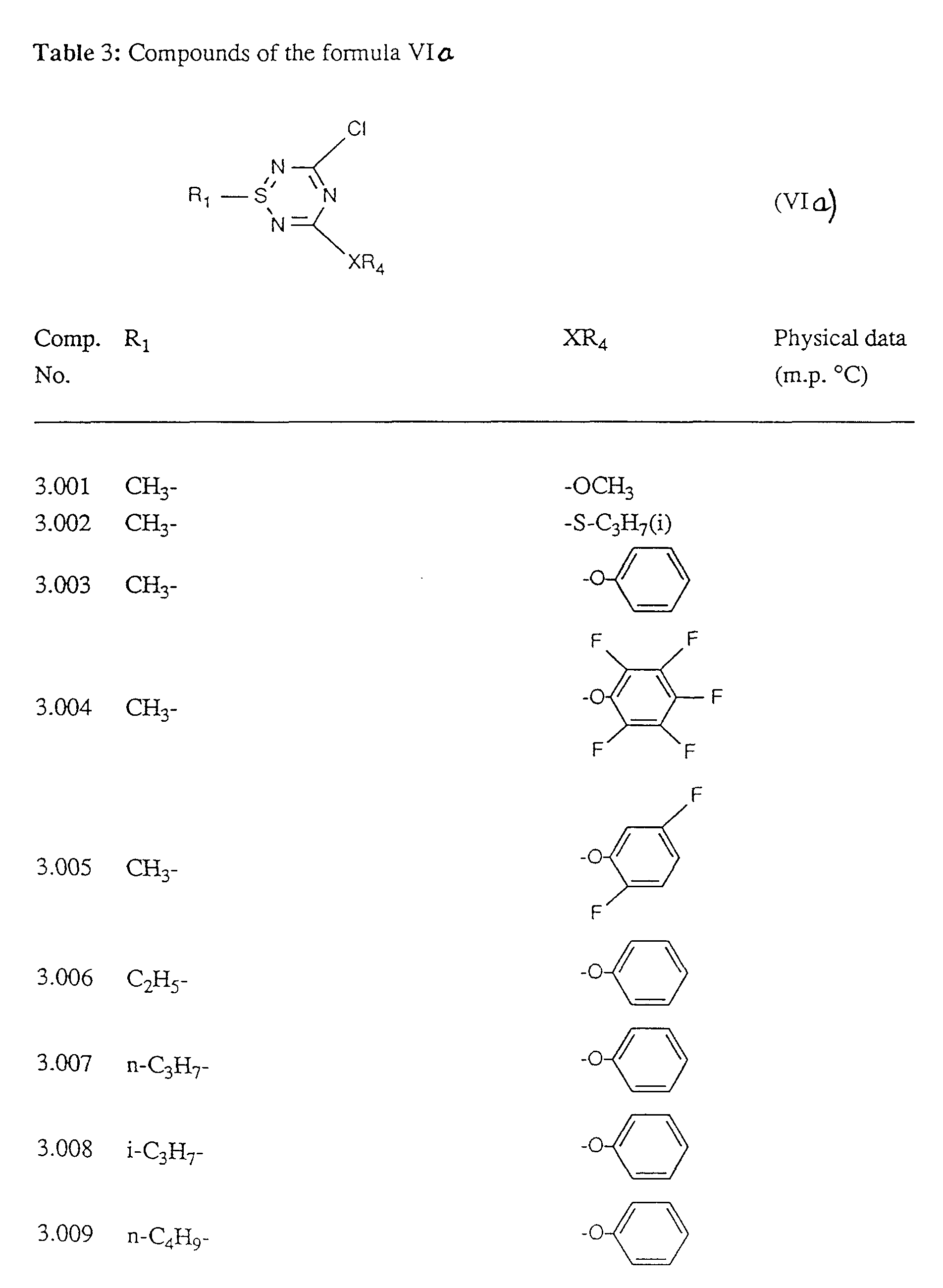

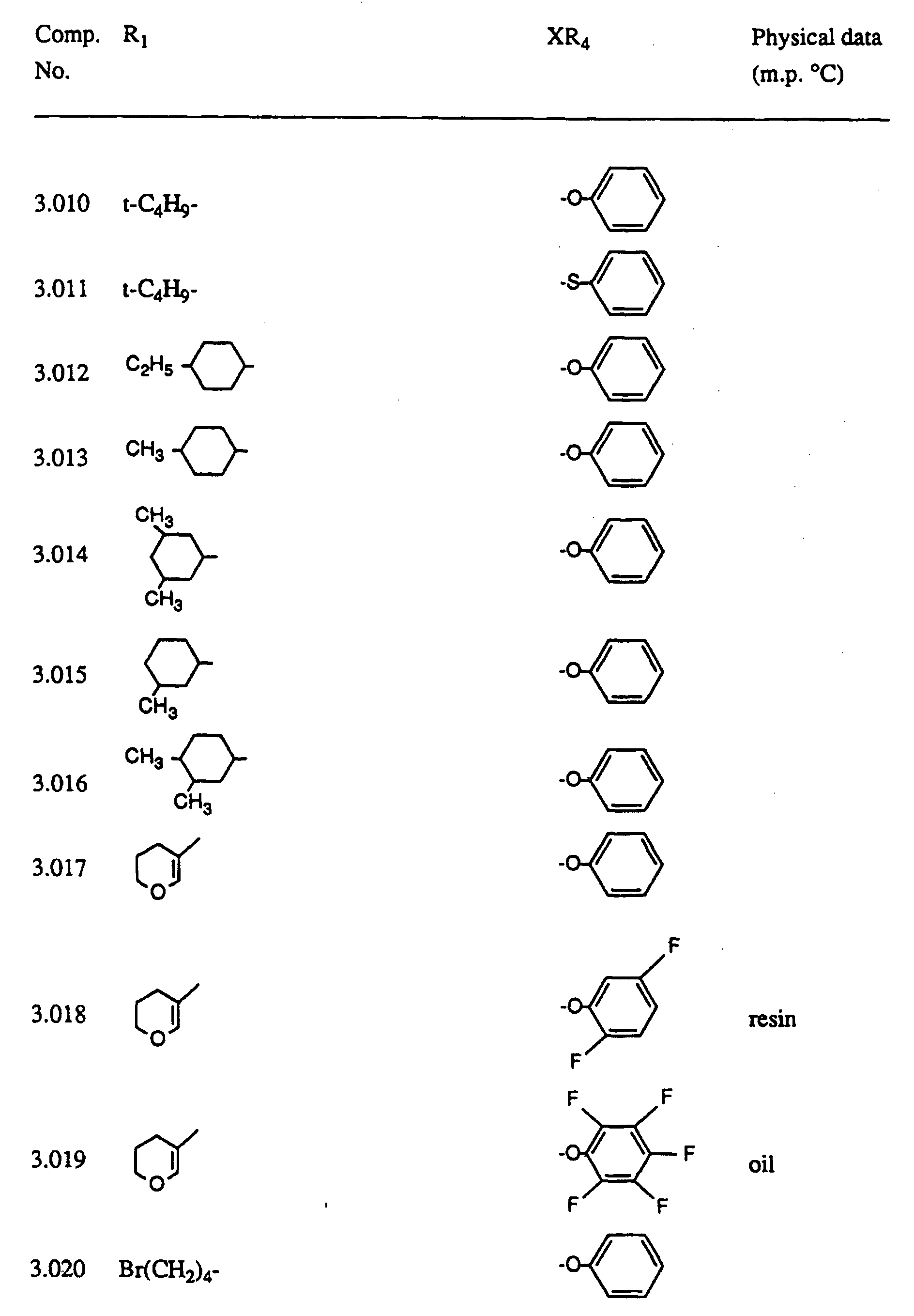

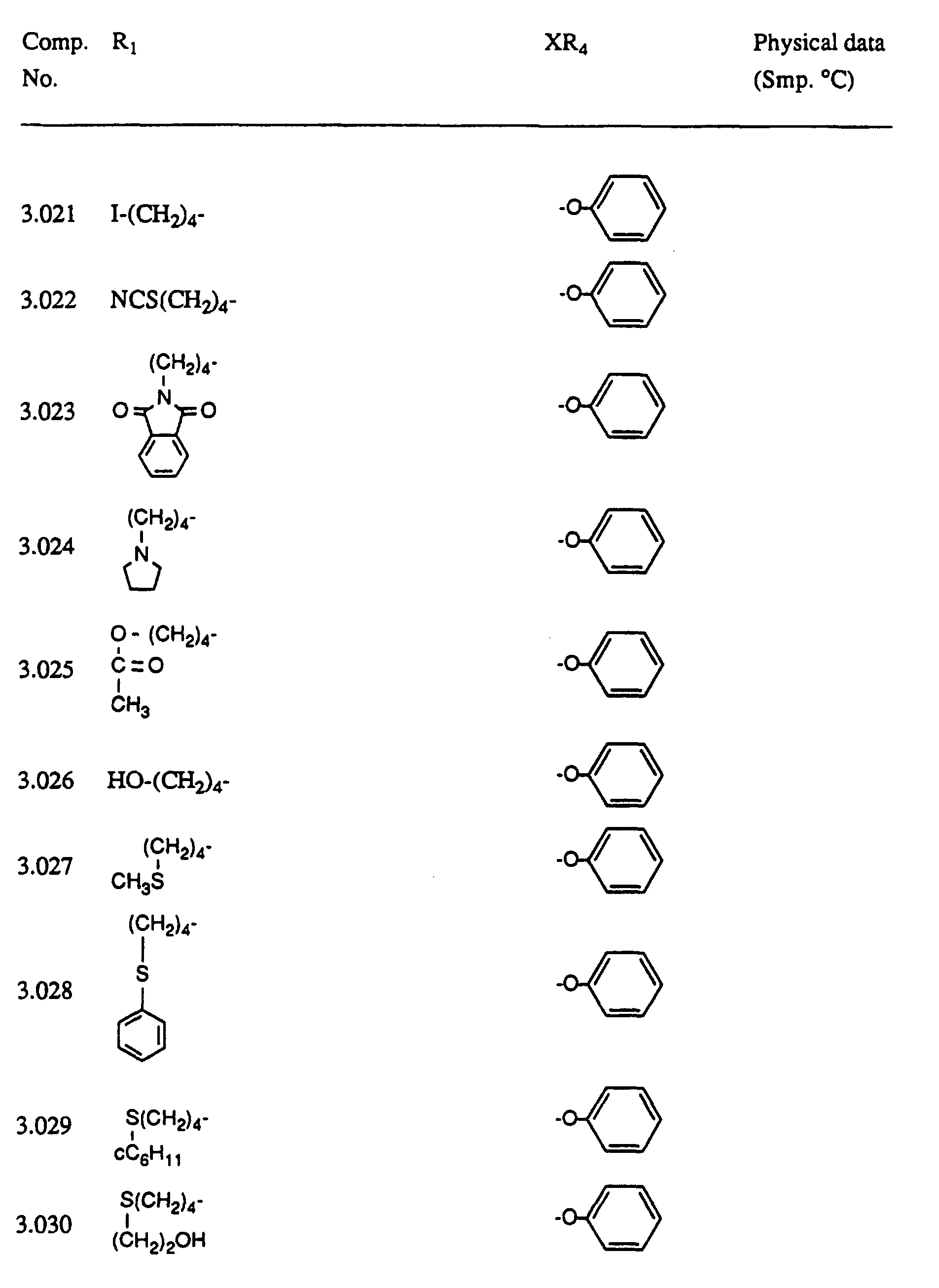

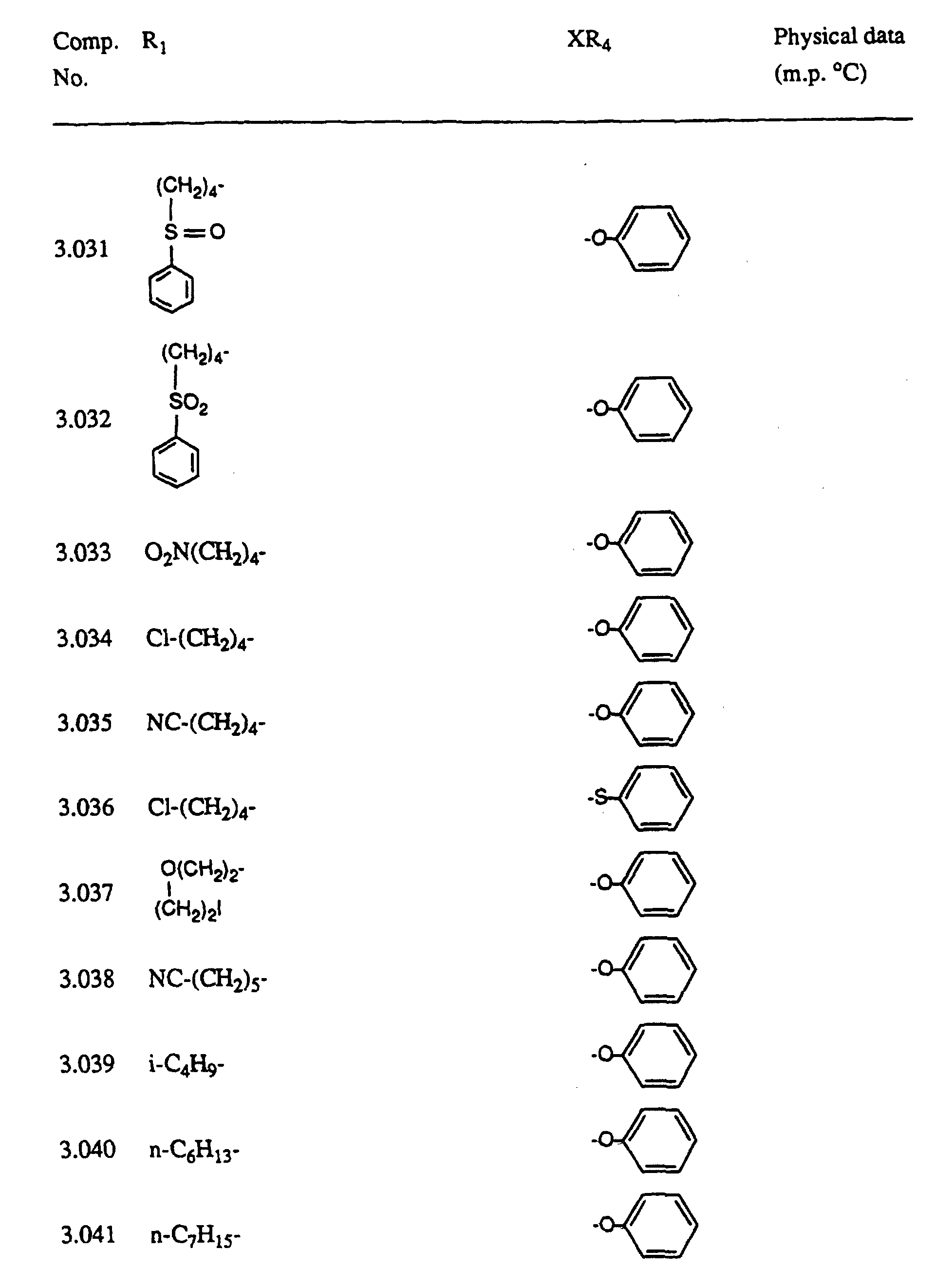

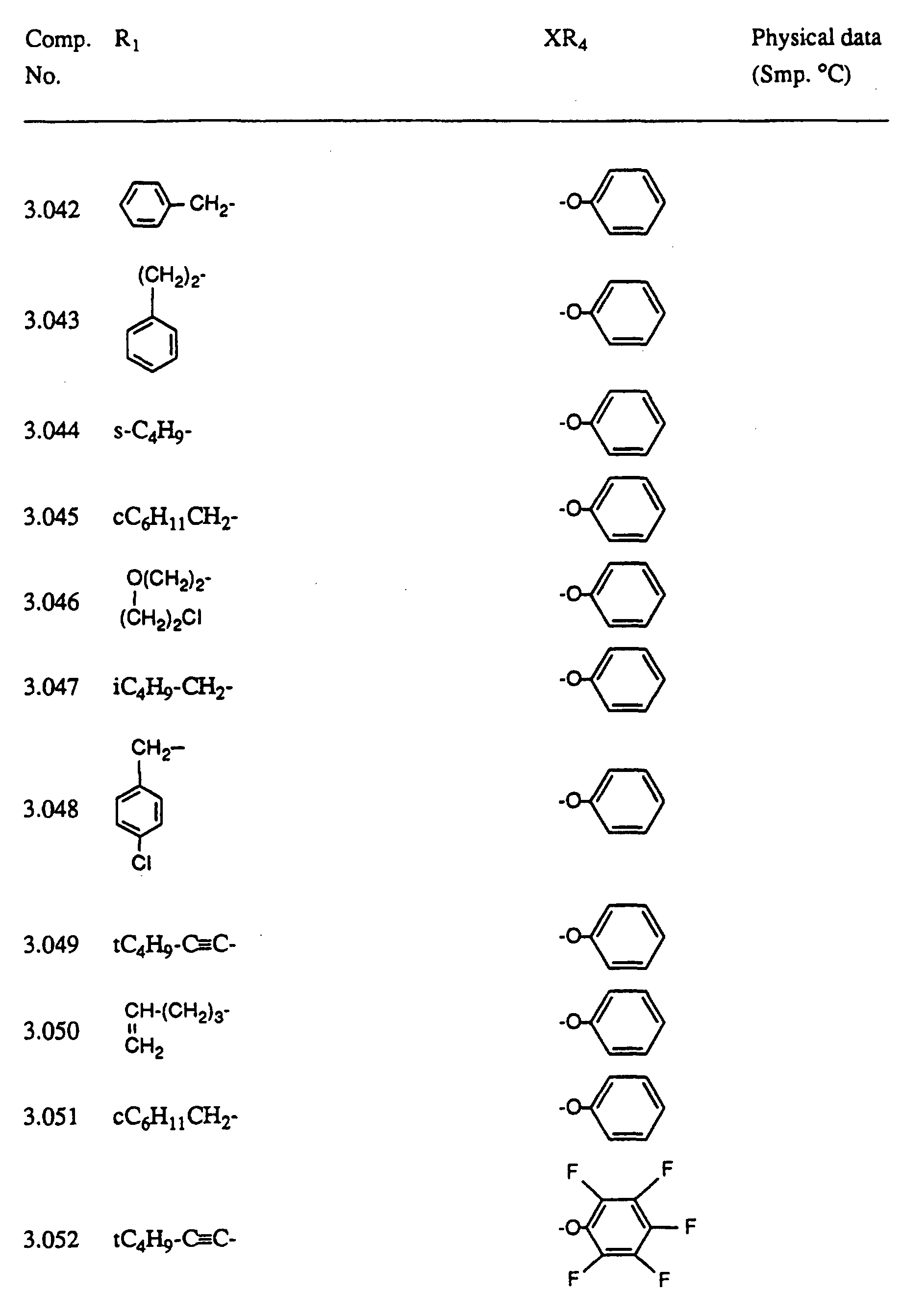

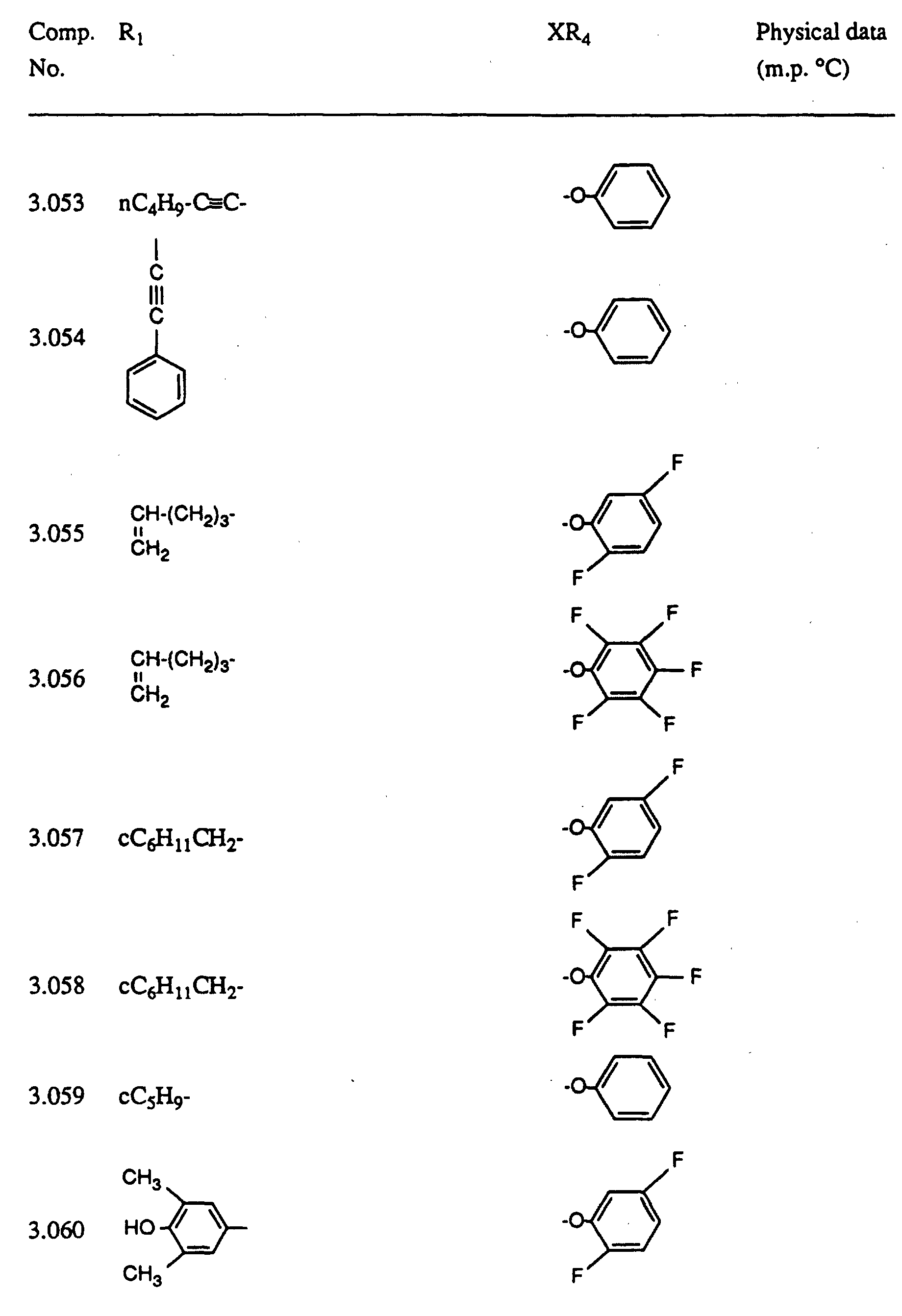

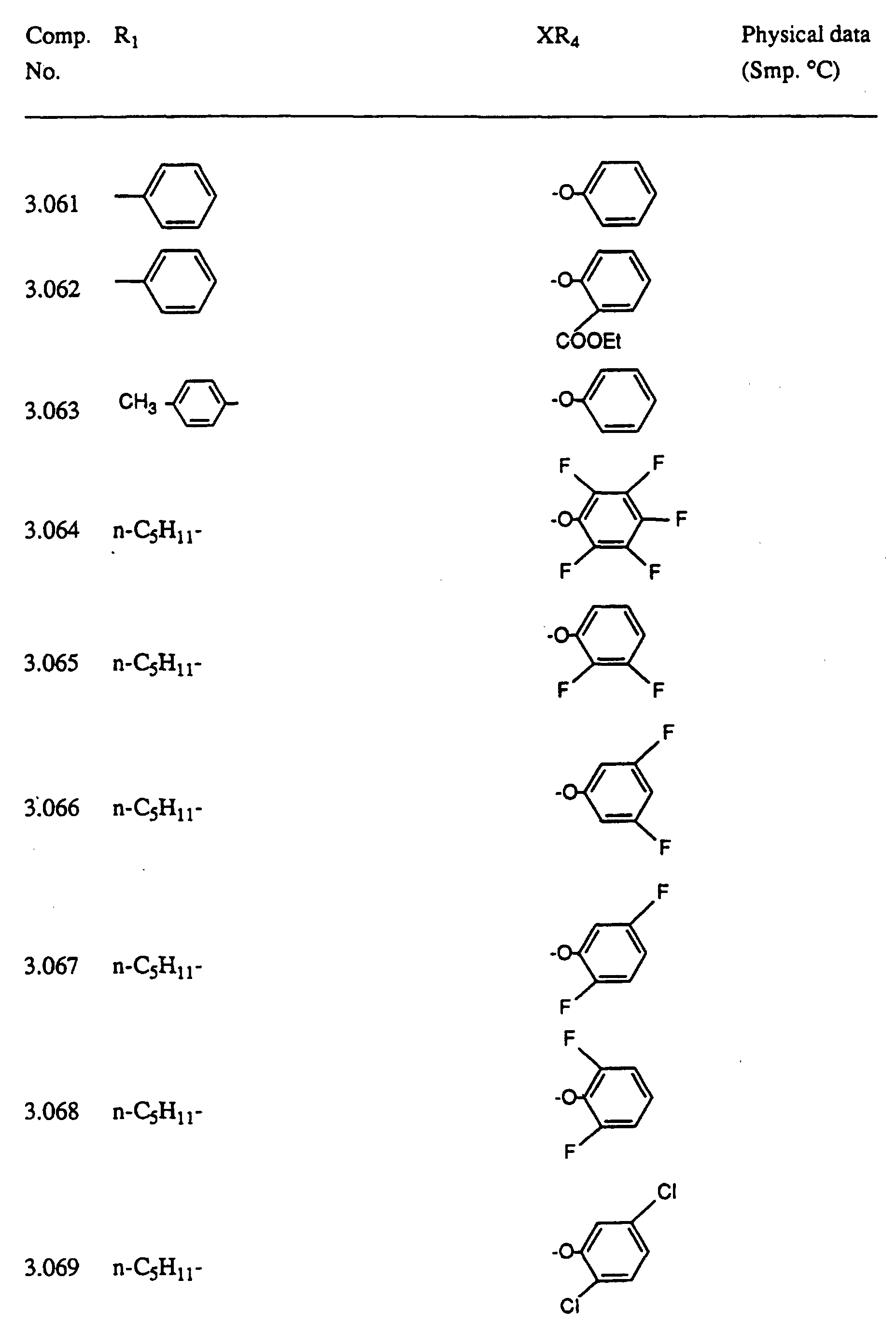

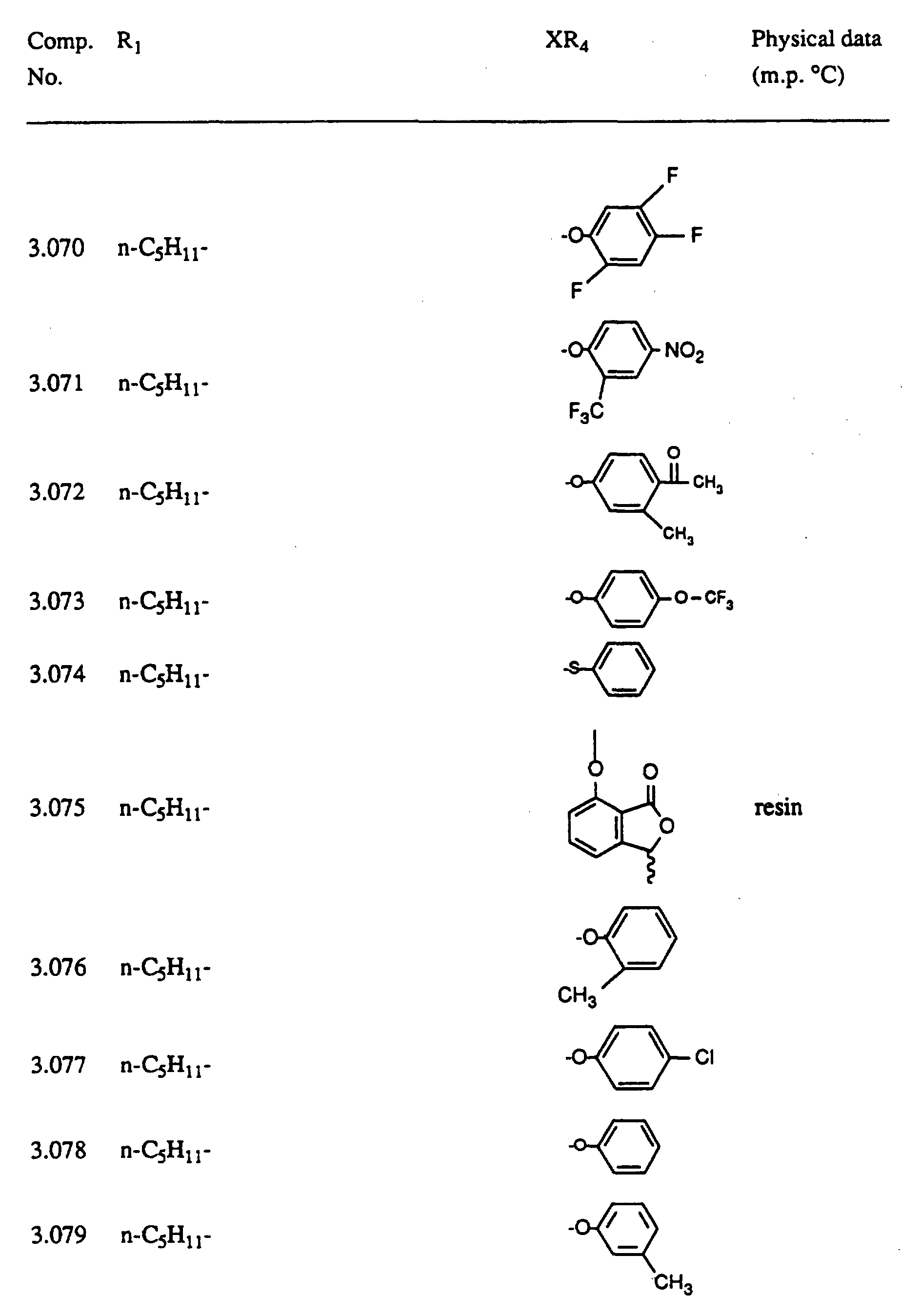

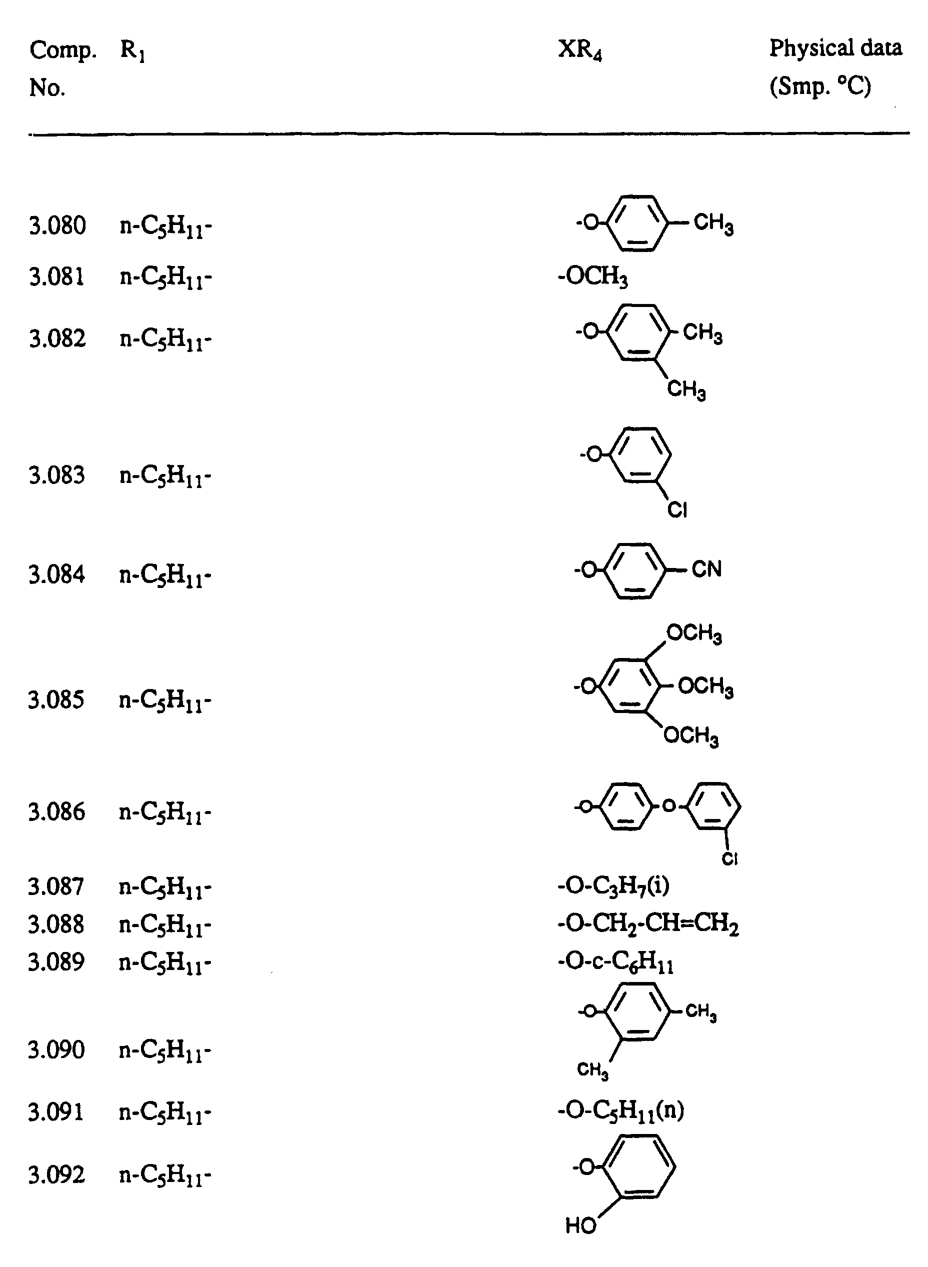

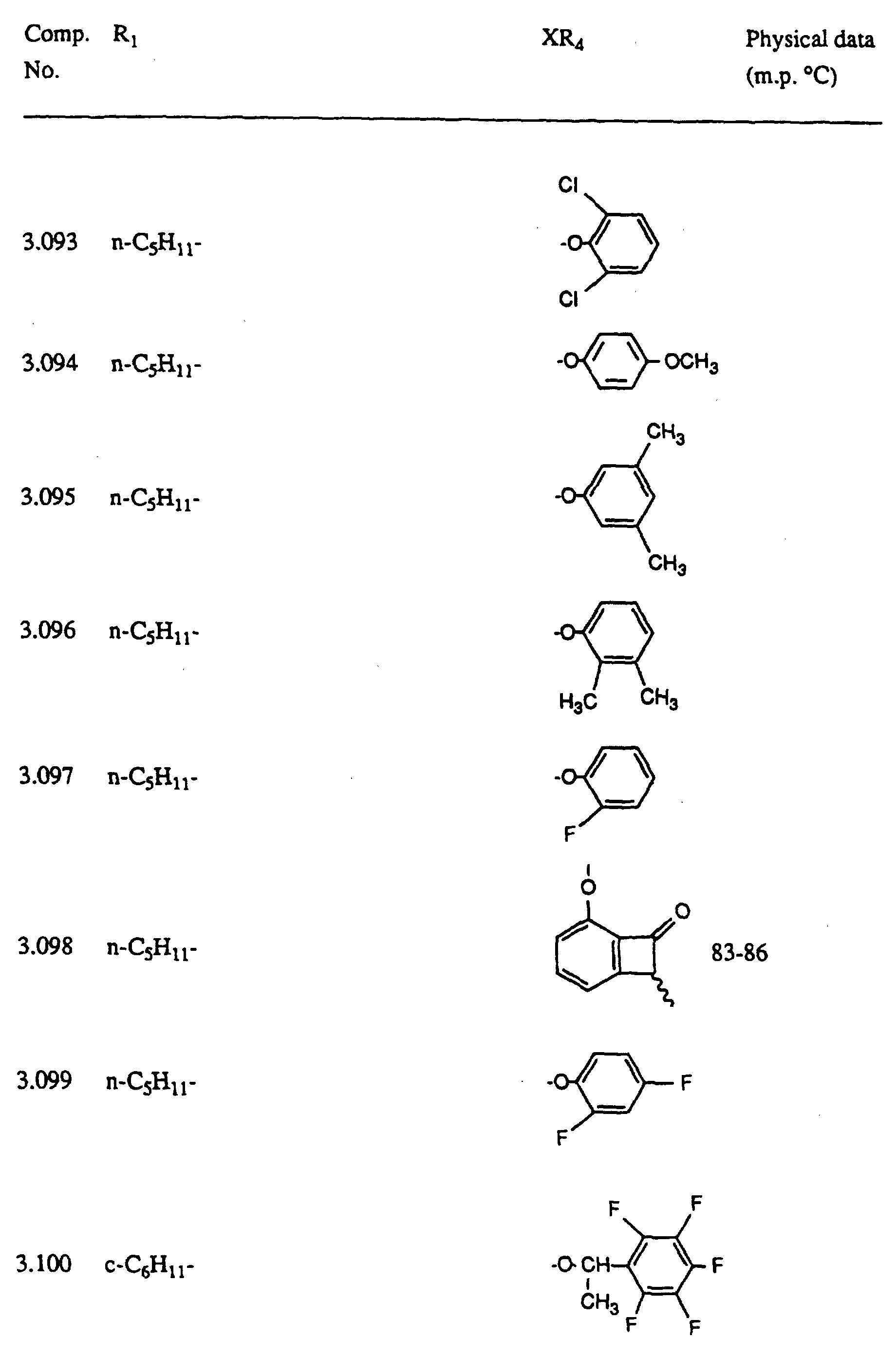

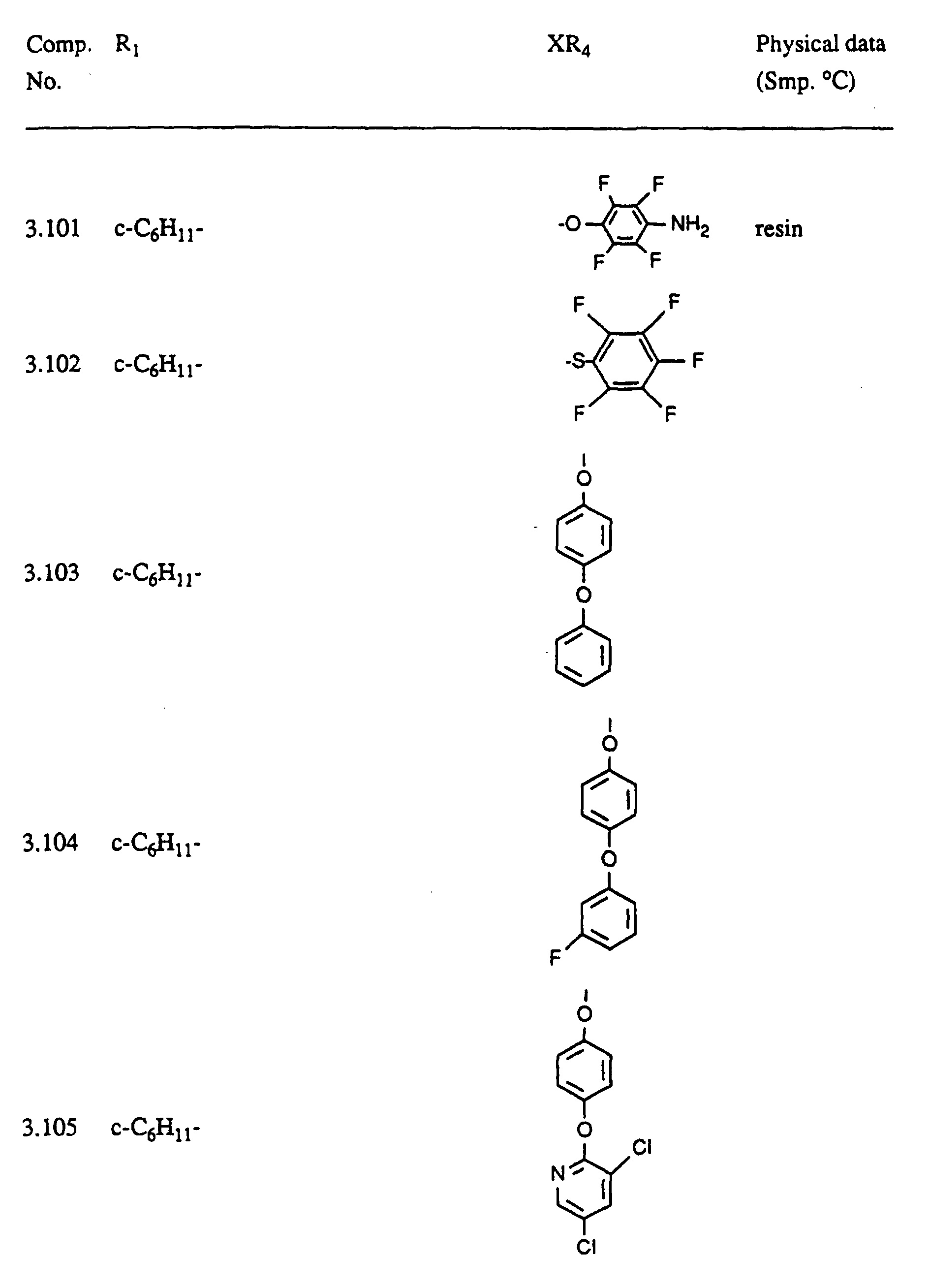

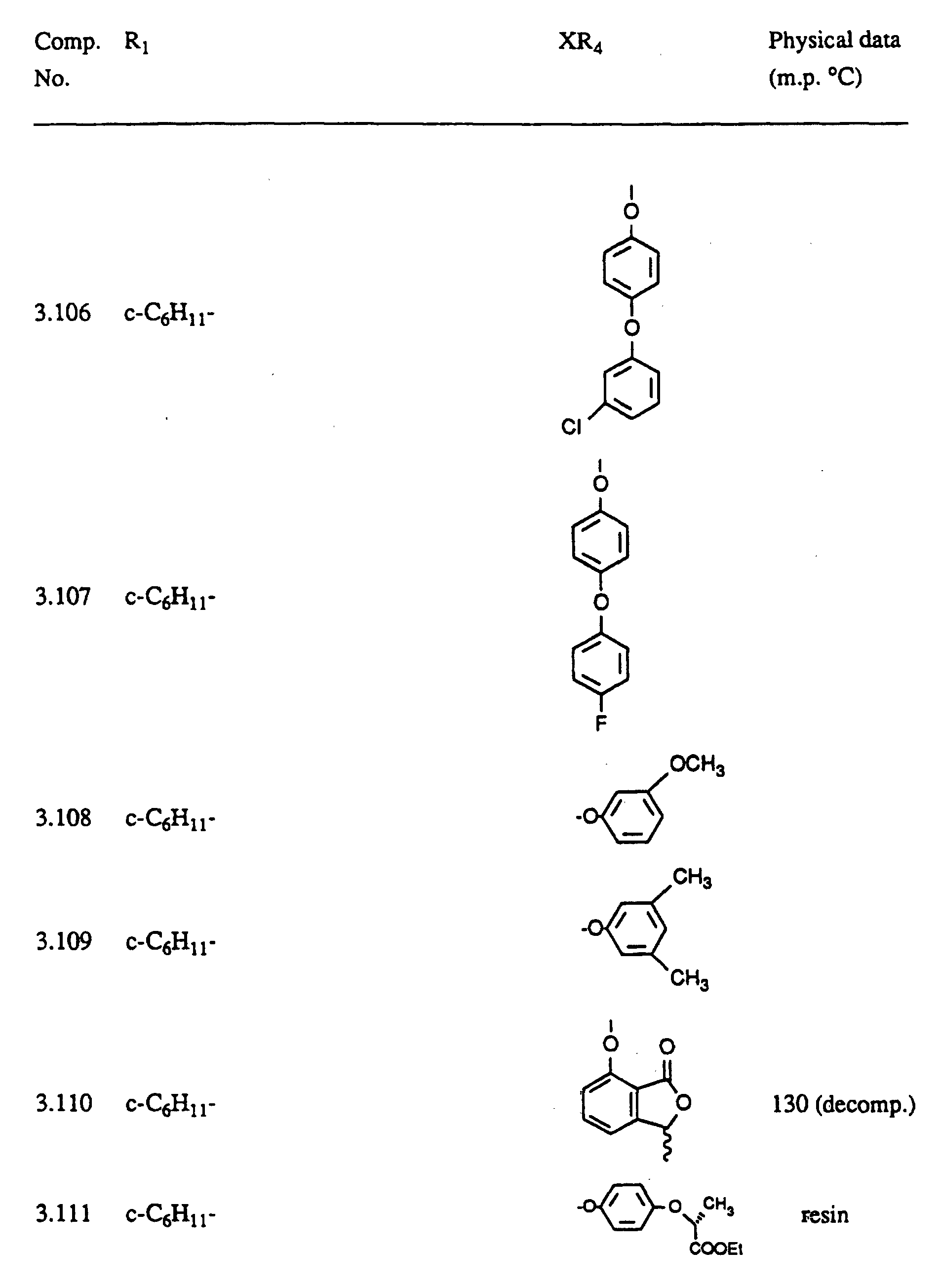

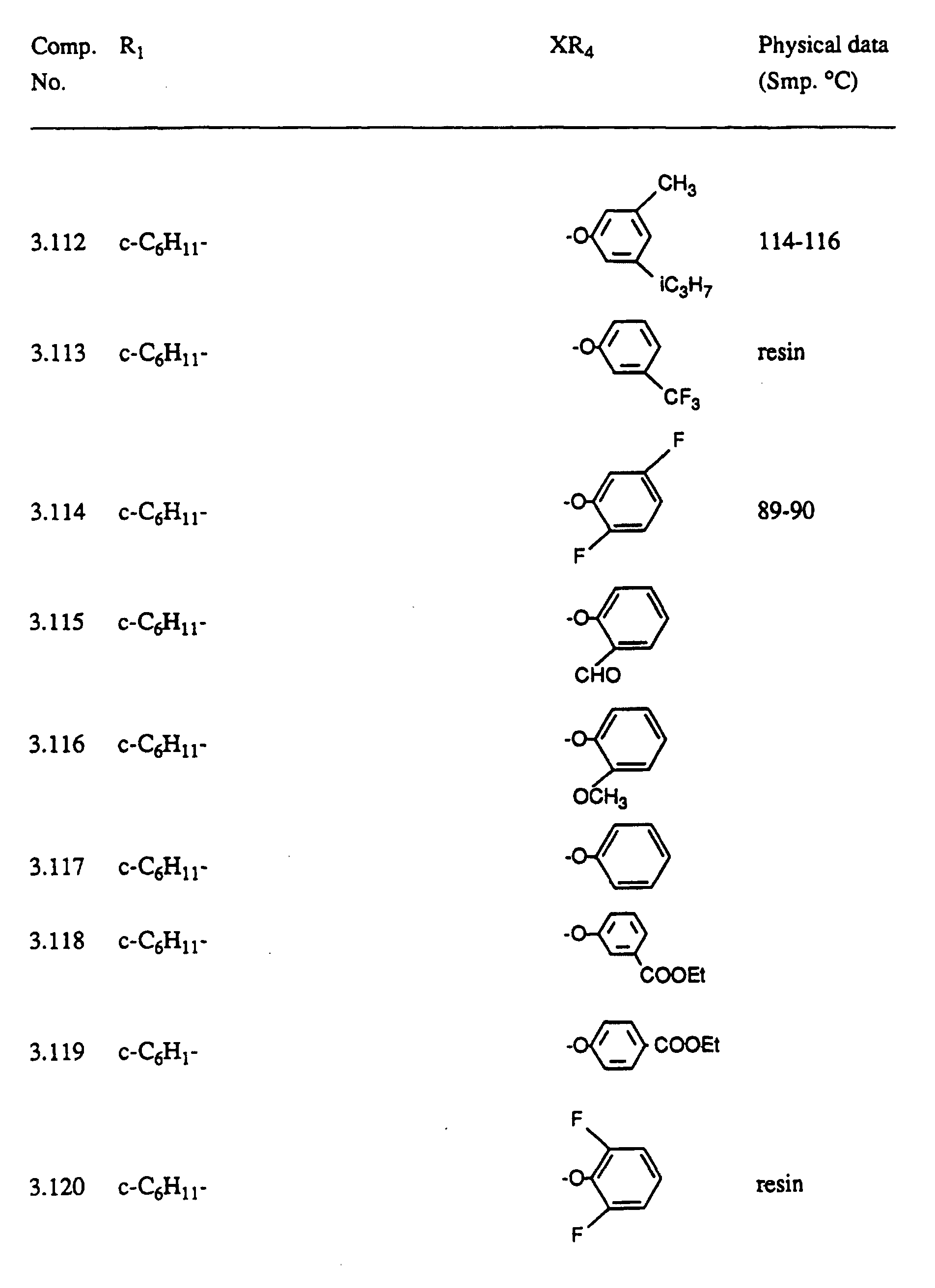

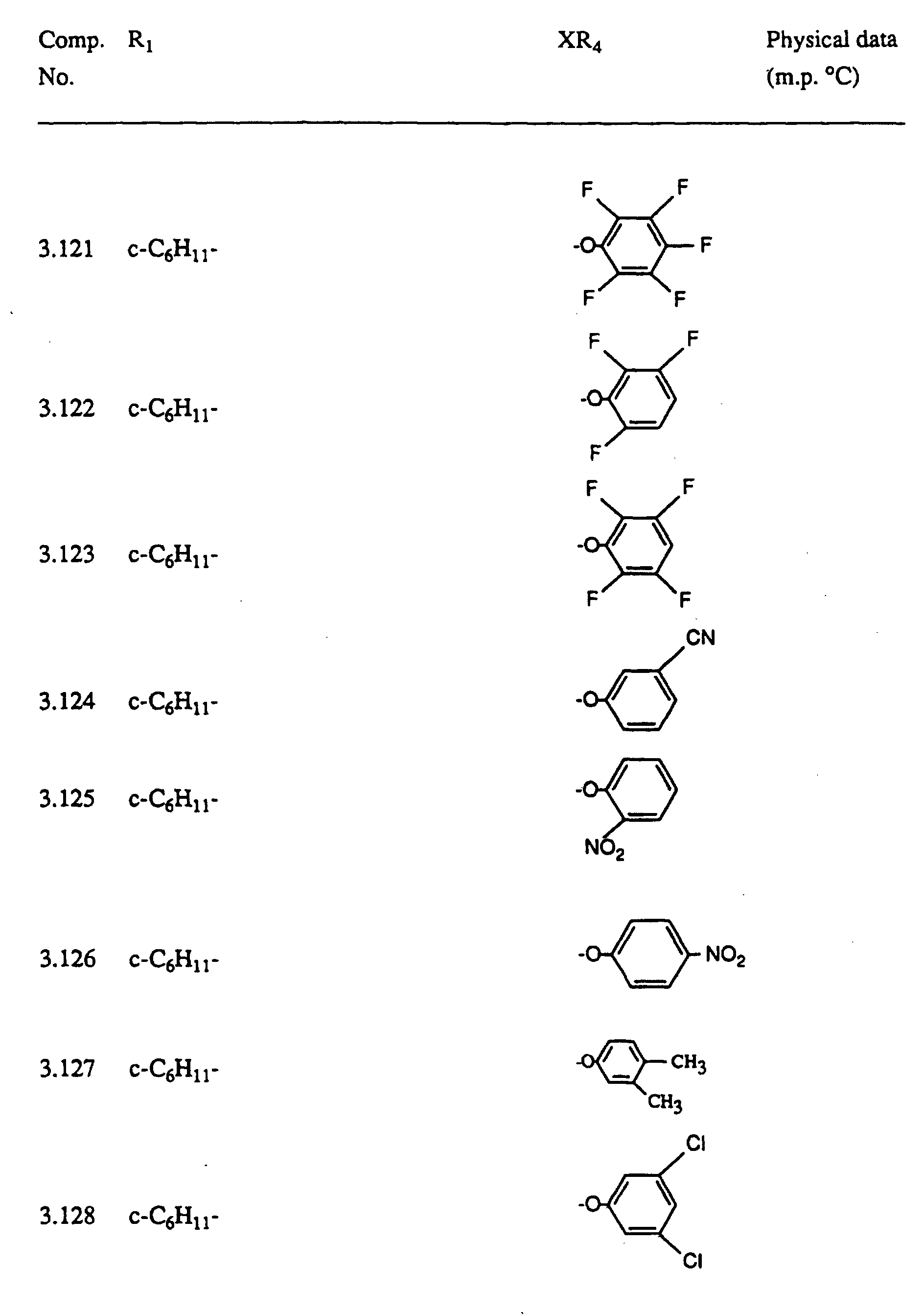

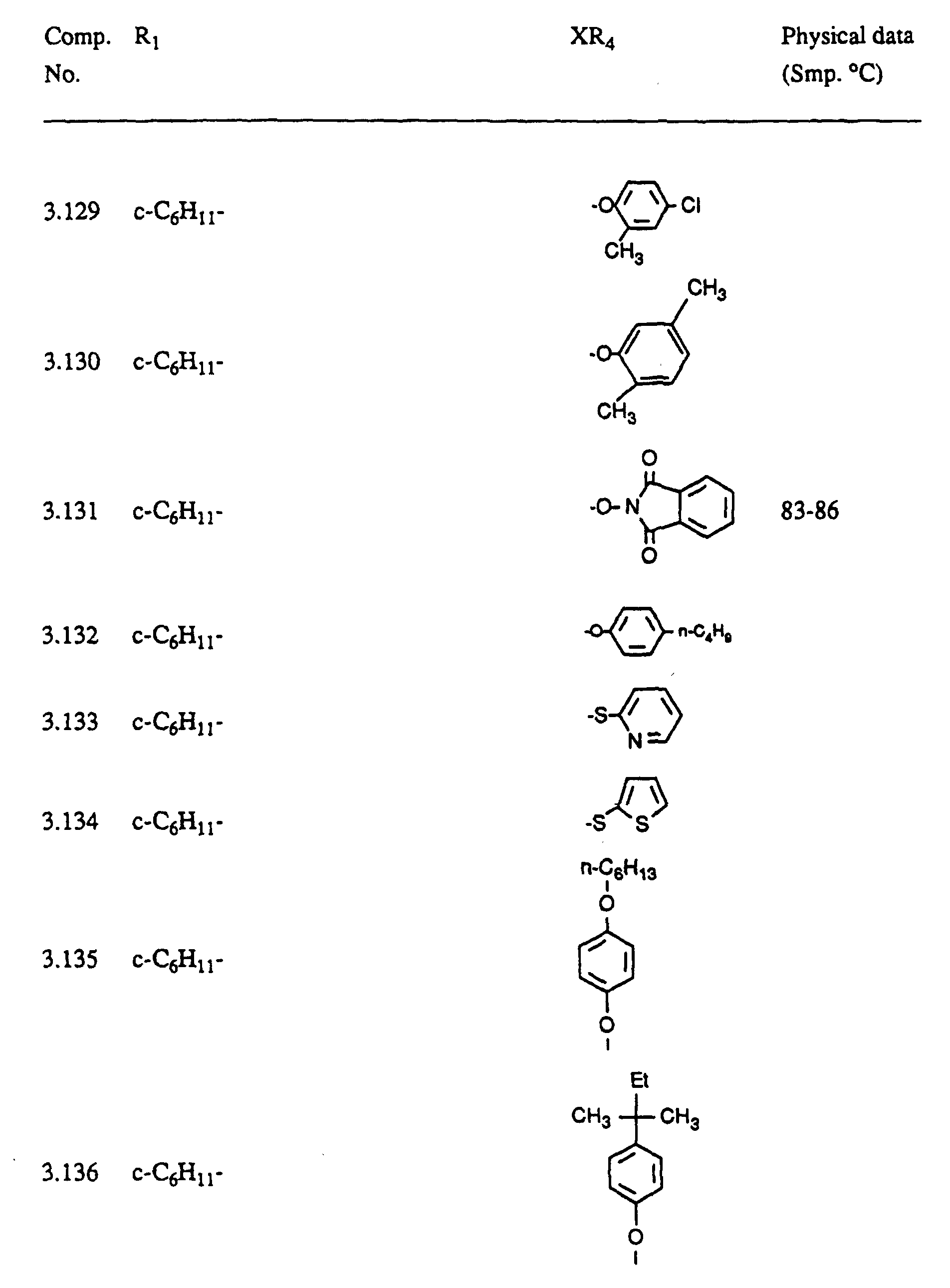

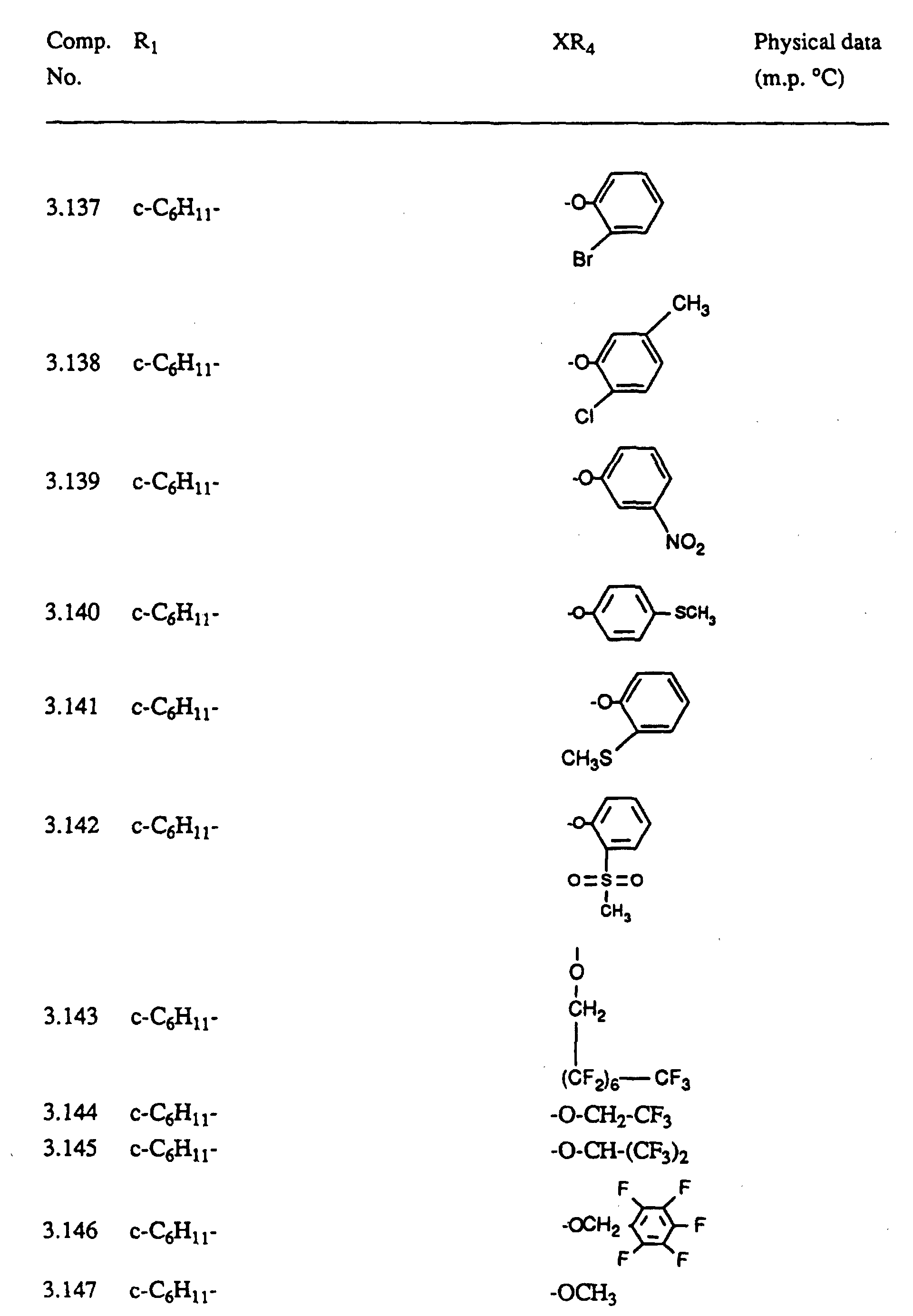

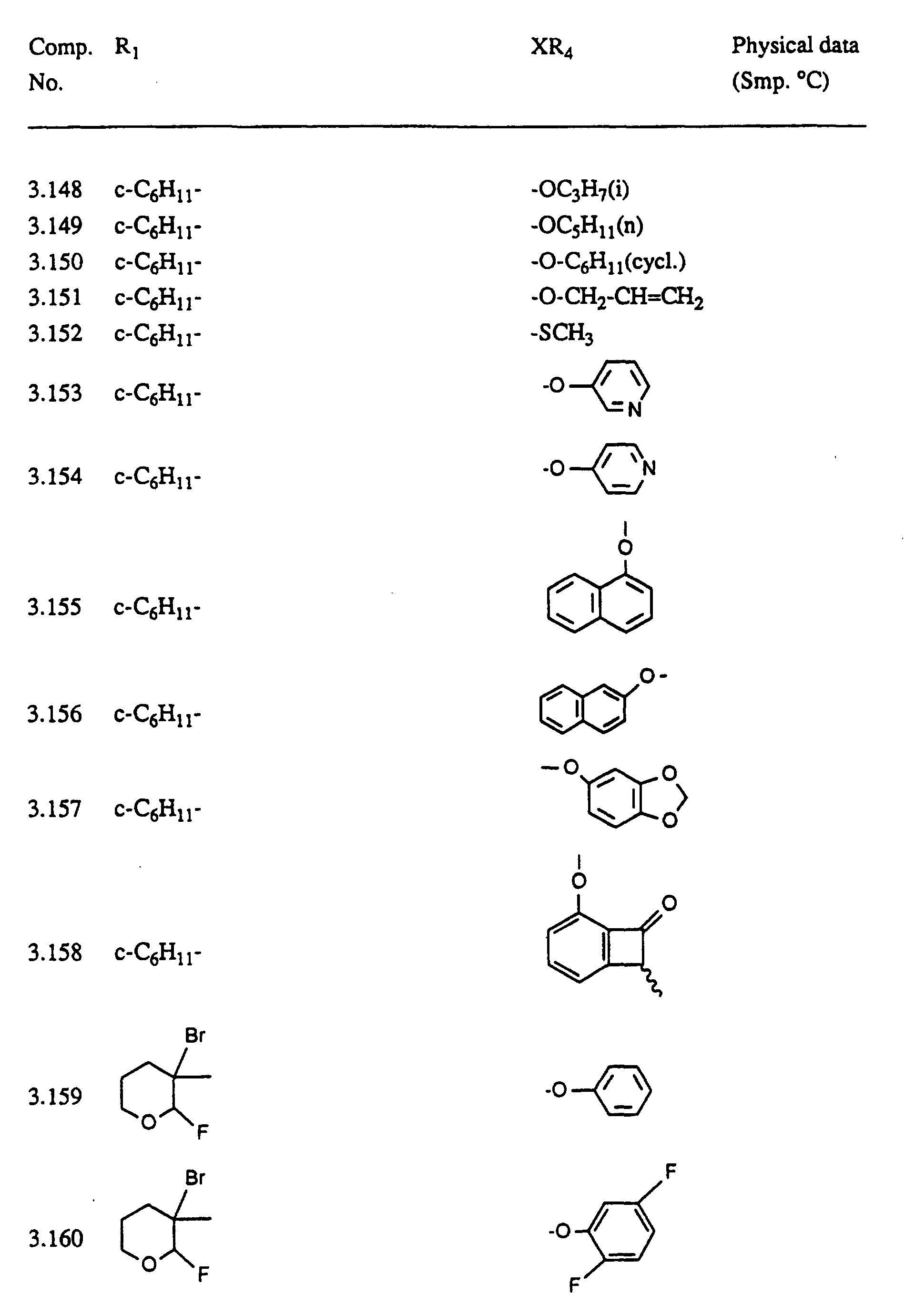

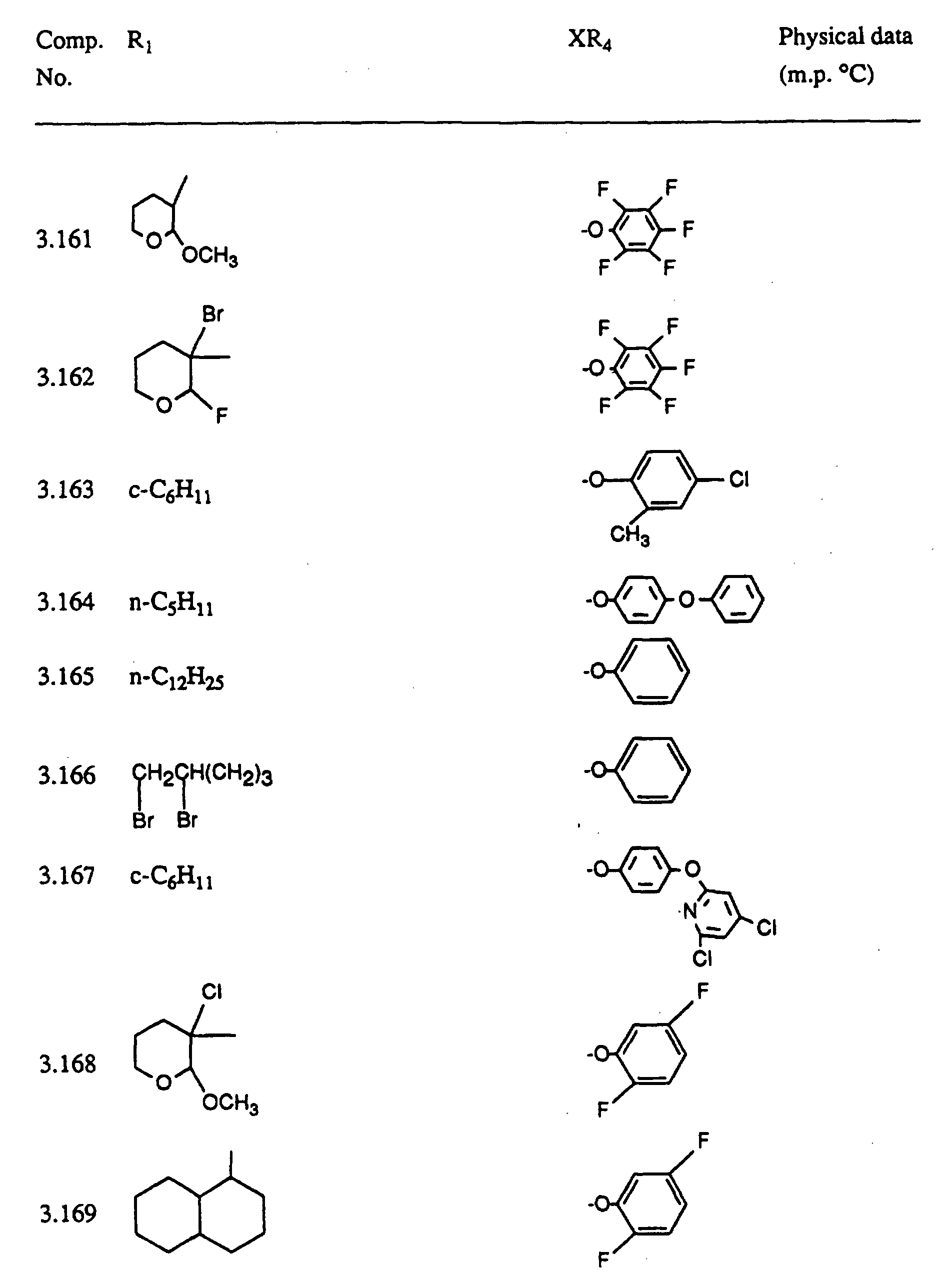

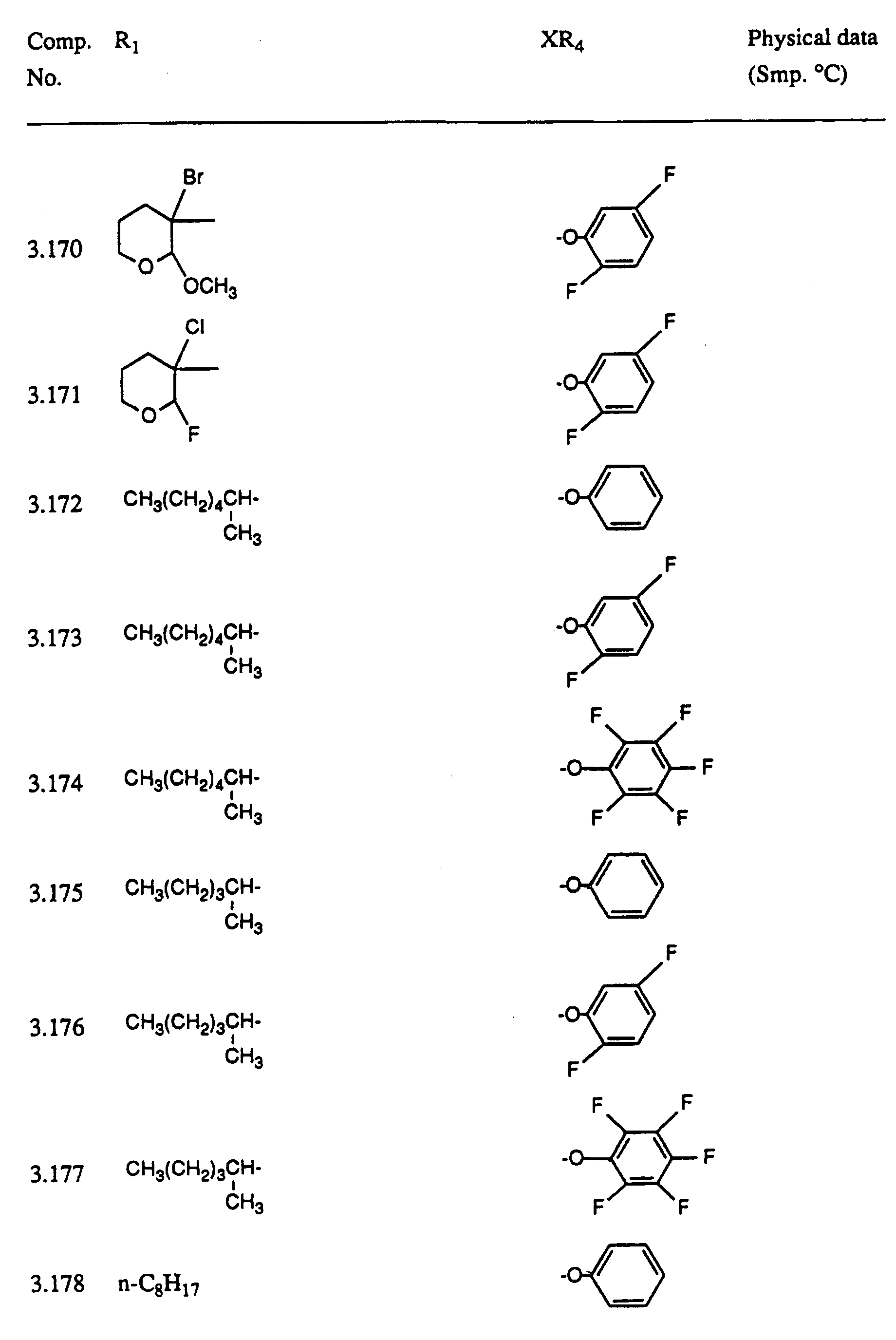

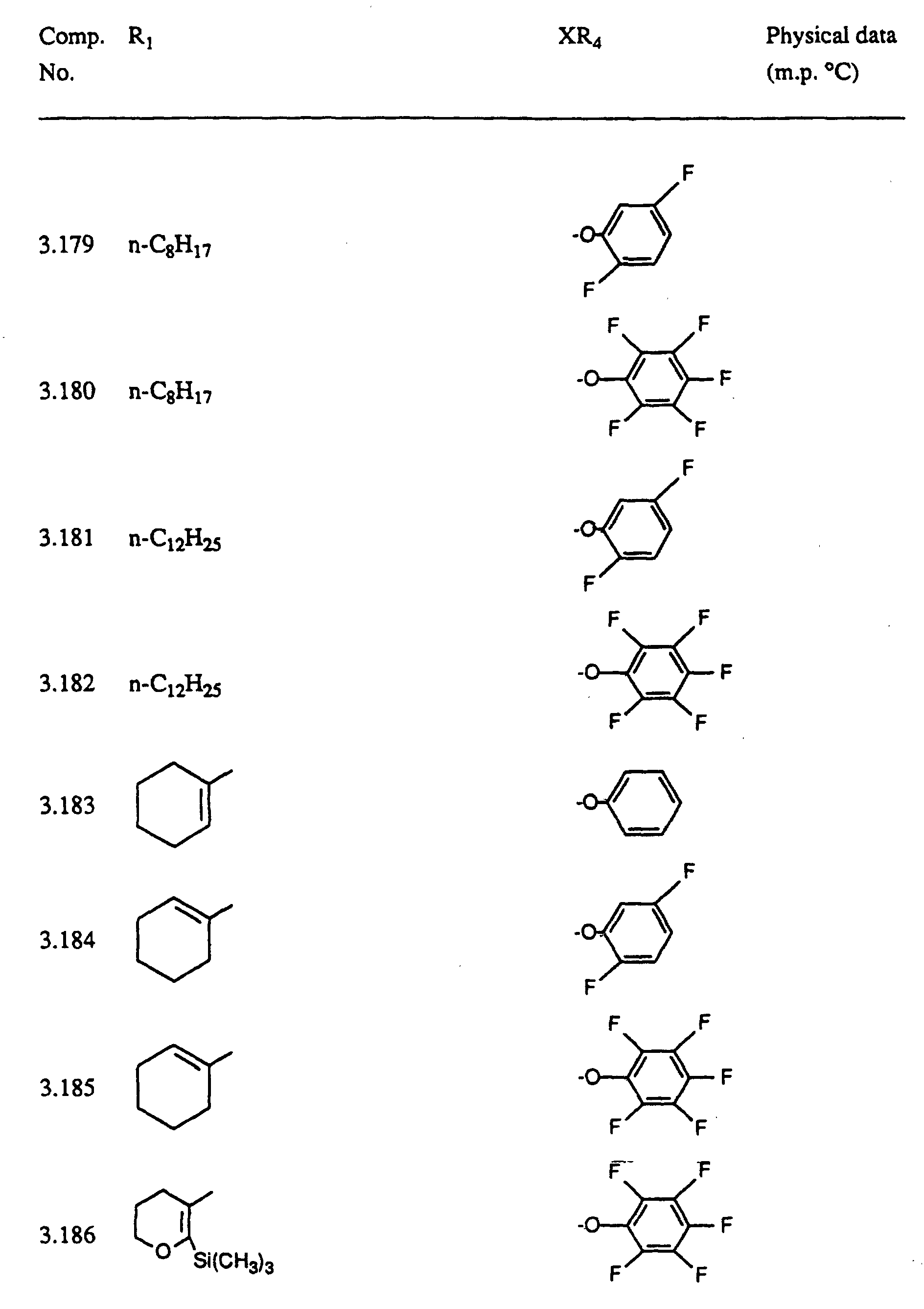

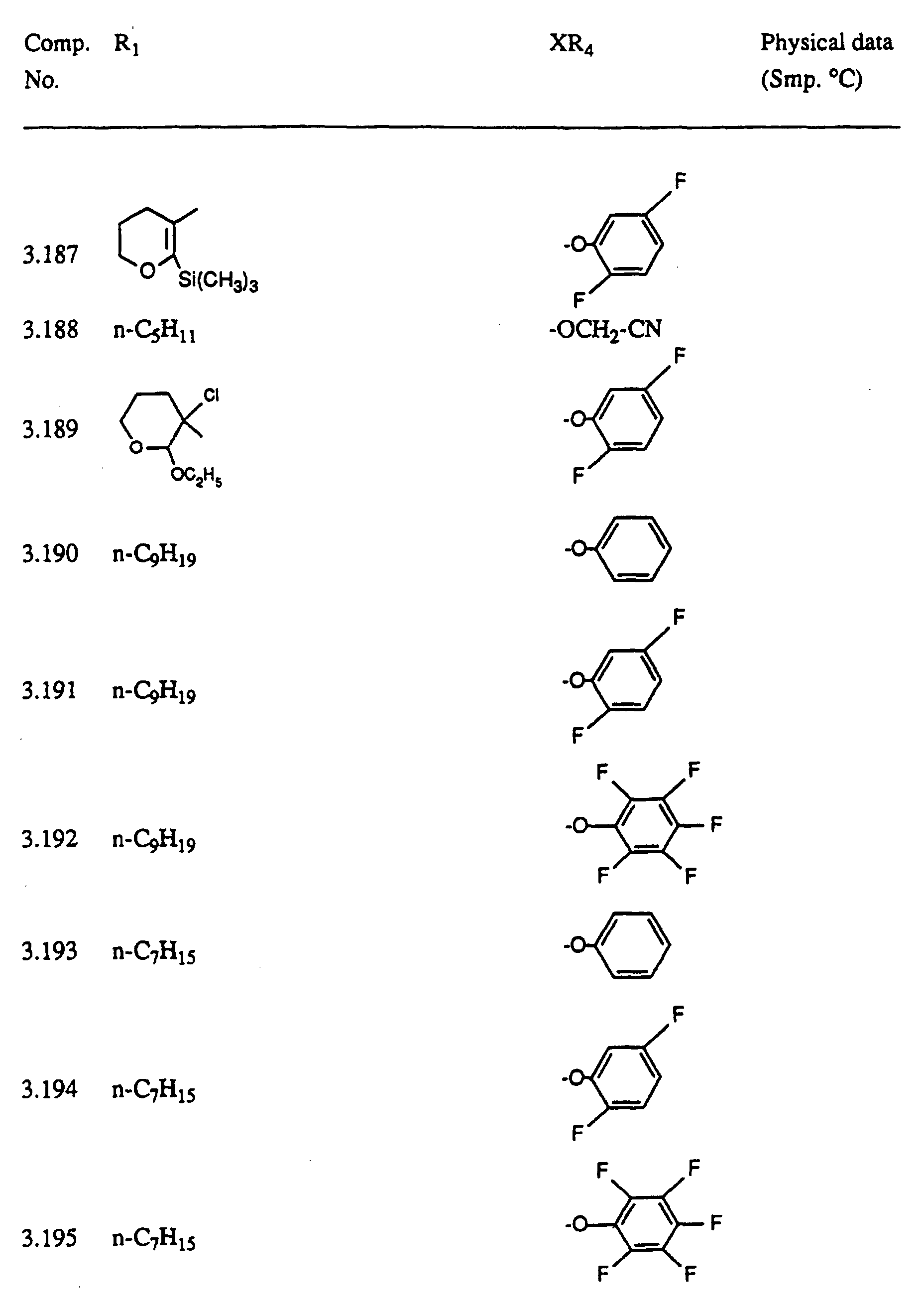

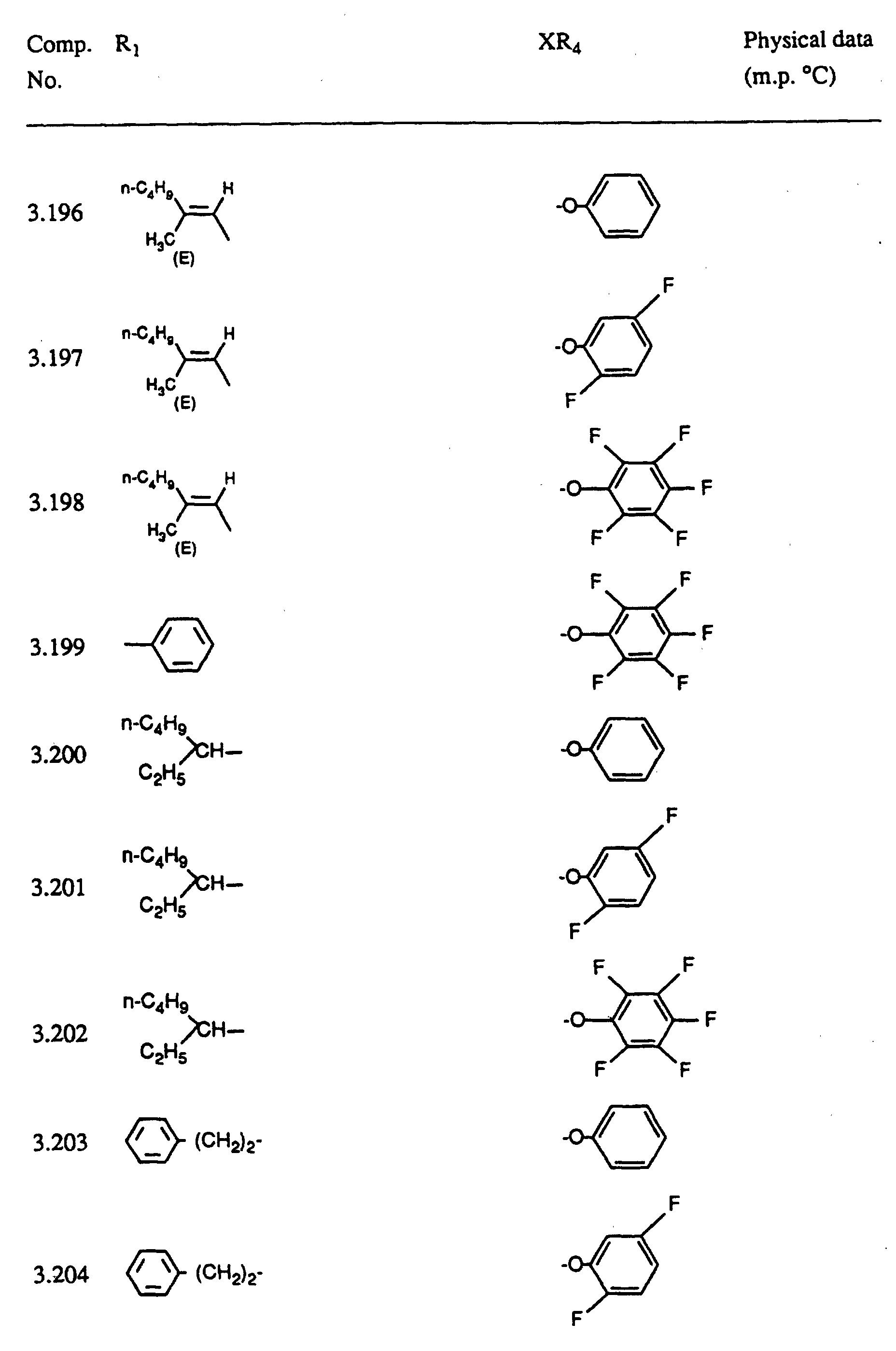

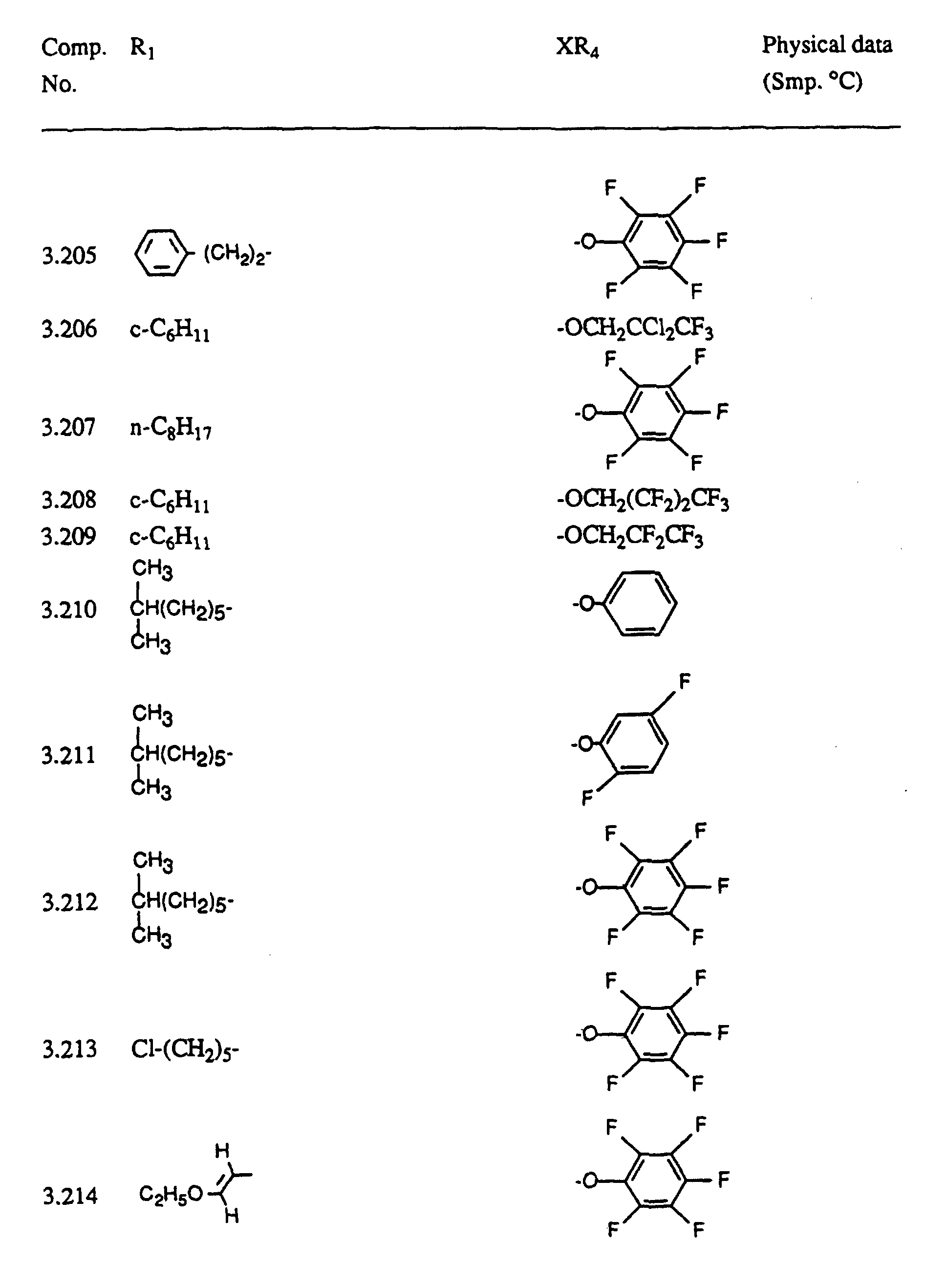

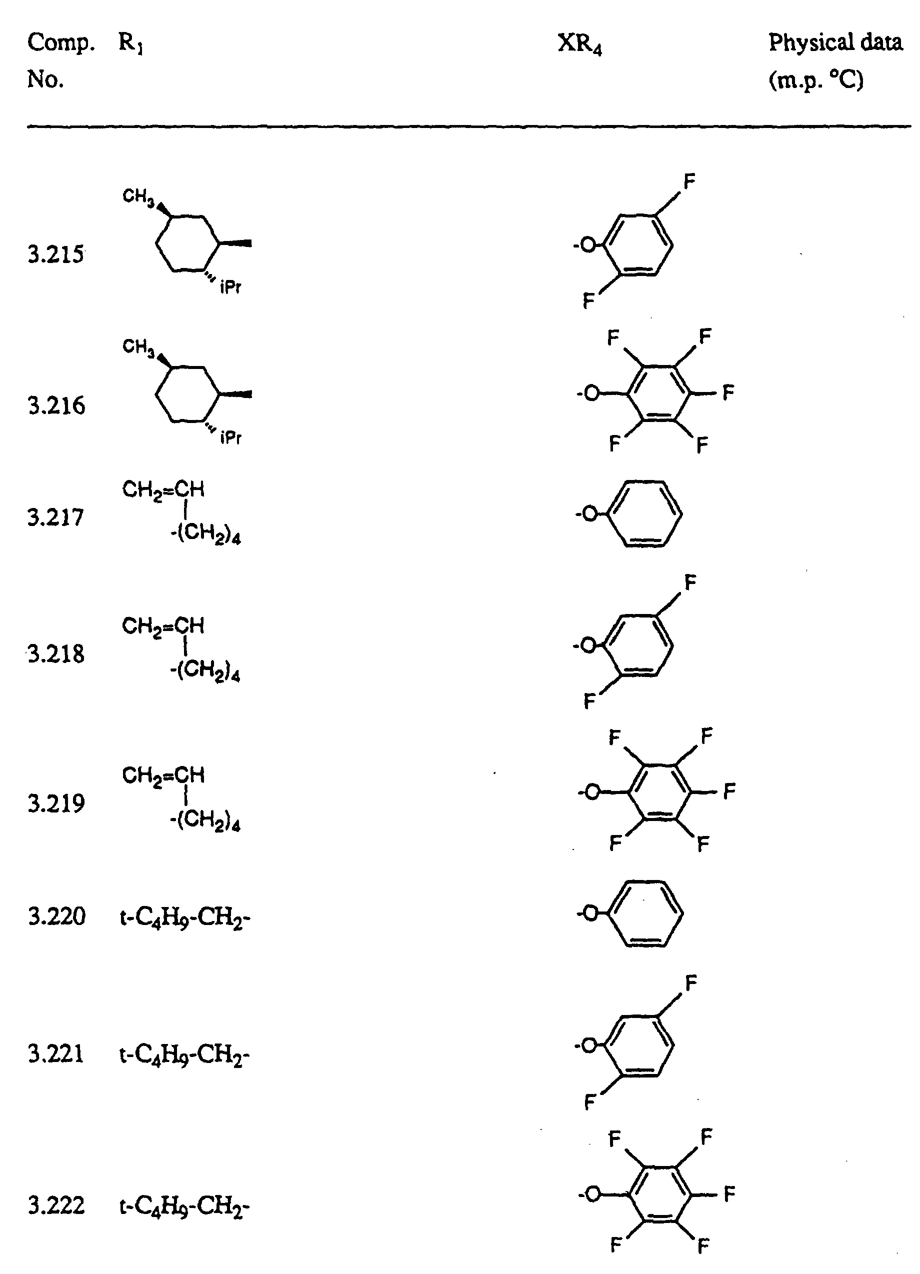

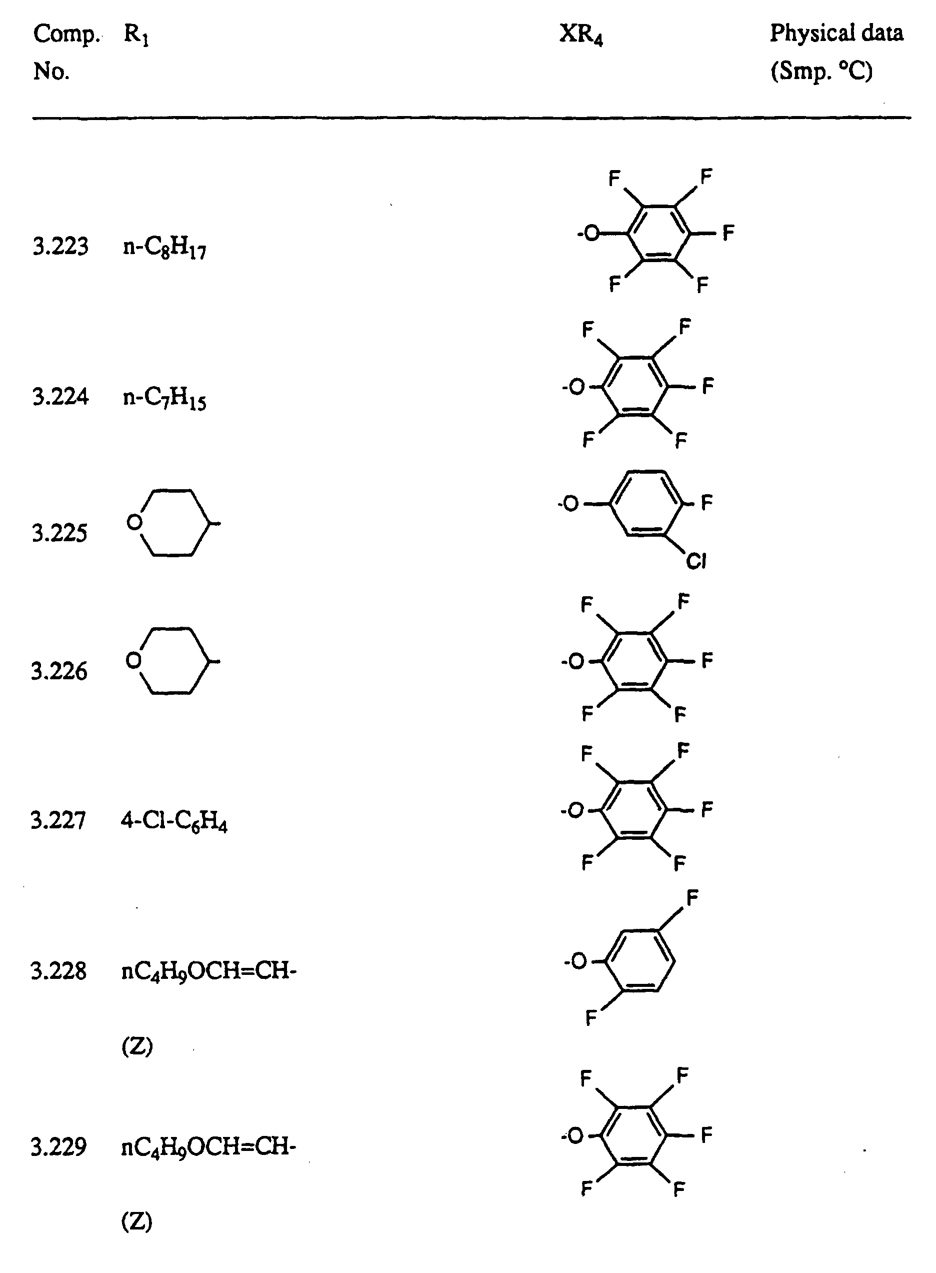

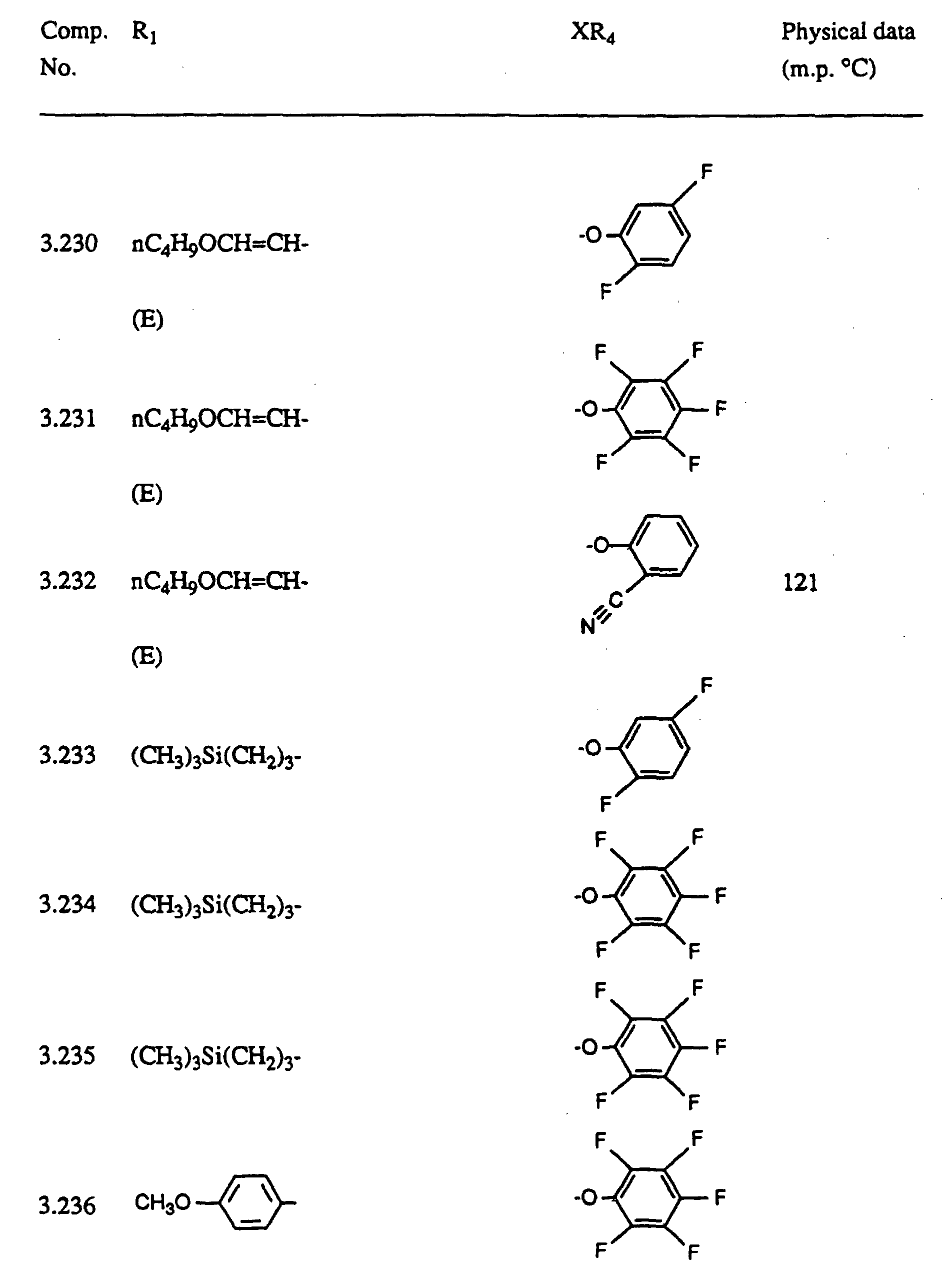

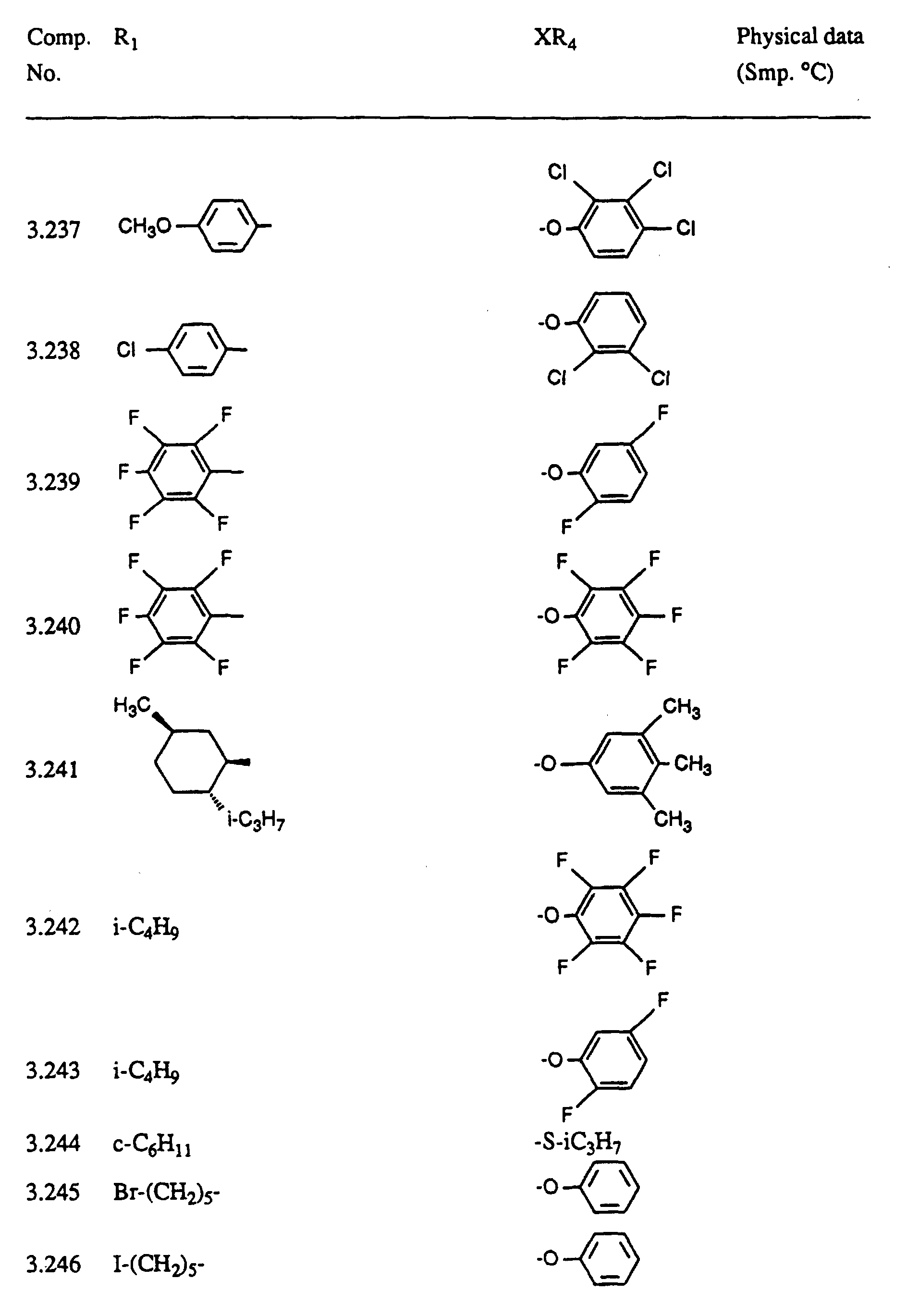

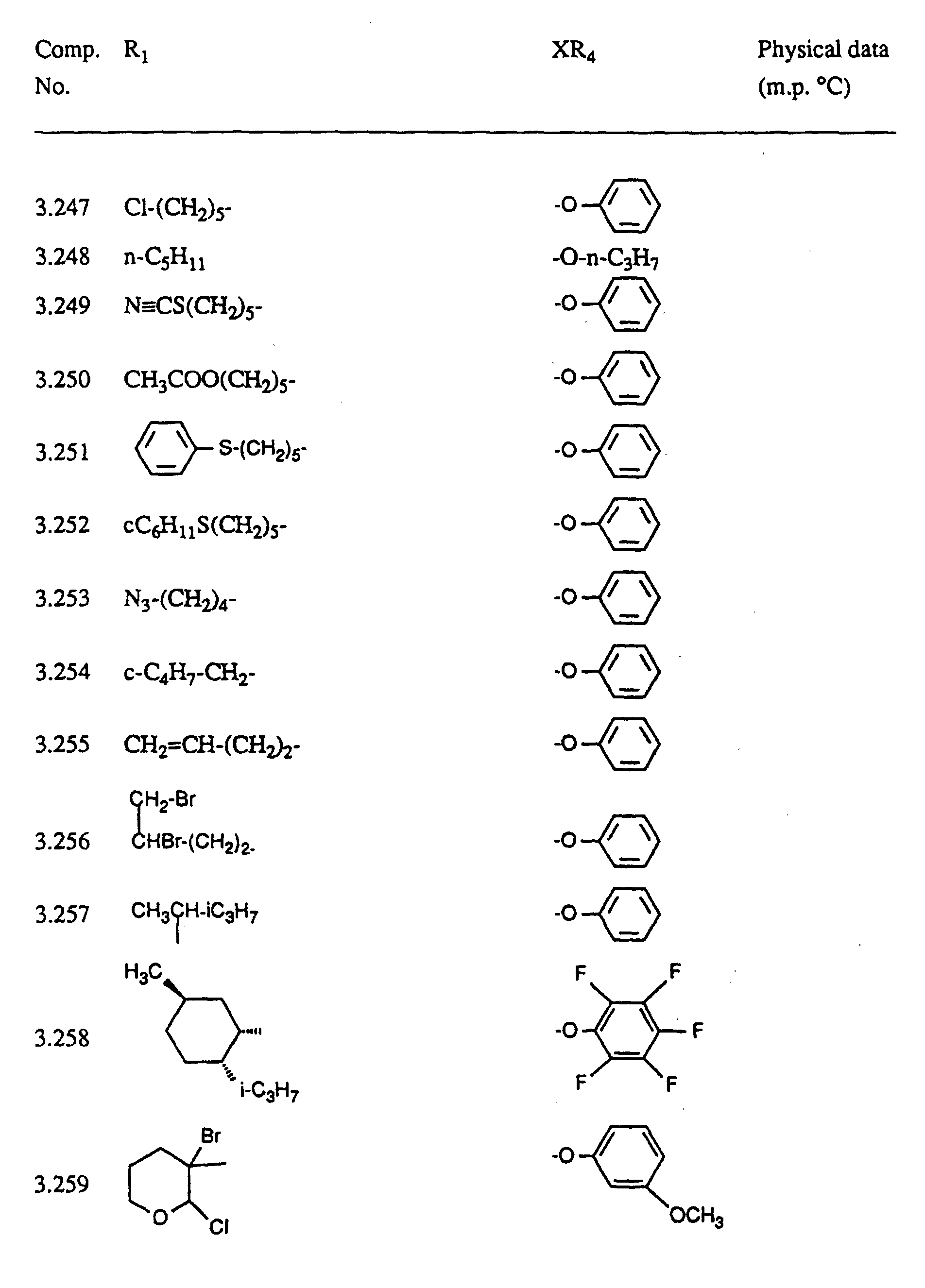

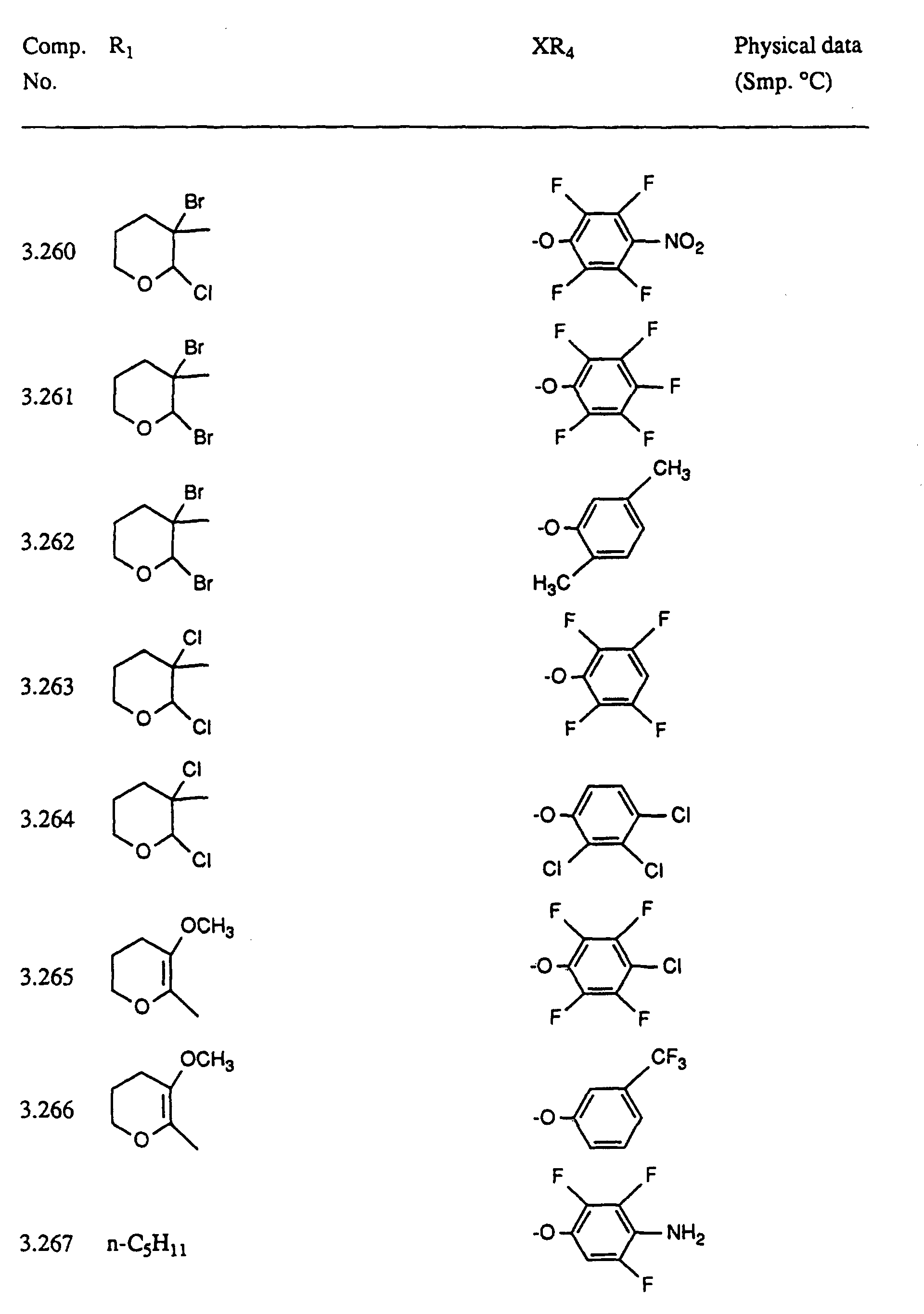

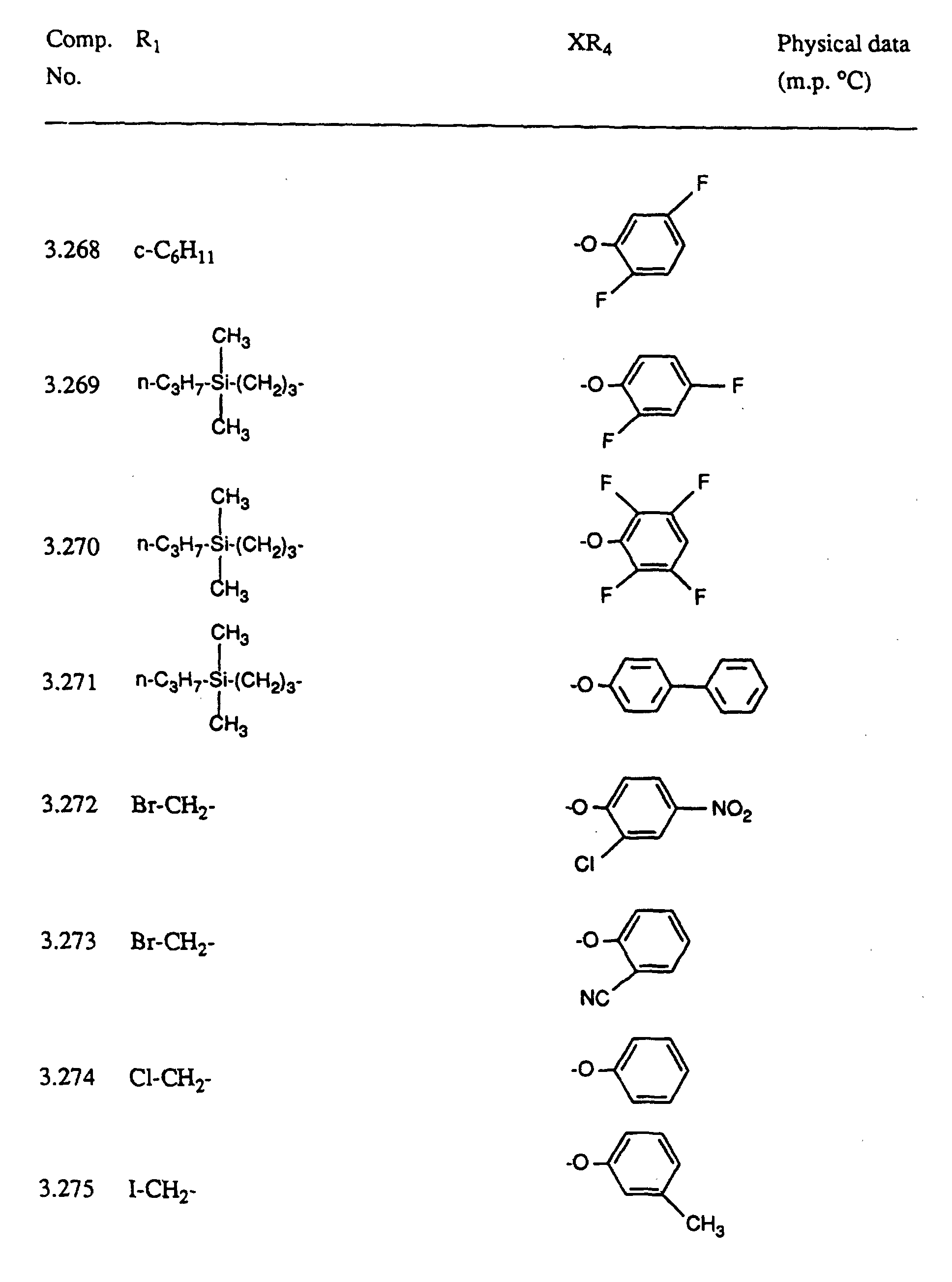

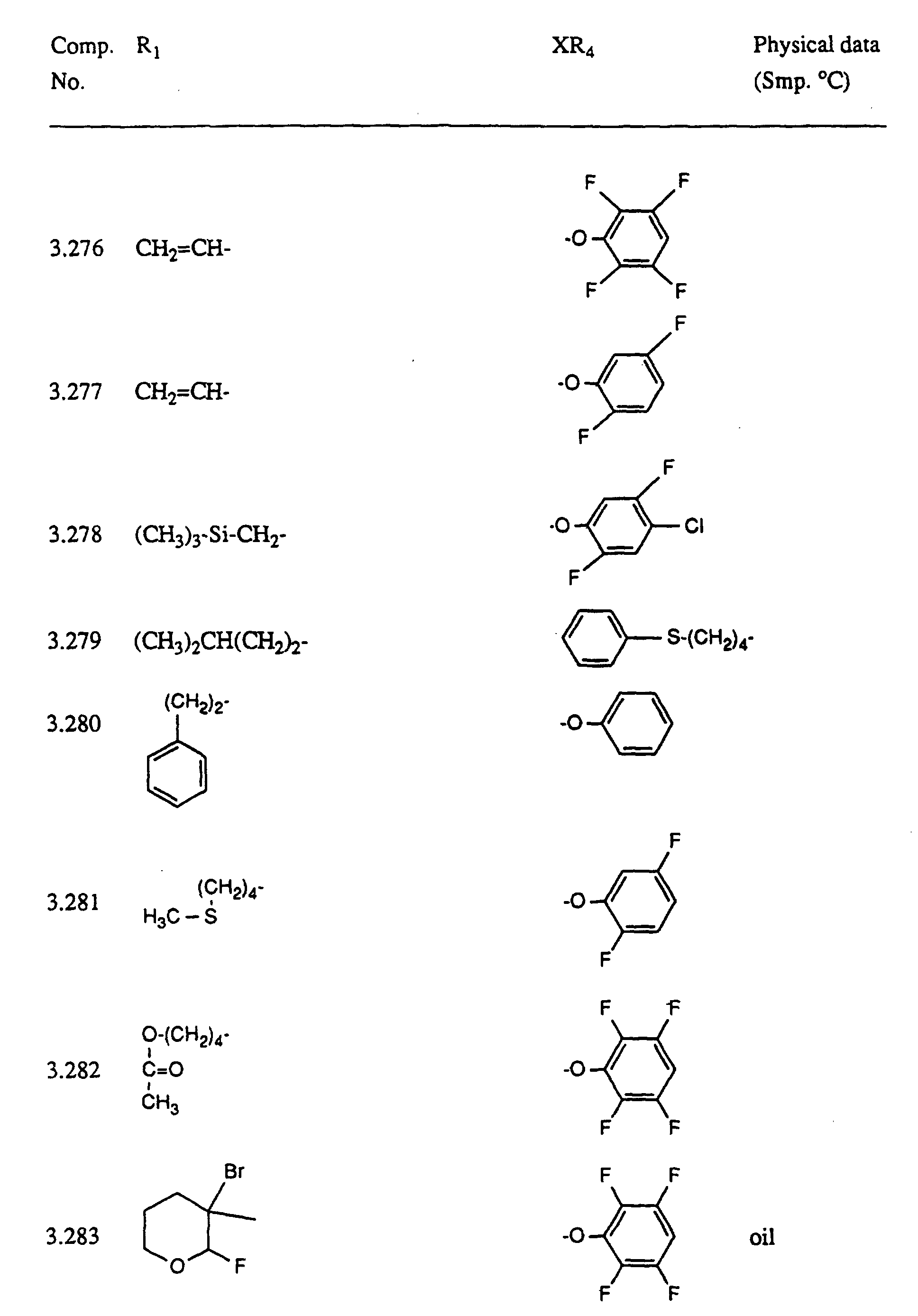

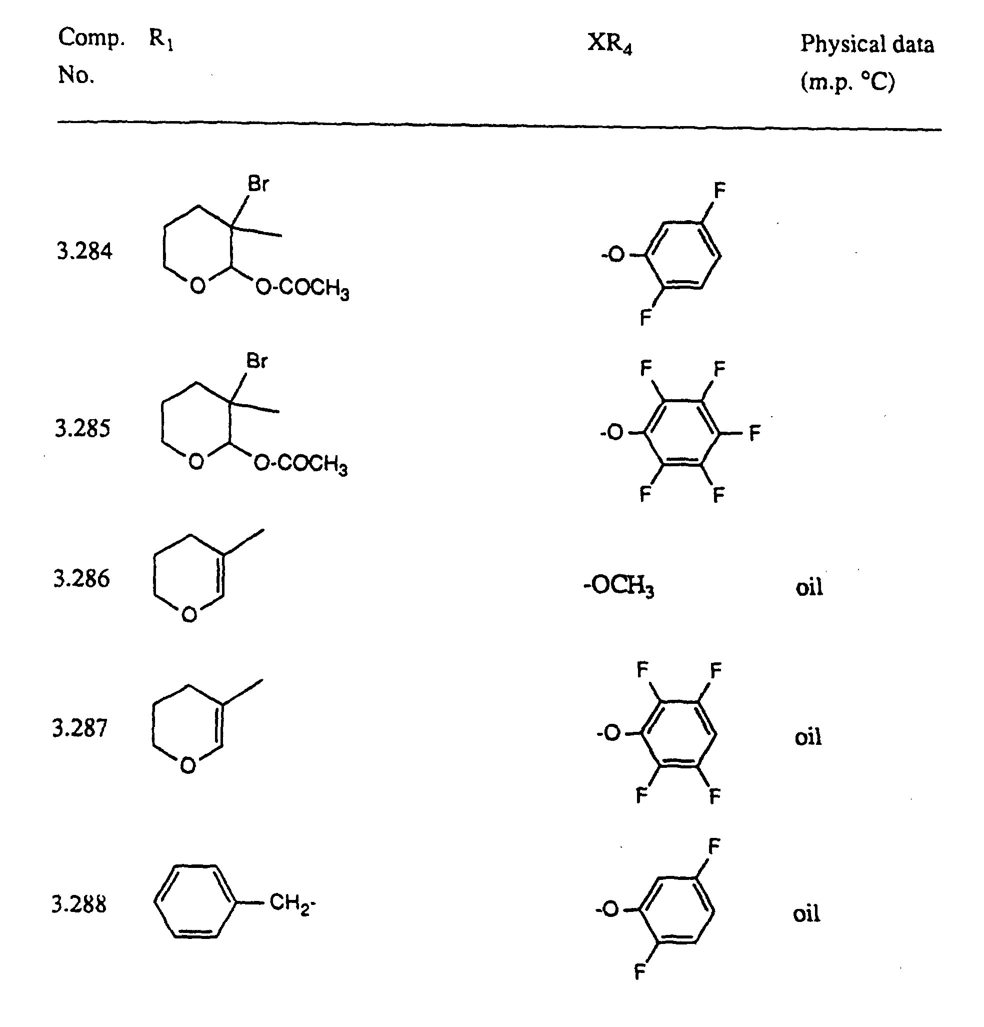

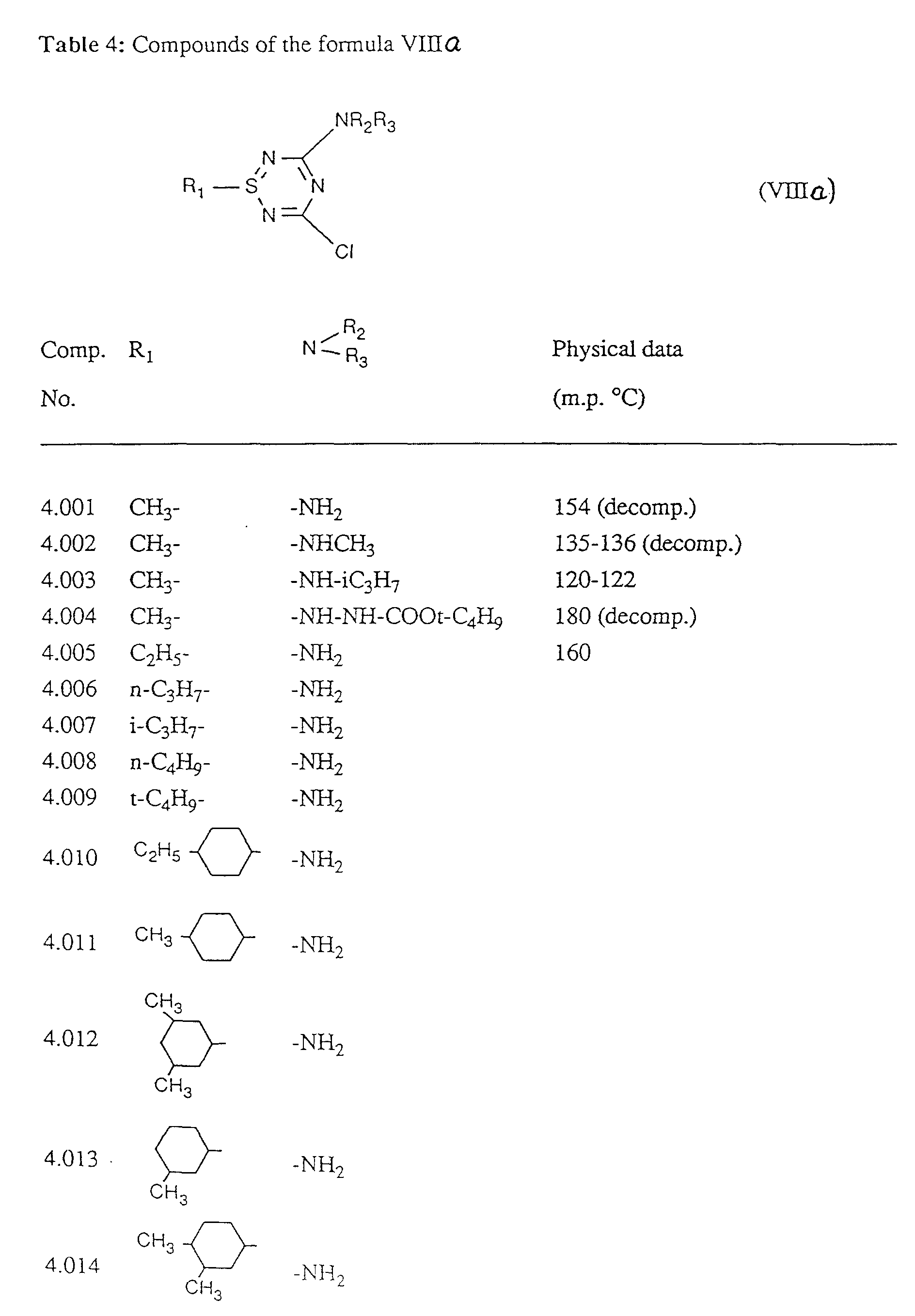

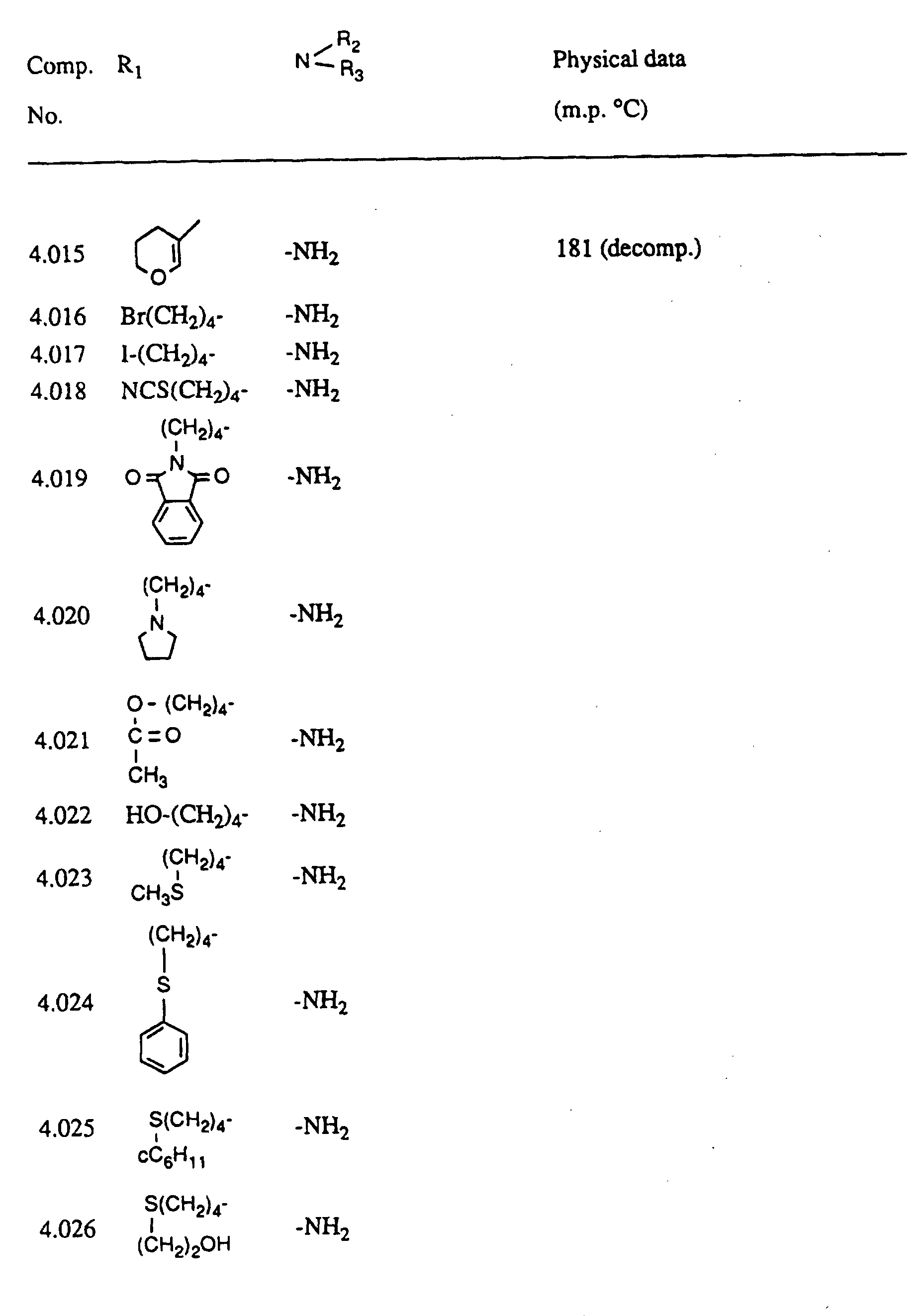

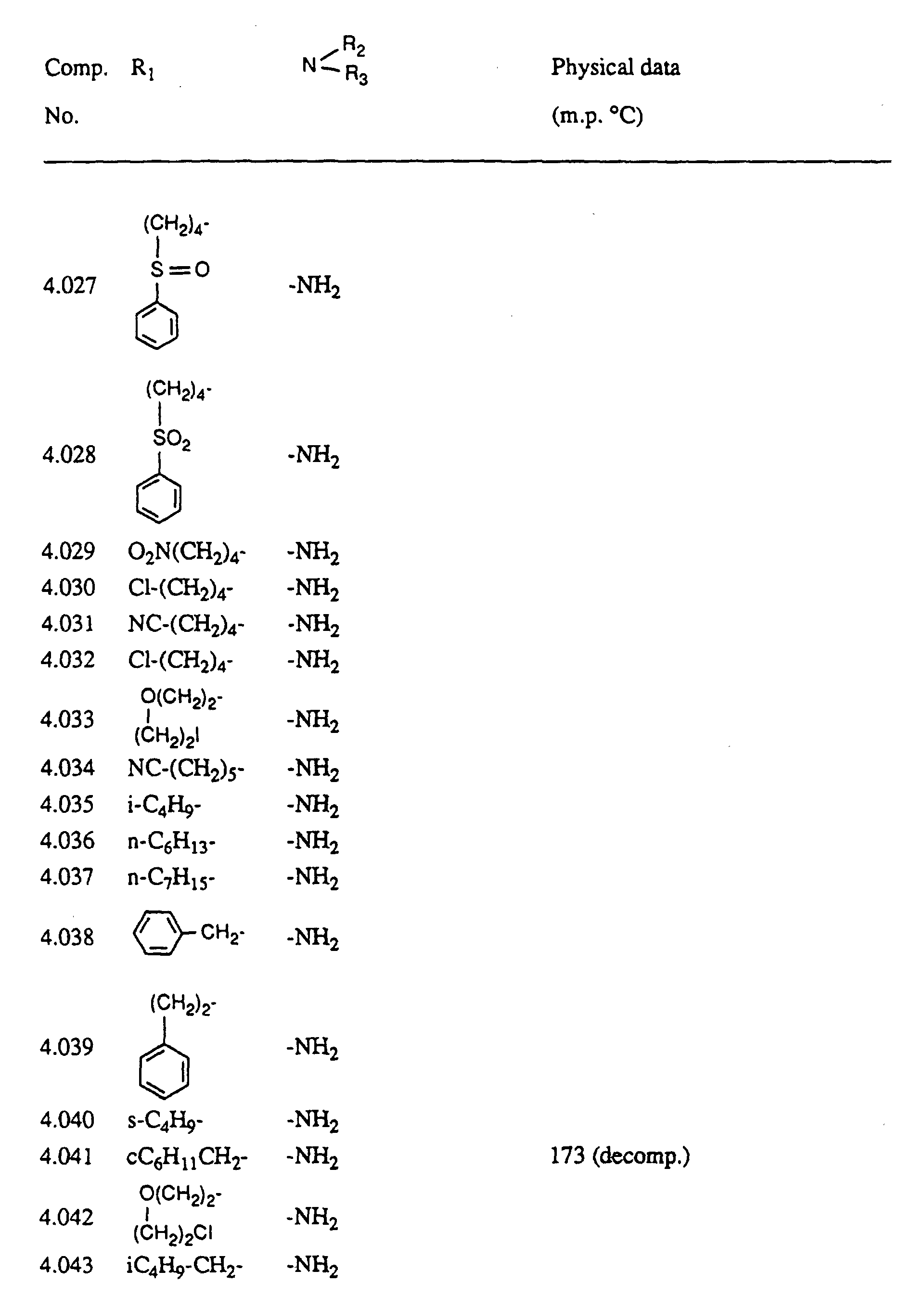

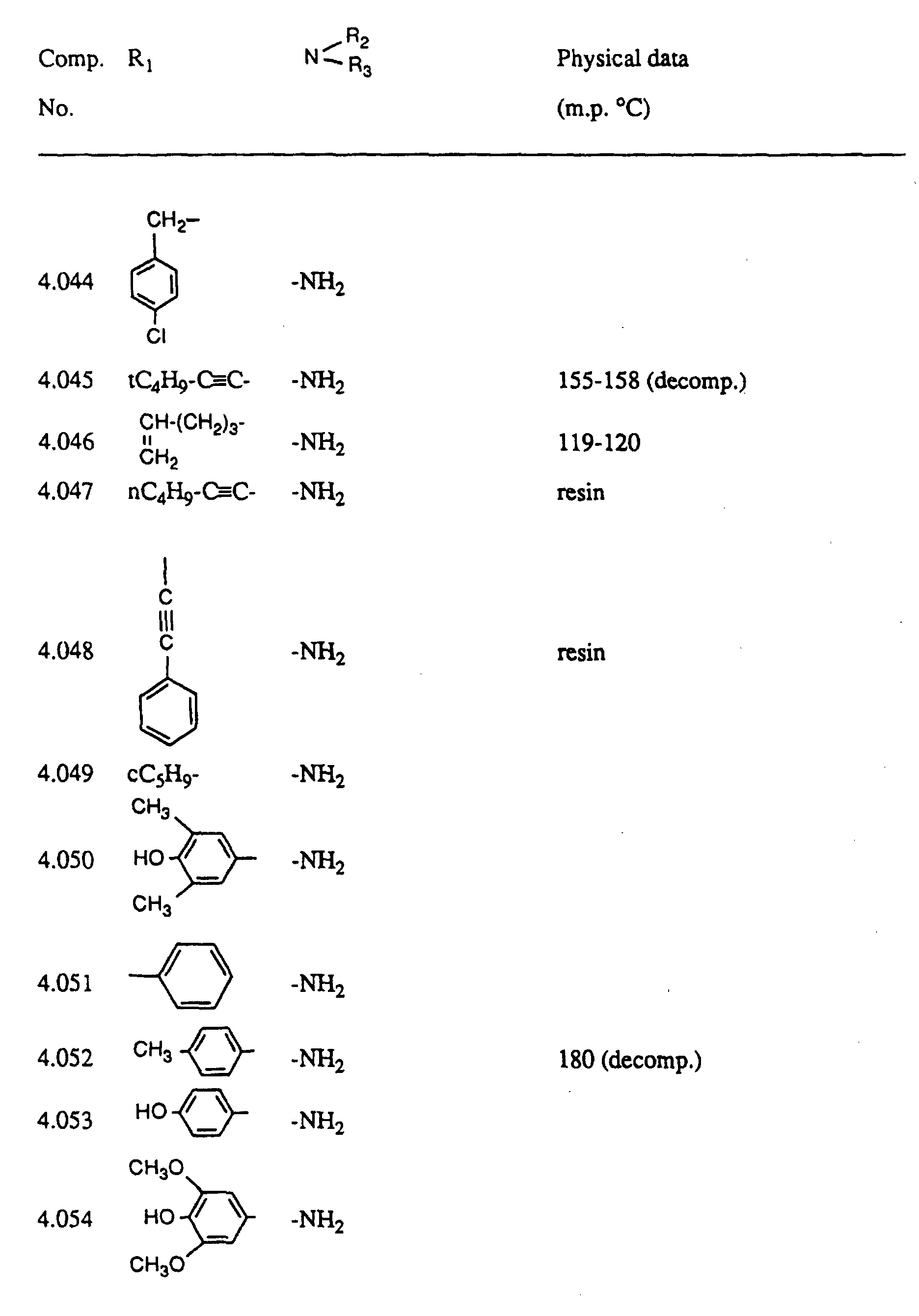

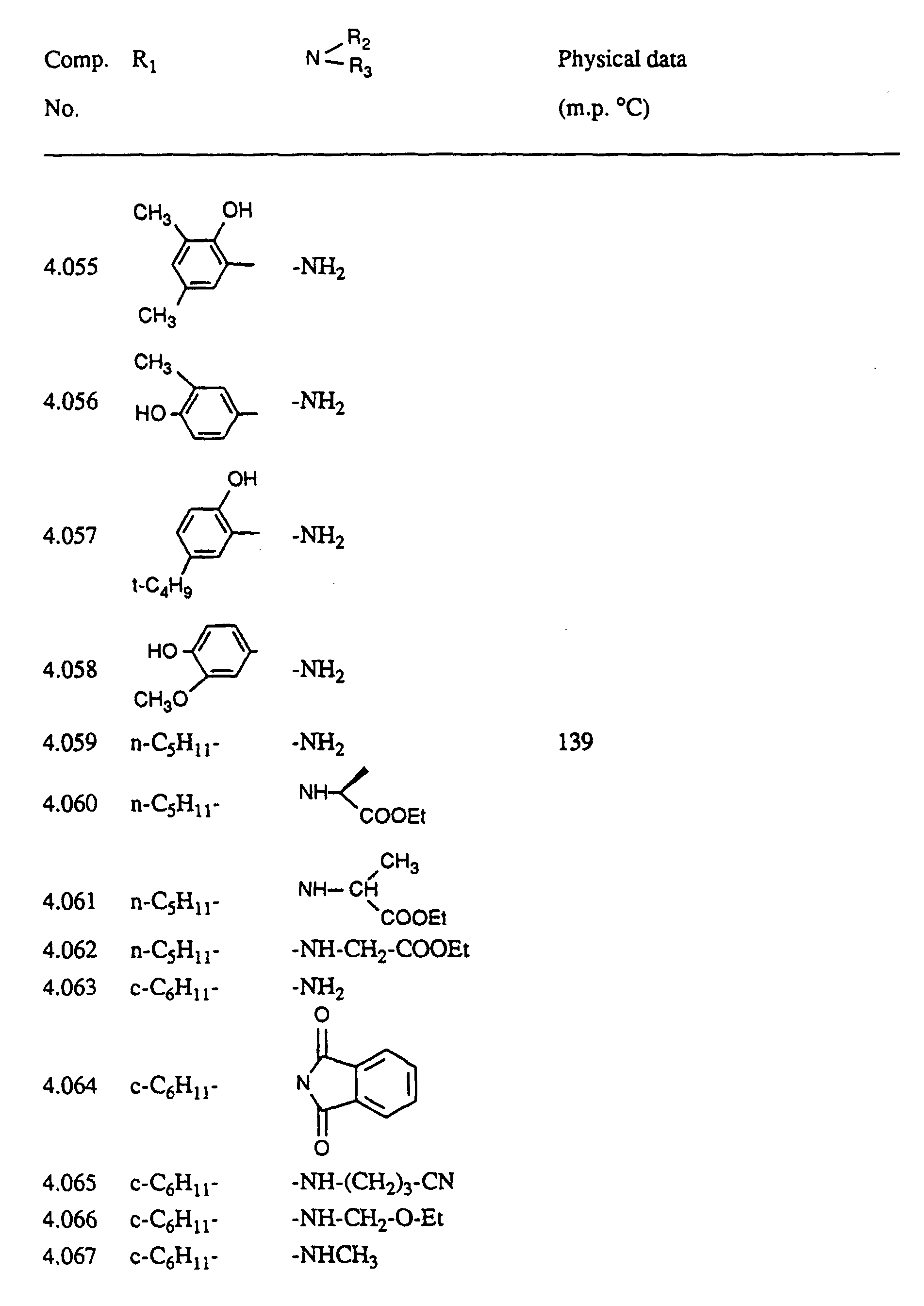

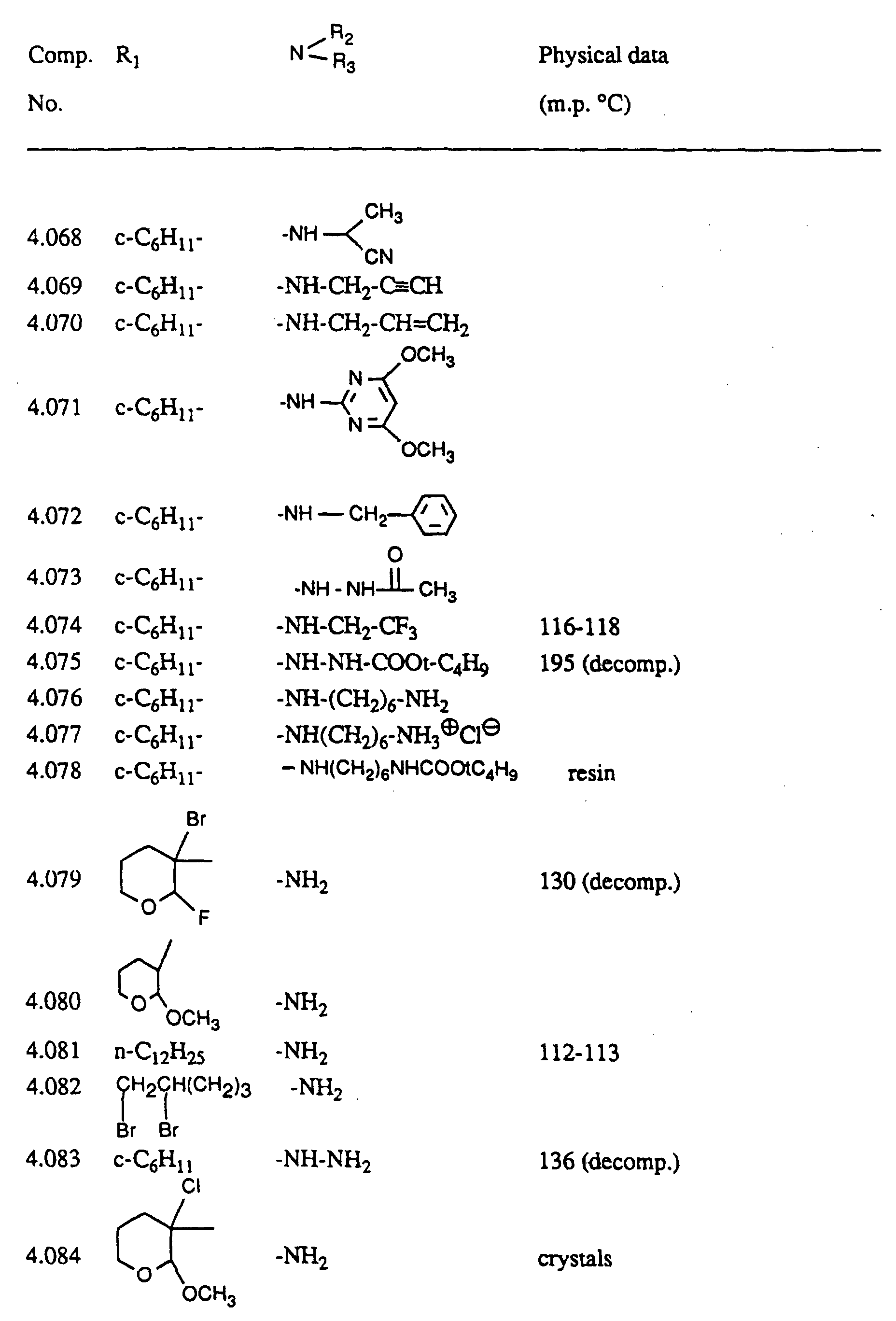

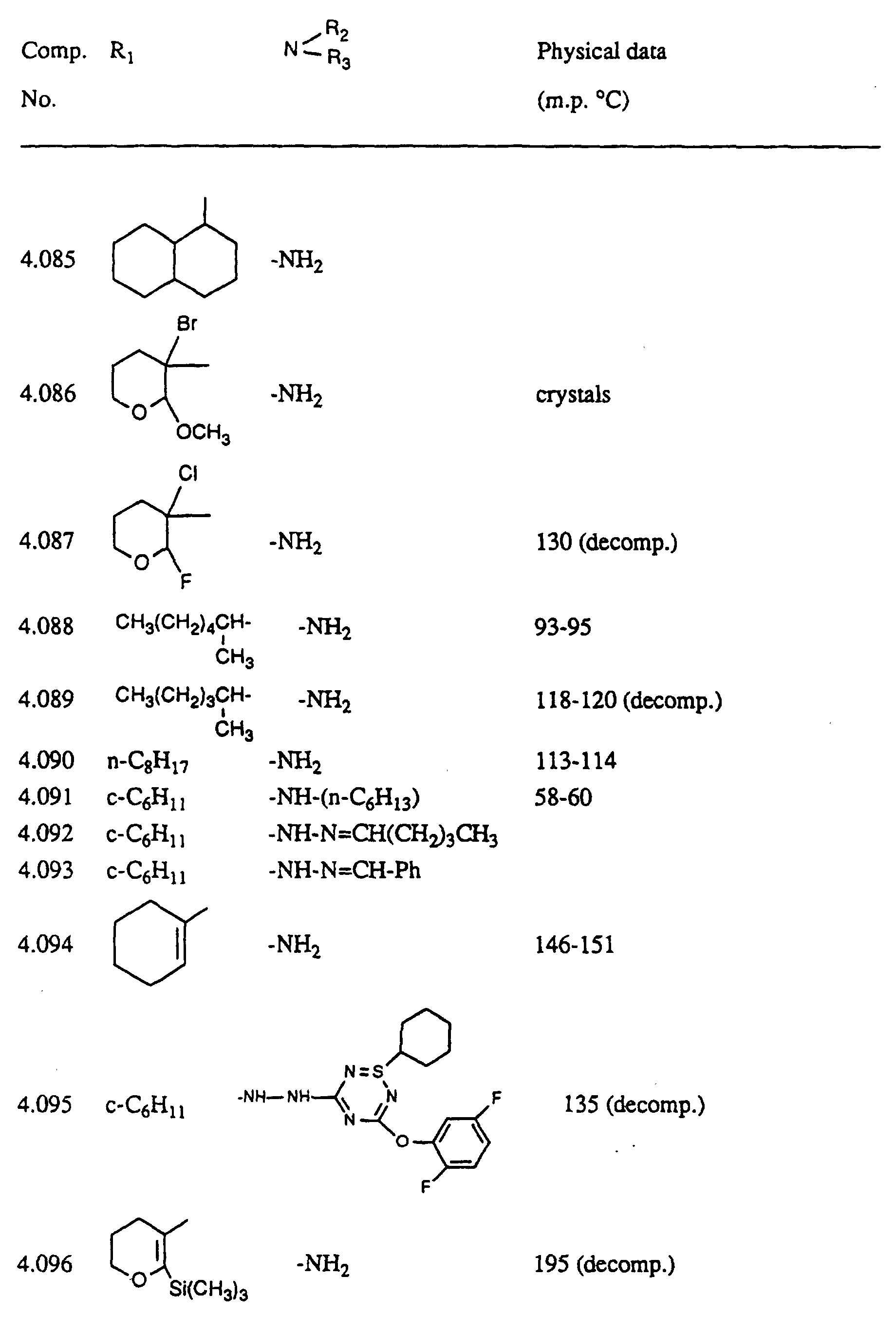

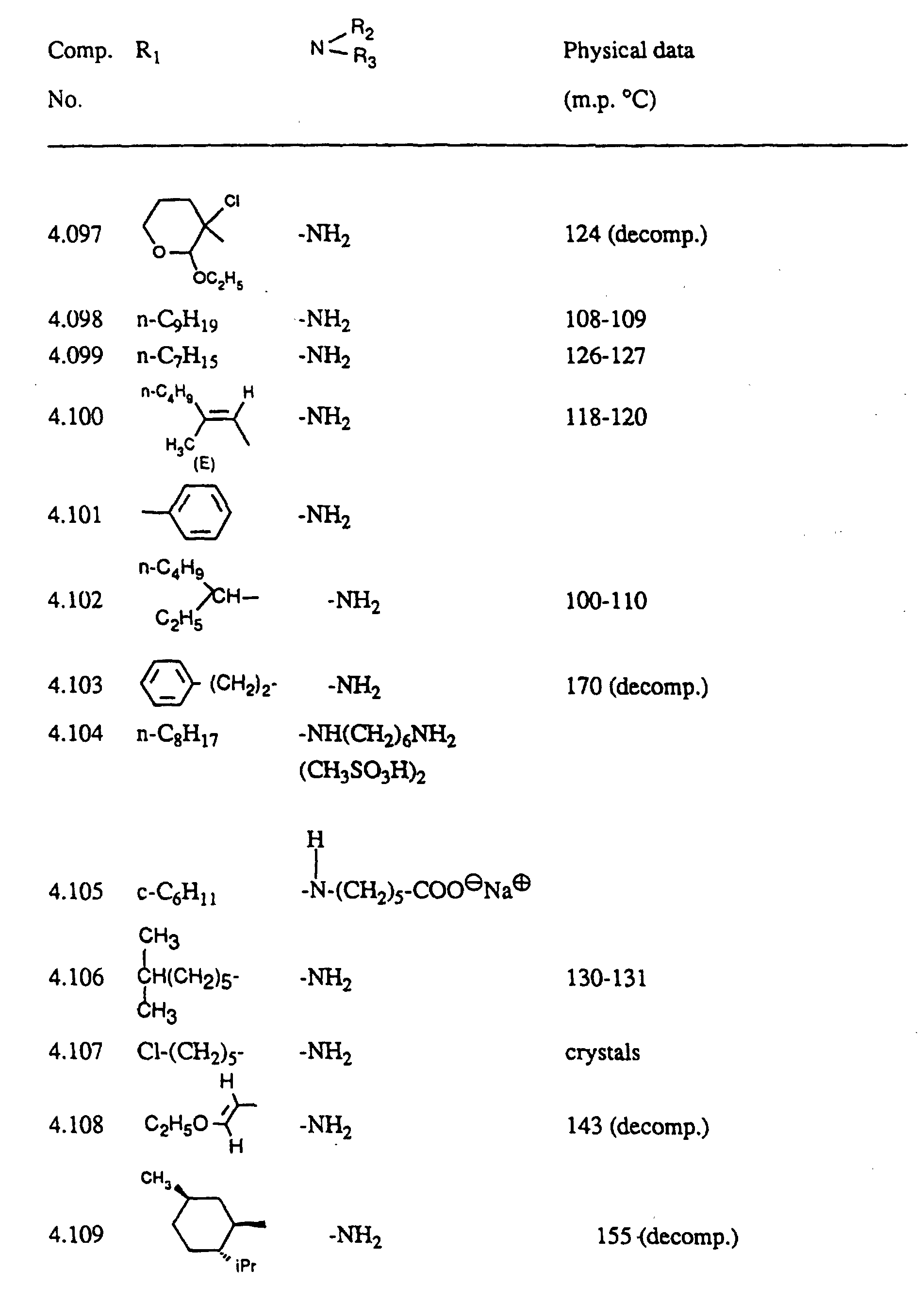

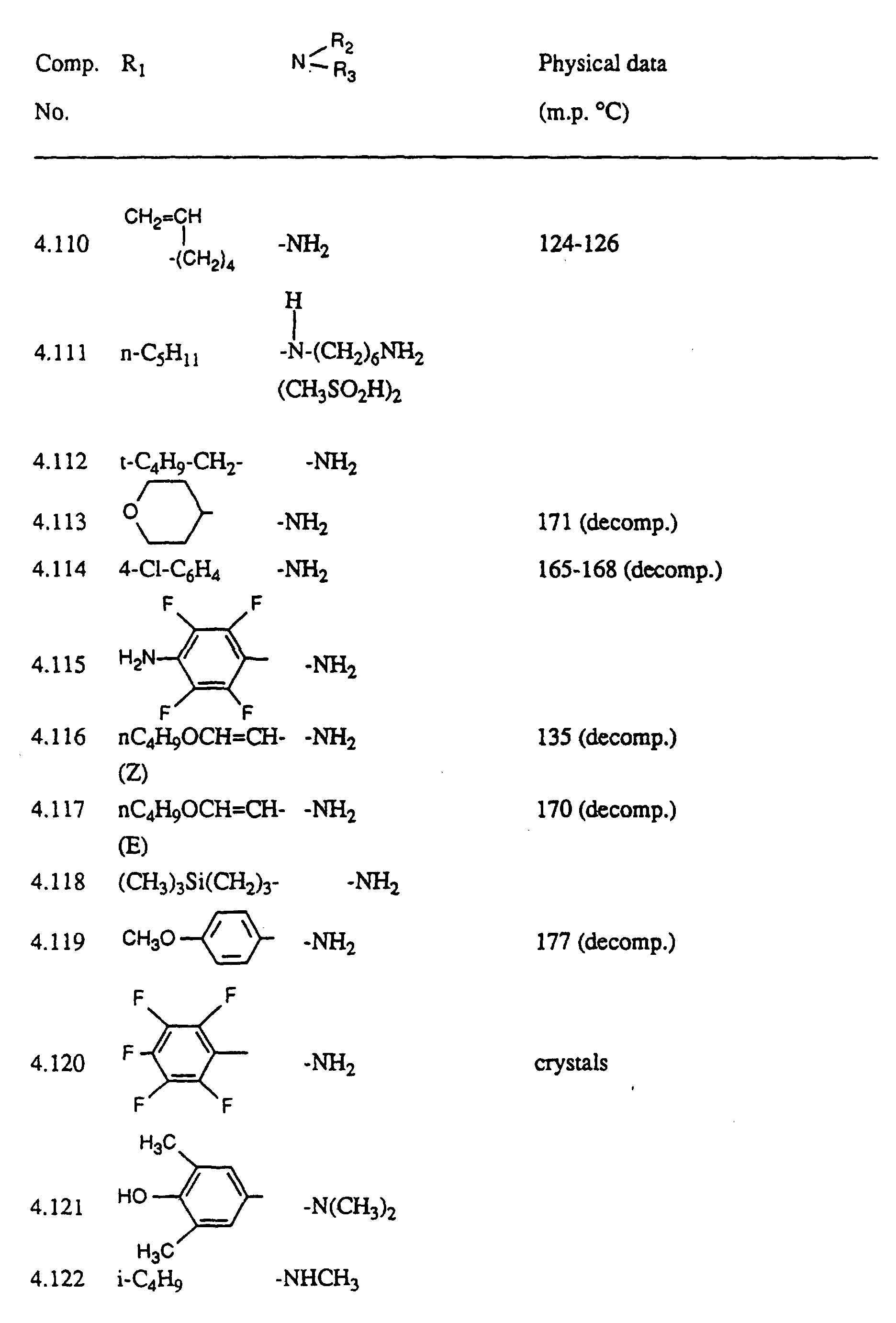

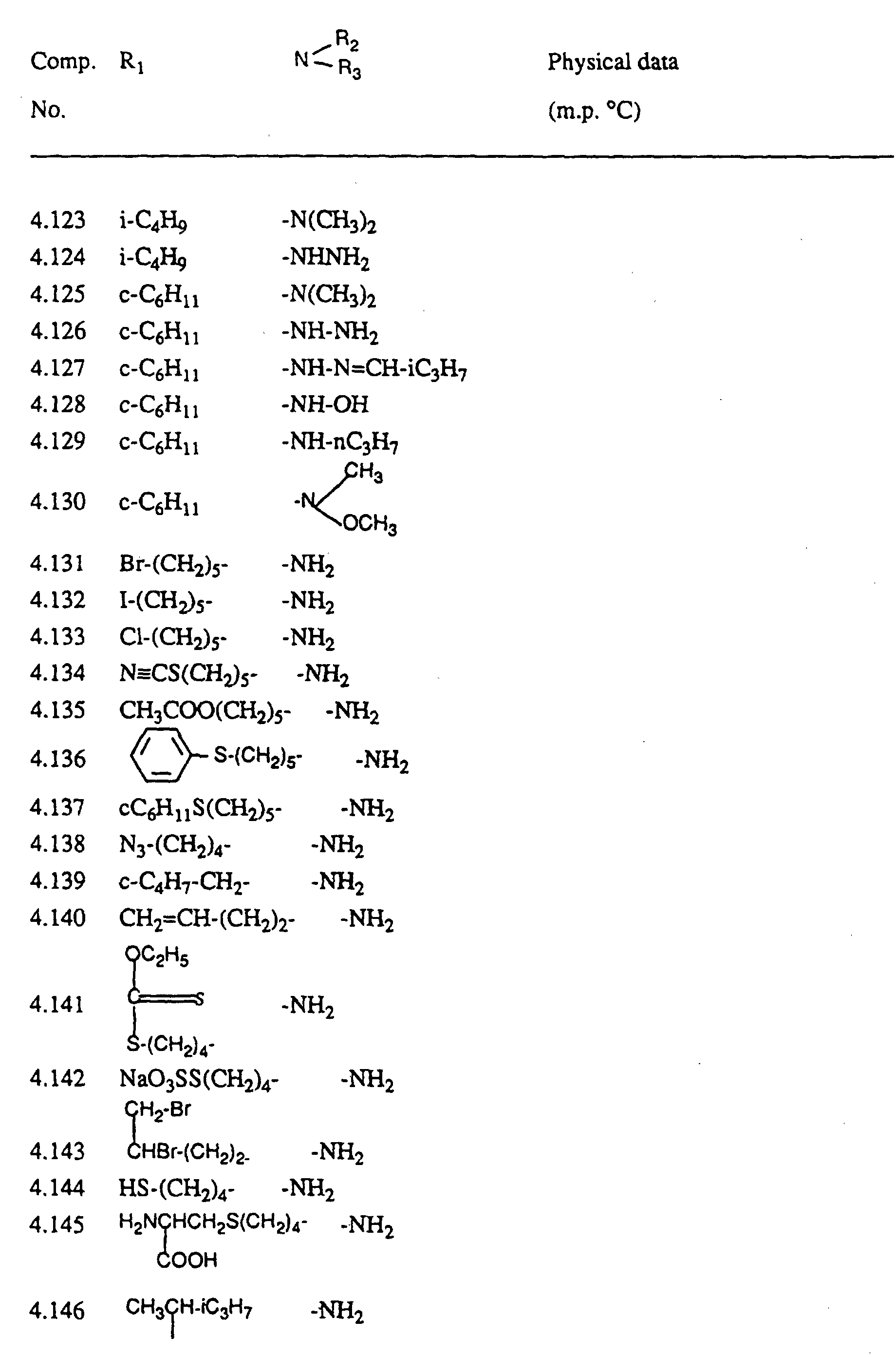

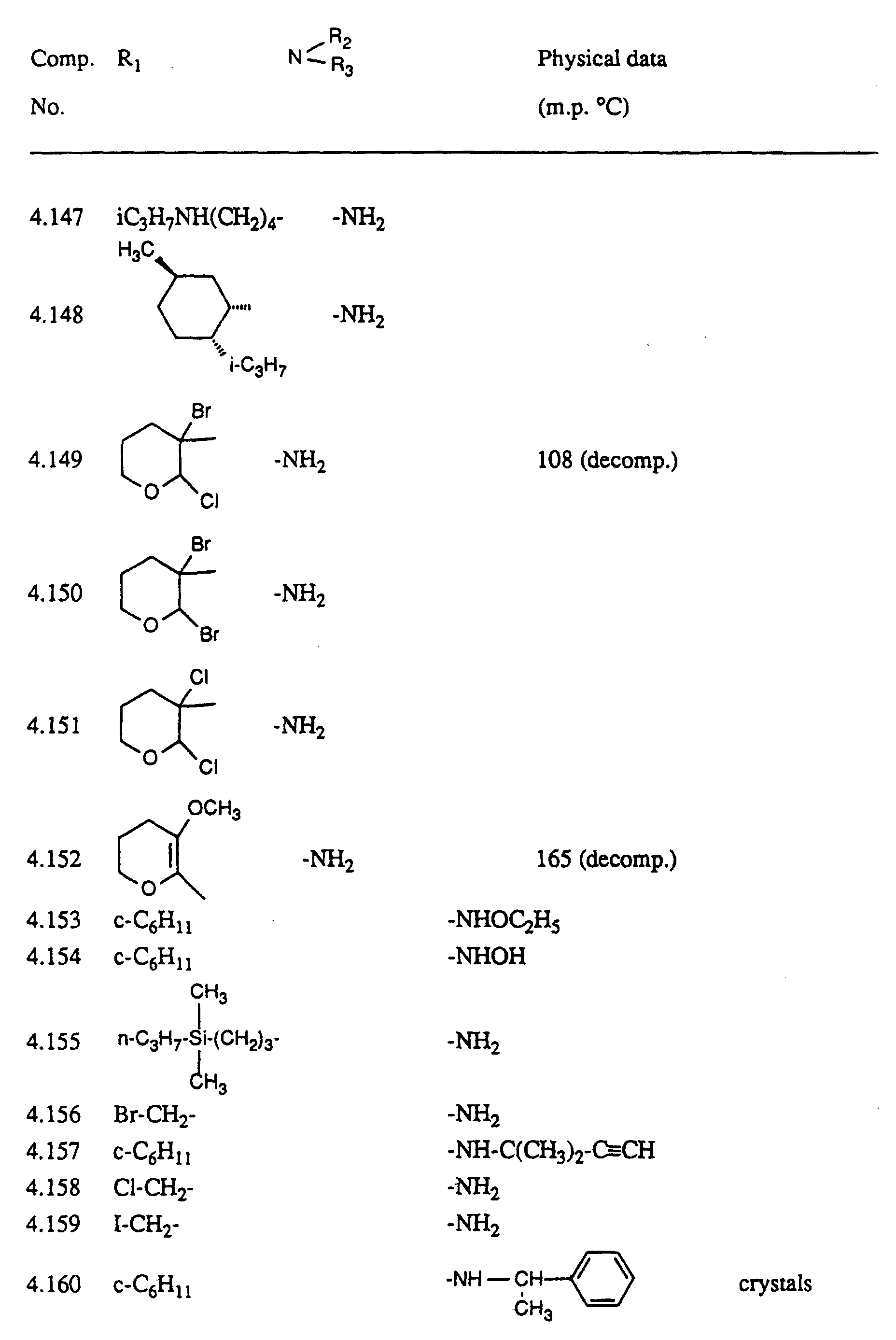

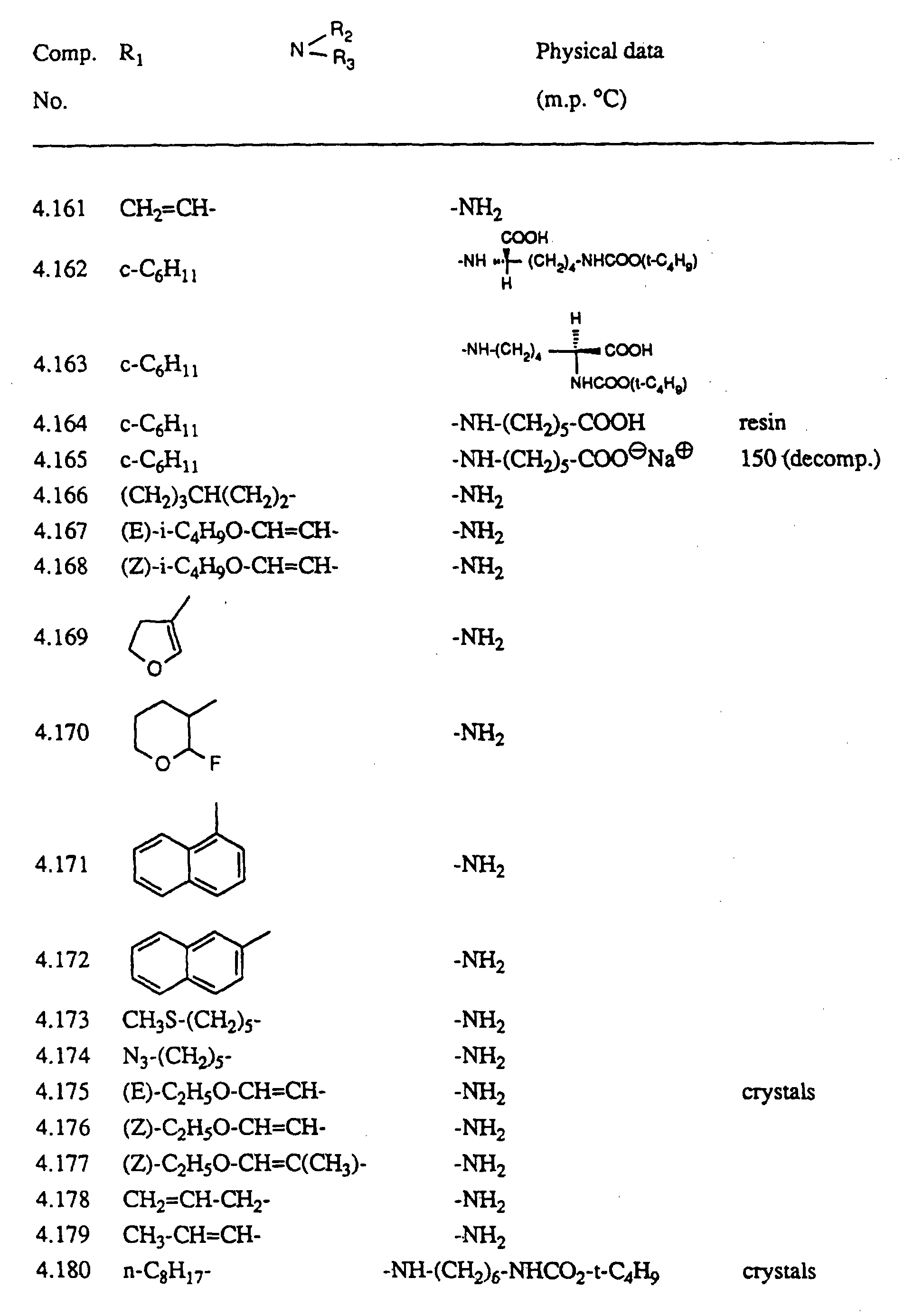

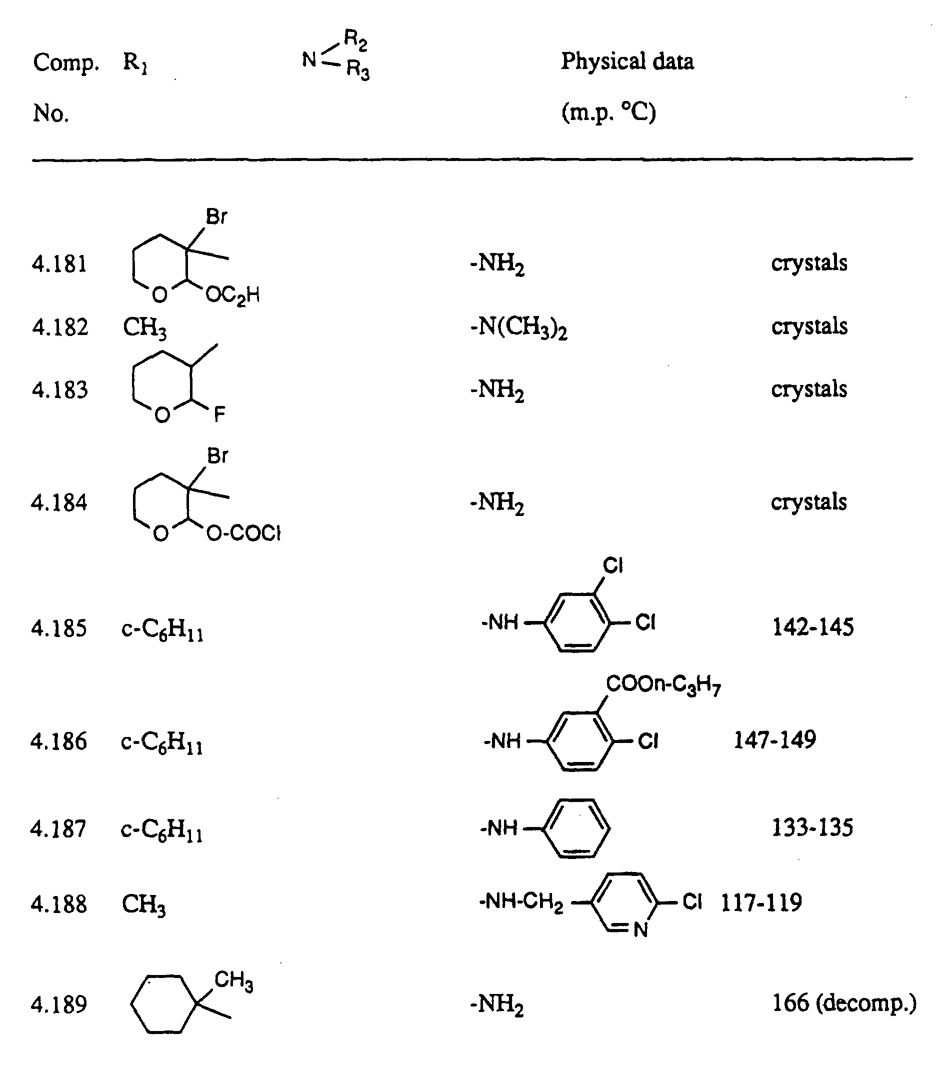

### Formulation Examples of active ingredients of the formula I (% = percent by weight)

| F1. Emulsion concentrates | a) | b) | c) | d) |
|---|---|---|---|---|
| | | | | |
| Active ingredient according to the table | 5 % | 10 % | 25 % | 50 % |
| Calcium dodecylbenzenesulfonate | 6 % | 8 % | 6 % | 8 % |
| Castor oil polyglycol ether (36 mol of EO) | 4 % | - | 4 % | 4 % |
| Octylphenol polyglycol ether (7-8 mol of EO) | - | 4 % | - | 2 % |
| Cyclohexanone | - | - | 10 % | 20 % |
| Mixture of aromatic hydrocarbons C₉-C₁₂ | 85 % | 78 % | 55 % | 16 % |

Emulsions of any desired concentration can be prepared from such concentrates by dilution with water.

| F2. Solutions | a) | b) | c) | d) |
|---|---|---|---|---|
| | | | | |
| Active ingredient according to the table | 5 % | 10 % | 50 % | 90 % |
| Dipropylene glycol methyl ether | - | 20 % | 20 % | - |
| Polyethylene glycol MW 400 | 20 % | 10 % | - | - |
| N-Methyl-2-pyrrolidone | - | - | 30 % | 10 % |
| Mixture of aromatic hydrocarbons C₉-C₁₂ | 7 5 % % | 60 % | - | - |

The solutions are suitable for use in the form of microdrops.

| F3. Wettable powders | a) | b) | c) | d) |
|---|---|---|---|---|
| | | | | |
| Active ingredient according to the table | 5 % | 25 % | 50 % | 80 % |
| Sodium lignosulfonate | 4 % | - | 3 % | - |
| Sodium lauryl sulfate | 2 % | 3 % | - | 4 % |
| Sodium diisobutylnaphthalenesulfonate | - | 6 % | 5 % | 6 % |
| Octylphenol polyglycol ether (7-8 mol of EO) | - | 1 % | 2 % | - |
| Highly disperse silica | 1 % | 3 % | 5 % | 10 % |
| Kaolin | 88 % | 62 % | 35 % | - |

The active ingredient is mixed thoroughly with the additives and the mixture is ground thoroughly in a suitable mill. This gives wettable powder which can be diluted with water to give suspensions of any concentration.

| F4. Coated granules | a) | b) | c) |
|---|---|---|---|
| | | | |
| Active ingredient according to the table | 0.1 % | 5 % | 15 % |
| Highly disperse silica | 0.9 % | 2 % | 2 % |
| Inorganic carrier (Ø0.1 - 1 mm) for example CaCO₃ or SiO₂ | 99.0 % | 93 % | 83 % |

The active ingredient is dissolved in methylene chloride, the solution is sprayed onto the carrier, and the solvent is subsequently evaporated in vacuo.

| F5. Coated granules | a) | b) | c) |
|---|---|---|---|
| | | | |
| Active ingredient according to the table | 0.1 % | 5 % | 15 % |
| Polyethylene glycol MW 200 | 1.0 % | 2 % | 3 % |
| Highly disperse silica | 0.9 % | 1 % | 2 % |
| Inorganic carrier (Ø0.1 - 1mm) for example CaCO₃ or SiO₂ | 98.0 % | 92 % | 80 % |

In a mixer, the finely ground active ingredient is applied uniformly to the carrier material which has been moistened with polyethylene glycol. This gives dust-free coated granules.

| F6. Extruder granules | a) | b) | c) | d) |
|---|---|---|---|---|
| | | | | |
| Active ingredient according to the table | 0.1% | 3 % | 5 % | 15 % |
| Sodium lignosulfonate | 1.5 % | 2 % | 3 % | 4 % |
| Carboxymethylcellulose | 1.4 % | 2 % | 2 % | 2 % |
| Kaolin | 97.0 % | 93 % | 90 % | 79 % |

The active ingredient is mixed with the additives, ground and moistened with water. This mixture is extruded and subsequently dried in a stream of air.

| F7. Dusts | a) | b) | c) |
|---|---|---|---|
| | | | |
| Active ingredient according to the table | 0.1 % | 1 % | 5 % |
| Talc | 39.9 % | 49 % | 35 % |
| Kaolin | 60.0 % | 50 % | 60 % |

Ready-to-use dusts are obtained by mixing the active ingedient with the carriers and grinding the mixture in a suitable mill.

| F8. Suspension concentrates | a) | b) | c) | d) |
|---|---|---|---|---|
| | | | | |
| Active ingredient according to the table | 3 % | 10 % | 25 % | 50 % |
| Ethylene glycol | 5 % | 5 % | 5 % | 5 % |
| Nonylphenol polyglycol ether (15 mol of EO) | - | 1 % | 2 % | - |
| Sodium lignosulfonate | 3 % | 3 % | 4 % | 5 % |
| Carboxymethylcellulose | 1 % | 1 % | 1 % | 1 % |
| 37% aqueous formaldehyde solution | 0.2% | 0.2 % | 0.2 % | 0.2 % |
| Silicone oil emulsion | 0.8 % | 0.8 % | 0.8 % | 0.8 % |
| Water | 87 % | 79 % | 62 % | 38 % |

The finely ground active ingredient is mixed intimately with the additives. This gives a suspension concentrate from which suspensions of any desired concentration can be prepared by dilution with water.

### Biological examples

### Example B1: Herbicidal action before emergence of the plants

Monocotyledon and dicotyledon test plants are sown in standard soil in plastic pots. Immediately after sowing, the test substances are sprayed on (500 l of water/ha) in the form of an aqueous suspension (prepared with the formulation of Example F1) to give a dose of 2 kg of a.s./ha. The test plants are subsequently grown in a greenhouse under optimal conditions. After a test period of 3 weeks, the experiment is evaluated on the basis of a nine-step key (1 = complete damage, 9 = no action). Scores of 1 to 4 (in particular 1 to 3) mean a good to very good herbicidal action.

**Table B1:**

| pre-emergence action: | | | | |
|---|---|---|---|---|
| Test plant: Active ingredient No. | Avena | Setaria | Sinapis | Stellaria |
| 1.015 | 1 | 2 | 1 | 1 |
| 1.016 | 1 | 1 | 1 | 1 |
| 1.018 | 1 | 2 | 1 | 3 |
| 1.019 | 1 | 1 | 1 | 1 |
| 1.021 | 1 | 4 | 1 | 1 |
| 1.039 | 1 | 2 | 9 | 3 |
| 1.060 | 3 | 3 | 3 | 1 |
| 1.075 | 2 | 1 | 1 | 1 |
| 1.076 | 2 | 2 | 4 | 3 |
| 1.078 | 3 | 1 | 1 | 1 |
| 1.079 | 3 | 1 | 4 | 1 |
| 1.081 | 2 | 2 | 3 | 1 |
| 1.108 | 2 | 1 | 6 | 4 |
| 1.112 | 2 | 1 | 3 | 1 |
| 1.113 | 2 | 2 | 3 | 1 |
| 1.121 | 4 | 2 | 1 | 1 |
| 1.128 | 1 | 1 | 1 | 1 |
| 1.134 | 1 | 1 | 1 | 1 |
| 1.136 | 1 | 2 | 1 | 1 |
| 1.137 | 2 | 1 | 1 | 1 |
| 1.140 | 2 | 2 | 1 | 1 |
| 1.141 | 1 | 1 | 1 | 1 |
| 1.150 | 2 | 3 | 5 | 1 |
| 1.161 | 1 | 1 | 1 | 1 |
| 1.191 | 1 | 1 | 3 | 1 |
| 1.235 | 2 | 2 | 1 | 1 |
| 1.237 | 1 | 1 | 1 | 1 |
| 1.240 | 2 | 2 | 1 | 1 |
| 1.241 | 1 | 4 | 1 | 1 |
| 1.259 | 2 | 1 | 1 | 9 |
| 1.278 | 2 | 2 | 3 | 5 |
| 1.282 | 6 | 3 | 1 | 3 |
| 1.293 | 2 | 4 | 4 | 2 |

The same results are obtained when the compounds of the formula I are formulated in accordance with suitable examples F2 to F8.

### Example B2: Post-emergence herbicidal action

Monocotyledon and dicotyledon test plants are grown in standard soil in the greenhouse in plastic pots and, in the 4- to 6-leaf stage, sprayed with an aqueous suspension of the test substances of the formula I, prepared with a 25% wettable powder {Example F3, b)), which corresponds to a dose of 2000 g of a.s./ha (5001 of water/ha). The test plants are subsequently grown on in the greenhouse under optimal conditions. After a test period of approximately 18 days, the experiment is evaluated using a nine-step key (1 = complete damage, 9 = no action). Scores of 1 to 4 (in particular 1 to 3) mean a good to very good herbicidal action. In this experiment, the compounds of the formula I have a powerful herbicidal action. The same results are obtained when the compounds of the formula I are formulated in accordance with Examples F1 to F2 and F4 to F8.

**Table B2:**

| post-emergence action: | | | | |
|---|---|---|---|---|
| Test plant: active ingredient No. | Avena | Setaria | Sinapis | Stellaria |
| 1.237 | 1 | 5 | 1 | 1 |
| 1.141 | 1 | 2 | 1 | 1 |
| 1.075 | 1 | 2 | 1 | 1 |
| 1.137 | 1 | 5 | 1 | 2 |
| 1.021 | 1 | 3 | 1 | 1 |

The same results are obtained when the compounds of the formula I are formulated in accordance with suitable examples F2 to F8.

## Claims

1. A compound of the formula I 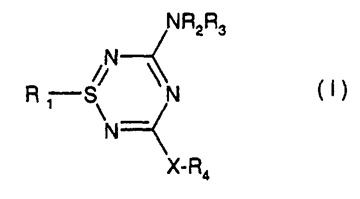 in which
R₁ is C₁-C₂₀alkyl, C₃-C₈cycloalkyl, C₃-C₈cycloalkyl-C₁-C₁₇alkyl, C₁-C₁₇alkyl-C₃-C₈cycloalkyl, C₂-C₁₇alkenyl, C₃-C₈cycloalkyl-C₂-C₁₇alkenyl, C₂-C₁₇alkenyl-C₃-C₈cycloalkyl, C₂-C₁₇alkynyl, C₃-C₈cycloalkyl-C₂-C₁₇alkynyl, C₂-C₁₇alkynyl-C₃-C₈cycloalkyl, C₅-C₈cycloalkenyl, C₅-C₈cycloalkenyl-C₁-C₁₇alkyl, C₁-C₁₇alkyl-C₅-C₈cycloalkenyl, C₅-C₈cycloalkenyl-C₂-C₁₇alkenyl, C₂-C₁₇alkenyl-C₅-C₈cycloalkenyl, C₅-C₈cycloalkenyl-C₂-C₁₇alkynyl, C₂-C₁₇alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₁₇alkynyl-C₂-C₁₇alkenyl or C₂-C₁₇alkenyl-C₂-C₁₇alkynyl, it being possible for these substituents to be unsubstituted or substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, phenyl, biphenyl, naphthyl or saturated or unsaturated heterocylyl having 3 to 8 ring atoms which, in addition to carbon atoms, has at least one hetero atom selected from nitrogen, oxygen and sulfur, it being possible for these phenyl, naphthyl and heterocyclyl substituents to be unsubstituted or substituted by halogen, cyano, nitro, -OR₅, -NR₁₀R₁₁ or -CONR₈R₉, or
R₁ is saturated or unsaturated heterocylyl having 3 to 8 ring atoms which, in addition to carbon atoms, has at least one hetero atom selected from nitrogen, oxygen and sulfur, the heterocycle being bonded to the thiatriazine ring via one of its carbon atoms and it being possible for the heterocycle to be substituted by halogen, cyano, nitro, =O, -OR₅, -S(O)ₙR₆, -COOR₇, -CONR₈R₉, -NR₁₀R₁₁, =NR₁₂, =N-NR₁₃R₁₄, C₁-C₁₇alkyl, C₂-C₁₇alkenyl, C₂-C₁₇alkynyl, C₃-C₈cycloalkyl, C₅-C₈cycloalkenyl, phenyl, biphenyl, naphthyl, C₁-C₁₇alkyl which is substituted by halogen, cyano, nitro, -OR₅, -CONR₈R₉ or -NR₁₀R₁₁, C₂-C₁₇alkenyl which is substituted by halogen, cyano, nitro, -OR₅, -CONR₈R₉ or -NR₁₀R₁₁, C₂-C₁₇alkynyl which is substituted by halogen, cyano, nitro, -OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁, phenyl which is substituted by halogen, cyano, nitro, -OR₅, -CONR₈R₉ or -NR₁₀R₁₁, biphenyl which is substituted by halogen, cyano, nitro, -OR₅, -CONR₈R₉ or -NR₁₀R₁₁, or naphthyl which is substituted by halogen, cyano, nitro, -OR₅, -CONR₈R₉ or -NR₁₀R₁₁, or
R₁ is phenyl, biphenyl, naphthyl, C₁-C₁₀alkylphenyl, C₁-C₁₀alkylbiphenyl, C₁-C₁₀alkylnaphthyl, C₂-C₁₀alkenylphenyl, C₂-C₁₀alkenylbiphenyl, C₂-C₁₀alkenylnaphthyl, C₂-C₁₀alkynylphenyl, C₂-C₁₀alkynylbiphenyl or C₂-C₁₀alkynylnaphthyl, it being possible for these substituents to be unsubstituted or substituted by halogen, cyano, nitro, -OR₅, -S(O)ₙR₆,-COOR₇, -CONR₈R₉ or -NR₁₀R₁₁, in which
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₇alkylcarbonyl, benzoyl, halobenzoyl, C₁-C₆alkylamino, C₂-C₈dialkylamino, -N=CH₂, -N=CH-C₁-C₄alkyl, -N=C(C₁-C₄alkyl)₂, C₃-C₆trialkylsilyl, C₃-C₇cycloalkyl, C₂-C₇alkenyl or C₃-C₇alkynyl,
n is 0, 1 or 2,
R₆ is hydrogen or cyano if n is 0 or
R₆ is C₁-C₅alkyl, C₁-C₅haloalkyl, C₁-C₅hydroxyalkyl, phenyl, naphthyl, C₁-C₄alkylphenyl, C₁-C₄alkylnaphthyl, halophenyl, halonaphthyl, saturated or unsaturated heterocyclyl or haloheterocyclyl having 3 to 8 ring atoms which, in addition to carbon atoms, at least one hetero atom selected from nitrogen, oxygen and sulfur, or C₃-C₇cycloalkyl,
R₇ is hydrogen, C₁-C₁₂alkyl, C₁-C₁₂haloalkyl, C₁-C₆nitroalkyl, C₁-C₆cyanoalkyl, phenyl, naphthyl, halophenyl, halonaphthyl, C₂-C₁₀alkoxyalkyl, C₁-C₁₀alkylcarbonyl, saturated or unsaturated heterocyclyl or haloheterocyclyl having 3 to 8 ring atoms which, in addition to carbon atoms, at least one hetero atom selected from nitrogen, oxygen and sulfur, C₃-C₇cycloalkyl, C₃-C₇halocycloalkyl, -N=CH₂, -N=CH-C₁-C₄alkyl, -N=C(C₁-C₄alkyl)₂ or C₂-C₆dialkylamino,
R₈ and R₉ independently of one another are hydrogen, phenyl, naphthyl, halophenyl, halonaphthyl, C₁-C₈alkyl, C₁-C₈haloalkyl, C₁-C₈alkoxy, phenoxy, naphthoxy, halophenoxy, halonaphthoxy, C₁-C₇cyanoalkyl, C₃-C₇alkenyl, C₃-C₇alkynyl or C₂-C₈alkoxyalkyl, or
R₈ and R₉ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur,
R₁₀ and R₁₁ independently of one another are hydrogen, phenyl, naphthyl, halophenyl, halonaphthyl, C₁-C₈alkyl, C₁-C₈haloalkyl, C₁-C₈alkoxy, phenoxy, naphthoxy, halophenoxy, halonaphthoxy, C₁-C₇cyanoalkyl, C₃-C₇alkenyl, C₃-C₇alkynyl, C₂-C₈alkoxyalkyl, C₁-C₆alkylamino, C₂-C₆dialkylamino, formyl, C₂-C₈alkylcarbonyl, phenylcarbonyl or naphthylcarbonyl, it being possible therein for the phenyl or naphthyl moiety to be substituted by C₁-C₄alkyl, halogen, C₁-C₄alkoxy, hydroxy, cyano, nitro or C₁-C₆alkoxycarbonyl, or R₁₀ and R₁₁ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur,
R₁₂ is hydrogen, C₁-C₈alkyl, C₁-C₈haloalkyl, phenyl, halophenyl, cyanophenyl, nitrophenyl, C₁-C₄alkylphenyl, C₁-C₄alkoxyphenyl, naphthyl, cyanonaphthyl, nitronaphthyl, halonaphthyl, C₁-C₄alkylnaphthyl, C₁-C₄alkoxynaphthyl, phenyl-C₁-C₆alkyl or naphthyl-C₁-C₆alkyl, and
R₁₃ and R₁₄ independently of one another are hydrogen, C₁-C₆alkyl, C₁-C₆alkylcarbonyl, phenyl, halophenyl, cyanophenyl, nitrophenyl, C₁-C₄alkylphenyl, C₁-C₄alkoxyphenyl, naphthyl, cyanonaphthyl, nitronaphthyl, halonaphthyl, C₁-C₄alkylnaphthyl or C₁-C₄alkoxynaphthyl,
R₂ and R₃ independently of one another are hydrogen, C₁-C₆alkyl, C₁-C₆alkyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, where M is ammonium or an alkali metal atom or alkaline earth metal atom, C₃-C₈cycloalkyl, C₁-C₅alkylcarbonyloxy, phenylcarbonyloxy, naphthylcarbonyloxy, C₁-C₆alkylamino, C₁-C₅alkoxycarbonyl, C₂-C₁₂dialkylamino, phenyl, naphthyl, phenoxy, naphthoxy, biphenyl, biphenyloxy, phenthio or naphthio, it being possible for the abovementioned aromatic rings to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or CONR₈R₉
R₂ and R₃ independently of one another are C₂-C₆alkenyl, C₂-C₆alkenyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, where M is ammonium or an alkali metal atom or alkaline earth metal atom, C₃-C₈cycloalkyl, C₂-C₅alkylcarbonyloxy, phenylcarbonyloxy, naphthylcarbonyloxy, C₁-C₆alkylamino, C₂-C₅alkoxycarbonyl, C₂-C₁₂dialkylamino, phenyl, naphthyl, phenoxy, naphthoxy, biphenyl, biphenyloxy, phenthio or naphthio, it being possible for the abovementioned aromatic rings to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or CONR₈R₉, or
R₂ and R₃ independently of one another are C₃-C₆alkynyl, C₃-C₆alkynyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, where M is ammonium or an alkali metal atom or alkaline earth metal atom, C₃-C₈cycloalkyl, C₁-C₅alkylcarbonyloxy, phenylcarbonyloxy, naphthylcarbonyloxy, C₁-C₆alkylamino, C₁-C₅alkoxycarbonyl, C₂-C₁₂dialkylamino, phenyl, naphthyl, phenoxy, naphthoxy, biphenyl, biphenyloxy, phenthio or naphthio, it being possible for the abovementioned aromatic rings to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or CONR₈R₉, or
R₂ and R₃ independently of one another are formyl, C₁-C₁₅alkylcarbonyl, C₂-C₁₅alkenylcarbonyl, C₄-C₉cycloalkylcarbonyl, C₆-C₉cycloalkenylcarbonyl or C₃-C₈cycloalkyl-C₁-C₆alkylcarbonyl, it being possible for these substituents to be substituted by halogen, cyano, nitro, hydroxyl, amino, C₁-C₆alkylamino, C₂-C₁₂dialkylamino, -COOH,-COOM, in which M is ammonium or an alkali metal atom or alkaline earth metal atom, or C₁-C₈alkoxycarbonyl, C₄-C₁₀cycloalkoxycarbonyl, C₁-C₈alkylaminocarbonyl or C₂-C₁₂dialkylaminocarbonyl, or
R₂ and R₃ independently of one another are saturated or unsaturated heterocyclyl or heterocyclylcarbonyl having 3 to 8 ring atoms which, in addition to carbon atoms, at least one hetero atom selected from nitrogen, oxygen and sulfur, heterocyclyl as defined above which is substituted by halogen, cyano, nitro, C₁-C₅alkyl, C₁-C₅alkoxy, C₁-C₅alkylcarbonyl, C₁-C₅alkylcarbonyloxy, C₁-C₆alkoxycarbonyl, aminocarbonyl, C₁-C₆alkylaminocarbonyl or C₂-C₁₁dialkylaminocarbonyl, or heterocyclylcarbonyl as defined above which is substituted by halogen, cyano, nitro, C₁-C₅alkyl, C₁-C₅alkoxy, C₁-C₅alkylcarbonyl, C₁-C₆alkoxycarbonyl, aminocarbonyl, C₁-C₆alkylamino or C₁-C₅alkylcarbonyloxy, or
R₂ and R₃ independently of one another are phenylcarbonyl, biphenylcarbonyl, naphthylcarbonyl, phenyl-C₁-C₆alkylcarbonyl, biphenyl-C₁-C₆alkylcarbonyl, naphthyl-C₁-C₆alkylcarbonyl, phenyl-C₂-C₆alkenylcarbonyl, biphenyl-C₂-C₆alkenylcarbonyl, naphthyl-C₂-C₆alkenylcarbonyl, phenyl-C₃-C₆alkynylcarbonyl, biphenyl-C₃-C₆alkynylcarbonyl or naphthyl-C₃-C₆alkynylcarbonyl, it being possible for these substituents to be substituted by C₁-C₅alkyl, C₁-C₅alkoxy, C₁-C₅alkylthio, C₁-C₅haloalkyl, C₁-C₅alkylcarbonyl, halogen, cyano, amino, nitro, -COOR₇, C₁-C₈alkoxycarbonyl, hydroxyl, C₁-C₅alkylsulfinyl, C₁-C₅alkylsulfonyl, C₁-C₆alkylaminocarbonyl or C₂-C₁₂dialkylaminocarbonyl, or
R₂ and R₃ independently of one another are phenyl, naphthyl or saturated or unsaturated heterocylyl having 3 to 8 ring atoms which, in addition to carbon atoms, has at least one hetero atom selected from nitrogen, oxygen and sulfur, it being possible for these substituents to be substituted by halogen, cyano, nitro, C₁-C₅alkyl, C₁-C₅alkoxy, C₁-C₅alkylthio, -COOH,-CONH₂, C₁-C₆alkylaminocarbonyl, C₂-C₁₀dialkylaminocarbonyl, C₁-C₅alkylcarbonyl or C₁-C₅alkoxycarbonyl, or
R₂ and R₃ together with the nitrogen atom to which they are bonded form a saturated or unsaturated heterocyclic ring having 3 to 8 ring atoms which, in addition to carbon atoms, at least one hetero atom selected from nitrogen, oxygen and sulfur, which heterocyclic ring is unsubstituted or substituted by C₁-C₅alkyl, C₁-C₅alkoxy, halogen, cyano or nitro, or
R₂ and R₃ independently of one another are amino, C₁-C₆alkylamino, C₂-C₈dialkylamino, phenylamino, naphthylamino, C₁-C₆alkylcarbonylamino, C₁-C₁₀alkoxycarbonylamino, hydroxyl, C₁-C₆alkoxy, C₁-C₆alkylcarbonyloxy, phenoxy, biphenyloxy or naphthoxy, X is O or S(O)ₓ, in which x is 0, 1 or 2, and
R₄ is C₁-C₈alkyl, C₂-C₈alkenyl, C₃-C₈alkynyl, C₃-C₈cycloalkyl, C₅-C₈cycloalkenyl, C₃-C₈cycloalkyl-C₁-C₄alkyl or C₁-C₄alkyl-C₃-C₈cycloalkyl, it being possible for these substituents to be unsubstituted or substituted by halogen, cyano, nitro, =O or -OR₅, or R₄ is phenyl, biphenyl, naphthyl, heterocyclyl, C₁-C₄alkylphenyl, C₁-C₄alkylnaphthyl, phenyl-C₁-C₄alkyl or naphthyl-C₁-C₄alkyl, it being possible for these substituents to be unsubstituted or substituted by halogen, cyano, nitro, amino, -COOH, hydroxyl, C₁-C₁₀alkyl, C₁-C₁₀alkyloxy, C₁-C₆alkylamino, di-C₂-C₈alkylamino, C₁-C₁₀alkylthio, C₁-C₁₀haloalkyl, C₁-C₁₀haloalkoxy, C₁-C₁₀haloalkylthio, C₂-C₁₀alkoxycarbonylalkoxy, C₁-C₆alkylsulfinyl, C₁-C₆alkylsulfonyl, C₁-C₆alkyloxycarbonyl, C₁-C₆alkylcarbonyl, -CONH₂, formyl, C₁-C₇alkylaminocarbonyl, C₂-C₁₁dialkylaminocarbonyl, C₃-C₆trialkylsilyl, C₁-C₁₀alkylcarbonylamino, C₁-C₁₀alkylcarbonyloxy, phenoxy, halophenoxy, pyridyloxy or pyridyloxy which is substituted by halogen, C₁-C₄alkyl, C₁-C₄alkoxy, cyano, nitro or amino, or 2 adjacent substituents on the phenyl or naphthyl ring R₄ form a carbocyclic or saturated or unsaturated heterocyclic ring having 3 to 8 ring atoms which, in addition to carbon atoms, at least one hetero atom selected from nitrogen, oxygen and sulfur, which carbocyclic or heterocyclic ring is unsubstituted or substituted by halogen, cyano, nitro, amino, -COOH, =O, C₁-C₁₀alkyl, C₁-C₁₀alkoxy, C₁-C₁₀alkylthio, C₁-C₁₀haloalkyl, hydroxyl, C₃-C₁₀alkoxycarbonylalkoxy, C₁-C₆alkylsulfinyl, C₁-C₆alkylsulfonyl, C₂-C₆alkyloxycarbonyl, C₂-C₆alkylcarbonyl, -CONH₂, formyl, C₂-C₇alkylaminocarbonyl, C₃-C₁₁ dialkylaminocarbonyl, C₃-C₆trialkylsilyl, C₂-C₁₀alkylcarbonylamino, C₂-C₁₀alkylcarbonyloxy, phenoxy, pyridyloxy or pyridyloxy which is substituted by halogen, C₁-C₄alkyl, C₁-C₄alkoxy, cyano, nitro or amino,
and salts of the compounds of the formula I,
the compounds of the formulae Ia, Ia₁, Ia₂, Ia₃ and Ia₄ being excluded: 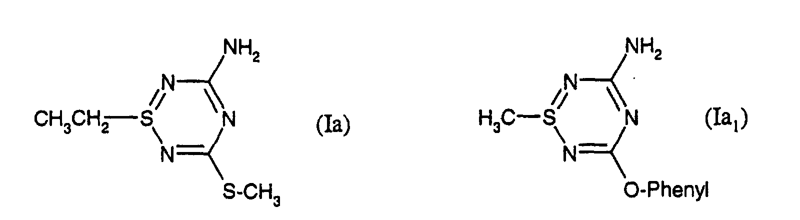 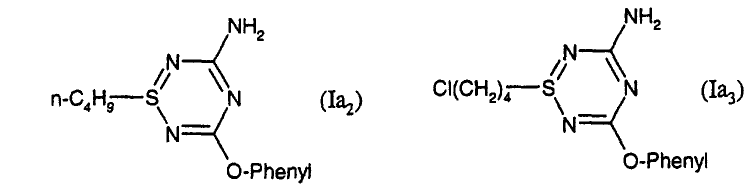 and

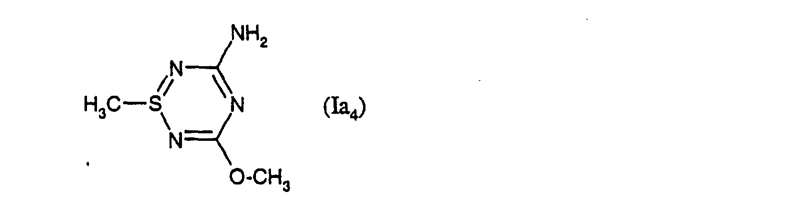

2. A compound according to claim 1, in which
R₁ is C₁-C₁₀alkyl, C₃-C₈cycloalkyl, C₃-C₈cycloalkyl-C₁-C₆alkyl, C₁-C₈alkyl-C₃-C₈cycloalkyl, C₂-C₁₀alkenyl, C₃-C₈cycloalkyl-C₂-C₆alkenyl, C₂-C₈alkenyl-C₃-C₈cycloalkyl, C₂-C₁₀alkynyl, C₃-C₈cycloalkyl-C₂-C₆alkynyl, C₂-C₈alkynyl-C₃-C₈cycloalkyl, C₅-C₈cycloalkenyl, C₅-C₈cycloalkenyl-C₁-C₆alkyl, C₁-C₈alkyl-C₅-C₈cycloalkenyl, C₅-C₈cycloalkenyl-C₂-C₆alkenyl, C₂-C₈alkenyl-C₅-C₈cycloalkenyl, C₅-C₈cycloalkenyl-C₂-C₆alkynyl, C₂-C₈alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₈alkynyl-C₂-C₆alkenyl or C₂-C₈alkenyl-C₂-C₆alkynyl, it being possible for these substituents to be unsubstituted or substituted by halogen, cyano, nitro, =O, -OR₅,-S(O)ₙR₆, -COOR₇, -CONR₈R₉, -NR₁₀R₁₁, =NR₁₂, phenyl, biphenyl, naphthyl or saturated or unsaturated heterocyclyl having 3 to 8 ring atoms which, in addition to carbon atoms has at least one hetero atom selected from nitrogen, oxygen and sulfur, it being possible for these phenyl, naphthyl and heterocyclyl substituents to be unsubstituted or substituted by halogen, cyano, nitro, -OR₅, -NR₁₀R₁₁ or -CONR₈R₉, or
R₁ is saturated or unsaturated heterocyclyl having 3 to 8 ring atoms which, in addition to carbon atoms has at least one hetero atom selected from nitrogen, oxygen and sulfur, the heterocycle being bonded to the thiatriazine ring via one of its carbon atoms and it being possible for the heterocycle to be substituted by halogen, cyano, nitro, =O, -OR₅, -S(O)ₙR₆, -COOR₇,-CONR₈R₉, -NR₁₀R₁₁, =NR₁₂, =N-NR₁₃R₁₄, C₁-C₈alkyl, C₂-C₈alkenyl, C₂-C₈alkynyl, C₃-C₈cycloalkyl, C₅-C₈cycloalkenyl, phenyl, biphenyl, naphthyl, C₁-C₈alkyl which is substituted by halogen, cyano, nitro, -OR₅, -CONR₈R₉ or -NR₁₀R₁₁, C₂-C₈alkenyl which is substituted by halogen, cyano, nitro, -OR₅, -CONR₈R₉ or -NR₁₀R₁₁, C₂-C₈alkynyl which is substituted by halogen, cyano, nitro, -OR₅, -CONR₈R₉ or -NR₁₀R₁₁, C₃-C₈cycloalkyl which is substituted by halogen, cyano, nitro, -OR₅, -CONR₈R₉ or -NR₁₀R₁₁, C₅-C₈cycloalkenyl which is substituted by halogen, cyano, nitro, -OR₅, -CONR₈R₉ or -NR₁₀R₁₁, phenyl which is substituted by halogen, cyano, nitro, C₁-C₆alkyl, C₁-C₆haloalkyl, -OR₅, -CONR₈R₉ or-NR₁₀R₁₁, biphenyl which is substituted by halogen, cyano, nitro, -OR₅, -CONR₈R₉ or-NR₁₀R₁₁, or naphthyl which is substituted by halogen, cyano, nitro, -OR₅, -CONR₈R₉ or-NR₁₀R₁₁, or
R₁ is phenyl, biphenyl, naphthyl, C₁-C₄alkylphenyl, C₁-C₄alkylbiphenyl, C₁-C₄alkylnaphthyl, C₂-C₄alkenylphenyl, C₂-C₄alkenylbiphenyl, C₂-C₄alkenylnaphthyl, C₂-C₄alkynylphenyl, C₂-C₄alkynylbiphenyl or C₂-C₄alkynylnaphthyl, it being possible for these substituents to be substituted by halogen, cyano, nitro, -OR₅, -S(O)ₙR₆, -COOR₇, -CONR₈R₉ or -NR₁₀R₁₁, in which
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₄alkylcarbonyl, benzoyl, halobenzoyl, C₁-C₄alkylamino, C₂-C₆dialkylamino, C₃-C₆trialkylsilyl, C₃-C₆cycloalkyl, C₂-C₄alkenyl or C₃-C₄alkynyl,
n is 0, 1 or 2,
R₆ is hydrogen or cyano if n is 0, or
R₆ is C₁-C₄alkyl, C₁-C₄haloalkyl, phenyl, C₁-C₄alkylphenyl, halophenyl, saturated or unsaturated heterocyclyl or haloheterocyclyl having 3 to 8 ring atoms which, in addition to carbon atoms, has at least one hetero atom selected from nitrogen, oxygen and sulfur, or C₃-C₆cycloalkyl,
R₇ is hydrogen, C₁-C₄alkyl, C₁-C₄haloalkyl, C₁-C₄cyanoalkyl, phenyl, halophenyl, C₂-C₄alkoxyalkyl, saturated or unsaturated heterocyclyl or haloheterocyclyl having 3 to 8 ring atoms which, in addition to carbon atoms, has at least one hetero atom selected from nitrogen, oxygen and sulfur,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or R₈ and R₉ together with the nitrogen atom to which they are bonded form a saturated or unsaturated heterocycle having 3 to 8 ring atoms which can be halogenated and which, in addition to carbon atoms, has at least one hetero atom selected from nitrogen, oxygen and sulfur,
R₁₀ and R₁₁ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be substituted by C₁-C₄alkyl, halogen, C₁-C₄alkoxy, hydroxyl, cyano, nitro or C₁-C₄alkoxycarbonyl, or
R₁₀ and R₁₁ together with the nitrogen atom to which they are bonded to form a saturated or unsaturated heterocycle having 3 to 8 ring atoms which can be halogenated and which, in addition to carbon atoms, has at least one hetero atom selected from nitrogen, oxygen and sulfur, and
R₁₂ is hydrogen, C₁-C₄alkyl, C₁-C₄haloalkyl, phenyl, halophenyl, cyanophenyl, nitrophenyl, C₁-C₄alkylphenyl, C₁-C₄alkoxyphenyl or phenyl-C₁-C₄alkyl.

3. A compound according to claim 2, in which
R₁ is C₁-C₁₀alkyl, C₃-C₈cycloalkyl, C₃-C₈cycloalkyl-C₁-C₆alkyl, C₁-C₈alkyl-C₃-C₈cycloalkyl, C₂-C₁₀alkenyl, C₃-C₈cycloalkyl-C₂-C₆alkenyl, C₂-C₈alkenyl-C₃-C₈cycloalkyl, C₂-C₁₀alkynyl, C₃-C₈cycloalkyl-C₂-C₆alkynyl, C₂-C₈alkynyl-C₃-C₈cycloalkyl, C₅-C₈cycloalkenyl, C₅-C₈cycloalkenyl-C₁-C₆alkyl, C₁-C₈alkyl-C₅-C₈cycloalkenyl, C₅-C₈cycloalkenyl-C₂-C₆alkenyl, C₂-C₈alkenyl-C₅-C₈cycloalkenyl, C₅-C₈cycloalkenyl-C₂-C₆alkynyl, C₂-C₈alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C₈cycloalkenyl, C₅-C₈cycloalkenyl-C₃-C₈cycloalkyl, C₂-C₈alkynyl-C₂-C₆alkenyl or C₂-C₈alkenyl-C₂-C₆alkynyl, it being possible for these substituents to be unsubstituted or substituted by halogen, cyano, =O, -OR₅, -COOR₇, - CONR₈R₉, -NR₁₀R₁₁ or phenyl, it being possible for this phenyl ring to be unsubstituted or substituted by halogen, or
R₁ is saturated or unsaturated heterocylyl having 3 to 8 ring atoms which, in addition to carbon atoms, has at least one hetero atom selected from nitrogen, oxygen and sulfur, the heterocycle being bonded to the thiatriazine ring via one of its carbon atoms and it being possible for the heterocycle to be substituted by halogen, cyano, =O, -OR₅, -S(O)ₙR₆, -COOR₇, -CONR₈R₉,-NR₁₀R₁₁, C₁-C₈alkyl, C₂-C₈alkenyl, C₂-C₈alkynyl, C₃-C₈cycloalkyl, C₅-C₈cycloalkenyl, phenyl, C₁-C₈alkyl which is substituted by halogen, cyano, -OR₅, -CONR₈R₉ or -NR₁₀R₁₁, C₂-C₈alkenyl which is substituted by halogen, cyano, -OR₅, -CONR₈R₉ or -NR₁₀R₁₁, C₂-C₈alkynyl which is substituted by halogen, cyano, -OR₅, -CONR₈R₉ or -NR₁₀R₁₁, C₃-C₈cycloalkyl which is substituted by halogen, cyano, -OR₅, -CONR₈R₉ or -NR₁₀R₁₁, C₅-C₈cycloalkenyl which is substituted by halogen, cyano, -OR₅, -CONR₈R₉ or -NR₁₀R₁₁, phenyl which is substituted by halogen, cyano, nitro, C₁-C₄alkyl, C₁-C₆haloalkyl, -OR₅, -CONR₈R₉ or -NR₁₀R₁₁, or
R₁ is phenyl, biphenyl, naphthyl, C₁-C₄alkylphenyl, C₁-C₄alkylbiphenyl, C₁-C₄alkylnaphthyl, C₂-C₄alkenylphenyl, C₂-C₄alkenylbiphenyl, C₂-C₄alkenylnaphthyl, C₂-C₄alkynylphenyl, C₂-C₄alkynylbiphenyl or C₂-C₄alkynylnaphthyl, it being possible for these substituents to be unsubstituted or substituted by halogen, cyano, -OR₅, -S(O)ₙR₆, -COOR₇, -CONR₈R₉ or-NR₁₀R₁₁, in which
R₅ is hydrogen, C₁-C₆alkyl, C₁-C₆haloalkyl, C₂-C₆alkoxyalkyl, C₁-C₆cyanoalkyl, phenyl-C₁-C₄alkyl, C₁-C₄alkylcarbonyl, benzoyl, halobenzoyl, C₃-C₆trialkylsilyl, C₃-C₆cycloalkyl, C₂-C₄alkenyl or C₃-C₄alkynyl,
n is 0, 1 or 2,
R₆ is hydrogen or cyano if n is 0 or
R₆ is C₁-C₄alkyl, C₁-C₄haloalkyl, phenyl or C₁-C₄alkylphenyl,
R₇ is hydrogen, C₁-C₄alkyl, C₁-C₄haloalkyl, C₁-C₄cyanoalkyl, phenyl, halophenyl, C₂-C₄alkoxyalkyl, saturated or unsaturated heterocyclyl or haloheterocyclyl having 3 to 8 ring atoms which, in addition to carbon atoms, at least one hetero atom selected from nitrogen, oxygen and sulfur,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or
R₈ and R₉ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur, R₁₀ and R₁₁ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be substituted by C₁-C₄alkyl, halogen, C₁-C₄alkoxy, hydroxyl, cyano, nitro or C₁-C₄alkoxycarbonyl, or R₁₀ and R₁₁ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur.

4. A compound according to claim 3, in which
R₁ is C₁-C₁₀alkyl, C₃-C₈cycloalkyl, C₃-C₈cycloalkyl-C₁-C₆alkyl, C₁-C₈alkyl-C₃-C₈cycloalkyl, C₂-C₁₀alkenyl, C₃-C₈cycloalkyl-C₂-C₆alkenyl, C₂-C₈alkenyl-C₃-C₈cycloalkyl, C₂-C₁₀alkynyl, C₃-C₈cycloalkyl-C₂-C₆alkynyl, C₂-C₈alkynyl-C3-C8cycloalkyl, C₅-C₈cycloalkenyl, C₅-C₈cycloalkenyl-C₁-C6alkyl, C₁-C₈alkyl-C₅-C₈cycloalkenyl, C₅-C₈cycloalkenyl-C₂-C₆alkenyl, C₂-C₈alkenyl-C₅-C₈cycloalkenyl, C₅-C₈cycloalkenyl-C₂-C₆alkynyl, C₂-C₈alkynyl-C₅-C₈cycloalkenyl, C₃-C₈cycloalkyl-C₃-C₈cycloalkyl, C₅-C₈cycloalkenyl-C₅-C₈cycloalkenyl, C₃-C₈cycloalkyl-C₅-C8cycloalkenyl, C₅-C₈cycloalkenyl-C₃-C₈cycloalkyl, C₂-C₈alkynyl-C₂-C₆alkenyl or C₂-C₈alkenyl-C₂-C₆alkynyl, it being possible for these substituents to be unsubstituted or substituted by halogen, cyano, =0, -OR₅, -COOR₇, -CONR₈R₉, -NR₁₀R₁₁ or phenyl, it being possible for this phenyl ring to be unsubstituted or substituted by halogen, or
R₁ is saturated or unsaturated heterocylyl having 3 to 8 ring atoms which, in addition to carbon atoms, has at least one hetero atom selected from nitrogen, oxygen and sulfur, the heterocycle being bonded to the thiatriazine ring via one of its carbon atoms and it being possible for the heterocycle to be substituted by halogen, cyano, =O, C₁-C₄alkoxy, C₁-C₈alkyl, C₂-C₈alkenyl, C₂-C₈alkynyl, phenyl, C₁-C₈alkyl which is substituted by halogen, C₂-C₈alkenyl which is substituted by halogen, C₂-C₈alkynyl which is substituted by halogen, phenyl which is substituted by halogen, cyano, nitro, C₁-C₄alkyl or C₁-C₄alkoxy, or
R₁ is phenyl, biphenyl, naphthyl, C₁-C₄alkylphenyl, C₁-C₄alkylbiphenyl, C₁-C₄alkylnaphthyl, C₂-C₄alkenylphenyl or C₂-C₄alkynylphenyl, it being possible for these substituents to be unsubstituted or substituted by halogen, cyano, -OR₅, -S(O)ₙR₆, -COOR₇, -CONR₈R₉ or-NR₁₀R₁₁, in which
R₅ is hydrogen, C₁-C₆alkyl, C₁-C₆haloalkyl, C₂-C₆alkoxyalkyl, C₁-C₆cyanoalkyl, phenyl-C₁-C₄alkyl, C₁-C₄alkylcarbonyl, benzoyl, halobenzoyl, C₃-C₆trialkylsilyl, C₃-C₆cycloalkyl, C₂-C₄alkenyl or C₃-C₄alkynyl,
n is 0, 1 or 2,
R₆ is hydrogen or cyano if n is 0, or
R₆ is C₁-C₄alkyl, C₁-C₄haloalkyl, phenyl or C₁-C₄alkylphenyl,
R₇ is hydrogen, C₁-C₄alkyl, C₁-C₄haloalkyl, C₁-C₄cyanoalkyl, phenyl, halophenyl, C₂-C₄alkoxyalkyl, saturated or unsaturated heterocyclyl or haloheterocyclyl having 3 to 8 ring atoms which, in addition to carbon atoms, at least one hetero atom selected from nitrogen, oxygen and sulfur,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or R₈ and R₉ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur, R₁₀ and R₁₁ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be unsubstituted or substituted by C₁-C₄alkyl, halogen, C₁-C₄alkoxy, hydroxyl, cyano, nitro or C₁-C₄alkoxycarbonyl, or
R₁₀ and R₁₁ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur.

5. A compound according to claim 1, in which
R₂ and R₃ independently of one another are hydrogen, C₁-C₆alkyl, C₁-C₆alkyl which is substituted by halogen, cyano, nitro, C₁-C₄alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₃-C₈cycloalkyl, C₁-C₅alkylcarbonyloxy, phenylcarbonyloxy, naphthylcarbonyloxy, C₁-C₆alkylamino, C₁-C₅alkoxycarbonyl, C₂-C₆dialkylamino or phenyl, it being possible for the phenyl ring to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or -CONR₈R₉, or
R₂ and R₃ independently of one another are C₂-C₆alkenyl, C₂-C₆alkenyl which is substituted by halogen, cyano, nitro, C₁-C₄alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₁-C₆alkylamino, C₂-C₅alkoxycarbonyl, C₂-C₆dialkylamino or phenyl, it being possible for the phenyl ring to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or -CONR₈R₉, or
R₂ and R₃ independently of one another are C₃-C₆alkynyl, C₃-C₆alkynyl which is substituted by halogen, cyano, nitro, C₁-C₈alkoxy, C₃-C₆trialkylsilyl, hydroxyl, amino, ammonium, tri-C₁-C₄alkylammonium, -COOH, -COOM, in which M is ammonium or an alkali metal atom or alkaline earth metal atom, or C₁-C₆alkylamino, C₂-C₅alkoxycarbonyl, C₂-C₆dialkylamino or phenyl, it being possible for the phenyl ring to be unsubstituted or substituted by halogen, cyano, nitro, -OR₅, -NR₁₀R₁₁, C₁-C₄alkyl, formyl, C₁-C₄alkylcarbonyl, COOR₇, C₁-C₄alkylthio, C₁-C₄alkylsulfonyl or -CONR₈R₉, or
R₂ and R₃ independently of one another are formyl, C₁-C₈alkylcarbonyl, C₂-C₈alkenylcarbonyl, C₄-C₉cycloalkylcarbonyl, C₆-C₉cycloalkenylcarbonyl or C₃-C₈cycloalkyl-C₁-C₆alkylcarbonyl,
it being possible for these substituents to be unsubstituted
or substituted by halogen, cyano, nitro, hydroxyl, amino, C₁-C₆alkylamino, C₂-C₇dialkylamino, -COOH,
-COOM, in which M is ammonium or an alkali metal atom or alkaline earth metal atom, or C₂-C₈alkoxycarbonyl, C₄-C₁₀cycloalkoxycarbonyl, C₁-C₈alkylaminocarbonyl or C₂-Cgdialkylaminocarbonyl, or
R₂ and R₃ independently of one another are saturated or unsaturated heterocyclyl or heterocyclylcarbonyl having 3 to 8 ring atoms which, in addition to carbon atoms, at least one hetero atom selected from nitrogen, oxygen and sulfur, heterocyclylas defined above which is substituted by halogen, cyano, nitro, C₁-C₅alkyl, C₁-C₅alkoxy, C₁-C₅alkylcarbonyl, C₁-C₅alkylcarbonyloxy, C₁-C₆alkoxycarbonyl, aminocarbonyl, C₁-C₆alkylaminocarbonyl or C₂-C₈dialkylaminocarbonyl, or heterocyclylcarbonyl which is substituted by halogen, cyano, nitro, C₁-C₄alkyl, C₁-C₄alkoxy, C₁-C₄alkylcarbonyl, C₁-C₄alkoxycarbonyl, aminocarbonyl, C₁-C₆alkylamino or C₁-C₅alkylcarbonyloxy, or
R₂ and R₃ independently of one another are phenylcarbonyl, biphenylcarbonyl, naphthylcarbonyl, phenyl-C₁-C₆alkylcarbonyl, biphenyl-C₁-C₆alkylcarbonyl, naphthyl-C₁-C₆alkylcarbonyl, phenyl-C₂-C₆alkenylcarbonyl, biphenyl-C₂-C₆alkenylcarbonyl or naphthyl-C₂-C₆alkenylcarbonyl, it being possible for these substituents to be unsubstituted or substituted by C₁-C₄alkyl, C₁-C₄alkoxy, C₁-C₄alkylthio, C₁-C₄haloalkyl, C₁-C₄alkylcarbonyl, halogen, cyano, amino, nitro, -COOR₇, C₁-C₅alkoxycarbonyl, hydroxyl, C₁-C₄alkylsulfonyl, C₁-C₅alkylaminocarbonyl or C₂-C₆dialkylaminocarbonyl, or
R₂ and R₃ independently of one another are phenyl, naphthyl or saturated or unsaturated heterocylyl having 3 to 8 ring atoms which, in addition to carbon atoms, has at least one hetero atom selected from nitrogen, oxygen and sulfur, it being possible for these substituents to be unsubstituted or substituted by halogen, cyano, nitro, C₁-C₄alkyl, C₁-C₄alkoxy, C₁-C₄alkylthio, -COOH, -CONH₂, C₁-C₅alkylaminocarbonyl, C₂-C₇dialkylaminocarbonyl, C₁-C₄alkylcarbonyl or C₁-C₅alkoxycarbonyl, or
R₂ and R₃ together with the nitrogen atom to which they are bonded form saturated or unsaturated heterocyclic ring having 3 to 8 ring atoms which, in addition to carbon atoms, at least one hetero atom selected from nitrogen, oxygen and sulfur, which heterocyclic ring is unsubstituted or substituted by C₁-C₄alkyl, C₁-C₄alkoxy, halogen, cyano or nitro, or
R₂ and R₃ independently of one another are amino, C₁-C₄alkylamino, C₂-C₆dialkylamino, phenylamino, C₁-C₅alkylcarbonylamino, C₁-C₅alkoxycarbonylamino, hydroxyl, C₁-C₄alkoxy, C₁-C₅alkylcarbonyloxy or phenoxy,
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₄alkylcarbonyl, benzoyl, halobenzoyl, C₁-C₄-alkylamino, C₂-C₆dialkylamino, C₃-C₆trialkylsilyl, C₃-C₆cycloalkyl, C₂-C₄alkenyl or C₃-C₄alkynyl,
R₇ is hydrogen, C₁-C₄alkyl, C₁-C₄halogenalkyl, C₁-C₄cyanoalkyl, phenyl, halophenyl, C₂-C₄alkoxyalkyl, saturated or unsaturated heterocyclyl or haloheterocyclyl having 3 to 8 ring atoms which, in addition to carbon atoms, at least one hetero atom selected from nitrogen, oxygen and sulfur,
R₈ and R₉ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl or C₂-C₄alkoxyalkyl, or
R₈ and R₉ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur,
R₁₀ and R₁₁ independently of one another are hydrogen, phenyl, halophenyl, C₁-C₄alkyl, C₁-C₄haloalkyl, C₁-C₄cyanoalkyl, C₃-C₄alkenyl, C₃-C₄alkynyl, C₂-C₄alkoxyalkyl, formyl, C₁-C₄alkylcarbonyl or phenylcarbonyl, it being possible therein for the phenyl moiety to be substituted by C₁-C₄alkyl, halogen, C₁-C₄alkoxy, hydroxyl, cyano, nitro or C₁-C₄alkoxycarbonyl, or
R₁₀ and R₁₁ together with the nitrogen atom to which they are bonded form a saturated or unsaturated heterocycle having 3 to 8 ring atoms which can be halogenated and which, in addition to carbon atoms, at least one hetero atom selected from nitrogen, oxygen and sulfur.

6. A compound according to claim 5, in which
R₂ and R₃ independently of one another are hydrogen, C₁-C₆alkyl, C₁-C₆alkyl which is substituted by halogen, hydroxyl, amino, ammonium, tri-C₁-C₄alkylammonium, -COOH,-COOM, where M is ammonium or an alkali metal atom or alkaline earth metal atom, or C₁-C₅alkylcarbonyloxy, phenylcarbonyloxy, C₁-C₆alkylamino, C₁-C₅alkoxycarbonyl or C₂-C₆dialkylamino, or
R₂ and R₃ independently of one another are formyl, C₁-C₈alkylcarbonyl, C₂-Cgalkenylcarbonyl, C₄-C₉cycloalkylcarbonyl, C₆-C₉cycloalkenylcarbonyl or C₃-C₈cycloalkyl-C₁-C₆alkylcarbonyl, it being possible for these substituents to be unsubstituted or substituted by halogen, cyano, hydroxyl, amino, -COOH or -COOM, where M is ammonium or an alkali metal atom or alkaline earth metal atom, or
R₂ and R₃ independently of one another are saturated or unsaturated heterocyclyl or heterocyclylcarbonyl having 3 to 8 ring atoms which, in addition to carbon atoms, at least one hetero atom selected from nitrogen, oxygen and sulfur, heterocyclyl as defined above which is substituted by halogen, cyano, nitro, C₁-C₅alkyl, C₁-C₅alkoxy or C₁-C₆alkoxycarbonyl, or heterocyclylcarbonyl as defined above which is substituted by halogen, cyano, nitro, C₁-C₄alkyl, C₁-C₄alkoxy or C₁-C₄alkoxycarbonyl, or
R₂ and R₃ independently of one another are phenylcarbonyl, biphenylcarbonyl, naphthylcarbonyl, phenyl-C₁-C₆alkylcarbonyl, biphenyl-C₁-C₆alkylcarbonyl, naphthyl-C₁-C₆alkylcarbonyl, phenyl-C₂-C₆alkenylcarbonyl, biphenyl-C₂-C₆alkenylcarbonyl or naphthyl-C₂-C₆alkenylcarbonyl, it being possible for these substituents to be unsubstituted or substituted by C₁-C₄alkyl, C₁-C₄alkoxy, C₁-C₄alkylthio, C₁-C₄haloalkyl, C₁-C₄alkylcarbonyl, halogen, cyano, amino, nitro, -COOH, C₁-C₅alkoxycarbonyl, hydroxyl or C₁-C₄alkylsulfonyl, or
R₂ and R₃ independently of one another are phenyl, naphthyl or saturated or unsaturated heterocylyl having 3 to 8 ring atoms which, in addition to carbon atoms, has at least one hetero atom selected from nitrogen, oxygen and sulfur, it being possible for these substituents to be unsubstituted or substituted by halogen, cyano, C₁-C₄alkyl, C₁-C₄alkoxy, C₁-C₄alkylthio, -COOH or C₁-C₅alkoxycarbonyl, or
R₂ and R₃ together with the nitrogen atom to which they are bonded form a a saturated or unsaturated heterocyclic ring having 3 to 8 ring atoms which, in addition to carbon atoms, at least one hetero atom selected from nitrogen, oxygen and sulfur, which heterocyclic ring is unsubstituted or substituted by C₁-C₄alkyl, C₁-C₄alkoxy, halogen or cyano.

7. A compound according to claim 6, in which R₂ and R₃ are hydrogen, or
R₂ and R₃ independently of one another are formyl, C₁-C₈alkylcarbonyl, C₂-Cgalkenylcarbonyl, C₄-C₉cycloalkylcarbonyl, C₆-C₉cycloalkenylcarbonyl or C₃-C₈cycloalkyl-C₁-C₆alkylcarbonyl, it being possible for these substituents to be unsubstituted or substituted by halogen, cyano, hydroxyl or amino, or
R₂ and R₃ are phenylcarbonyl, it being possible for the phenyl ring to be unsubstituted or substituted by C₁-C₄-alkyl, C₁-C₄-alkoxy, C₁-C₄-alkylthio, C₁-C₄haloalkyl, halogen, cyano, nitro, -COOH, C₁-C₅alkoxycarbonyl, hydroxyl or C₁-C₄alkylsulfonyl.

8. A compound according to claim 1, in which X is O or S(O)ₓ, where x is 0, 1 or 2, and
R₄ is methyl which is substituted by halogen, cyano, nitro or OR₅, or
R₄ is C₂-C₈alkyl, C₂-C₈alkenyl, C₃-C₈alkynyl, C₃-C₈cycloalkyl, C₅-C₈cycloalkenyl C₃-C₈cycloalkyl-C₁-C₄alkyl or C₁-C₄alkyl-C₃-C₈cycloalkyl, it being possible for these substituents to be unsubstituted or substituted by halogen, cyano, nitro, =O or -OR₅, or
R₄ is phenyl which is substituted by halogen, cyano, nitro, amino, -COOH, hydroxyl, C₁-C₄alkyl, C₁-C₄alkyloxy, C₁-C₄alkylthio, C₁-C₄haloalkyl, C₁-C₄haloalkoxy, C₁-C₄haloalkylthio, C₂-C₆alkoxycarbonylalkoxy, C₁-C₄alkylsulfinyl, C₁-C₄alkylsulfonyl, C₁-C₆alkyloxycarbonyl, C₁-C₆alkylcarbonyl, -CONH₂, formyl, C₁-C₅alkylaminocarbonyl, C₂-C₇dialkylaminocarbonyl, C₁-C₄alkylamino, C₂-C₆dialkylamino,
C₃-C₆trialkylsilyl, C₁-C₆alkylcarbonylamino, C₁-C₆alkylcarbonyloxy, phenoxy, halophenoxy or pyridyloxy, or
R₄ is biphenyl, naphthyl, saturated or unsaturated heterocylyl having 3 to 8 ring atoms which, in addition to carbon atoms, has at least one hetero atom selected from nitrogen, oxygen and sulfur, C₁-C₄alkylphenyl, C₁-C₄alkylnaphthyl, phenyl-C₁-C₄alkyl or naphthyl-C₁-C₄alkyl, it being possible for these substituents to be unsubstituted or substituted by halogen, cyano, nitro, amino, -COOH, hydroxyl, C₁-C₄alkyl, C₁-C₄alkyloxy, C₁-C₄alkylthio, C₁-C₄haloalkyl, C₁-C₄haloalkoxy, C₁-C₄haloalkylthio, C₂-C₆-alkoxycarbonylalkoxy, C₁-C₄alkylsulfinyl, C₁-C₄alkylsulfonyl, C₁-C₆alkyloxycarbonyl, C₁-C₆alkylcarbonyl, -CONH₂, formyl, C₁-C₅alkylaminocarbonyl, C₂-C₇dialkylaminocarbonyl,
C₃-C₆trialkylsilyl, C₁-C₆alkylcarbonylamino, C₁-C₆alkylcarbonyloxy, phenoxy, halophenoxy or pyridyloxy, and
R₅ is hydrogen, C₁-C₆alkyl, C₁-C₆haloalkyl, C₂-C₆alkoxyalkyl, C₁-C₆cyanoalkyl, phenyl, halophenyl, C₁-C₄alkoxyphenyl, phenyl-C₁-C₄alkyl, C₁-C₄alkylcarbonyl, benzoyl, halobenzoyl, C₁-C₄alkylamino, C₂-C₆dialkylamino, C₃-C₆trialkylsilyl, C₃-C₆cycloalkyl, C₂-C₄alkenyl or C₃-C₄alkynyl.

9. A compound according to claim 8, in which
X is O or S, and
R₄ is methyl which is substituted by halogen or cyano, or
R₄ is C₂-C₈alkyl, C₂-C₈alkenyl, C₃-C₈alkynyl, C₃-C₈cycloalkyl, C₅-C₈cycloalkenyl, C₃-C₈cycloalkyl-C₁-C₄alkyl or C₁-C₄alkyl-C₃-C₈cycloalkyl, it being possible for these substituents to be unsubstituted or substituted by halogen or cyano, or
R₄ is phenyl which is substituted by halogen, cyano, nitro, amino, C₁-C₄alkyl, C₁-C₄alkyloxy, C₁-C₄alkylthio, C₁-C₄haloalkyl, C₁-C₄haloalkoxy, C₂-C₆alkoxycarbonylalkoxy, C₁-C₄alkylsulfonyl, C₁-C₆alkyloxycarbonyl, C₁-C₆alkylcarbonyl, formyl, C₁-C₄alkylamino, C₂-C₆dialkylamino,
C₃-C₆trialkylsilyl or C₂-C₆alkylcarbonyloxy, or
R₄ is biphenyl, naphthyl, saturated or unsaturated heterocylyl having 3 to 8 ring atoms which, in addition to carbon atoms, has at least one hetero atom selected from nitrogen, oxygen and sulfur, C₁-C₄alkylphenyl, C₁-C₄alkylnaphthyl, phenyl-C₁-C₄alkyl or naphthyl-C₁-C₄alkyl, it being possible for these substituents to be unsubstituted or substituted by halogen, cyano, nitro, amino, C₁-C₄alkyl, C₁-C₄alkyloxy, C₁-C₄alkylthio, C₁-C₄haloalkyl, C₁-C₄haloalkoxy, C₂-C₆alkoxycarbonylalkoxy, C₁-C₄alkylsulfonyl, C₁-C₆alkyloxycarbonyl, C₁-C₆alkylcarbonyl, C₃-C₆trialkylsilyl or C₁-C₆alkylcarbonyloxy.

10. A compound according to claim 9, in which X is O and
R₄ is phenyl which is substituted by halogen, cyano, nitro, amino, C₁-C₄alkyl, C₁-C₄alkyloxy, C₁-C₄alkylthio, C₁-C₄haloalkyl, halomethoxy, C₁-C₄alkylsulfonyl, C₁-C₄alkylamino or C₂-C₆dialkylamino.

11. A process for the preparation of the compounds according to claim 1, which comprises
(1) reacting a compound of the formula II 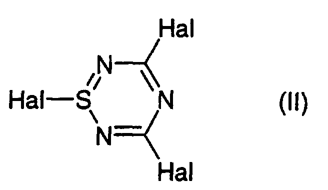 in which Hal substituents independently of one another are fluorine, chlorine or bromine, with an organometal compound of the formula III
R₁-M (III),
in which R₁ is as defined in claim 1 and M is a monovalent or polyvalent metal atom,
(2a) reacting the resulting compound of the formula IV 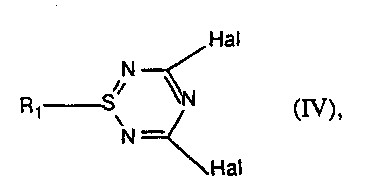 in which R₁ is as defined in claim 1 and Hal radicals independently of one another are fluorine, chlorine or bromine, with the compound of the formula V
H-XR₄ (V),
in which X and R₄ are as defined above, to give the compound of the formula VI 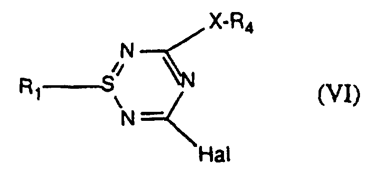 and
(3a) then converting these compounds with the compounds of the formula VII
M₁-NR₂R₃ (VII),
in which R₂ and R₃ are as defined above and M₁ is hydrogen or a metal atom into the end products of the formula I; or
(2b) reacting the compounds of the formula IV with the compounds of the formula VII to give the compounds of the formula VIII 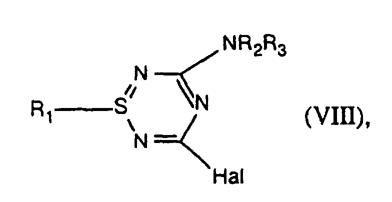 in which R₁, R₂ and R₃ are as defined in claim 1 and Hal is fluorine, chlorine or bromine, and
(3b) then converting these compounds with the compounds of the formula V into the end products of the formula I.

12. A herbicidal and plant-growth-inhibiting composition, which comprises one or more compounds of the formula I according to claim 1.

13. A composition according to claim 12, which comprises between 0.1 % and 95 % of active ingredient of the formula 1.

14. A method of controlling undesirable plant growth, which comprises applying an effective amount of an active ingredient of the formula I according to claim 1, or a composition comprising this active ingredient, to the plants or their environment.

15. A method according to claim 14, wherein an amount of active ingredient of between 0.001 and 5 kg, in particular between 0.005 to 2 kg, is applied per hectare.

16. A method of inhibiting plant growth, which comprises applying an effective amount of an active ingredient of the formula I according to claim 1, or a composition comprising this active ingredient, to the plants or their environment.

17. A method according to claim 14 for the selective pre- or post-emergence control of weeds in crops of useful plants.

18. The use of a composition according to claim 12 for the selective pre- or post-emergence control of weeds in crops of useful plants.

19. Seed treated with a composition according to claim 12.

20. A compound of formula IV 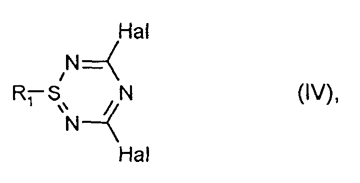 wherein R₁ is as defined in claim 1, and Hal independently of each other are fluorine, chlorine or bromine.

21. A compound of formula VI 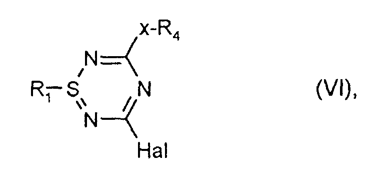 wherein R₁, X and R₄ are as defined in claim 1, and Hal is fluorine, chlorine or bromine.

22. A compound of formula VIII 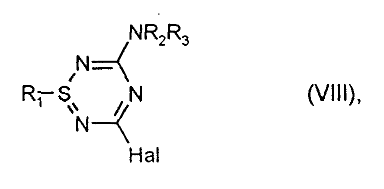 wherein R₁, R₂ and R₃ are as defined in claim 1, and Hal is fluorine, chlorine or bromine.

## Patentansprüche

1. Verbindung der Formel I 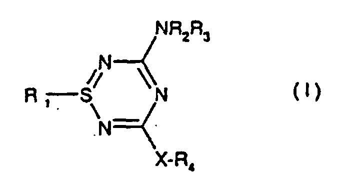 worin
R₁ C₁-C₂₀-Alkyl, C₃-C₈-Cycloalkyl, C₃-C₈-Cycloalkyl-C₁-C₁₇-alkyl, C₁-C₁₇-Alkyl-C₃-C₈-cycloalkyl, C₂-C₁₇-Alkenyl, C₃-C₈-Cycloalkyl-C₂-C₁₇-alkenyl, C₂-C₁₇-Alkenyl-C₃-C₈-cycloalkyl, C₂-C₁₇-Alkinyl, C₃-C₈-Cycloalkyl-C₂-C₁₇-alkinyl, C₂-C₁₇-Alkinyl-C₃-C₈-cycloalkyl, C₅-C₈-Cycloalkenyl, C₅-C₈-Cycloalkenyl-C₁-C₁₇-alkyl, C₁-C₁₇-Alkyl-C₅-C₈-cycloalkenyl, C₅-C₈-Cycloalkenyl-C₂-C₁₇-alkenyl, C₂-C₁₇-Alkenyl-C₅-C₈-cycloalkenyl, C₅-C₈-Cycloalkenyl-C₂-C₁₇-alkinyl, C₂-C₁₇-Alkinyl-C₅-C₈-cycloalkenyl, C₃-C₈-Cycloalkyl-C₃-C₈-cycloalkyl, C₅-C₈-Cycloalkenyl-C₅-C₈-cycloalkenyl, C₃-C₈-Cycloalkyl-C₅-C₈-cycloalkenyl, C₅-C₈-Cycloalkenyl-C₃-C₈-cycloalkyl, C₂-C₁₇-Alkinyl-C₂-C₁₇-alkenyl oder C₂-C₁₇-Alkenyl-C₂-C₁₇-alkinyl ist, wobei diese Substituenten gegebenenfalls mit Halogen, Cyano, Nitro, =O, -OR₅, -S(O)ₙR₆, -COOR₇, -CONR₈R₉, -NR₁₀R₁₁, =NR₁₂, =N-NR₁₃R₁₄, Phenyl, Biphenyl, Naphthyl oder gegebenenfalls gesättigtem Heterocyclyl mit 3 bis 8 Ringatomen substituiert sein können, das zusätzlich zu Kohlenstoffatomen mindestens ein Heteroatom, ausgewählt aus Stickstoff, Sauerstoff und Schwefel, aufweist, wobei diese Phenyl-, Naphthyl- und Heterocyclylsubstituenten gegebenenfalls mit Halogen, Cyano, Nitro, -OR₅, -NR₁₀R₁₁ oder -CONR₈R₉ substituiert sein können, oder
R₁ gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, ist, wobei der Heterocyclus über eines seiner Kohlenstoffatome an den Thiatriazinring gebunden ist und der Heterocyclus mit Halogen, Cyano, Nitro, =O, -OR₅, -S(O)ₙR₆, -COOR₇, -CONR₈R₉, -NR₁₀R₁₁, =NR₁₂, =N-NR₁₃R₁₄, C₁-C₁₇-Alkyl, C₂-C₁₇-Alkenyl, C₂-C₁₇-Alkinyl, C₃-C₈-Cycloalkyl, C₅-C₈-Cycloalkenyl, Phenyl, Biphenyl, Naphthyl, C₁-C₁₇-Alkyl substituiert mit Halogen, Cyano, Nitro, -OR₅, -CONR₈R₉ oder -NR₁₀R₁₁, C₂-C₁₇-Alkenyl substituiert mit Halogen, Cyano, Nitro, -OR₅, -CONR₈R₉ oder -NR₁₀R₁₁, C₂-C₁₇-Alkinyl substituiert mit Halogen, Cyano, Nitro, -OR₅, -CONR₈R₉ oder -NR₁₀R₁₁, C₃-C₈-Cycloalkyl substituiert mit Halogen, Cyano, Nitro, -OR₅, -CONR₈R₉ oder -NR₁₀R₁₁, C₅-C₈-Cycloalkenyl substituiert mit Halogen, Cyano, Nitro, -OR₅, -CONR₈R₉ oder -NR₁₀R₁₁, Phenyl substituiert mit Halogen, Cyano, Nitro, -OR₅, -CONR₈R₉ oder -NR₁₀R₁₁, Biphenyl substituiert mit Halogen, Cyano, Nitro, -OR₅, -CONR₈R₉ oder -NR₁₀R₁₁, oder Naphthyl substituiert mit Halogen, Cyano, Nitro, -OR₅, -CONR₈R₉ oder -NR₁₀R₁₁, substituiert sein kann, oder
R₁ Phenyl, Biphenyl, Naphthyl, C₁-C₁₀-Alkylphenyl, C₁-C₁₀-Alkylbiphenyl, C₁-C₁₀-Alkylnaphthyl, C₂-C₁₀-Alkenylphenyl, C₂-C₁₀-Alkenylbiphenyl, C₂-C₁₀-Alkenylnaphthyl, C₂-C₁₀-Alkinylphenyl, C₂-C₁₀-Alkinylbiphenyl oder C₂-C₁₀-Alkinylnaphthyl ist, wobei diese Substituenten gegebenenfalls mit Halogen, Cyano, Nitro, -OR₅, -S(O)ₙR₆, -COOR₇, -CONR₈R₉ oder -NR₁₀R₁₁ substituiert sein können, wobei
R₅ Wasserstoff, C₁-C₆-Alkyl, C₁-C₆-Halogenalkyl, C₂-C₆-Alkoxyalkyl, C₁-C₆-Cyanoalkyl, Phenyl, Halogenphenyl, C₁-C₄-Alkoxyphenyl, Phenyl-C₁-C₄-alkyl, C₁-C₇-Alkylcarbonyl, Benzoyl, Halogenbenzoyl, C₁-C₆-Alkylamino, C₂-C₈-Dialkylamino, -N=CH₂, -N=CH-C₁-C₄-Alkyl, -N=C(C₁-C₄-Alkyl)₂, C₃-C₆-Trialkylsilyl, C₃-C₇-Cycloalkyl, C₂-C₇-Alkenyl oder C₃-C₇-Alkinyl ist,
n 0, 1 oder 2 ist,
R₆ Wasserstoff oder Cyano ist, wenn n 0 ist, oder
R₆ C₁-C₅-Alkyl, C₁-C₅-Halogenalkyl, C₁-C₅-Hydroxyalkyl, Phenyl, Naphthyl, C₁-C₄-Alkylphenyl, C₁-C₄-Alkylnaphthyl, Halogenphenyl, Halogennaphthyl, gegebenenfalls gesättigtes Heterocyclyl oder Halogenheterocyclyl mit 3 bis 8 Ringatomen, die zusätzlich zu Kohlenstoffatomen mindestens ein Heteroatom, ausgewählt aus Stickstoff, Sauerstoff und Schwefel, aufweisen, oder C₃-C₇-Cycloalkyl ist,
R₇ Wasserstoff, C₁-C₁₂-Alkyl, C₁-C₁₂-Halogenalkyl, C₁-C₆-Nitroalkyl, C₁-C₆-Cyanoalkyl, Phenyl, Naphthyl, Halogenphenyl, Halogennaphthyl, C₂-C₁₀-Alkoxyalkyl, C₁-C₁₀-Alkylcarbonyl, gegebenenfalls gesättigtes Heterocyclyl oder Halogenheterocyclyl mit 3 bis 8 Ringatomen, die zusätzlich zu Kohlenstoffatomen mindestens ein Heteroatom, ausgewählt aus Stickstoff, Sauerstoff und Schwefel, aufweisen, C₃-C₇-Cycloalkyl, C₃-C₇-Halogencycloalkyl, -N=CH₂, -N=CH-C₁-C₄-Alkyl, -N=C(C₁-C₄-Alkyl)₂ oder C₂-C₆-Dialkylamino ist,
R₈ und R₉ unabhängig voneinander Wasserstoff, Phenyl, Naphthyl, Halogenphenyl, Halogennaphthyl, C₁-C₈-Alkyl, C₁-C₈-Halogenalkyl, C₁-C₈-Alkoxy, Phenoxy, Naphthoxy, Halogenphenoxy, Halogennaphthoxy, C₁-C₇-Cyanoalkyl, C₃-C₇-Alkenyl, C₃-C₇-Alkinyl oder C₂-C₈-Alkoxyalkyl sind, oder
R₈ und R₉ zusammen mit dem Stickstoffatom, an das sie gebunden sind, einen gegebenenfalls gesättigten Heterocyclus mit 3 bis 8 Ringatomen, der halogeniert sein kann, und zusätzlich zu Kohlenstoffatomen mindestens ein Heteroatom, ausgewählt aus Stickstoff, Sauerstoff und Schwefel, aufweist, bilden,
R₁₀ und R₁₁ unabhängig voneinander Wasserstoff, Phenyl, Naphthyl, Halogenphenyl, Halogennaphthyl, C₁-C₈-Alkyl, C₁-C₈-Halogenalkyl, C₁-C₈-Alkoxy, Phenoxy, Naphthoxy, Halogenphenoxy, Halogennaphthoxy, C₁-C₇-Cyanoalkyl, C₃-C₇-Alkenyl, C₃-C₇-Alkinyl, C₂-C₈-Alkoxyalkyl, C₁-C₆-Alkylamino, C₂-C₆-Dialkylamino, Formyl, C₂-C₈-Alkylcarbonyl, Phenylcarbonyl oder Naphthylcarbonyl sind, wobei der Phenyl- oder Naphthylteil darin mit C₁-C₄-Alkyl, Halogen, C₁-C₄-Alkoxy, Hydroxyl, Cyano, Nitro oder C₁-C₆-Alkoxycarbonyl substituiert sein kann, oder
R₁₀ und R₁₁ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden,
R₁₂ Wasserstoff, C₁-C₈-Alkyl, C₁-C₈-Halogenalkyl, Phenyl, Halogenphenyl, Cyanophenyl, Nitrophenyl, C₁-C₄-Alkylphenyl, C₁-C₄-Alkoxyphenyl, Naphthyl, Cyanonaphthyl, Nitronaphthyl, Halogennaphthyl, C₁-C₄-Alkylnaphthyl, C₁-C₄-Alkoxynaphthyl, Phenyl-C₁-C₆-alkyl oder Naphthyl-C₁-C₆-alkyl ist, und
R₁₃ und R₁₄ unabhängig voneinander Wasserstoff, C₁-C₆-Alkyl, C₁-C₆-Alkylcarbonyl, Phenyl, Halogenphenyl, Cyanophenyl, Nitrophenyl, C₁-C₄-Alkylphenyl, C₁-C₄-Alkoxyphenyl, Naphthyl, Cyanonaphthyl, Nitronaphthyl, Halogennaphthyl, C₁-C₄-Alkylnaphthyl oder C₁-C₄-Alkoxynaphthyl sind,
R₂ und R₃ unabhängig voneinander Wasserstoff, C₁-C₆-Alkyl, C₁-C₆-Alkyl substituiert mit Halogen, Cyano, Nitro, C₁-C₈-Alkoxy, C₃-C₆-Trialkylsilyl, Hydroxyl, Amino, Ammonium, Tri-C₁-C₄-alkylammonium, -COOH, COOM, worin M Ammonium oder ein Alkalimetallatom oder Erdalkalimetallatom ist, C₃-C₈-Cycloalkyl, C₁-C₅-Alkylcarbonyloxy, Phenylcarbonyloxy, Naphthylcarbonyloxy, C₁-C₆-Alkylamino, C₁-C₅-Alkoxycarbonyl, C₂-C₁₂-Dialkylamino, Phenyl, Naphthyl, Phenoxy, Naphthoxy, Biphenyl, Biphenyloxy, Phenthio oder Naphthio sind, wobei die genannten aromatischen Ringe gegebenenfalls mit Halogen, Cyano, Nitro, -OR₅, -NR₁₀R₁₁, C₁-C₄-Alkyl, Formyl, C₁-C₄-Alkylcarbonyl, COOR₇, C₁-C₄-Alkylthio, C₁-C₄-Alkylsulfonyl oder CONR₈R₉ substituiert sein können, sind; oder
R₂ und R₃ unabhängig voneinander C₂-C₆-Alkenyl, C₂-C₆-Alkenyl substituiert mit Halogen, Cyano, Nitro, C₁-C₈-Alkoxy, C₃-C₆-Trialkylsilyl, Hydroxyl, Amino, Ammonium, Tri-C₁-C₄-alkylammonium, -COOH, -COOM, worin M Ammonium oder ein Alkalimetallatom oder Erdalkalimetallatom ist, C₃-C₈-Cycloalkyl, C₂-C₅-Alkylcarbonyloxy, Phenylcarbonyloxy, Naphthylcarbonyloxy, C₁-C₆-Alkylamino, C₂-C₅-Alkoxycarbonyl, C₂-C₁₂-Dialkylamino, Phenyl, Naphthyl, Phenoxy, Naphthoxy, Biphenyl, Biphenyloxy, Phenthio oder Naphthio, wobei die genannten aromatischen Ringe gegebenenfalls mit Halogen, Cyano, Nitro, -OR₅, -NR₁₀R₁₁, C₁-C₄-Alkyl, Formyl, C₁-C₄-Alkylcarbonyl, COOR₇, C₁-C₄-Alkylthio, C₁-C₄-Alkylsulfonyl oder -CONR₈R₉ substituiert sein können, sind; oder
R₂ und R₃ unabhängig voneinander C₃-C₆-Alkinyl, C₃-C₆-Alkinyl substituiert mit Halogen, Cyano, Nitro, C₁-C₈-Alkoxy, C₃-C₆-Trialkylsilyl, Hydroxyl, Amino, Ammonium, Tri-C₁-C₄-alkylammonium, -COOH, -COOM, worin M Ammonium oder ein Alkalimetallatom oder Erdalkalimetallatom ist, C₃-C₈-Cycloalkyl, C₁-C₅-Alkylcarbonyloxy, Phenylcarbonyloxy, Naphthylcarbonyloxy, C₁-C₆-Alkylamino, C₁-C₅-Alkoxycarbonyl, C₂-C₁₂-Dialkylamino, Phenyl, Naphthyl, Phenoxy, Naphthoxy, Biphenyl, Biphenyloxy, Phenthio oder Naphthio, wobei die genannten aromatischen Ringe gegebenenfalls mit Halogen, Cyano, Nitro, -OR₅, -NR₁₀R₁₁, C₁-C₄-Alkyl, Formyl, C₁-C₄-Alkylcarbonyl, COOR₇, C₁-C₄-Alkylthio, C₁-C₄-Alkylsulfonyl oder -CONR₈R₉ substituiert sein können, sind; oder
R₂ und R₃ unabhängig voneinander Formyl, C₁-C₁₅-Alkylcarbonyl, C₂-C₁₅-Alkenylcarbonyl, C₄-C₉-Cycloalkylcarbonyl, C₆-C₉-Cycloalkenylcarbonyl oder C₃-C₈-Cycloalkyl-C₁-C₆-alkylcarbonyl, wobei diese Substituenten mit Halogen, Cyano, Nitro, Hydroxyl, Amino, C₁-C₆-Alkylamino, C₂-C₁₂-Dialkylamino, -COOH, -COOM, worin M Ammonium oder ein Alkalimetallatom oder Erdalkalimetallatom ist, C₁-C₈-Alkoxycarbonyl, C₄-C₁₀-Cycloalkoxycarbonyl, C₁-C₈-Alkylaminocarbonyl oder C₂-C₁₂-Dialkylaminocarbonyl substituiert sein können, sind; oder
R₂ und R₃ unabhängig voneinander gegebenenfalls gesättigtes Heterocyclyl oder Heterocyclylcarbonyl mit 3 bis 8 Ringatomen, das zusätzlich zu Kohlenstoffatomen mindestens ein Heteroatom, ausgewählt aus Stickstoff, Sauerstoff und Schwefel, aufweist, Heterocyclyl, wie vorstehend definiert, substituiert mit Halogen, Cyano, Nitro, C₁-C₅-Alkyl, C₁-C₅-Alkoxy, C₁-C₅-Alkylcarbonyl, C₁-C₅-Alkylcarbonyloxy, C₁-C₆-Alkoxycarbonyl, Aminocarbonyl, C₁-C₆-Alkylaminocarbonyl oder C₂-C₁₁-Dialkylaminocarbonyl, oder Heterocyclylcarbonyl, wie vorstehend definiert, substituiert mit Halogen, Cyano, Nitro, C₁-C₅-Alkyl, C₁-C₅-Alkoxy, C₁-C₅-Alkylcarbonyl, C₁-C₆-Alkoxycarbonyl, Aminocarbonyl, C₁-C₆-Alkylamino oder C₁-C₅-Alkylcarbonyloxy, sind; oder
R₂ und R₃ unabhängig voneinander Phenylcarbonyl, Biphenylcarbonyl, Naphthylcarbonyl, Phenyl-C₁-C₆-alkylcarbonyl, Biphenyl-C₁-C₆-alkylcarbonyl, Naphthyl-C₁-C₆-alkylcarbonyl, Phenyl-C₂-C₆-alkenylcarbonyl, Biphenyl-C₂-C₆-alkenylcarbonyl, Naphthyl-C₂-C₆-alkenylcarbonyl, Phenyl-C₃-C₆-alkinylcarbonyl, Biphenyl-C₃-C₆-alkinylcarbonyl oder Naphthyl-C₃-C₆-alkinylcarbonyl, wobei diese Substituenten mit C₁-C₅-Alkyl, C₁-C₅-Alkoxy, C₁-C₅-Alkylthio, C₁-C₅-Halogenalkyl, C₁-C₅-Alkylcarbonyl, Halogen, Cyano, Amino, Nitro, -COOR₇, C₁-C₈-Alkoxycarbonyl, Hydroxyl, C₁-C₅-Alkylsulfinyl, C₁-C₅-Alkylsulfonyl, C₁-C₆-Alkylaminocarbonyl oder C₂-C₁₂-Dialkylaminocarbonyl substituiert sein können, sind; oder
R₂ und R₃ unabhängig voneinander Phenyl, Naphthyl oder gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, wobei diese Substituenten mit Halogen, Cyano, Nitro, C₁-C₅-Alkyl, C₁-C₅-Alkoxy, C₁-C₅-Alkylthio, -COOH, -CONH₂, C₁-C₆-Alkylaminocarbonyl, C₂-C₁₀-Dialkylaminocarbonyl, C₁-C₅-Alkylcarbonyl oder C₁-C₅-Alkoxycarbonyl substituiert sein können, sind; oder
R₂ und R₃ zusammen mit dem Stickstoffatom, an das sie gebunden sind, einen gegebenenfalls gesättigten heterocyclischen Ring mit 3 bis 8 Ringatomen, der zusätzlich zu Kohlenstoffatomen mindestens ein Heteroatom, ausgewählt aus Stickstoff, Sauerstoff und Schwefel, aufweist, bilden, wobei der heterocyclische Ring mit C₁-C₅-Alkyl, C₁-C₅-Alkoxy, Halogen, Cyano oder Nitro substituiert sein kann, oder
R₂ und R₃ unabhängig voneinander Amino, C₁-C₆-Alkylamino, C₂-C₈-Dialkylamino, Phenylamino, Naphthylamino, C₁-C₆-Alkylcarbonylamino, C₁-C₁₀-Alkoxycarbonylamino, Hydroxyl, C₁-C₆-Alkoxy, C₁-C₆-Alkylcarbonyloxy, Phenoxy, Biphenyloxy oder Naphthoxy sind,
X O oder S(O)ₓ ist, worin x 0, 1 oder 2 ist, und
R₄ C₁-C₈-Alkyl, C₂-C₈-Alkenyl, C₃-C₈-Alkinyl, C₃-C₈-Cycloalkyl, C₅-C₈-Cycloalkenyl, C₃-C₈-Cycloalkyl-C₁-C₄-alkyl oder C₁-C₄-Alkyl-C₃-C₈-cycloalkyl ist, worin diese Substituenten gegebenenfalls mit Halogen, Cyano, Nitro, =O oder -OR₅ substituiert sein können, oder
R₄ Phenyl, Biphenyl, Naphthyl, Heterocyclyl, C₁-C₄-Alkylphenyl, C₁-C₄-Alkylnaphthyl, Phenyl-C₁-C₄-alkyl oder Naphthyl-C₁-C₄-alkyl ist, wobei diese Substituenten gegebenenfalls mit Halogen, Cyano, Nitro, Amino, -COOH, Hydroxyl, C₁-C₁₀-Alkyl, C₁-C₁₀-Alkyloxy, C₁-C₆-Alkylamino, Di-C₂-C₈-alkylamino, C₁-C₁₀-Alkylthio, C₁-C₁₀-Halogenalkyl, C₁-C₁₀-Halogenalkoxy, C₁-C₁₀-Halogenalkylthio, C₂-C₁₀-Alkoxycarbonylalkoxy, C₁-C₆-Alkylsulfinyl, C₁-C₆-Alkylsulfonyl, C₁-C₆-Alkyloxycarbonyl, C₁-C₆-Alkylcarbonyl, -CONH₂, Formyl, C₁-C₇-Alkylaminocarbonyl, C₂-C₁₁-Dialkylaminocarbonyl, C₃-C₆-Trialkylsilyl, C₁-C₁₀-Alkylcarbonylamino, C₁-C₁₀-Alkylcarbonyloxy, Phenoxy, Halogenphenoxy, Pyridyloxy oder Pyridyloxy substituiert mit Halogen, C₁-C₄-Alkyl, C₁-C₄-Alkoxy, Cyano, Nitro oder Amino, substituiert sein können, oder 2 einander benachbarte Substituenten am Phenyl- oder Naphthylring R₄ einen carbocyclischen oder gegebenenfalls gesättigten heterocyclischen Ring mit 3 bis 8 Ringatomen, der zusätzlich zu Kohlenstoffatomen mindestens ein Heteroatom, ausgewählt aus Stickstoff, Sauerstoff und Schwefel, aufweist, bilden, wobei der carbocyclische oder der heterocyclische Ring mit Halogen, Cyano, Nitro, Amino, -COOH, =O, C₁-C₁₀-Alkyl, C₁-C₁₀-Alkoxy, C₁-C₁₀-Alkylthio, C₁-C₁₀-Halogenalkyl, Hydroxyl, C₃-C₁₀-Alkoxycarbonylalkoxy, C₁-C₆-Alkylsulfinyl, C₁-C₆-Alkylsulfonyl, C₂-C₆-Alkyloxycarbonyl, C₂-C₆-Alkylcarbonyl, -CONH₂, Formyl, C₂-C₇-Alkylaminocarbonyl, C₃-C₁₁-Dialkylaminocarbonyl, C₃-C₆-Trialkylsilyl, C₂-C₁₀-Alkylcarbonylamino, C₂-C₁₀-Alkylcarbonyloxy, Phenoxy, Pyridyloxy oder Pyridyloxy substituiert mit Halogen, C₁-C₄-Alkyl, C₁-C₄-Alkoxy, Cyano, Nitro oder Amino, substituiert sein kann,
und Salze der Verbindungen der Formel I,
wobei die Verbindungen der Formeln la, la₁, la₂, la₃ und la₄ ausgeschlossen sind: 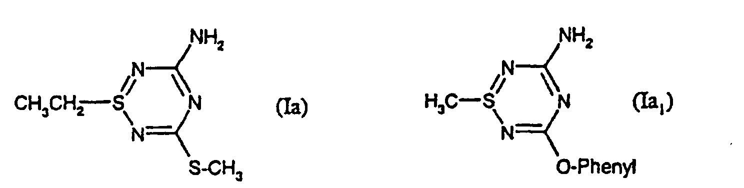 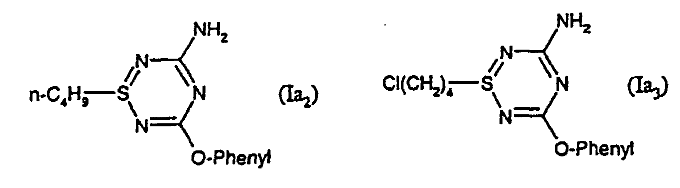 und

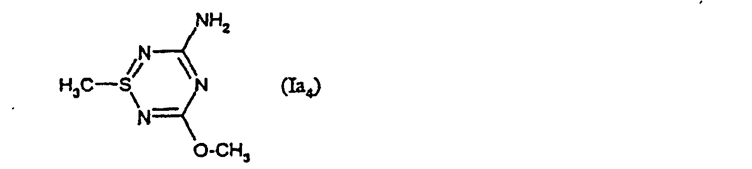

2. Verbindungen nach Anspruch 1, worin
R₁ C₁-C₁₀-Alkyl, C₃-C₈-Cycloalkyl, C₃-C₈-Cycloalkyl-C₁-C₆-alkyl, C₁-C₈-Alkyl-C₃-C₈-cycloalkyl, C₂-C₁₀-Alkenyl, C₃-C₈-Cycloalkyl-C₂-C₆-alkenyl, C₂-C₈-Alkenyl-C₃-C₈-cycloalkyl, C₂-C₁₀-Alkinyl, C₃-C₈-Cycloalkyl-C₂-C₆-alkinyl, C₂-C₈-Alkinyl-C₃-C₈-cycloalkyl, C₅-C₈-Cycloalkenyl, C₅-C₈-Cycloalkenyl-C₁-C₆-alkyl, C₁-C₈-Alkyl-C₅-C₈-cycloalkenyl, C₅-C₈-Cycloalkenyl-C₂-C₆-alkenyl, C₂-C₈-Alkenyl-C₅-C₈-cycloalkenyl, C₅-C₈-Cycloalkenyl-C₂-C₆-alkinyl, C₂-C₈-Alkinyl-C₅-C₈-cycloalkenyl, C₃-C₈-Cycloalkyl-C₃-C₈-cycloalkyl, C₅-C₈-Cycloalkenyl-C₅-C₈-cycloalkenyl, C₃-C₈-Cycloalkyl-C₅-C₈-cycloalkenyl, C₅-C₈-Cycloalkenyl-C₃-C₈-cycloalkyl, C₂-C₈-Alkinyl-C₂-C₆-alkenyl oder C₂-C₈-Alkenyl-C₂-C₆-alkinyl ist, wobei diese Substituenten gegebenenfalls mit Halogen, Cyano, Nitro, =O, -OR₅, -S(O)ₙR₆, -COOR₇, -CONR₈R₉, -NR₁₀R₁₁, =NR₁₂, Phenyl, Biphenyl, Naphthyl oder gegebenenfalls gesättigtem Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, substituiert sein können, wobei diese Phenyl-, Naphthyl- und Heterocyclylsubstituenten gegebenenfalls mit Halogen, Cyano, Nitro, -OR₅, -NR₁₀R₁₁ oder -CONR₈R₉ substituiert sein können, oder
R₁ gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, ist, wobei der Heterocyclus über eines seiner Kohlenstoffatome an den Thiatriazinring gebunden ist und der Heterocyclus mit Halogen, Cyano, Nitro, =O, -OR₅, -S(O)ₙR₆, -COOR₇, -CONR₈R₉, -NR₁₀R₁₁, =NR₁₂, =N-NR₁₃R₁₄, C₁-C₈-Alkyl, C₂-C₈-Alkenyl, C₂-C₈-Alkinyl, C₃-C₈-Cycloalkyl, C₅-C₈-Cycloalkenyl, Phenyl, Biphenyl, Naphthyl, C₁-C₈-Alkyl substituiert mit Halogen, Cyano, Nitro, -OR₅, -CONR₈R₉ oder -NR₁₀R₁₁, C₂-C₈-Alkenyl substituiert mit Halogen, Cyano, Nitro, -OR₅, -CONR₈R₉ oder -NR₁₀R₁₁, C₂-C₈-Alkinyl substituiert mit Halogen, Cyano, Nitro, -OR₅, -CONR₈R₉ oder -NR₁₀R₁₁, C₃-C₈-Cycloalkyl substituiert mit Halogen, Cyano, Nitro, -OR₅, -CONR₈R₉ oder -NR₁₀R₁₁, C₅-C₈-Cycloalkenyl substituiert mit Halogen, Cyano, Nitro, -OR₅, -CONR₈R₉ oder -NR₁₀R₁₁, Phenyl substituiert mit Halogen, Cyano, Nitro, C₁-C₆-Alkyl, C₁-C₆-Halogenalkyl, -OR₅, -CONR₈R₉ oder -NR₁₀R₁₁, Biphenyl substituiert mit Halogen, Cyano, Nitro, -OR₅, -CONR₈R₉ oder -NR₁₀R₁₁, oder Naphthyl substituiert mit Halogen, Cyano, Nitro, -OR₅, -CONR₈R₉ oder -NR₁₀R₁₁, substituiert sein kann, oder
R₁ Phenyl, Biphenyl, Naphthyl, C₁-C₄-Alkylphenyl, C₁-C₄-Alkylbiphenyl, C₁-C₄-Alkylnaphthyl, C₂-C₄-Alkenylphenyl, C₂-C₄-Alkenylbiphenyl, C₂-C₄-Alkenylnaphthyl, C₂-C₄-Alkinylphenyl, C₂-C₄-Alkinylbiphenyl oder C₂-C₄-Alkinylnaphthyl ist, wobei diese Substituenten mit Halogen, Cyano, Nitro, -OR₅, -S(O)ₙR₆, -COOR₇, -CONR₈R₉ oder -NR₁₀R₁₁ substituiert sein können, worin
R₅ Wasserstoff, C₁-C₆-Alkyl, C₁-C₆-Halogenalkyl, C₂-C₆-Alkoxyalkyl, C₁-C₆-Cyanoalkyl, Phenyl, Halogenphenyl, C₁-C₄-Alkoxyphenyl, Phenyl-C₁-C₄-alkyl, C₁-C₄-Alkylcarbonyl, Benzoyl, Halogenbenzoyl, C₁-C₄-Alkylamino, C₂-C₆-Dialkylamino, C₃-C₆-Trialkylsilyl, C₃-C₆-Cycloalkyl, C₂-C₄-Alkenyl oder C₃-C₄-Alkinyl ist,
n 0, 1 oder 2 ist,
R₆ Wasserstoff oder Cyano ist, wenn n 0 ist, oder
R₆ C₁-C₄-Alkyl, C₁-C₄-Halogenalkyl, Phenyl, C₁-C₄-Alkylphenyl, Halogenphenyl, gegebenenfalls gesättigtes Heterocyclyl oder Halogenheterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, oder C₃-C₆-Cycloalkyl ist,
R₇ Wasserstoff, C₁-C₄-Alkyl, C₁-C₄-Halogenalkyl, C₁-C₄-Cyanoalkyl, Phenyl, Halogenphenyl, C₂-C₄-Alkoxyalkyl, gegebenenfalls gesättigtes Heterocyclyl oder Halogenheterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, ist,
R₈ und R₉ unabhängig voneinander Wasserstoff, Phenyl, Halogenphenyl, C₁-C₄-Alkyl, C₁-C₄-Halogenalkyl, C₁-C₄-Cyanoalkyl, C₃-C₄-Alkenyl, C₃-C₄-Alkinyl oder C₂-C₄-Alkoxyalkyl sind, oder
R₈ und R₉ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden,
R₁₀ und R₁₁ unabhängig voneinander Wasserstoff, Phenyl, Halogenphenyl, C₁-C₄-Alkyl; C₁-C₄-Halogenalkyl, C₁-C₄-Cyanoalkyl, C₃-C₄-Alkenyl, C₃-C₄-Alkinyl, C₂-C₄-Alkoxyalkyl, Formyl, C₁-C₄-Alkylcarbonyl oder Phenylcarbonyl sind, wobei der Phenylteil darin mit C₁-C₄-Alkyl, Halogen, C₁-C₄-Alkoxy, Hydroxyl, Cyano, Nitro oder C₁-C₄-Alkoxycarbonyl substituiert sein kann, oder
R₁₀ und R₁₁ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden, und
R₁₂ Wasserstoff, C₁-C₄-Alkyl, C₁-C₄-Halogenalkyl, Phenyl, Halogenphenyl, Cyanophenyl, Nitrophenyl, C₁-C₄-Alkylphenyl, C₁-C₄-Alkoxyphenyl oder Phenyl-C₁-C₄-alkyl ist.

3. Verbindung nach Anspruch 2, worin
R₁ C₁-C₁₀-Alkyl, C₃-C₈-Cycloalkyl, C₃-C₈-Cycloalkyl-C₁-C₆-alkyl, C₁-C₈-Alkyl-C₃-C₈-cycloalkyl, C₂-C₁₀-Alkenyl, C₃-C₈-Cycloalkyl-C₂-C₆-alkenyl, C₂-C₈-Alkenyl-C₃-C₈-cycloalkyl, C₂-C₁₀-Alkinyl, C₃-C₈-Cycloalkyl-C₂-C₆-alkinyl, C₂-C₈-Alkinyl-C₃-C₈-cycloalkyl, C₅-C₈-Cycloalkenyl, C₅-C₈-Cycloalkenyl-C₁-C₆-alkyl, C₁-C₈-Alkyl-C₅-C₈-cycloalkenyl, C₅-C₈-Cycloalkenyl-C₂-C₆-alkenyl, C₂-C₈-Alkenyl-C₅-C₈-cycloalkenyl, C₅-C₈-Cycloalkenyl-C₂-C₆-alkinyl, C₂-C₈-Alkinyl-C₅-C₈-cycloalkenyl, C₃-C₈-Cycloalkyl-C₃-C₈-cycloalkyl, C₅-C₈-Cycloalkenyl-C₅-C₈-cycloalkenyl, C₃-C₈-Cycloalkyl-C₅-C₈-cycloalkenyl, C₅-C₈-Cycloalkenyl-C₃-C₈-cycloalkyl, C₂-C₈-Alkinyl-C₂-C₆-alkenyl oder C₂-C₈-Alkenyl-C₂-C₆-alkinyl ist, wobei diese Substituenten gegebenenfalls mit Halogen, Cyano, =O, -OR₅, -COOR₇, -CONR₈R₉, -NR₁₀R₁₁ oder Phenyl substituiert sein können, wobei dieser Phenylring gegebenenfalls mit Halogen substituiert sein kann, oder
R₁ gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, ist, wobei der Heterocyclus über eines seiner Kohlenstoffatome an den Thiatriazinring gebunden ist und der Heterocyclus mit Halogen, Cyano, =O, -OR₅, -S(O)ₙR₆, -COOR₇, -CONR₈R₉, -NR₁₀R₁₁, C₁-C₈-Alkyl, C₂-C₈-Alkenyl, C₂-C₈-Alkinyl, C₃-C₈-Cycloalkyl, C₅-C₈-Cycloalkenyl, Phenyl, C₁-C₈-Alkyl substituiert mit Halogen, Cyano, -OR₅, -CONR₈R₉ oder -NR₁₀R₁₁, C₂-C₈-Alkenyl substituiert mit Halogen, Cyano, -OR₅, -CONR₈R₉ oder -NR₁₀R₁₁, C₂-C₈-Alkinyl substituiert mit Halogen, Cyano, -OR₅, -CONR₈R₉ oder -NR₁₀R₁₁, C₃-C₈-Cycloalkyl substituiert mit Halogen, Cyano, -OR₅, -CONR₈R₉ oder -NR₁₀R₁₁, C₅-C₈-Cycloalkenyl substituiert mit Halogen, Cyano, -OR₅, -CONR₈R₉ oder -NR₁₀R₁₁, Phenyl substituiert mit Halogen, Cyano, Nitro, C₁-C₄-Alkyl, C₁-C₆-Halogenalkyl, -OR₅, -CONR₈R₉ oder -NR₁₀R₁₁, substituiert sein kann, oder
R₁ Phenyl, Biphenyl, Naphthyl, C₁-C₄-Alkylphenyl, C₁-C₄-Alkylbiphenyl, C₁-C₄-Alkylnaphthyl, C₂-C₄-Alkenylphenyl, C₂-C₄-Alkenylbiphenyl, C₂-C₄-Alkenylnaphthyl, C₂-C₄-Alkinylphenyl, C₂-C₄-Alkinylbiphenyl oder C₂-C₄-Alkinylnaphthyl ist, wobei diese Substituenten gegebenenfalls mit Halogen, Cyano, -OR₅, -S(O)ₙR₆, -COOR₇, -CONR₈R₉ oder -NR₁₀R₁₁ substituiert sein können, worin
R₅ Wasserstoff, C₁-C₆-Alkyl, C₁-C₆-Halogenalkyl, C₂-C₆-Alkoxyalkyl, C₁-C₆-Cyanoalkyl, Phenyl-C₁-C₄-alkyl, C₁-C₄-Alkylcarbonyl, Benzoyl, Halogenbenzoyl, C₃-C₆-Trialkylsilyl, C₃-C₆-Cycloalkyl, C₂-C₄-Alkenyl oder C₃-C₄-Alkinyl ist,
n 0, 1 oder 2 ist,
R₆ Wasserstoff oder Cyano ist, wenn n 0 ist, oder
R₆ C₁-C₄-Alkyl, C₁-C₄-Halogenalkyl, Phenyl oder C₁-C₄-Alkylphenyl ist,
R₇ Wasserstoff, C₁-C₄-Alkyl, C₁-C₄-Halogenalkyl, C₁-C₄-Cyanoalkyl, Phenyl, Halogenphenyl, C₂-C₄-Alkoxyalkyl, gegebenenfalls gesättiges Heterocyclyl oder Halogenheterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, ist,
R₈ und R₉ unabhängig voneinander Wasserstoff, Phenyl, Halogenphenyl, C₁-C₄-Alkyl, C₁-C₄-Halogenalkyl, C₁-C₄-Cyanoalkyl, C₃-C₄-Alkenyl, C₃-C₄-Alkinyl oder C₂-C₄-Alkoxyalkyl sind, oder
R₈ und R₉ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden,
R₁₀ und R₁₁ unabhängig voneinander Wasserstoff, Phenyl, Halogenphenyl, C₁-C₄-Alkyl, C₁-C₄-Halogenalkyl, C₁-C₄-Cyanoalkyl, C₃-C₄-Alkenyl, C₃-C₄-Alkinyl, C₂-C₄-Alkoxyalkyl, Formyl, C₁-C₄-Alkylcarbonyl oder Phenylcarbonyl sind, wobei der Phenylteil darin mit C₁-C₄-Alkyl, Halogen, C₁-C₄-Alkoxy, Hydroxyl, Cyano, Nitro oder C₁-C₄-Alkoxycarbonyl substituiert sein kann, oder
R₁₀ und R₁₁ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden.

4. Verbindungen nach Anspruch 3, worin
R₁ C₁-C₁₀-Alkyl, C₃-C₈-Cycloalkyl, C₃-C₈-Cycloalkyl-C₁-C₆-alkyl, C₁-C₈-Alkyl-C₃-C₈-cycloalkyl, C₂-C₁₀-Alkenyl, C₃-C₈-Cycloalkyl-C₂-C₆-alkenyl, C₂-C₈-Alkenyl-C₃-C₈-cycloalkyl, C₂-C₁₀-Alkinyl, C₃-C₈-Cycloalkyl-C₂-C₆-alkinyl, C₂-C₈-Alkinyl-C₃-C₈-cycloalkyl, C₅-C₈-Cycloalkenyl, C₅-C₈-Cycloalkenyl-C₁-C₆-alkyl, C₁-C₈-Alkyl-C₅-C₈-cycloalkenyl, C₅-C₈-Cycloalkenyl-C₂-C₆-alkenyl, C₂-C₈-Alkenyl-C₅-C₈-cycloalkenyl, C₅-C₈-Cycloalkenyl-C₂-C₆-alkinyl, C₂-C₈-Alkinyl-C₅-C₈-cycloalkenyl, C₃-C₈-Cycloalkyl-C₃-C₈-cycloalkyl, C₅-C₈-Cycloalkenyl-C₅-C₈-cycloalkenyl, C₃-C₈-Cycloalkyl-C₅-C₈-cycloalkenyl, C₅-C₈-Cycloalkenyl-C₃-C₈-cycloalkyl, C₂-C₈-Alkinyl-C₂-C₆-alkenyl oder C₂-C₈-Alkenyl-C₂-C₆-alkinyl ist, wobei diese Substituenten gegebenenfalls mit Halogen, Cyano, =O, -OR₅, -COOR₇, -CONR₈R₉, -NR₁₀R₁₁ oder Phenyl substituiert sein können, wobei dieser Phenylring gegebenenfalls mit Halogen substituiert sein kann, oder
R₁ gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, ist, wobei der Heterocyclus über eines seiner Kohlenstoffatome an den Thiatriazinring gebunden ist und der Heterocyclus mit Halogen, Cyano, =O, C₁-C₄-Alkoxy, C₁-C₈-Alkyl, C₂-C₈-Alkenyl, C₂-C₈-Alkinyl, Phenyl, C₁-C₈-Alkyl substituiert mit Halogen, C₂-C₈-Alkenyl substituiert mit Halogen, C₂-C₈-Alkinyl substituiert mit Halogen, Phenyl substituiert mit Halogen, Cyano, Nitro, C₁-C₄-Alkyl oder C₁-C₄-Alkoxy, substituiert sein kann, oder
R₁ Phenyl, Biphenyl, Naphthyl, C₁-C₄-Alkylphenyl, C₁-C₄-Alkylbiphenyl, C₁-C₄-Alkylnaphthyl, C₂-C₄-Alkenylphenyl oder C₂-C₄-Alkinylphenyl ist, wobei diese Substituenten gegebenenfalls mit Halogen, Cyano, -OR₅, -S(O)ₙR₆, -COOR₇, -CONR₈R₉ oder -NR₁₀R₁₁ substituiert sein können, worin
R₅ Wasserstoff, C₁-C₆-Alkyl, C₁-C₆-Halogenalkyl, C₂-C₆-Alkoxyalkyl, C₁-C₆-Cyanoalkyl, Phenyl-C₁-C₄-alkyl, C₁-C₄-Alkylcarbonyl, Benzoyl, Halogenbenzoyl, C₃-C₆-Trialkylsilyl, C₃-C₆-Cycloalkyl, C₂-C₄-Alkenyl oder C₃-C₄-Alkinyl ist,
n 0, 1 oder 2 ist,
R₆ Wasserstoff oder Cyano ist, wenn n 0 ist, oder
R₆ C₁-C₄-Alkyl, C₁-C₄-Halogenalkyl, Phenyl oder C₁-C₄-Alkylphenyl ist,
R₇ Wasserstoff, C₁-C₄-Alkyl, C₁-C₄-Halogenalkyl, C₁-C₄-Cyanoalkyl, Phenyl, Halogenphenyl, C₂-C₄-Alkoxyalkyl, gegebenenfalls gesättigtes Heterocyclyl oder Halogenheterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, ist,
R₈ und R₉ unabhängig voneinander Wasserstoff, Phenyl, Halogenphenyl, C₁-C₄-Alkyl, C₁-C₄-Halogenalkyl, C₁-C₄-Cyanoalkyl, C₃-C₄-Alkenyl, C₃-C₄-Alkinyl oder C₂-C₄-Alkoxyalkyl sind, oder
R₈ und R₉ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden,
R₁₀ und R₁₁ unabhängig voneinander Wasserstoff, Phenyl, Halogenphenyl, C₁-C₄-Alkyl, C₁-C₄-Halogenalkyl, C₁-C₄-Cyanoalkyl, C₃-C₄-Alkenyl, C₃-C₄-Alkinyl, C₂-C₄-Alkoxyalkyl, Formyl, C₁-C₄-Alkylcarbonyl oder Phenylcarbonyl sind, wobei der Phenylteil darin gegebenenfalls mit C₁-C₄-Alkyl, Halogen, C₁-C₄-Alkoxy, Hydroxyl, Cyano, Nitro oder C₁-C₄-Alkoxycarbonyl substituiert sein kann, oder
R₁₀ und R₁₁ zusammen mit dem Stickstoffatom, an das sie gebunden sind, einen gegebenenfalls gesättigten Heterocyclus mit 3 bis 8 Ringatomen, der halogeniert seiri kann, und der zusätzlich zu Kohlenstoffatomen mindestens ein Heteroatom, ausgewählt aus Stickstoff, Sauerstoff und Schwefel, aufweist, bilden.

5. Verbindungen nach Anspruch 1, worin
R₂ und R₃ unabhängig voneinander Wasserstoff, C₁-C₆-Alkyl, C₁-C₆-Alkyl substituiert mit Halogen, Cyano, Nitro, C₁-C₄-Alkoxy, C₃-C₆-Trialkylsilyl, Hydroxyl, Amino, Ammonium, Tri-C₁-C₄-alkylammonium, -COOH, -COOM, worin M Ammonium oder ein Alkalimetallatom oder Erdalkalimetallatom ist, oder C₃-C₈-Cycloalkyl, C₁-C₅-Alkylcarbonyloxy, Phenylcarbonyloxy, Naphthylcarbonyloxy, C₁-C₆-Alkylamino, C₁-C₅-Alkoxycarbonyl, C₂-C₆-Dialkylamino oder Phenyl sind, wobei der Phenylring gegebenenfalls mit Halogen, Cyano, Nitro, -OR₅, -NR₁₀R₁₁, C₁-C₄-Alkyl, Formyl, C₁-C₄-Alkylcarbonyl, COOR₇, C₁-C₄-Alkylthio, C₁-C₄-Alkylsulfonyl oder -CONR₈R₉ substituiert sein kann, oder
R₂ und R₃ unabhängig voneinander C₂-C₆-Alkenyl, C₂-C₆-Alkenyl substituiert mit Halogen, Cyano, Nitro, C₁-C₄-Alkoxy, C₃-C₆-Trialkylsilyl, Hydroxyl, Amino, Ammonium, Tri-C₁-C₄-alkylammonium, -COOH, -COOM, worin M Ammonium oder ein Alkalimetallatom oder Erdalkalimetallatom ist, C₁-C₆-Alkylamino, C₂-C₅-Alkoxycarbonyl, C₂-C₆-Dialkylamino oder Phenyl sind, wobei der Phenylring gegebenenfalls mit Halogen, Cyano, Nitro, -OR₅, -NR₁₀R₁₁, C₁-C₄-Alkyl, Formyl, C₁-C₄-Alkylcarbonyl, COOR₇, C₁-C₄-Alkylthio, C₁-C₄-Alkylsulfonyl oder -CONR₈R₉ substituiert sein kann, oder
R₂ und R₃ unabhängig voneinander C₃-C₆-Alkinyl, C₃-C₆-Alkinyl substituiert mit Halogen, Cyano, Nitro, C₁-C₈-Alkoxy, C₃-C₆-Trialkylsilyl, Hydroxyl, Amino, Ammonium, Tri-C₁-C₄-alkylammonium, -COOH, -COOM, worin M Ammonium oder ein Alkalimetallatom oder Erdalkalimetallatom ist, oder C₁-C₆-Alkylamino, C₂-C₅-Alkoxycarbonyl, C₂-C₆-Dialkylamino oder Phenyl sind, wobei der Phenylring gegebenenfalls mit Halogen, Cyano, Nitro, -OR₅, -NR₁₀R₁₁, C₁-C₄-Alkyl, Formyl, C₁-C₄-Alkylcarbonyl, COOR₇, C₁-C₄-Alkylthio, C₁-C₄-Alkylsulfonyl oder -CONR₈R₉ substituiert sein kann, oder
R₂ und R₃ unabhängig voneinander Formyl, C₁-C₈-Alkylcarbonyl, C₂-C₈-Alkenylcarbonyl, C₄-C₉-Cycloalkylcarbonyl, C₆-C₉-Cycloalkenylcarbonyl oder C₃-C₈-Cycloalkyl-C₁-C₆-alkylcarbonyl sind, wobei diese Substituenten gegebenenfalls mit Halogen, Cyano, Nitro, Hydroxyl, Amino, C₁-C₆-Alkylamino, C₂-C₇-Dialkylamino, -COOH, -COOM, worin M Ammonium oder ein Alkalimetallatom oder Erdalkalimetallatom ist, oder C₂-C₈-Alkoxycarbonyl, C₄-C₁₀-Cycloalkoxycarbonyl, C₁-C₈-Alkylaminocarbonyl oder C₂-C₈-Dialkylaminocarbonyl substituiert sein können, oder
R₂ und R₃ unabhängig voneinander gegebenenfalls gesättigtes Heterocyclyl oder Heterocyclylcarbonyl mit 3 bis 8 Ringatomen, das zusätzlich zu Kohlenstoffatomen mindestens ein Heteroatom, ausgewählt aus Stickstoff, Sauerstoff und Schwefel, aufweist, Heterocyclyl, wie vorstehend definiert, substituiert mit Halogen, Cyano, Nitro, C₁-C₅-Alkyl, C₁-C₅-Alkoxy, C₁-C₅-Alkylcarbonyl, C₁-C₅-Alkylcarbonyloxy, C₁-C₆-Alkoxycarbonyl, Aminocarbonyl, C₁-C₆-Alkylaminocarbonyl oder C₂-C₈-Dialkylaminocarbonyl, oder Heterocyclylcarbonyl substituiert mit Halogen, Cyano, Nitro, C₁-C₄-Alkyl, C₁-C₄-Alkoxyl C₁-C₄-Alkylcarbonyl, C₁-C₄-Alkoxycarbonyl, Aminocarbonyl, C₁-C₆-Alkylamino oder C₁-C₅-Alkylcarbonyloxy, sind, oder
R₂ und R₃ unabhängig voneinander Phenylcarbonyl, Biphenylcarbonyl, Naphthylcarbonyl, Phenyl-C₁-C₆-alkylcarbonyl, Biphenyl-C₁-C₆-alkylcarbonyl, Naphthyl-C₁-C₆-alkylcarbonyl, Phenyl-C₂-C₆-alkenylcarbonyl, Biphenyl-C₂-C₆-alkenylcarbonyl oder Naphthyl-C₂-C₆-alkenylcarbonyl sind, wobei diese Substituenten gegebenenfalls mit C₁-C₄-Alkyl, C₁-C₄-Alkoxy, C₁-C₄-Alkylthio, C₁-C₄-Halogenalkyl, C₁-C₄-Alkylcarbonyl, Halogen, Cyano, Amino, Nitro, -COOR₇, C₁-C₅-Alkoxycarbonyl, Hydroxyl, C₁-C₄-Alkylsulfonyl, C₁-C₅-Alkylaminocarbonyl oder C₂-C₆-Dialkylaminocarbonyl substituiert sein können, oder
R₂ und R₃ unabhängig voneinander Phenyl, Naphthyl oder gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, sind, wobei diese Substituenten gegebenenfalls mit Halogen, Cyano, Nitro, C₁-C₄-Alkyl, C₁-C₄-Alkoxy, C₁-C₄-Alkylthio, -COOH, -CONH₂, C₁-C₅-Alkylaminocarbonyl, C₂-C₇-Dialkylaminocarbonyl, C₁-C₄-Alkylcarbonyl oder C₁-C₅-Alkoxycarbonyl substituiert sein können, oder
R₂ und R₃ zusammen mit dem Stickstoffatom, an das sie gebunden sind, einen gegebenenfalls gesättigten heterocyclischen Ring mit 3 bis 8 Ringatomen, der zusätzlich zu Kohlenstoffatomen mindestens ein Heteroatom, ausgewählt aus Stickstoff, Sauerstoff und Schwefel, aufweist, bilden, wobei der heterocyclische Ring mit C₁-C₄-Alkyl, C₁-C₄-Alkoxy, Halogen, Cyano oder Nitro substituiert sein kann, oder
R₂ und R₃ unabhängig voneinander Amino, C₁-C₄-Alkylamino, C₂-C₆-Dialkylamino, Phenylamino, C₁-C₅-Alkylcarbonylamino, C₁-C₅-Alkoxycarbonylamino, Hydroxyl, C₁-C₄-Alkoxy, C₁-C₅-Alkylcarbonyloxy oder Phenoxy sind,
R₅ Wasserstoff, C₁-C₆-Alkyl, C₁-C₆-Halogenalkyl, C₂-C₆-Alkoxyalkyl, C₁-C₆-Cyanoalkyl, Phenyl, Halogenphenyl, C₁-C₄-Alkoxyphenyl, Phenyl-C₁-C₄-alkyl, C₁-C₄-Alkylcarbonyl, Benzoyl, Halogenbenzoyl, C₁-C₄-Alkylamino, C₂-C₆-Dialkylamino, C₃-C₆-Trialkylsilyl, C₃-C₆-Cycloalkyl, C₂-C₄-Alkenyl oder C₃-C₄-Alkinyl ist,
R₇ Wasserstoff, C₁-C₄-Alkyl, C₁-C₄-Halogenalkyl, C₁-C₄-Cyanoalkyl, Phenyl, Halogenphenyl, C₂-C₄-Alkoxyalkyl, gegebenenfalls gesättigtes Heterocyclyl oder Halogenheterocyclyl mit 3 bis 8 Ringatomen, die zusätzlich zu Kohlenstoffatomen mindestens ein Heteroatom, ausgewählt aus Stickstoff, Sauerstoff und Schwefel, aufweisen, ist,
R₈ und R₉ unabhängig voneinander Wasserstoff, Phenyl, Halogenphenyl, C₁-C₄-Alkyl, C₁-C₄-Halogenalkyl, C₁-C₄-Cyanoalkyl, C₃-C₄-Alkenyl, C₃-C₄-Alkinyl oder C₂-C₄-Alkoxyalkyl sind, oder
R₈ und R₉ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden,
R₁₀ und R₁₁ unabhängig voneinander Wasserstoff, Phenyl, Halogenphenyl, C₁-C₄-Alkyl, C₁-C₄-Halogenalkyl, C₁-C₄-Cyanoalkyl, C₃-C₄-Alkenyl, C₃-C₄-Alkinyl, C₂-C₄-Alkoxyalkyl, Formyl, C₁-C₄-Alkylcarbonyl oder Phenylcarbonyl sind, wobei der Phenylteil darin mit C₁-C₄-Alkyl, Halogen, C₁-C₄-Alkoxy, Hydroxyl, Cyano, Nitro oder C₁-C₄-Alkoxycarbonyl substituiert sein kann, oder
R₁₀ und R₁₁ zusammen mit dem Stickstoffatom, an das sie gebunden sind, einen gegebenenfalls gesättigten Heterocyclus mit 3 bis 8 Ringatomen, der halogeniert sein kann, und der zusätzlich zu Kohlenstoffatomen mindestens ein Heteroatom, ausgewählt aus Stickstoff, Sauerstoff und Schwefel, aufweist, bilden.

6. Verbindung nach Anspruch 5, worin
R₂ und R₃ unabhängig voneinander Wasserstoff, C₁-C₆-Alkyl, C₁-C₆-Alkyl substituiert mit Halogen, Hydroxyl, Amino, Ammonium, Tri-C₁-C₄-alkylammonium, -COOH, -COOM, worin M Ammonium oder ein Alkalimetallatom oder Erdalkalimetallatom ist, oder C₁-C₅-Alkylcarbonyloxyl Phenylcarbonyloxy, C₁-C₆-Alkylamino, C₁-C₅-Alkoxycarbonyl oder C₂-C₆-Dialkylamino sind, oder
R₂ und R₃ unabhängig voneinander Formyl, C₁-C₈-Alkylcarbonyl, C₂-C₈-Alkenylcarbonyl, C₄-C₉-Cycloalkylcarbonyl, C₆-C₉-Cycloalkenylcarbonyl oder C₃-C₈-Cycloalkyl-C₁-C₆-alkylcarbonyl sind, wobei diese Substituenten gegebenenfalls mit Halogen, Cyano, Hydroxyl, Amino, -COOH oder -COOM, worin M Ammonium oder ein Alkalimetallatom oder Erdalkalimetallatom ist, substituiert sein können, oder
R₂ und R₃ unabhängig voneinander gegebenenfalls gesättigtes Heterocyclyl oder Heterocyclylcarbonyl mit 3 bis 8 Ringatomen, das zusätzlich zu Kohlenstoffatomen mindestens ein Heteroatom, ausgewählt aus Stickstoff, Sauerstoff und Schwefel, aufweist, Heterocyclyl, wie vorstehend definiert, substituiert mit Halogen, Cyano, Nitro, C₁-C₅-Alkyl, C₁-C₅-Alkoxy oder C₁-C₆-Alkoxycarbonyl, oder Heterocyclylcarbonyl, wie vorstehend definiert, substituiert mit Halogen, Cyano, Nitro, C₁-C₄-Alkyl, C₁-C₄-Alkoxy oder C₁-C₄-Alkoxycarbonyl, sind oder
R₂ und R₃ unabhängig voneinander Phenylcarbonyl, Biphenylcarbonyl, Naphthylcarbonyl, Phenyl-C₁-C₆-alkylcarbonyl, Biphenyl-C₁-C₆-alkylcarbonyl, Naphthyl-C₁-C₆-alkylcarbonyl, Phenyl-C₂-C₆-alkenylcarbonyl, Biphenyl-C₂-C₆-alkenylcarbonyl oder Naphthyl-C₂-C₆-alkenylcarbonyl sind, wobei diese Substituenten gegebenenfalls mit C₁-C₄-Alkyl, C₁-C₄-Alkoxy, C₁-C₄-Alkylthio, C₁-C₄-Halogenalkyl, C₁-C₄-Alkylcarbonyl, Halogen, Cyano, Amino, Nitro, -COOH, C₁-C₅-Alkoxycarbonyl, Hydroxyl oder C₁-C₄-Alkylsulfonyl substituiert sein können, sind oder
R₂ und R₃ unabhängig voneinander Phenyl, Naphthyl oder gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, sind, wobei diese Substituenten gegebenenfalls mit Halogen, Cyano, C₁-C₄-Alkyl, C₁-C₄-Alkoxy, C₁-C₄-Alkylthio, -COOH oder C₁-C₅-Alkoxycarbonyl substituiert sein können, oder
R₂ und R₃ zusammen mit dem Stickstoffatom, an das sie gebunden sind, einen gegebenenfalls gesättigten heterocyclischen Ring mit 3 bis 8 Ringatomen, der zusätzlich zu Kohlenstoffatomen mindestens ein Heteroatom, ausgewählt aus Stickstoff, Sauerstoff und Schwefel, aufweist, bilden, wobei der heterocyclische Ring mit C₁-C₄-Alkyl, C₁-C₄-Alkoxy, Halogen oder Cyano substituiert sein kann.

7. Verbindung nach Anspruch 6, worin
R₂ und R₃ Wasserstoff sind, oder
R₂ und R₃ unabhängig voneinander Formyl, C₁-C₈-Alkylcarbonyl, C₂-C₈-Alkenylcarbonyl, C₄-C₉-Cycloalkylcarbonyl, C₆-C₉-Cycloalkenylcarbonyl oder C₃-C₈-Cycloalkyl-C₁-C₆-alkylcarbonyl sind, wobei diese Substituenten gegebenenfalls mit Halogen, Cyano, Hydroxyl oder Amino substituiert sein können, oder
R₂ und R₃ Phenylcarbonyl sind, wobei der Phenylring gegebenenfalls mit C₁-C₄-Alkyl, C₁-C₄-Alkoxy, C₁-C₄-Alkylthio, C₁-C₄-Halogenalkyl, Halogen, Cyano, Nitro, -COOH, C₁-C₅-Alkoxycarbonyl, Hydroxyl oder C₁-C₄-Alkylsulfonyl substituiert sein kann.

8. Verbindung nach Anspruch 1, worin X O oder S(O)ₓ ist, worin x 0, 1 oder 2 ist, und
R₄ Methyl substituiert mit Halogen, Cyano, Nitro oder OR₅ ist, oder
R₄ C₂-C₈-Alkyl, C₂-C₈-Alkenyl, C₃-C₈-Alkinyl, C₃-C₈-Cycloalkyl, C₅-C₈-Cycloalkenyl, C₃-C₈-Cycloalkyl-C₁-C₄-alkyl oder C₁-C₄-Alkyl-C₃-C₈-cycloalkyl ist, worin diese Substituenten gegebenenfalls mit Halogen, Cyano, Nitro, =O oder -OR₅ substituiert sein können, oder
R₄ Phenyl substituiert mit Halogen, Cyano, Nitro, Amino, -COOH, Hydroxyl, C₁-C₄-Alkyl, C₁-C₄-Alkyloxy, C₁-C₄-Alkylthio, C₁-C₄-Halogenalkyl, C₁-C₄-Halogenalkoxy, C₁-C₄-Halogenalkylthio, C₂-C₆-Alkoxycarbonylalkoxy, C₁-C₄-Alkylsulfinyl, C₁-C₄-Alkylsulfonyl, C₁-C₆-Alkyloxycarbonyl, C₁-C₆-Alkylcarbonyl, -CONH₂, Formyl, C₁-C₅-Alkylaminocarbonyl, C₂-C₇-Dialkylaminocarbonyl, C₁-C₄-Alkylamino, C₂-C₆-Dialkylamino, C₃-C₆-Trialkylsilyl, C₁-C₆-Alkylcarbonylamino, C₁-C₆-Alkylcarbonyloxy, Phenoxy, Halogenphenoxy oder Pyridyloxy, ist oder
R₄ Biphenyl, Naphthyl, gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, C₁-C₄-Alkylphenyl, C₁-C₄-Alkylnaphthyl, Phenyl-C₁-C₄-alkyl oder Naphthyl-C₁-C₄-alkyl ist, wobei diese Substituenten gegebenenfalls mit Halogen, Cyano, Nitro, Amino, -COOH, Hydroxyl, C₁-C₄-Alkyl, C₁-C₄-Alkyloxy, C₁-C₄-Alkylthio, C₁-C₄-Halogenalkyl, C₁-C₄-Halogenalkoxy, C₁-C₄-Halogenalkylthio, C₂-C₆-Alkoxycarbonylalkoxy, C₁-C₄-Alkylsulfinyl, C₁-C₄-Alkylsulfonyl, C₁-C₆-Alkyloxycarbonyl, C₁-C₆-Alkylcarbonyl, -CONH₂, Formyl, C₁-C₅-Alkylaminocarbonyl, C₂-C₇-Dialkylaminocarbonyl, C₃-C₆-Trialkylsilyl, C₁-C₆-Alkylcarbonylamino, C₁-C₆-Alkylcarbonyloxy, Phenoxy, Halogenphenoxy oder Pyridyloxy substituiert sein können, und
R₅ Wasserstoff, C₁-C₆-Alkyl, C₁-C₆-Halogenalkyl, C₂-C₆-Alkoxyalkyl, C₁-C₆-Cyanoalkyl, Phenyl, Halogenphenyl, C₁-C₄-Alkoxyphenyl, Phenyl-C₁-C₄-alkyl, C₁-C₄-Alkylcarbonyl, Benzoyl, Halogenbenzoyl, C₁-C₄-Alkylamino, C₂-C₆-Dialkylamino, C₃-C₆-Trialkylsilyl, C₃-C₆-Cycloalkyl, C₂-C₄-Alkenyl oder C₃-C₄-Alkinyl ist.

9. Verbindungen nach Anspruch 8, worin
X O oder S ist, und
R₄ Methyl substituiert mit Halogen oder Cyano ist, oder
R₄ C₂-C₈-Alkyl, C₂-C₈-Alkenyl, C₃-C₈-Alkinyl, C₃-C₈-Cycloalkyl, C₅-C₈-Cycloalkenyl, C₃-C₈-Cycloalkyl-C₁-C₄-alkyl oder C₁-C₄-Alkyl-C₃-C₈-cycloalkyl ist, worin diese Substituenten gegebenenfalls mit Halogen oder Cyano substituiert sein können, oder R₄ Phenyl substituiert mit Halogen, Cyano, Nitro, Amino, C₁-C₄-Alkyl, C₁-C₄-Alkyloxy, C₁-C₄-Alkylthio, C₁-C₄-Halogenalkyl, C₁-C₄-Halogenalkoxy, C₂-C₆-Alkoxycarbonylalkoxy, C₁-C₄-Alkylsulfonyl, C₁-C₆-Alkyloxycarbonyl, C₁-C₆-Alkylcarbonyl, Formyl, C₁-C₄-Alkylamino, C₂-C₆-Dialkylamino, C₃-C₆-Trialkylsilyl oder C₂-C₆-Alkylcarbonyloxy ist, oder
R₄ Biphenyl, Naphthyl, gegebenenfalls gesättigtes Heterocyclyl mit 3 bis 8 Ringatomen, das zusätzlich zu Kohlenstoffatomen mindestens ein Heteroatom, ausgewählt aus Stickstoff, Sauerstoff und Schwefel, aufweist, C₁-C₄-Alkylphenyl, C₁-C₄-Alkylnaphthyl, Phenyl-C₁-C₄-alkyl oder Naphthyl-C₁-C₄-alkyl ist, wobei diese Substituenten gegebenenfalls mit Halogen, Cyano, Nitro, Amino, C₁-C₄-Alkyl, C₁-C₄-Alkyloxy, C₁-C₄-Alkylthio, C₁-C₄-Halogenalkyl, C₁-C₄-Halogenalkoxy, C₂-C₆-Alkoxycarbonylalkoxy, C₁-C₄-Alkylsulfonyl, C₁-C₆-Alkyloxycarbonyl, C₁-C₆-Alkylcarbonyl, C₃-C₆-Trialkylsilyl oder C₁-C₆-Alkylcarbonyloxy substituiert sein können.

10. Verbindungen nach Anspruch 9, worin
X O ist, und
R₄ Phenyl substituiert mit Halogen, Cyano, Nitro, Amino, C₁-C₄-Alkyl, C₁-C₄-Alkyloxy, C₁-C₄-Alkylthio, C₁-C₄-Halogenalkyl, Halogenmethoxy, C₁-C₄-Alkylsulfonyl, C₁-C₄-Alkylamino oder C₂-C₆-Dialkylamino ist.

11. Verfahren zur Herstellung der Verbindungen nach Anspruch 1, das umfasst
(1) Umsetzen einer Verbindung der Formel II 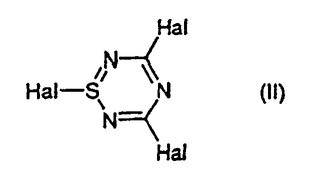 worin Hal-Substituenten unabhängig voneinander Fluor, Chlor oder Brom sind, mit einer metallorganischen Verbindung der Formel III
R₁-M (III),
worin R₁ wie in Anspruch 1 definiert ist und M ein ein- oder mehrwertiges Metallatom ist,
(2a) Umsetzen der erhaltenen Verbindung der Formel IV 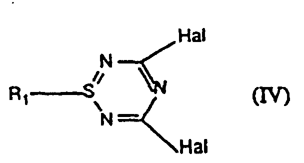 worin R₁ wie in Anspruch 1 definiert ist, und Hal-Reste unabhängig voneinander Fluor, Chlor oder Brom sind, mit der Verbindung der Formel V
H-XR₄ (V),
worin X und R₄ wie vorstehend definiert sind, zu der Verbindung der Formel VI 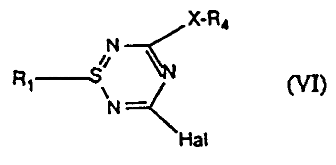 und
(3a) dann Umsetzen dieser Verbindungen mit den Verbindungen der Formel VII
M₁-NR₂R₃ (VII),
worin R₂ und R₃ wie vorstehend definiert sind, und M₁ Wasserstoff oder ein Metallatom ist, zu den Endprodukten der Formel I; oder
(2b) Umsetzen der Verbindungen der Formel IV mit den Verbindungen der Formel VII zu den Verbindungen der Formel VIII 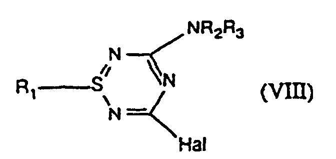 worin R₁, R₂ und R₃ wie in Anspruch 1 definiert sind, und Hal Fluor, Chlor oder Brom ist, und
(3b) dann Umwandeln dieser Verbindungen mit den Verbindungen der Formel V in die Endprodukte der Formel I.

12. Herbizides und den Pflanzenwuchs hemmendes Mittel, das eine oder mehrere Verbindungen der Formel I nach Anspruch 1 umfasst.

13. Mittel nach Anspruch 12, das zwischen 0,1 % und 95% Wirkstoff der Formel I umfasst.

14. Verfahren zur Bekämpfung unerwünschten Pflanzenwachstums, das Applizieren einer wirksamen Menge eines Wirkstoffs der Formel I nach Anspruch 1, oder ein diesen Wirkstoff enthaltendes Mittel auf die Pflanzen oder deren Lebensraum umfasst.

15. Verfahren nach Anspruch 14, wobei eine Wirkstoffmenge zwischen 0,001 und 5 kg, insbesondere zwischen 0,005 bis 2 kg, pro Hektar appliziert wird.

16. Verfahren zur Hemmung des Pflanzenwachstums, das Applizieren einer wirksamen Menge eines Wirkstoffs der Formel I nach Anspruch 1, oder ein diesen Wirkstoff enthaltendes Mittel auf die Pflanzen oder deren Lebensraum umfasst.

17. Verfahren nach Anspruch 14 zur selektiven pre- oder post-emergenten Bekämpfung von Unkräutern in Nutzpflanzenkulturen.

18. Verwendung eines Mittels nach Anspruch 12 zur selektiven pre- oder post-emergenten Bekämpfung von Unkräutern in Nutzpflanzenkulturen.

19. Saatgut, behandelt mit einem Mittel nach Anspruch 12.

20. Verbindung der Formel IV 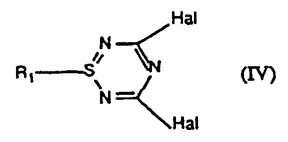 worin R₁ wie in Anspruch 1 definiert ist und Hal unabhängig voneinander Fluor, Chlor oder Brom sind.

21. Verbindung der Formel VI 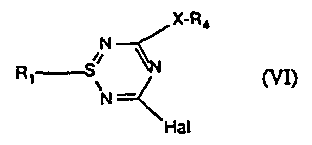 worin R₁, X und R₄ wie in Anspruch 1 definiert sind und Hal Fluor, Chlor oder Brom ist.

22. Verbindung der Formel VIII 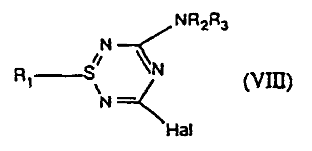 worin R₁, R₂ und R₃ wie in Anspruch 1 definiert sind und Hal Fluor, Chlor oder Brom ist.

## Revendications

1. Composé de formule I 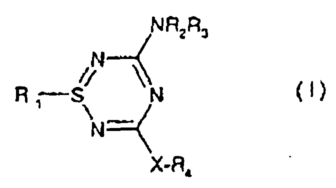 dans laquelle
R₁ représente un groupe alkyle en C₁-C₂₀, cycloalkyle en C₃-C₈, (cycloalkyle en C₃-C₈) (alkyle en C₁-C₁₇), (alkyle en C₁-C₁₇) (cycloalkyle en C₃-C₈), alcényle en C₂-C₁₇, (cycloalkyle en C₃-C₈) (alcényle en C₂-C₇), (alcényle en C₂-C₁₇) (cycloalkyle en C₃-C₈), alkynyle en C₂-C₁₇, (cycloalkyle en C₃-C₈) (alkynyle en C₂-C₁₇), (alkynyle en C₂-C₁₇) (cycloalkyle en C₃-C₈), cycloalcényle en C₅-C₈, (cycloalcényle en C₅-C₈) (alkyle en C₁-C₁₇), (alkyle en C₁-C₁₇) (cycloalcényle en C₅-C₈), (cycloalcényle en C₅-C₈) (alcényle en C₂-C₁₇), (alcényle en C₂-C₁₇) (cycloalcényle C₅-C₈), (cycloalcényle en C₅-C₈) (alkynyle en C₂-C₁₇), (alkynyle en C₂-C₁₇) (cycloalcényle en C₅-C₈), (cycloalkyle en C₃-C₈)(cycloalkyle en C₃-C₈), (cycloalcényle en C₅-C₈) (cycloalcényle en C₅-C₈), (cycloalkyle en C₃-C₈) (cycloalcényle en C₅-C₈), (cycloalcényle en C₅-C₈) (cycloalkyle en C₃-C₈), (alkynyle en C₂-C₁₇) (alcényle en C₂-C₁₇) ou (alcényle en C₂-C₁₇)(alkynyle en C₂-C₁₇), ces substituants pouvant être non substitués ou substitués par un atome d'halogène, un groupe cyano, nitro, =O, -OR₅,-S(O)ₙR₆, -COOR₇, -CONR₈R₉, -NR₁₀R₁₁, =NR₁₂, =N-NR₁₃R₁₄, phényle, biphényle, naphtyle ou un hététérocyclyle saturé ou insaturé comportant 3 à 8 atomes dans le cycle, qui, en plus des atomes de carbone, comprend au moins un hétéroatome choisi parmi un atome d'azote, d'oxygène et de soufre, ces substituants phényle, naphtyle et hétérocyclyle pouvant être non substitués ou substitués par un atome d'halogène un groupe cyano, nitro, -O, -OR₅, -NR₁₀R₁₁, ou-CONR₈R₉, ou bien R¹ représente un groupe hétérocyclyle saturé ou insaturé comportant 3 à 8 atomes dans le cycle, qui, en plus des atomes de carbone, comprend au moins un hétéroatome choisi parmi un atome d'azote, d'oxygène et de soufre, l'hétérocycle étant lié au cycle thiatriazine par l'intermédiaire d'un de ses atomes de carbone, et l'hétérocycle pouvant être substitué par un atome d'halogène, un groupe cyano, nitro, =O, -OR₅, -S(O)ₙR₆,-COOR₇, -CONR₈R₉, -NR₁₀R₁₁, =NR₁₂, -N-NR₁₃R₁₄, alkyle en C₁-C₁₇, alcényle en C₂-C₁₇, alkynyle en C₂-C₁₇, cycloalkyle en C₃-C₈, cycloalcényle en C₅-C₈, phényle, biphényle, naphtyle, alkyle en C₁-C₁₇ qui est substitué par un atome d'halogène, un groupe cyano, nitro, -OR₅, -CONR₈R₉ ou -NR₁₀R₁₁, alcényle en C₂-C₁₇, qui est substitué par un atome d'halogène, un groupe cyano, nitro, -OR₅, -CONR₈R₉ ou -NR₁₀R₁₁, alkynyle en C₂-C₁₇, qui est substitué par un atome d'halogène, un groupe cyano, nitro, -OR₅, -CONR₈R₉ ou -NR₁₀R₁₁, cycloalkyle en C₃-C₈, qui est substitué par un atome d'halogène, un groupe cyano, nitro, -OR₅, -CONR₈R₉ ou -NR₁₀R₁₁, cycloalcényle en C₅-C₈, qui est substitué par un atome d'halogène, un groupe cyano, nitro, -OR₅, -CONR₈R₉ ou -NR₁₀R₁₁, phényle, qui est substitué par un atome d'halogène, un groupe cyano, nitro, -OR₅, -CONR₈R₉ ou -NR₁₀R₁₁, biphényle, qui est substitué par un atome d'halogène, un groupe cyano, nitro, -OR₅, -CONR₈R₉ ou -NR₁₀R₁₁, ou naphtyle, qui est substitué par un atome d'halogène, un groupe cyano, nitro, -OR₅, -CONR₈R₉ ou-NR₁₀R₁₁, ou bien R₁ représente un groupe phényle, biphényle, naphtyle, (alkyle en C₁-C₁₀)phényle, (alkyle en C₁-C₁₀)biphényle, (alkyle en C₁-C₁₀)naphtyle, (alcényle en C₂-C₁₀)phényle, (alcényle en C₂-C₁₀)biphényle, (alcényle en C₂-C₁₀)naphtyle, (alkynyle en C₂-C₁₀)phényle, (alkynyle en C₂-C₁₀)biphényle ou (alkynyle en C₂-C₁₀)naphtyle, ces substituants pouvant être non substitués ou substitués par un atome d'halogène, un groupe cyano, nitro, -OR₅, -S(O)ₙR₆, -COOR₇, -CONR₈R₉ ou -NR₁₀R₁₁, dans lesquels
R₅ représente un atome d'hydrogène, un groupe alkyle en C₁-C₆, halogénoalkyle en C₁-C₆, alcoxyalkyle en C₁-C₆, cyanoalkyle en C₁-C₆, phényle, halogénophényle, alcoxyphényle en C₁-C₄, phényl-(alkyle en C₁-C₄), alkylcarbonyle en C₁-C₇, benzoyle, halogénobenzoyle, alkylamino en C₁-C₆, dialkylamino en C₂-C₈, -N=CH₂, -N=CH-(alkyle en C₁-C₄) -N=C(alkyle en C₁-C₄)₂, trialkylsilyle en C₃-C₆, cycloalkyle en C₃-C₇, alcényle en C₂-C₇ ou alkynyle en C₃-C₇,
n vaut 0, 1 ou 2,
R₆ représente un atome d'hydrogène ou un groupe cyano si n vaut 0 ou bien
R₆ représente un groupe alkyle en C₁-C₅, halogénoalkyle en C₁-C₅, hydroxyalkyle en C₁-C₅, phényle, naphtyle, (alkyle en C₁-C₄)naphtyle, halogénophényle, halogénonaphtyle, hétérocyclyle saturé ou insaturé ou halogénohétérocyclyle comportant 3 à 8 atomes dans le cycle, qui, en plus des atomes de carbone comportent au moins un hétéroatome choisi parmi un atome d'azote, d'oxygène ou de soufre ou un groupe cycloalkyle en C₃-C₇,
R₇ représente un atome d'hydrogène, groupe alkyle en C₁-C₁₂, halogénoalkyle en C₁-C₁₂, nitroalkyle en C₁-C₆, cyanoalkyle en C₁-C₆, phényle, naphtyle, halogénophényle, halogénonaphtyle, alcoxyalkyle en C₂-C₁₀, alcoxycarbonyle en C₁-C₁₀, un groupe hétérocyclyle ou halogénohétérocyclyle saturé ou insaturé comportant 3 à 8 atomes dans le cycle, qui, en plus des atomes de carbone comportent au moins un hétéroatome choisi parmi un atome d'azote, d'oxygène ou de soufre, un groupe cycloalkyle en C₃-C₇, halogénocycloalkyle en C₃-C₇,, -N=CH₂, -N=CH (alkyle en C₁-C₄), -N=C (alkyle en C₁-C₄)₂, ou dialkylamino en C₂-C₆
R₈ et R₉ représentent indépendamment l'un de l'autre un atome d'hydrogène, un groupe phényle, naphtyle, halogénophényle, halogénonaphtyle, alkyle en C₁-C₅, halogénoalkyle en C₁-C₈, alcoxy en C₁-C₈, phénoxy, naphtoxy, halogénophénoxy, halogénonaphtoxy, cyanoalkyle en C₁-C₇, alcényle en C₃-C₇, alkynyle en C₃-C₇ ou alcoxyalkyle en C₂-C₈, ou bien R₈ et R₉ pris ensemble avec l'atome d'azote auquel ils sont liés forment un hétérocycle saturé ou insaturé comportant 3 à 8 atomes de cycle qui peuvent être halogénés, et qui, en plus des atomes de carbone, comprennent au moins un hétéroatome choisi parmi un atome d'azote, d'oxygène ou de soufre,
R₁₀ et R₁₁, indépendamment l'un de l'autre, représentent un atome d'hydrogène, un groupe phényle, naphtyle, halogénophényle, halogénonaphtyle, alkyle en C₁-C₈, halogénoalkyle en C₁-C₈, alcoxy en C₁-C₈, phénoxy, naphtoxy, halogénophénoxy, halogénonaphtoxy, cyanoalkyle en C₁-C₇, alcényle en C₃-C₇, alkynyle en C₃-C₇, alcoxyalkyle en C₂-C₈, alkylamino en C₁-C₆, dialkylamino en C₂-C₆, formyle, alkylcarbonyle en C₂-C₈, phénylcarbonyle ou naphtylcarbonyle, les parties phényle ou naphtyle pouvant être substituées par un groupe alkyle en C₁-C₄, un atome d'halogène, un groupe alcoxy en C₁-C₄, hydroxy, cyano, nitro ou alcoxycarbonyle en C₁-C₆, ou
R₁₀ et R₁₁ pris ensemble avec l'atome d'azote auquel ils sont liés forment un hétérocycle saturé ou insaturé comprenant 3 à 8 atomes de cycle qui peuvent être halogénés, et qui, en plus des atomes de carbone, comprennent au moins un hétéroatome choisi parmi un atome d'azote, d'oxygène ou de soufre,
R₁₂ représente un atome d'hydrogène, un groupe alkyle en C₁-C₈, halogénoalkyle en C₁-C₈, phényle, halogénophényle, cyanophényle, nitrophényle, (alkyle en C₁-C₄)phényle, (alcoxy en C₁-C₄)phényle, naphtyle, cyanonaphtyle, nitronaphtyle, halogénonaphtyle, (alkyle en C₁-C₄)naphtyle, (alcoxy en C₁-C₄)naphtyle, phényl-(alkyle en C₁-C₆), ou naphtyl-(alkyle en C₁-C₆), et
R₁₃ et R₁₄ représentent indépendamment l'un de l'autre un groupe alkyle en C₁-C₆, alkylcarbonyle C₁-C₆, phényle, halogénophényle, cyanophényle, nitrophényle, (alkyle en C₁-C₄)phényle, (alcoxy en C₁-C₄)phényle, naphtyle, cyanonaphtyle, nitronaphtyle, halogénonaphtyle, (alkyle en C₁-C₄)-naphtyle, ou (alcoxy en C₁-C₄)-naphtyle,
R₂ et R₃ représentent indépendamment l'un de l'autre un atome d'hydrogène, un groupe alkyle en C₁-C₆, alkyle en C₁-C₆ qui est substitué par un atome d'halogène, un groupe cyano, nitro, alcoxy en C₁-C₈, trialkylsilyle en C₃-C₆, hydroxyle, amino, ammonium, tri-(alkyle en C₁-C₄)ammonium, -COOH, -COOM, où M représente un groupe ammonium ou un atome de métal alcalin ou un atome de métal alcalino-terreux, un groupe cycloalkyle en C₃-C₈, (alkyle en C₁-C₅) carbonyloxy, phénylcarbonyloxy, naphtylcarbonyloxy, alkylamino en C₁-C₆, alcoxycarbonyle en C₁-C₅, dialkylamino en C₁-C₁₂, phényle, naphtyle, phénoxy, naphtoxy, biphényle, biphényloxy, phénylthio ou naphthio; les cycles aromatiques mentionnés ci-dessus pouvant être non substitués ou substitués par un atome d'halogène, un groupe cyano, nitro, -OR₅, -NR₁₀R₁₁, alkyle en C₁-C₄, formyle, alkylcarbonyle en C₁-C₄, COOR₇, alkylthio en C₁-C₄, alkylsulfonyle en C₁-C₄, ou CONR₈R₉,
R₂ et R₃ représentent indépendamment l'un de l'autre un groupe alcényle en C₂-C₆, alcényle en C₂-C₆, qui est substitué par un atome d'halogène, un groupe cyano, nitro, alcoxy en C₁-C₈, trialkylsilyle en C₃-C₆, hydroxyle, amino, ammonium, tri-(alkyle en C₁-C₄)ammonium, -COOH, -COOM, où M représente un groupe ammonium ou un atome de métal alcalin ou un atome de métal alcalino-terreux, un groupe cycloalkyle en C₃-C₈, (alkyle en C₁-C₅) carbonyloxy, phénylcarbonyloxy, naphtylcarbonyloxy, alkylamino en C₁-C₆, alcoxycarbonyle en C₁-C₅, dialkylamino en C₁-C₁₂, phényle, naphtyle, phénoxy, naphtoxy, biphényle, biphényloxy, phénylthio ou naphthio; les cycles aromatiques mentionnés ci-dessus pouvant être non substitués ou substitués par un atome d'halogène, un groupe cyano, nitro, -OR₅, -NR₁₀R₁₁, alkyle en C₁-C₄, formyle, alkylcarbonyle en C₁-C₄, COOR₇, alkylthio en C₁-C₄, alkylsulfonyle en C₁-C₄, ou CONR₈R₉, ou
R₂ et R₃ représentent indépendamment l'un de l'autre un groupe alkynyle en C₃-C₆, alkynyle en C₃-C₆ qui est substitué par un atome d'halogène, un groupe cyano, nitro, alcoxy en C₁-C₈, trialkylsilyle en C₃-C₆, hydroxyle, amino, ammonium, tri-(alkyle en C₁-C₄)ammonium, -COOH, -COOM, où M représente un groupe ammonium ou un atome de métal alcalin ou un atome de métal alcalino-terreux, un groupe cycloalkyle en C₃-C₈, (alkyle en C₁-C₅)-carbonyloxy, phénylcarbonyloxy, naphtylcarbonyloxy, alkylamino en C₁-C₆, alcoxycarbonyle en C₁-C₅, dialkylamino en C₁-C₁₂, phényle, naphtyle, phénoxy, naphtoxy, biphényle, biphényloxy, phénthio ou naphthio; les cycles aromatiques mentionnés ci-dessus pouvant être non substitués ou substitués par un atome d'halogène, un groupe cyano, nitro, -OR₅, -NR₁₀R₁₁, alkyle en C₁-C₄, formyle, alkylcarbonyle en C₁-C₄, COOR₇, alkylthio en C₁-C₄, alkylsulfonyle en C₁-C₄, ou CONR₈R₉, ou
R₂ et R₃ représentent indépendamment l'un de l'autre un groupe formyle, alkylcarbonyle en C₁-C₁₅, alcénylcarbonyle en C₂-C₁₅, cycloalkylcarbonyle en C₄-C₉, cycloalcénylcarbonyle en C₆-C₉, (cycloalkyle en C₃-C₈)-(alkylcarbonyle en C₁-C₆₎), ces substituants pouvant être substitués par un atome d'halogène, un groupe cyano, nitro, hydroxyle, amino, alkylamino en C₁-C₆, dialkylamino en C₂-C₆, -COOH, -COOM, dans lequel M représente un groupe ammonium ou un atome de métal alcalin ou un atome de métal alcalino-terreux, ou alcoxycarbonyle en C₁-C₈, cycloalcoxycarbonyle en C₄-C₁₀, alkylaminocarbonyle en C₁-C₈, ou dialkylaminocarbonyle en C₂-C₁₂, ou
R₂ et R₃ représentent indépendamment l'un de l'autre un groupe hétérocyclyle ou un hétérocyclylcarbonyle saturé ou insaturé ayant 3 à 8 atomes dans le cycle, qui, en plus des atomes de carbone, comprennent au moins un hétéroatome choisi parmi un atome d'azote, d'oxygène et de soufre, l'hétérocyclyle défini ci-dessus qui est substitué par un atome d'halogène, un groupe cyano, nitro, alkyle en C₁-C₅, alcoxy en C₁-C₅, alkylcarbonyle en C₁-C₅, alkylcarbonyloxy en C₁-C₅, alcoxycarbonyle en C₁-C₅, aminocarbonyle, alkylaminocarbonyle en C₁-C₆, ou dialkylaminocarbonyle en C₂-C₁₂, ou hétérocyclylcarbonyle tel que défini ci-dessus qui est substitué par un atome d'halogène, un groupe cyano, nitro, alkyle en C₁-C₅, alcoxy en C₁-C₅, alkylcarbonyle en C₁-C₅, alcoxycarbonyle en C₁-C₆, aminocarbonyle, alkylamino en C₁-C₆, ou alkylcarbonyloxy en C₁-C₅, ou
R₂ et R₃ représentent indépendamment l'un de l'autre un groupe phénylcarbonyle, biphénylcarbonyle, naphtylcarbonyle, phényl-(alkylcarbonyle en C₁-C₆), biphényl-(alkylcarbonyle en C₁-C₆), naphtyl(alkylcarbonyle en C₁-C₆), phényl-(alcénylcarbonyle en C₂-C₆), biphényl-(alcénylcarbonyle en C₂-C₆), naphtyl-(alcénylcarbonyle en C₂-C₆), phényl-(alkynylcarbonyle en C₃-C₆), biphényl-(alkynylcarbonyle en C₃-C₆) ou naphtyl-(alkynylcarbonyle en C₃-C₆), ces substituants pouvant être non substitués ou substitués par un groupe alkyle en C₁-C₅, alcoxy en C₁-C₅, alkylthio en C₁-C₅, halogénoalkyle en C₁-C₅, alkylcarbonyle en C₁-C₅, un atome d'halogène, un groupe cyano, amino, nitro, -COOR₇, alcoxycarbonyle en C₁-C₈, hydroxyle, alkylsulfinyle en C₁-C₅, alkylsulfonyle en C₁-C₅, alkylaminocarbonyle en C₁-C₆, ou dialkylaminocarbonyle en C₂-C₁₂, ou
R₂ et R₃ représentent indépendamment l'un de l'autre un groupe phényle, naphtyle, groupe hétérocyclyle saturé ou insaturé ayant 3 à 8 atomes dans le cycle, qui, en plus des atomes de carbone, comprennent au moins un hétéroatome choisi parmi un atome d'azote, d'oxygène et de soufre, ces substituants pouvant être substitués par un atome d'halogène, un groupe cyano, nitro, alkyle en C₁-C₅, alcoxy en C₁-C₅, alkylthio en C₁-C₅, -COOH, -CONH₂, alkylaminocarbonyle en C₁-C₆, dialkylaminocarbonyle en C₂-C₁₀, alkylcarbonyle en C₁-C₅ ou alcoxycarbonyle en C₁-C₅ ou
R₂ et R₃ pris ensemble avec l'atome d'azote auquel ils sont liés forment un hétérocycle saturé ou insaturé comportant 3 à 8 atomes dans le cycle, qui, en plus des atomes de carbone, comprend au moins un hétéroatome choisi parmi un atome d'azote, d'oxygène et de soufre, cet hetérocycle étant non substitué ou substitué par un groupe alkyle en C₁-C₅, alcoxy en C₁-C₅, un atome d'halogène, un groupe cyano ou nitro, ou
R₂ et R₃ représentent indépendamment l'un de l'autre un groupe amino, alkylamino en C₁-C₆, dialkylamino en C₂-C₈, phénylamino, naphtylamino, alkylcarbonylamino en C₁-C₆, alcoxycarbonylamino en C₁-C₁₀, hydroxyle, alcoxy en C₁-C₆, alkylcarbonyloxy en C₁-C₆, phénoxy, biphénoxy ou naphtoxy, X représente O ou S(O)ₓ, dans lequel x vaut 0, 1 ou 2, et
R₄ représente un groupe alkyle en C₁-C₈, alcényle en C₂-C₈, alkynyle en C₃-C₈, cycloalkyle en C₃-C₈, cycloalcényle en C₅-C₈, (cycloalkyle en C₃-C₈) (alkyle en C₁-C₄), ou (alkyle en C₁-C₄) (cycloalkyle en C₃-C₈), ces substituants pouvant être non substitués ou substitués par un atome d'halogène, un groupe cyano, nitro, -O ou -OR₅, ou
R₄ représente un groupe phényle, biphényle, naphtyle, hétérocyclyle, (alkyle en C₁-C₄)phényle, (alkyle en C₁-C₄)naphtyle, phényl(alkyle en C₁-C₄), ou naphtyl(alkyle en C₁-C₄), ces substituants pouvant être non substitués ou substitués par un atome d'halogène, un groupe cyano, nitro, amino, -COOH, hydroxyle, alkyle en C₁-C₁₀, alkyloxy en C₁-C₁₀, alkylamino en C₁-C₆, dialkylamino en C₂-C₈, alkylthio en C₁-C₁₀, halogénoalkyle en C₁-C₁₀, halogénoalcoxy en C₁-C₁₀, halogénoalkylthio en C₁-C₁₀, alcoxycarbonylalcoxy en C₂-C₁₀, alkylsulfinyle en C₁-C₆, alkylsulfonyle en C₁-C₆, alkyloxycarbonyle en C₁-C₆, alkylcarbonyle en C₁-C₆, -CONH₂, formyle, alkylaminocarbonyle en C₁-C₇, dialkylaminocarbonyle en C₂-C₁₁, trialkylsilyle en C₃-C₆, alkylcarbonylamino en C₁-C₁₀, alkylcarbonyloxy en C₁-C₁₀, phénoxy, halogénophénoxy, pyridyloxy, ou pyridyloxy qui est substitué par un atome d'halogène, un groupe alkyle en C₁-C₄, alcoxy en C₁-C₄, cyano, nitro ou amino, ou deux substituants adjacents sur le cycle phényle ou naphtyle R₄ forment un noyau carbocyclique ou hétérocyclique saturé ou insaturé comportant 3 à 8 atomes de cycle, qui, en plus des atomes de carbone, a au moins un hétéroatome choisi parmi un atome d'azote, d'oxygène et de soufre, ce carbocycle ou cet hétérocycle étant substitué ou non substitué par un atome d'halogène, un groupe cyano, nitro, amino, -COOH, =O, alkyle en C₁-C₁₀, alcoxy en C₁-C₁₀, alkylthio en C₁-C₁₀, halogénoalkyle en C₁-C₁₀, hydroxyle, alcoxycarbonyloxy en C₃-C₁₀, alkylsulfinyle en C₁-C₆, alkylsulfonyle en C₁-C₆, alkyloxycarbonyle en C₂-C₆, alkylcarbonyle en C₂-C₆, -CONH₂, formyle, alkylaminocarbonyle en C₂-C₇, dialkylaminocarbonyle en C₃-C₁₁, trialkylsilyle en C₃-C₆, alkylcarbonylamino en C₂-C₁₀, alkylcarbonyloxy en C₂-C₁₀, phénoxy, pyridyloxy, ou pyridyloxy qui est substitué par un atome d'halogène, un groupe alkyle en C₁-C₄, alcoxy en C₁-C₄, cyano, nitro ou amino, et les sels des composés de formules I,
les composés de formule Ia, Ia1, Ia2 et Ia4 étant exclus : 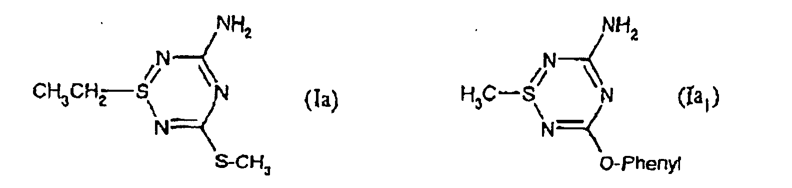 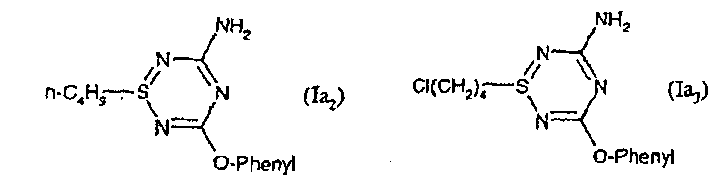 and

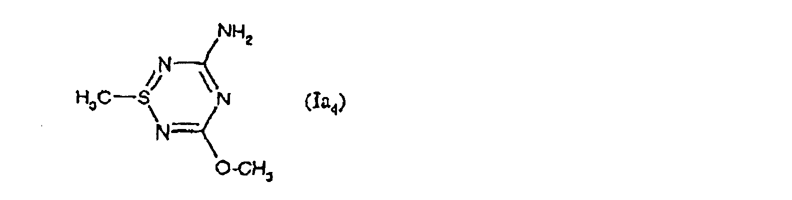

2. Composé selon la revendication 1, dans lequel
R₁ représente un groupe alkyle en C₁-C₁₀, cycloalkyle en C₃-C₈, (cycloalkyle en C₃-C₈) (alkyle en C₁-C₆), (alkyle en C₁-C₈) (cycloalkyle en C₃-C₈), alcényle en C₂-C₁₀, (cycloalkyle en C₃-C₈) (alcényle en C₂-C₆), (alcényle en C₂-C₈)(cycloalkyle en C₃-C₈), alkynyle en C₂-C₁₀, (cycloalkyle en C₃-C₈) (alkynyle en C₂-C₆), (alkynyle en C₂-C₈)(cycloalkyle en C₃-C₈), cycloalcényle en C₅-C₈, (cycloalcényle en C₅-C₈) (alkyle en C₁-C₆), (alkyle en C₁-C₈) (cycloalcényle en C₅-C₈), (cycloalcényle en C₅-C₈) (alcényle en C₂-C₆), (alcényle en C₂-C₈)(cycloalcényle C₅-C₈), (cycloalcényle en C₅-C₈) (alkynyle en C₂-C₆), (alkynyle en C₂-C₈) (cycloalcényle en C₅-C₈), (cycloalkyle en C₃-C₈) (cycloalkyle en C₃-C₈), (cycloalcényle en C₅-C₈) (cycloalcényle en C₅-C₈), (cycloalkyle en C₃-C₈) (cycloalcényle en C₅-C₈), (cycloalcényle en C₅-C₈) (cycloalkyle en C₃-C₈), (alkynyle en C₂-C₈) (alcényle en C₂-C₆) ou (alcényle en C₂-C₈) (alkynyle en C₂-C₆), ces substituants pouvant être non substitués ou substitués par un atome d'halogène, un groupe cyano, nitro, =O, -OR₅, -S(O)ₙR₆, -COOR₇, -CONR₈R₉, -NR₁₀R₁₁, =NR₁₂, phényle, biphényle, naphtyle ou hététérocyclyle saturé ou insaturé comportant 3 à 8 atomes dans le cycle, qui, en plus des atomes de carbone, comprend au moins un hétéroatome choisi parmi un atome d'azote, d'oxygène et de soufre, ces substituants phényle, naphtyle et hétérocyclyle pouvant être non substitués ou substitués par un atome d'halogène un groupe cyano, nitro, -O, -OR₅, -NR₁₀R₁₁, ou -CONR₈R₉, ou
R¹ représente un groupe hétérocyclyle saturé ou insaturé comportant 3 à 8 atomes dans le cycle, qui, en plus des atomes de carbone, comprend au moins un hétéroatome choisi parmi un atome d'azote, d'oxygène et de soufre, l'hétérocycle étant lié au cycle thiatriazine par l'intermédiaire d'un de ses atomes de carbone, et l'hétérocycle pouvant être substitué par un atome d'halogène, un groupe cyano, nitro, =O, -OR₅, -S(O)ₙR₆,-COOR₇, -CONR₈R₉, -NR₁₀R₁₁, =NR₁₂, =N-NR₁₃R₁₄, alkyle en C₁-C₈, alcényle en C₂-C₈, alkynyle en C₂-C₈, cycloalkyle en C₃-C₈, cycloalcényle en C₅-C₈, phényle, biphényle, naphtyle, alkyle en C₁-C₈ qui est substitué par un atome d'halogène, un groupe cyano, nitro, -OR₅, -CONR₈R₉ ou -NR₁₀R₁₁, alcényle en C₂-C₈, qui est substitué par un atome d'halogène, un groupe cyano, nitro, -OR₅, -CONR₈R₉ ou -NR₁₀R₁₁, alkynyle en C₂-C₈, qui est substitué par un atome d'halogène, un groupe cyano, nitro, -OR₅, -CONR₈R₉ ou -NR₁₀R₁₁, cycloalkyle en C₃-C₈, qui est substitué par un atome d'halogène, un groupe cyano, nitro, -OR₅, -CONR₈R₉ ou -NR₁₀R₁₁, cycloalcényle en C₅-C₈, qui est substitué par un atome d'halogène, un groupe cyano, nitro, -OR₅, -CONR₈R₉ ou -NR₁₀R₁₁, phényle, qui est substitué par un atome d'halogène, un groupe cyano, nitro, alkyle en C₁-C₆, halogénoalkyle en C₁-C₆, -OR₅, -CONR₈R₉ ou-NR₁₀R₁₁, biphényle, qui est substitué par un atome d'halogène, un groupe cyano, nitro, -OR₅, -CONR₈R₉ ou-NR₁₀R₁₁, ou naphtyle, qui est substitué par un atome d'halogène, un groupe cyano, nitro, -OR₅, -CONR₈R₉ ou-NR₁₀R₁₁, ou
R₁ représente un groupe phényle, biphényle, naphtyle, (alkyle en C₁-C₄)-phényle, (alkyle en C₁-C₄)-biphényle, (alkyle en C₁-C₄)-naphtyle, (alcényle en C₂-C₄)-phényle, (alcényle en C₂-C₄)-biphényle, (alkyle en C₁-C₄)-naphtyle, (alkynyle en C₂-C₁₀)phényle, (alkynyle en C₂-C₄)biphényle ou (alkynyle en C₁-C₄)-naphtyle, ces substituants pouvant être non substitués ou substitués par un atome d'halogène, un groupe cyano, nitro, -OR₅, -S(O)ₙR₆, -COOR₇, -CONR₈R₉ ou-NR₁₀R₁₁, dans lesquels
R₅ représente un atome d'hydrogène, un groupe alkyle en C₁-C₆, halogénoalkyle en C₁-C₆, alcoxyalkyle en C₁-C₆, cyanoalkyle en C₁-C₆, phényle, halogénophényle, alcoxyphényle en C₁-C₄, phényl-(alkyle en C₁-C₄), (alkyle en C₁-C₄)-carbonyle, benzoyle, halogénobenzoyle, alkylamino en C₁-C₄, dialkylamino en C₁-C₆, trialkylsilyle en C₃-C₆, cycloalkyle en C₃-C₆, alcényle en C₂-C₄ ou alkynyle en C₃-C₄,
n vaut 0, 1 ou 2,
R₆ représente un atome d'hydrogène ou un groupe cyano si n vaut 0, ou
R₆ représente un groupe alkyle en C₁-C₄, halogénoalkyle en C₁-C₄, phényle, (alkyle en C₁-C₄)phényle, halogénophényle, alcoxyalkyl en C₁-C₄, hétérocyclyle saturé ou insaturé ou halogénohétérocyclyle comportant 3 à 8 atomes dans le cycle, qui, en plus des atomes de carbone comportent au moins un hétéroatome choisi parmi un atome d'azote, d'oxygène ou de soufre, ou cycloalkyle en C₃-C₆,
R₇ représente un atome d'hydrogène, groupe alkyle en C₁-C₄, halogénoalkyle en C₁-C₄, cyanoalkyle en C₁-C₄, phényle, halogénophényle, alcoxyalkyle en C₂-C₄, hétérocyclyle ou halogénohétérocyclyle saturé ou insaturé comportant 3 à 8 atomes dans le cycle, qui, en plus des atomes de carbone comportent au moins un hétéroatome choisi parmi un atome d'azote, d'oxygène et de soufre,
R₈ et R₉ représentent indépendamment l'un de l'autre un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄ ou alcoxyalkyle en C₂-C₄, ou
R₈ et R₉ pris ensemble avec l'atome d'azote auquel ils sont liés forment un hétérocycle saturé ou insaturé comportant 3 à 8 atomes de cycle qui peuvent être halogénés, et qui., en plus des atomes de carbone, comprennent au moins un hétéroatome choisi parmi un atome d'azote, d'oxygène ou de soufre,
R₁₀ et R₁₁, indépendamment l'un de l'autre, représentent un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄, alcoxyalkyle en C₂-C₄, formyle, alkylcarbonyle en C₂-C₄ ou phénylcarbonyle, les parties phényle pouvant être substituées par un groupe alkyle en C₁-C₄, un atome d'halogène, un groupe alcoxy en C₁-C₄, hydroxyle, cyano, nitro ou alcoxycarbonyle en C₁-C₆, ou
R₁₀ et R₁₁ pris ensemble avec l'atome d'azote auquel ils sont liés forment un hétérocycle saturé ou insaturé comprenant 3 à 8 atomes de cycle qui peuvent être halogénés, et qui, en plus des atomes de carbone, comprennent au moins un hétéroatome choisi parmi un atome d'azote, d'oxygène ou de soufre,
R₁₂ représente un atome d'hydrogène, un groupe alkyle en C₁-C₄, halogénoalkyle en C₁-C₄, Phényle, halogénophényle, cyanophényle, nitrophényle, (alkyle en C₁-C₄)-phényle, (alcoxy en C₁-C₄)-phényle ou phényl-(alkyle en C₁-C₄).

3. Composé selon la revendication 2, dans lequel
R₁ représente un groupe alkyle en C₁-C₁₀, cycloalkyle en C₃-C₈, (cycloalkyle en C₃-C₆) (alkyle en C₁-C₆), (alkyle en C₁-C₈) (cycloalkyle en C₃-C₈), alcényle en C₂-C₁₀, (cycloalkyle en C₃-C₈) (alcényle en C₂-C₆), (alcényle en C₂-C₈) (cycloalkyle en C₃-C₈), alkynyle en C₂-C₁₀, (cycloalkyle en C₃-C₈)(alkynyle en C₂-C₆), (alkynyle en C₂-C₈) (cycloalkyle en C₃-C₈), cycloalcényle en C₅-C₈, (cycloalcényle en C₅-C₈) (alkyle en C₁-C₆), (alkyle en C₁-C₈) (cycloalcényle en C₅-C₈), (cycloalcényle en C₅-C₈) (alcényle en C₂-C₆), (alcényle en C₂-C₈) (cycloalcényle C₅-C₈), (cycloalcényle en C₅-C₈) (alkynyle en C₂-C₆), (alkynyle en C₂-C₈) (cycloalcényle en C₅-C₈), (cycloalkyle en C₃-C₈) (cycloalkyle en C₃-C₈), (cycloalcényle en C₅-C₈) (cycloalcényle en C₅-C₈), (cycloalkyle en C₃-C₈) (cycloalcényle en C₅-C₈), (cycloalcényle en C₅-C₈) (cycloalkyle en C₃-C₈), (alkynyle en C₂-C₈) (alcényle en C₂-C₆) ou (alcényle en C₂-C₈) (alkynyle en C₂-C₆), ces substituants pouvant être non substitués ou substitués par un atome d'halogène, un groupe cyano, =O,-OR₅, -COOR₇, -CONR₈R₉, -NR₁₀R₁₁, ou phényle, ce cycle phényle pouvant être non substitué ou substitué par un atome d'halogène, ou
R¹ représente un groupe hétérocyclyle saturé ou insaturé comportant 3 à 8 atomes dans le cycle, qui, en plus des atomes de carbone, comprend au moins un hétéroatome choisi parmi un atome d'azote, d'oxygène et de soufre, l'hétérocycle étant lié au cycle thiatriazine par l'intermédiaire d'un de ses atomes de carbone, et l'hétérocycle pouvant être substitué par un atome d'halogène, un groupe cyano, , =O, -OR₅, -S(O)ₙR₆, -COOR₇,-CONR₈R₉, -NR₁₀R₁₁, alkyle en C₁-C₈, alcényle en C₂-C₈, alkynyle en C₂-C₈, cycloalkyle en C₃-C₈, cycloalcényle en C₅-C₈, phényle, alkyle en C₁-C₈ qui est substitué par un atome d'halogène, un groupe cyano, -OR₅, -CONR₈R₉ ou-NR₁₀R₁₁, alcényle en C₂-C₈, qui est substitué par ⁻un atome d'halogène, un groupe cyano, -OR₅, -CONR₈R₉ ou -NR₁₀R₁₁, alkynyle en C₂-C₈, qui est substitué par un atome d'halogène, un groupe cyano, -OR₅, -CONR₈R₉ ou -NR₁₀R₁₁, cycloalkyle en C₃-C₈, qui est substitué par un atome d'halogène, un groupe cyano, -OR₅, -CONR₈R₉ ou -NR₁₀R₁₁, cycloalcényle en C₅-C₈, qui est substitué par un atome d'halogène, un groupe cyano, -OR₅, -CONR₈R₉ ou -NR₁₀R₁₁, phényle, qui est substitué par un atome d'halogène, un groupe cyano, nitro, alkyle en C₁-C₄, halogénoalkyle en C₁-C₆, -OR₅, -CONR₈R₉ ou -NR₁₀R₁₁, ou
R₁ représente un groupe phényle, biphényle, naphtyle, (alkyle en C₁-C₄)-phényle, (alkyle en C₁-C₄)-biphényle, (alkyle en C₁-C₄)-naphtyle, (alcényle en C₂-C₄)-phényle, (alcényle en C₂-C₄)-biphényle, (alcényle en C₂-C₄)-naphtyle, (alkynyl en C₂-C₄)-phényle, (alkynyl en C₂-C₄)-biphényle ou (alkynyle en C₂-C₄)-naphtyle, ces substituants pouvant être non substitués ou substitués par un atome d'halogène, un groupe cyano, -OR₅, -S(O)ₙR₆, -COOR₇, -CONR₈R₉ ou -NR₁₀R₁₁, dans lesquels
R₅ représente un atome d'hydrogène, un groupe alkyle en C₁-C₆, halogénoalkyle en C₁-C₆, alcoxyalkyle en C₁-C₆, cyanoalkyle en C₁-C₆, phényl-(alkyle en C₁-C₄), (alkyle en C₁-C₄)-carbonyle, benzoyle, halogénobenzoyle, trialkylsilyle en C₃-C₆, cycloalkyle en C₃-C₆, alcényle en C₂-C₄ ou alkynyle en C₃-C₄,
n vaut 0, 1 ou 2,
R₆ représente un atome d'hydrogène ou un groupe cyano si n vaut 0 ou bien
R₆ représente un groupe alkyle en C₁-C₄, halogénoalkyle en C₁-C₄, phényle, (alkyle en C₁-C₄)-phényle,
R₇ représente un atome d'hydrogène, groupe alkyle en C₁-C₄, halogénoalkyle en C₁-C₄, cyanoalkyle en C₁-C₄, phényle, halogénophényle, alcoxyalkyle en C₂-C₄, hétérocyclyle ou halogénohétérocyclyle saturé ou insaturé comportant 3 à 8 atomes dans le cycle, qui, en plus des atomes de carbone comportent au moins un hétéroatome choisi parmi un atome d'azote, d'oxygène et de soufre,
R₈ et R₉ représentent indépendamment l'un de l'autre un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄ ou alcoxyalkyle en C₂-C₄, ou
R₈ et R₉ pris ensemble avec l'atome d'azote auquel ils sont liés forment un hétérocycle saturé ou insaturé comportant 3 à 8 atomes de cycle qui peuvent être halogénés, et qui, en plus des atomes de carbone, comprennent au moins un hétéroatome choisi parmi un atome d'azote, d'oxygène ou de soufre,
R₁₀ et R₁₁, indépendamment l'un de l'autre, représentent un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄, alcoxyalkyle en C₂-C₄, formyle, alkylcarbonyle en C₁-C₄ ou phénylcarbonyle, la partie phényle pouvant être substituée par un groupe alkyle en C₁-C₄, un atome d'halogène, un groupe alcoxy en C₁-C₄, hydroxyle, cyano, nitro ou alcoxycarbonyle en C₁-C₆, ou
R₁₀ et R₁₁ pris ensemble avec l'atome d'azote auquel ils sont liés forment un hétérocycle saturé ou insaturé comprenant 3 à 8 atomes de cycle qui peuvent être halogénés, et qui, en plus des atomes de carbone, comprennent au moins un hétéroatome choisi parmi un atome d'azote, d'oxygène ou de soufre,

4. composé selon la revendication 3, dans lequel
R₁ représente un groupe alkyle en C₁-C₁₀, cycloalkyle en C₃-C₈, (cycloalkyle en C₃-C₈) (alkyle en C₁-C₆), (alkyle en C₁-C₈) (cycloalkyle en C₃-C₈), alcényle en C₂-C₁₀, (cycloalkyle en C₃-C₈) (alcényle en C₂-C₆), (alcényle en C₂-C₈) (cycloalkyle en C₃-C₈), alkynyle en C₂-C₁₀, (cycloalkyle en C₃-C₈) (alkynyle en C₂-C₆), (alkynyle en C₂-C₈) (cycloalkyle en C₃-C₈), cycloalcényle en C₅-C₈, (cycloalcényle en C₅-C₈) (alkyle en C₁-C₆), (alkyle en C₁-C₈) (cycloalcényle en C₅-C₈), (cycloalcényle en C₅-C₈) (alcényle en C₂-C₆), (alcényle en C₂-C₈) (cycloalcényle C₅-C₈), (cycloalcényle en C₅-C₈) (alkynyle en C₂-C₆), (alkynyle en C₂-C₈) (cycloalcényle en C₅-C₈), (cycloalkyle en C₃-C₈) (cycloalkyle en C₃-C₈), (cycloalcényle en C₅-C₈) (cycloalcényle en C₅-C₈), (cycloalkyle en C₃-C₈) (cycloalcényle en C₅-C₈), (cycloalcényle en C₅-C₈) (cycloalkyle en C₃-C₈), (alkynyle en C₂-C₈) (alcényle en C₂-C₆) ou (alcényle en C₂-C₈) (alkynyle en C₂-C₆), ces substituants pouvant être non substitués ou substitués par un atome d'halogène, un groupe cyano, =O, -OR₅, -COOR₇, -CONR₈R₉, -NR₁₀R₁₁, ou phényle, ce cycle phényle, pouvant être non substitué ou substitué par un atome d'halogène, ou
R¹ représente un groupe hétérocyclyle saturé ou insaturé comportant 3 à 8 atomes dans le cycle, qui, en plus des atomes de carbone, comprend au moins un héteoatome choisi parmi un atome d'azote, d'oxygène et de soufre, l'hétérocycle étant lié au cycle thiatriazine par l'intermédiaire d'un de ses atomes de carbone, et l'hétérocycle pouvant être substitué par un atome d'halogène, un groupe cyano, =O, alcoxy en C₁-C₄, alkyle en C₁-C₈, alcényle en C₁-C₈, alkynyle en C₁-C₈, phényle, alkyle en C₁-C₈ qui est substitué par un atome d'halogène, alcényle en C₂-C₈, qui est substitué par un atome d'halogène, alkynyle en C₂-C₈, qui est substitué par un atome d'halogène, phényle qui est substitué un atome d'halogène, un groupe cyano, nitro, alkyle en C₁-C₄, alcoxy en C₁-C₄, ou
R₁ représente un groupe phényle, biphényle, naphtyle, (alkyle en C₁-C₄)-phényle, (alkyle en C₁-C₄)-biphényle, (alkyle en C₁-C₄)-naphtyle, (alcényle en C₁-C₄)-phényle ou (alkynyle en C₂-C₄)-phényle, ces substituants pouvant être non substitués ou substitués par un atome d'halogène, un groupe cyano, -OR₅, -S(O)ₙR₆, -COOR₇, -CONR₈R₉ ou -NR₁₀R₁₁, dans lesquels
R₅ représente un atome d'hydrogène, un groupe alkyle en C₁-C₆, halogénoalkyle en C₁-C₆, alcoxyalkyle en C₁-C₆, cyanoalkyle en C₁-C₆, Phényl-(alkyle en C₁-C₄), (alkyle en C₁-C₄)-carbonyle, benzoyle, halogénobenzoyle, trialkylsilyle en C₃-C₆, cycloalkyle en C₃-C₆, alcényle en C₂-C₄ ou alkynyle en C₃-C₄,
n vaut 0, 1 ou 2,
R₆ représente un atome d'hydrogène ou un groupe cyano si n vaut 0 ou bien
R₆ représente un groupe alkyle en C₁-C₄, halogénoalkyle en C₁-C₄, phényle, (alkyle en C₁-C₄)-phényle,
R₇ représente un atome d'hydrogène, groupe alkyle en C₁-C₄, halogénoalkyle en C₁-C₄, cyanoalkyle en C₁-C₄, phényle, halogénophényle, alcoxyalkyle en C₂-C₄, hétérocyclyle ou halogénohétérocyclyle saturé ou insaturé comportant 3 à 8 atomes dans le cycle, qui, en plus des atomes de carbone comportent au moins un hétéroatome choisi parmi un atome d'azote, d'oxygène et de soufre,
R₈ et R₉ représentent indépendamment l'un de l'autre un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄ ou alcoxyalkyle en C₂-C₄, ou
R₈ et R₉ pris ensemble avec l'atome d'azote auquel ils sont liés forment un hétérocycle saturé ou insaturé comportant 3 à 8 atomes de cycle qui peuvent être halogénés, et qui, en plus des atomes de carbone, comprennent au moins un hétéroatome choisi parmi un atome d'azote, d'oxygène ou de soufre,
R₁₀ et R₁₁, indépendamment l'un de l'autre, représentent un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄, alcoxyalkyle en C₂-C₄, formyle, alkylcarbonyle en C₂-C₄ ou phénylcarbonyle, la partie phényle pouvant être substituée par un groupe alkyle en C₁-C₄, un atome d'halogène, un groupe alcoxy en C₁-C₄, hydroxyle, cyano, nitro ou alcoxycarbonyle en C₁-C₆, ou
R₁₀ et R₁₁ pris ensemble avec l'atome d'azote auquel ils sont liés forment un hétérocycle saturé ou insaturé comprenant 3 à 8 atomes de cycle qui peuvent être halogénés, et qui, en plus des atomes de carbone, comprennent au moins un hétéroatome choisi parmi un atome d'azote, d'oxygène ou de soufre.

5. Composé selon la revendication 1, dans lequel
R₂ et R₃ représentent indépendamment l'un de l'autre un atome d'hydrogène, un groupe alkyle en C₁-C₆, alkyle en C₁-C₆ qui est substitué par un atome d'halogène, un groupe cyano, nitro, alcoxy en C₁-C₈, trialkylsilyle en C₃-C₆, hydroxyle, amino, ammonium, tri-(alkyle en C₁-C₄)ammonium, -COOH, -COOM, où M représente un groupe ammonium ou un atome de métal alcalin ou un atome de métal alcalino-terreux, un groupe cycloalkyle en C₃-C₄, (alkyle en C₁-C₅) carbonyloxy, phénylcarbonyloxy, naphtylcarbonyloxy, alkylamino en C₁-C₆, alcoxycarbonyle en C₁-C₅, dialkylamino en C₂-C₆ ou phényle, le cycle phényle pouvant être non substitué ou substitué par un atome d'halogène, un groupe cyano, nitro, -OR₅, -NR₁₀R₁₁, alkyle en C₁-C₄, formyle, alkylcarbonyle en C₁-C₄, COOR₇, alkylthio en C₁-C₄, alkylsulfonyle en C₁-C₄, ou CONR₈R₉, ou
R₂ et R₃ représentent indépendamment l'un de l'autre un groupe alcényle en C₂-C₆, alcényle en C₂-C₆, qui est substitué par un atome d'halogène, un groupe cyano, nitro, alcoxy en C₁-C₄, trialkylsilyle en C₃-C₆, hydroxyle, amino, ammonium, tri-(alkyle en C₁-C₄)ammonium, -COOH, -COOM, où M représente un groupe ammonium ou un atome de métal alcalin ou un atome de métal alcalino-terreux, ou alkylamino en C₁-C₆, alcoxycarbonyle en C₁-C₅, dialkylamino en C₁-C₁₂ ou phényle, le cycle phényle pouvant être non substitué ou substitué par un atome d'halogène, un groupe cyano, nitro, -OR₅, -NR₁₀R₁₁, alkyle en C₁-C₄, formyle, alkylcarbonyle en C₁-C₄, COOR₇, alkylthio en C₁-C₄, alkylsulfonyle en C₁-C₄, ou CONR₈R₉, ou
R₂ et R₃ représentent indépendamment l'un de l'autre un groupe alkynyle en C₃-C₆, alkynyle en C₃-C₆, qui est substitué par un atome d'halogène, un groupe cyano, nitro, alcoxy en C₁-C₈, trialkylsilyle en C₃-C₆, hydroxyle, amino, ammonium, tri-(alkyle en C₁-C₄)ammonium, -COOH, -COOM, où M représente un groupe ammonium ou un atome de métal alcalin ou un atome de métal alcalino-terreux, ou alkylamino en C₁-C₆, alcoxycarbonyle en C₁-C₅, dialkylamino en C₂-C₆ ou phényle, le cycle phényle pouvant être non substitué ou substitué par un atome d'halogène, un groupe cyano, nitro, -OR₅, -NR₁₀R₁₁, alkyle en C₁-C₄, formyle, alkylcarbonyle en C₁-C₄, COOR₇, alkylthio en C₁-C₄, alkylsulfonyle en C₁-C₄, ou CONR₈R₉, ou
R₂ et R₃ représentent indépendanunent l'un de l'autre un groupe formyle, alkylcarbonyle en C₁-C₈, alcénylcarbonyle en C₂-C₈, cycloalkylcarbonyle en C₄-C₉, cycloalcénylcarbonyle en C₆-C₉, (cycloalkyle en C₃-C₈) (alkylcarbonyle en C₁-C₆₎), ces substituants pouvant être non substitués ou substitués par un atome d'halogène, un groupe cyano, nitro, hydroxyle, amino, alkylamino en C₁-C₆, dialkylamino en C₂-C₆, -COOH, -COOM, dans lequel M représente un groupe ammonium ou un atome de métal alcalin ou un atome de métal alcalino-terreux, ou alcoxycarbonyle en C₂-C₈, cycloalcoxycarbonyle en C₄-C₁₀, alkylaminocarbonyle en C₁-C₈, ou dialkylaminocarbonyle en C₂-C₁₂, ou
R₂ et R₃ représentent indépendamment l'un de l'autre un groupe hétérocyclyle ou un hétérocyclylcarbonyle saturé ou insaturé ayant 3 à 8 atomes dans le cycle, qui, en plus des atomes de carbone, comprennent au moins un hétéroatome choisi parmi un atome d'azote, d'oxygène et de soufre, l'hétérocyclyle défini ci-dessus qui est substitué par un atome d'halogène, un groupe cyano, nitro, alkyle en C₁-C₅, alcoxy en C₁-C₅, alkylcarbonyle en C₁-C₅, alkylcarbonyloxy en C₁-C₅, alcoxycarbonyle en C₁-C₆, aminocarbonyle, alkylaminocarbonyle en C₁-C₆, ou dialkylaminocarbonyle en C₂-C₈, ou hétérocyclylcarbonyle qui est substitué par un atome d'halogène, un groupe cyano, nitro, alkyle en C₁-C₄, alcoxy en C₁-C₄, alkylcarbonyle en C₁-C₄, alcoxycarbonyle en C₁-C₄, aminocarbonyle, alkylamino en C₁-C₆, ou alkylcarbonyloxy en C₁-C₅, ou
R₂ et R₃ représentent indépendamment l'un de l'autre un groupe phénylcarbonyle, biphénylcarbonyle, naphtylcarbonyle, phényl-(alkylcarbonyle en C₁-C₆), biphényl-(alkylcarbonyle en C₁-C₆), naphtyl(alkylcarbonyle en C₁-C₆), phényl-(alcénylcarbonyle en C₂-C₆), biphényl-(alcénylcarbonyle en C₂-C₆), naphtyl-(alcénylcarbonyle en C₂-C₆), ces substituants pouvant être non substitués ou substitués par un groupe alkyle en C₁-C₄, alcoxy en C₁-C₄, alkylthio en C₁-C₄, halogénoalkyle en C₁-C₄, alkylcarbonyle en C₁-C₄, un atome d'halogène, un groupe cyano, amino, nitro, -COOR₇, alcoxycarbonyle en C₁-C₅, hydroxyle, alkylsulfonyle en C₁-C₄, alkylaminocarbonyle en C₁-C₅, ou dialkylaminocarbonyle en C₂-C₆, ou
R₂ et R₃ représentent indépendamment l'un de l'autre un groupe phényle, naphtyle, groupe hétérocyclyle saturé ou insaturé ayant 3 à 8 atomes dans le cycle, qui, en plus des atomes de carbone, comprennent au moins un hétéroatome choisi parmi un atome d'azote, d'oxygène et de soufre, ces substituants pouvant être non substitués ou substitués par un atome d'halogène, un groupe cyano, nitro, alkyle en C₁-C₄, alcoxy en C₁-C₄, alkylthio en C₁-C₄, -COOH, -CONH₂, alkylaminocarbonyle en C₁-C₅, dialkylaminocarbonyle en C₂-C₇, alkylcarbonyle en C₁-C₄ ou alcoxycarbonyle en C₁-C₅ ou
R₂ et R₃ pris ensemble avec l'atome d'azote auquel ils sont liés forment un hétérocycle saturé ou insaturé comportant 3 à 8 atomes dans le cycle, qui, en plus des atomes de carbone, comprend au moins un hétéroatome choisi parmi un atome d'azote, d'oxygène et de soufre, cet hétérocycle étant non substitué ou substitué par un groupe alkyle en C₁-C₅, alcoxy en C₁-C₄, un atome d'halogène, un groupe cyano ou nitro, ou
R₂ et R₃ représentent indépendamment l'un de l'autre un groupe amino, alkylamino en C₁-C₄, dialkylamino en C₂-C₆, phénylamino, alkylcarbonylamino en C₁-C₅, alcoxycarbonylamino en C₁-C₅, hydroxyle, alcoxy en C₁-C₄, alkylcarbonyloxy en C₁-C₅ ou phénoxy,
R₅ représente un atome d'hydrogène, un groupe alkyle en C₁-C₆, halogénoalkyle en C₁-C₆, alcoxyalkyle en C₂-C₆, cyanoalkyle en C₁-C₆, phényle, halogénophényle, (alcoxy en C₁-C₄)-phényle, phényl-(alkyle en C₁-C₄), (alkyle en C₁-C₄)-carbonyle, benzoyle, halogénobenzoyle, alkylamino C₁-C₄, dialkylamino C₂-C₆, trialkylsilyle en C₃-C₆, cycloalkyle en C₃-C₆, alcényle en C₂-C₄ ou alkynyle en C₃-C₄,
R₇ représente un atome d'hydrogène, groupe alkyle en C₁-C₄, halogénoalkyle en C₁-C₄, cyanoalkyle en C₁-C₄, phényle, halogénophényle, alcoxyalkyle en C₂-C₄, hétérocyclyle ou halogénohétérocyclyle saturé ou insaturé comportant 3 à 8 atomes dans le cycle, qui, en plus des atomes de carbone comportent au moins un hétéroatome choisi parmi un atome d'azote, d'oxygène et de soufre,
R₈ et R₉ représentent indépendamment l'un de l'autre un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄ ou alcoxyalkyle en C₂-C₄, ou
R₈ et R₉ pris ensemble avec l'atome d'azote auquel ils sont liés forment un hétérocycle saturé ou insaturé comportant 3 à 8 atomes de cycle qui peuvent être halogénés, et qui, en plus des atomes de carbone, comprennent au moins un hétéroatome choisi parmi un atome d'azote, d'oxygène ou de soufre,
R₁₀ et R₁₁, indépendamment l'un de l'autre, représentent un atome d'hydrogène, un groupe phényle, halogénophényle, alkyle en C₁-C₄, halogénoalkyle en C₁-C₄, cyanoalkyle en C₁-C₄, alcényle en C₃-C₄, alkynyle en C₃-C₄, alcoxyalkyle en C₂-C₄, formyle, alkylcarbonyle en C₂-C₄ ou phénylcarbonyle, la partie phényle pouvant être substituée par un groupe alkyle en C₁-C₄, un atome d'halogène, un groupe alcoxy en C₁-C₄, hydroxyle, cyano, nitro ou alcoxycarbonyle en C₁-C₄, ou
R₁₀ et R₁₁ pris ensemble avec l'atome d'azote auquel ils sont liés forment un hétérocycle saturé ou insaturé comprenant 3 à 8 atomes de cycle qui peuvent être halogénés, et qui, en plus des atomes de carbone, comprennent au moins un hétéroatome choisi parmi un atome d'azote, d'oxygène ou de soufre.

6. Composé selon la revendication 1, dans lequel
R₂ et R₃ représentent indépendamment l'un de l'autre un atome d'hydrogène, un groupe alkyle en C₁-C₆, alkyle en C₁-C₆ qui est substitué par un atome d'halogène, un groupe hydroxyle, amino, ammonium, tri-(alkyle en C₁-C₄)ammonium, -COOH, -COOM, où M représente un groupe ammonium ou un atome de métal alcalin ou un atome de métal alcalino-terreux, un groupe (alkyle en C₁-C₅)carbonyloxy, phénylcarbonyloxy, alkylamino en C₁-C₆, alcoxycarbonyle en C₁-C₅, dialkylamino en C₂-C₆ ou
R₂ et R₃ représentent indépendamment l'un de l'autre un groupe formyle, alkylcarbonyle en C₁-C₈, alcénylcarbonyle en C₂-C₈, cycloalkylcarbonyle en C₄-C₉, cycloalcénylcarbonyle en C₆-C₉, (cycloalkyle en C₃-C₈) (alkylcarbonyle en C₁-C₆₎), ces substituants pouvant être non substitués ou substitués par un atome d'halogène, un groupe cyano, nitro, hydroxyle, amino, -COOH, -COOM, dans lequel M représente un groupe ammonium ou un atome de métal alcalin ou un atome de métal alcalino-terreux, ou
R₂ et R₃ représentent indépendamment l'un de l'autre un groupe hétérocyclyle ou un hétérocyclylcarbonyle saturé ou insaturé ayant 3 à 8 atomes dans le cycle, qui, en plus des atomes de carbone, comprennent au moins un hétéroatome choisi parmi un atome d'azote, d'oxygène et de soufre, l'hétérocyclyle défini ci-dessus qui est substitué par un atome d'halogène, un groupe cyano, nitro, alkyle en C₁-C₅, alcoxy en C₁-C₅ ou alcoxycarbonyle en C₁-C₆, ou hétérocyclylcarbonyle tel que défini ci-dessus qui est substitué par un atome d'halogène, un groupe cyano, nitro, alkyle en C₁-C₄, alcoxy en C₁-C₄, alcoxycarbonyle en C₁-C₄, ou
R₂ et R₃ représentent indépendamment l'un de l'autre un groupe phénylcarbonyle, biphénylcarbonyle, naphtylcarbonyle, phényl-(alkylcarbonyle en C₁-C₆), biphényl-(alkylcarbonyle en C₁-C₆), naphtyl(alkylcarbonyle en C₁-C₆), phényl-(alcénylcarbonyle en C₂-C₆), biphényl-(alcénylcarbonyle en C₂-C₆) ou naphtyl-(alcénylcarbonyle en C₂-C₆), ces substituants pouvant étre non substitués ou substitués par un groupe alkyle en C₁-C₄, alcoxy en C₁-C₄, alkylthio en C₁-C₄, halogénoalkyle en C₁-C₄, alkylcarbonyle en C₁-C₄, un atome d'halogène, un groupe cyano, amino, nitro, -COOH, alcoxycarbonyle en C₁-C₅, hydroxyle, ou alkylsulfonyle en C₁-C₄, ou
R₂ et R₃ représentent indépendamment l'un de l'autre un groupe phényle, naphtyle, groupe hétérocyclyle saturé ou insaturé ayant 3 à 8 atomes dans le cycle, qui, en plus des atomes de carbone, comprend au moins un hétéroatome choisi parmi un atome d'azote, d'oxygène et de soufre, ces substituants pouvant être non substitués ou substitués par un atome d'halogène, un groupe cyano, alkyle en C₁-C₄, alcoxy en C₁-C₄, alkylthio en C₁-C₄, ou alcoxycarbonyle en C₁-C₅, ou
R₂ et R₃ pris ensemble avec l'atome d'azote auquel ils sont liés forment un hétérocycle saturé ou insaturé comportant 3 à 8 atomes dans le cycle, qui, en plus des atomes de carbone, comprend au moins un hétéroatome choisi parmi un atome d'azote, d'oxygène et de soufre, cet hétérocycle étant non substitué ou substitué par un groupe alkyle en C₁-C₄, alcoxy en C₁-C₄, un atome d'halogène ou un groupe cyano.

7. Composé selon la revendication 6, dans lequel
R₂ et R₃ représentent un atome d'hydrogène, ou
R₂ et R₃ représentent indépendamment l'un de l'autre un groupe formyle, alkylcarbonyle en C₁-C₈, alcénylcarbonyle en C₂-C₈, cycloalkylcarbonyle en C₄-C₉, cycloalcénylcarbonyle en C₆-C₉, (cycloalkyle en C₃-C₈) (alkylcarbonyle en C₁-C₆), ces substituants pouvant être non substitués ou substitués par un atome d'halogène, un groupe cyano, hydroxyle ou amino, ou
R₂ et R₃ représentent un groupe phénylcarbonyle, le cycle phényle pouvant être non substitué ou substitué par un groupe alkyle en C₁-C₄, alcoxy en C₁-C₄, alkylthio en C₁-C₄, halogénoalkyle en C₁-C₄, un atome d'halogène, un groupe cyano, nitro, -COOH, alcoxycarbonyle en C₁-C₅, hydroxyle, ou alkylsulfonyle en C₁-C₄.

8. Composé selon la revendication 1, dans lequel X représente O ou S(O)ₓ, dans lequel x vaut 0, 1 ou 2, et
R₄ représente un groupe méthyle qui est substitué par un atome d'halogène, un groupe cyano, nitro ou -OR₅, ou
R₄ représente un groupe alkyle en C₁-C₈, alcényle en C₂-C₈, alkynyle en C₃-C₈, cycloalkyle en C₃-C₈, cycloalcényle en C₅-C₈, (cycloalkyle en C₃-C₈) (alkyle en C₁-C₄), ou (alkyle en C₁-C₄) (cycloalkyle en C₃-C₈), ces substituants pouvant être non substitués ou substitués par un atome d'halogène, un groupe cyano, nitro, =O ou -OR₅, ou
R₄ représente un groupe phényle qui est substitué par un atome d'halogène, un groupe cyano, nitro, -COOH, hydroxyle, alkyle en C₁-C₄, alkyloxy en C₁-C₄, alkylthio en C₁-C₄, halogénoalkyle en C₁-C₄, halogénoalcoxy en C₁-C₄, halogénoalkylthio en C₁-C₄, alcoxycarbonylalcoxy en C₂-C₆, alkylsulfinyle en C₁-C₄, alkylsulfonyle en C₁-C₄, alkyloxycarbonyle en C₁-C₆, alkylcarbonyle en C₁-C₆, -CONH₂, formyle, alkylaminocarbonyle en C₁-C₅, dialkylaminocarbonyle en C₂-C₇, alkylamino en C₁-C₄, dialkylamino en C₁-C₄, trialkylsilyle en C₃-C₆, alkylcarbonylamino en C₁-C₆, alkylcarbonyloxy en C₁-C₆, phénoxy, halogénophénoxy, pyridyloxy, ou
R₄ représente un groupe biphényle, naphtyle, hétérocyclyle saturé ou insaturé comportant 3 à 8 atomes de cycle, qui, en plus des atomes de carbone, a au moins un hétéroatome choisi parmi un atome d'azote, d'oxygène et de soufre, (alkyle en C₁-C₄)phényle, (alkyle en C₁-C₄)naphtyle, phényl(alkyle en C₁-C₄), ou naphtyl(alkyle en C₁-C₄), ces substituants pouvant être non substitués ou substitués par un atome d'halogène, un groupe cyano, nitro, amino, -COOH, hydroxyle, alkyle en C₁-C₄, alkyloxy en C₁-C₄, alkylthio en C₁-C₄, halogénoalkyle en C₁-C₄, halogénoalcoxy en C₁-C₄, halogénoalkylthio en C₁-C₄, alcoxycarbonylalcoxy en C₂-C₆, alkylsulfinyle en C₁-C₄, alkylsulfonyle en C₁-C₄, alkyloxycarbonyle en C₁-C₆, alkylcarbonyle en C₁-C₆, -CONH₂, formyle, alkylaminocarbonyle en C₁-C₅, dialkylaminocarbonyle en C₂-C₇, trialkylsilyle en C₃-C₆, alkylcarbonylamino en C₁-C₆, alkylcarbonyloxy en C₁-C₆, phénoxy, halogénophénoxy, pyridyloxy, et
R₅ représente un atome d'hydrogène, un groupe alkyle en C₁-C₆, halogénoalkyle en C₁-C₆, alcoxyalkyle en C₂-C₆, cyanoalkyle en C₁-C₆, phényle, halogénophényle, phényl-(alcoxy en C₁-C₄), phényl-(alkyle en C₁-C₄), (alkyle en C₁-C₄)-carbonyle, benzoyle, halogénobenzoyle, alkylamino C₁-C₄, dialkylamino C₂-C₆, trialkylsilyle en C₃-C₆, cycloalkyle en C₃-C₆, alcényle en C₂-C₄ ou alkynyle en C₃-C₄.

9. Composé selon la revendication 8, dans lequel
X représente O ou S, et
R₄ représente un groupe méthyle qui est substitué par un atome d'halogène ou un groupe cyano, ou
R₄ représente un groupe alkyle en C₂-C₈, alcényle en C₂-C₈, alkynyle en C₃-C₈, cycloalkyle en C₃-C₈, cycloalcényle en C₅-C₈, (cycloalkyle en C₃-C₈) (alkyle en C₁-C₄), ou (alkyle en C₁-C₄) (cycloalkyle en C₃-C₈), ces substituants pouvant être non substitués ou substitués par un atome d'halogène, un groupe cyano, ou
R₄ représente un groupe phényle qui est substitué par un atome d'halogène, un groupe cyano, nitro, amino, alkyle en C₁-C₄, alkyloxy en C₁-C₄, alkylthio en C₁-C₄, halogénoalkyle en C₁-C₄, halogénoalcoxy en C₁-C₄, alcoxycarbonylalcoxy en C₂-C₆, alkylsulfonyle en C₁-C₄, alkyloxycarbonyle en C₁-C₆, alkylcarbonyle en C₁-C₆, formyle, alkylamino en C₁-C₄, dialkylamino en C₁-C₄, trialkylsilyle en C₃-C₆ ou alkylcarbonyloxy en C₂-C₆, ou
R₄ représente un groupe biphényle, naphtyle, hétérocyclyle saturé ou insaturé comportant 3 à 8 atomes de cycle, qui, en plus des atomes de carbone, a au moins un hétéroatome choisi parmi un atome d'azote, d'oxygène et de soufre, (alkyle en C₁-C₄)-phényle, (alkyle en C₁-C₄)-naphtyle, phényl-(alkyle en C₁-C₄), ou naphtyl-(alkyle en C₁-C₄), ces substituants pouvant être non substitués ou substitués par un atome d'halogène, un groupe cyano, nitro, amino, alkyle en C₁-C₄, alkyloxy en C₁-C₄, alkylthio en C₁-C₄, halogénoalkyle en C₁-C₄, halogénoalcoxy en C₁-C₄, alcoxycarbonylalcoxy en C₂-C₆, alkylsulfonyle en C₁-C₄, alkyloxycarbonyle en C₁-C₆, alkylcarbonyle en C₁-C₆, trialkylsilyle en C₃-C₆, alkylcarbonyloxy en C₁-C₆.

10. Composé selon la revendication 9, dans lequel
X représente O, et
R₄ représente un groupe phényle qui est substitué par un atome d'halogène, un groupe cyano, nitro, amino, alkyle en C₁-C₄, alkyloxy en C₁-C₄, alkylthio en C₁-C₄, halogénoalkyle en C₁-C₄, halogénométhoxy, alkylsulfonyle en C₁-C₄, alkylamino en C₁-C₄ ou dialkylamino en C₂-C₆.

11. Procédé pour la préparation des composés selon la revendication 1, qui comprend les étapes consistant :
(1) à faire réagir un composé de formule II 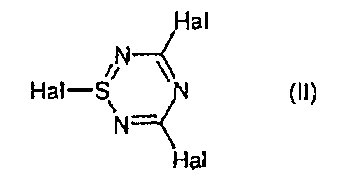 dans laquelle les substituants Hal représentent indépendamment les uns des autres un atome de fluor, de chlore ou de brome, avec un composé organométallique de formule III
R₁-M (III)
dans laquelle R₁ est tel que défini dans la revendication 1, et M représente un atome de métal monovalent ou polyvalent,
(2a) à faire réagir le composé obtenu de formule (IV) 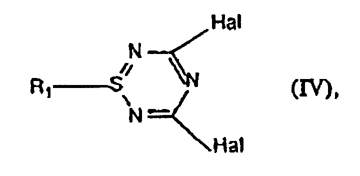 dans laquelle R₁ est tel que défini dans la revendication 1 et les radicaux Hal représentent indépendamment les uns des autres un atome de fluor, de chlore ou de brome, avec le composé de formule (V)
II-XR₄ (V)
dans laquelle X et R₄ sont tels que définis ci-dessus, pour obtenir le composé de formule VI 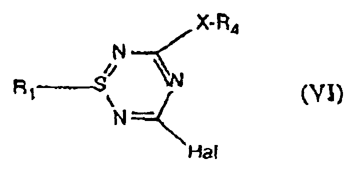 et
(3a) à convertir ensuite ces composés avec les composés de formule VII
M₁-NR₂R₃ (VII),
dans laquelle R₂ et R₃ sont tels que définis ci-dessus et M₁ représente un atome d'hydrogène ou un atome de métal en produits finaux de formule I ; ou
(2b) à faire réagir les composés de formule IV avec les composés de formule VII pour obtenir les composés de formule VIII 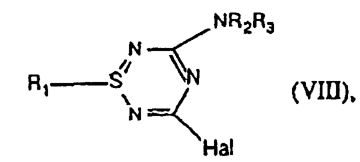 dans laquelle R₁, R₂ et R₃ sont tels que définis dans la revendication 1 et Hal représente un atome de fluor, de chlore ou de brome, et
(3b) à convertir ensuite ces composés avec les composés de formule V en produits finaux de formule I.

12. Composition herbicide et inhibant la croissance des plantes, qui comprend un ou plusieurs composés de formule I selon la revendication 1.

13. Composition selon la revendication 12, qui comprend entre 0,1% et 95% de l'ingrédient actif de formule I.

14. Méthode de lutte contre la croissance des plantes indésirables, qui comprend l'application d'une quantité efficace d'un ingrédient actif de formule I selon la revendication 1, ou d'une composition comprenant cet ingrédient actif, aux plantes ou à leur environnement.

15. Méthode selon la revendication 14, dans laquelle une quantité de l'ingrédient actif comprise entre 0,001 et 5 kg, en particulier entre 0,005 et 2 kg est appliquée par hectare.

16. Méthode d'inhibition de la croissance des plantes, qui comprend l'application d'une quantité efficace d'un ingrédient actif de formule I selon la revendication 1, ou d'une composition comprenant cet ingrédient actif, aux plantes ou à leur environnement.

17. Méthode selon la revendication 14, pour la lutte sélective contre les mauvaises herbes avant ou après émergence dans les cultures de plantes utiles.

18. Utilisation d'une composition selon la revendication 12 pour la lutte sélective contre les mauvaises herbes avant ou après émergence dans des cultures de plantes utiles.

19. Semences traitées par une composition selon la revendication 12.

20. Composé de formule IV 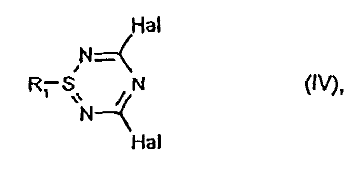 dans laquelle R₁ est tel que défini dans la revendication 1, et Hal représentent indépendamment l'un de l'autre un atome de fluor, de chlore ou de brome.

21. Composé de formule VI 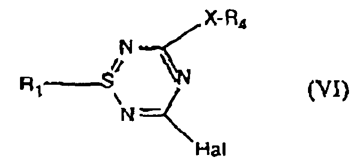 dans laquelle R₁, X et R₄ sont tels que définis dans la revendication 1, et Hal représente un atome de fluor, de chlore ou de brome.

22. Composé de formule VIII 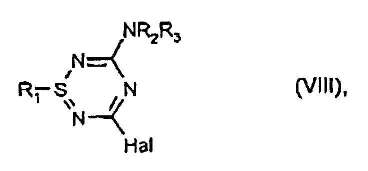 dans laquelle R₁, R₂ et R₃ sont tels que définis dans la revendication 1, et Hal représente un atome de fluor, de chlore ou de brome.
